# EUROPEAN PATENT APPLICATION

(11) **EP 3 384 938 A1**
(43) Date of publication of application: **10.10.2018**
(21) Application number: 18159455.7
(22) Date of filing: 11.09.2012
(51) Int. Cl.: A61K 48/00, C07K 14/47, A61K 45/06

(54) **ENGINEERED NUCLEIC ACIDS AND METHODS OF USE THEREOF**

(30) Priority: 12.09.2011 US 201161533554 P
(62) Divisional of application: 12831612.2
(71) Applicant: Moderna Therapeutics, Inc., Cambridge, Massachusetts 02141 (US)
(72) Inventor: Bancel, Stephane, Cambridge, MA 02141 (US); Schrum, Jason P., Philadelphia, PA 19127 (US); Aristarkhov, Alexander, Chestnut Hill, MA 02467 (US)
(74) Representative: Hoffmann Eitle

(57) **Abstract**

Provided are compositions and methods for delivering biological moieties such as modified nucleic acids into cells to kill or reduce the growth of viruses. Such compositions and methods include the use of modified messenger RNAs, and are useful to treat or prevent viral infection, or to improve a subject's health or wellbeing.

## Description

### CROSS REFERENCE TO RELATED APPLICATIONS

This application claims priority to US Provisional Application No. 61/533,554, filed September 12, 2011, entitled Engineered Nucleic Acids and Methods of Use Thereof.

### BACKGROUND OF THE INVENTION

Naturally occurring RNAs are synthesized from four basic ribonucleotides: ATP, CTP, UTP and GTP, but may contain post-transcriptionally modified nucleotides. Further, over one hundred natural nucleotide modifications have been identified in all RNA species (Rozenski, J, Crain, P, and McCloskey, J. (1999). The RNA Modification Database: 1999 update. Nucl Acids Res 27: 196-197). Nucleotides are modified in RNA to alter functional, structural, or catalytic roles of the parent RNA molecule. More recently, nucleotide modifications have been described to play a role in differentiating host cell RNA species from invading pathogenic RNA species. However, the precise mechanism by which nucleotide modifications alter the host immune response machinery and subsequently affect the translation efficiency of mRNA is unclear.

There is a need in the art for biological modalities to address the modulation of intracellular translation of nucleic acids.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described herein. The materials, methods, and examples are illustrative only and not intended to be limiting. Other features of the disclosure are apparent from the following detailed description and the claims.

### SUMMARY OF THE INVENTION

Provided herein are modified nucleic acids encoding anti-viral polypeptides (AVPs), e.g., anti-viral polypeptides described herein, precursors thereof, or partially or fully processed forms of these precursors. In certain embodiments, the anti-viral polypeptide has one or more of anti-bacterial, anti-fungal, anti-protozoal, anti-tumor/cancer, anti-parasitic, or anti-prion activity. In certain embodiments, the modified nucleic acids comprise mRNA. In particular embodiments, the modified mRNA (mmRNA) is derived from cDNA. In certain embodiments, the mmRNA comprises at least two nucleoside modifications. In certain embodiments, these nucleoside modifications are 5-methylcytosine and pseudouridine.

Provided herein are isolated nucleic acids (*e.g*., modified mRNAs encoding an anti-viral polypeptide described herein) comprising a translatable region and at least two different nucleoside modifications, wherein the nucleic acid exhibits reduced degradation in a cell into which the nucleic acid is introduced, relative to a corresponding unmodified nucleic acid. For example, the degradation rate of the nucleic acid is reduced by at least 10%, 20%, 30%, 40%, 50%, 60%, 70%, 80%, or 90%, compared to the degradation rate of the corresponding unmodified nucleic acid. In certain embodiments, the nucleic acids comprise RNA, DNA, TNA, GNA, or a hybrid thereof. In certain embodiments, the nucleic acids comprise messenger RNA (mRNA). In certain embodiments, the mRNA does not substantially induce an innate immune response of the cell into which the mRNA is introduced. In certain embodiments, the mRNA comprises at least one nucleoside selected from the group consisting of pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1 -deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine. In certain embodiments, the mRNA comprises at least one nucleoside selected from the group consisting of 5-aza-cytidine, pseudoisocytidine, 3-methyl-cytidine, N4-acetylcytidine, 5-formylcytidine, N4-methylcytidine, 5-hydroxymethylcytidine, 1 -methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine. In other embodiments, the mRNA comprises at least one nucleoside selected from the group consisting of 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine. In yet other embodiments, the mRNA comprises at least one nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some embodiments, the nucleic acids provided herein comprise a 5' untranslated region (UTR) and/or a 3'UTR, wherein each of the two different nucleoside modifications are independently present in the 5'UTR and/or 3'UTR. In some embodiments, nucleic acids are provided herein, wherein at least one of the two different nucleoside modifications are present in the translatable region. In some embodiments, nucleic acids provided herein are capable of binding to at least one polypeptide that prevents or reduces an innate immune response of a cell into which the nucleic acid is introduced.

Further provided herein are isolated nucleic acids (*e.g*., modified mRNAs described herein) comprising (i) a translatable region encoding an anti-viral polypeptide, *e.g*., an anti-viral polypeptide described herein, (ii) at least one nucleoside modification, and (iii) at least one intronic nucleotide sequence capable of being excised from the nucleic acid.

Further provided herein are isolated nucleic acids (*e.g*., modified mRNAs described herein) comprising (i) a translatable region encoding an anti-viral polypeptide, *e.g*., an anti-microbial polypeptide described herein, (ii) at least two different nucleoside modifications, and (iii) a degradation domain.

Further provided herein are isolated nucleic acids (*e.g.*, modified mRNAs described herein) comprising i) a translatable region encoding an anti-viral polypeptide, *e.g.*, an anti-viral polypeptide described herein, and ii) at least two different nucleoside modifications, wherein the translatable region encodes a polypeptide variant having an altered activity relative to a reference polypeptide. In certain embodiments, isolated mRNAs are provided, wherein the altered activity comprises an increased activity or wherein the altered activity comprises a decreased activity.

Further provided herein are non-enzymatically synthesized nucleic acids (*e.g.,* modified mRNAs described herein) comprising at least one nucleoside modification, and comprising a translatable region encoding an anti-viral polypeptide, *e.g*., an anti-viral polypeptide described herein. In certain embodiments, the non-enzymatically synthesized mRNA comprise at least two different nucleoside modifications.

Further provided herein are isolated nucleic acids (*e.g*., modified mRNAs described herein) comprising a noncoding region and at least one nucleoside modification that reduces an innate immune response of a cell into which the nucleic acid is introduced, wherein the nucleic acid sequesters one or more translational machinery components. In certain embodiments, the isolated nucleic acids comprising a noncoding region and at least one nucleoside modification described herein are provided in an amount effective to reduce protein expression in the cell. In certain embodiments, the translational machinery component is a ribosomal protein or a transfer RNA (tRNA). In certain embodiments, the nucleic acid comprises a small nucleolar RNA (sno-RNA), microRNA (miRNA), small interfering RNA (siRNA) or Piwi-interacting RNA (piRNA).

Further provided herein are isolated nucleic acids (*e.g.,* modified mRNAs described herein) comprising (i) a first translatable region, (ii) at least one nucleoside modification, and (iii) an internal ribosome entry site (IRES). In certain embodiments, the IRES is obtained from a picornavirus, a pest virus, a polio virus, an encephalomyocarditis virus, a foot-and-mouth disease virus, a hepatitis C virus, a classical swine fever virus, a murine leukemia virus, a simian immune deficiency virus or a cricket paralysis virus. In certain embodiments, the isolated nucleic acid further comprises a second translatable region. In certain embodiments, the isolated nucleic acid further comprises a Kozak sequence. In some embodiments, the first translatable region encodes an anti-viral polypeptide, e.g., an anti-viral polypeptide described herein. In some embodiments, the second translatable region encodes an anti-viral polypeptide, *e.g.,* an anti-viral polypeptide described herein. In some embodiments, the first translatable region encodes an anti-viral polypeptide, *e.g.,* an anti-viral polypeptide described herein. In some embodiments, the first and the second translatable regions encode anti-viral polypeptides, *e.g.,* anti-viral polypeptides described herein.

Further, provided herein are compositions (*e.g.,* pharmaceutical compositions) comprising the modified nucleic acids described herein. In certain embodiments, the composition further comprises a pharmaceutically acceptable carrier. In certain embodiments, the composition is formulated for systemic or local administration. In certain embodiments, the composition is formulated for intravenous administration. In certain embodiments, the composition is formulated for oral administration. In certain embodiments, the composition is formulated for topical administration. In certain embodiments, the composition is formulated for administration via a dressing (*e.g.,* wound dressing). In certain embodiments, the composition is formulated for administration via a bandage (*e.g.,* adhesive bandage). In certain embodiments, the composition is formulated for administration by inhalation. In certain embodiments, the composition is formulated for rectal administration. In certain embodiments, the composition is formulated for vaginal administration. In certain embodiments, the compositions comprise naked modified nucleic acids. In other embodiments, the modified nucleic acid is complexed or encapsulated. In another embodiment, the administration of the composition described herein may be administered at least once.

Provided herein are pharmaceutical compositions comprising: (i) an effective amount of a synthetic messenger ribonucleic acid (mRNA) encoding an anti-viral polypeptide, *e.g*., an anti-viral polypeptide described herein; and (ii) a pharmaceutically acceptable carrier, wherein i) the mRNA comprises pseudouridine, 5'methyl-cytidine or a combination thereof, or ii) the mRNA does not comprise a substantial amount of a nucleotide or nucleotides selected from the group consisting of uridine, cytidine, and a combination of uridine and cytidine, and wherein the composition is suitable for repeated intravenous administration to a mammalian subject in need thereof. In some embodiments, the anti-viral polypeptide is under 10kDa, *e.g.,* under 8kDa, 6kDa, 4kDa, 2kDa, or lkDa. In some embodiments, the anti-viral polypeptide comprises or consists of from about 6 to about 100 amino acids, *e.g.,* from about 6 to about 75 amino acids, about 6 to about 50 amino acids, about 6 to about 25 amino acids, about 25 to about 100 amino acids, about 50 to about 100 amino acids, or about 75 to about 100 amino acids. In certain embodiments, the anti-viral polypeptide comprises or consists of from about 15 to about 45 amino acids. In some embodiments, the anti-viral polypeptide is substantially cationic. In some embodiments, the anti-viral polypeptide is substantially amphipathic. In certain embodiments, the anti-viral polypeptide is substantially cationic and amphipathic. In some embodiments, the anti-viral polypeptide is cytostatic to a virus. In some embodiments, the anti-viral polypeptide is cytotoxic to a virus. In some embodiments, the anti-viral polypeptide is cytostatic and cytotoxic to a virus. In some embodiments, the anti-viral polypeptide is cytostatic to a bacterium, fungus, protozoan, parasite, prion, or combination thereof. In some embodiments, the anti-viral polypeptide is cytotoxic to a bacterium, fungus, protozoan, parasite, prion, or combination thereof. In certain embodiments, the anti-viral polypeptide is cytostatic and cytotoxic to a bacterium, fungus, protozoan, parasite, prion, or a combination thereof. In some embodiments, the anti-viral polypeptide is cytotoxic to a tumor or cancer cell (*e.g.,* a human cancer cell). In some embodiments, the anti-viral polypeptide is cytostatic to a tumor or cancer cell (*e.g.,* a human cancer cell). In certain embodiments, the anti-viral polypeptide is cytotoxic and cytostatic to a tumor or cancer cell (*e.g.,* a human cancer cell). In some embodiments, the anti-viral polypeptide is a secreted polypeptide. In certain embodiments, the anti-viral polypeptide is selected from the group consisting of anti-viral polypeptides and/or SEQ ID NOs: 1-1762. In certain embodiments, the anti-viral polypeptide comprises or consists of enfuvirtide (SEQ ID NO: 30). In some embodiments, the composition (*e.g*., pharmaceutical composition) provided herein further comprises a lipid-based transfection reagent. In some embodiments, the synthetic messenger ribonucleic acid (mRNA) encoding an anti-viral polypeptide, *e.g.,* an anti-viral polypeptide described herein, lacks at least one destabilizing element.

Further provided herein are pharmaceutical compositions comprising and/or consisting essentially of: (i) an effective amount of a synthetic messenger ribonucleic acid (mRNA) encoding an anti-viral polypeptide, *e.g.,* an anti-viral polypeptide described herein; (ii) a cell penetration agent; and (iii) a pharmaceutically acceptable carrier, wherein i) the mRNA comprises pseudouridine, 5'methyl-cytidine or a combination thereof, or ii) the mRNA does not comprise a substantial amount of a nucleotide or nucleotides selected from the group consisting of uridine, cytidine, and a combination of uridine and cytidine, and wherein the composition is suitable for repeated intravenous administration to a mammalian subject in need thereof.

Provided herein are methods of treating a subject having and/or being suspected of having a viral infection and/or a disease, disorder, or condition, *e.g.,* a disease, disorder, or condition associated with a viral infection, the methods comprising administering to a subject in need of such treatment a composition described herein in an amount sufficient to treat the viral infection and/or disease, disorder, or condition. In specific embodiments, the disease, disorder, or condition is associated with one or more cellular and/or molecular changes affecting, for example, the level, activity, and/or localization of an anti-viral polypeptide, *e.g.,* an anti-viral polypeptide described herein, precursors thereof, or a partially or fully processed form of these precursors. In certain embodiments, the methods of treating a subject having or being suspected of having a viral infection and/or a disease, disorder, or condition, *e.g*., a disease, disorder, or condition associated with a viral infection, comprise administering to the subject in need of such treatment a composition comprising a modified nucleic acid described herein in an amount sufficient to kill or reduce the growth of viruses, and/or to modulate one or more activities associated with, therefore to treat the viral infection and/or disease, disorder, or condition in the subject.

Further provided herein are methods of treating or preventing a viral infection of a target animal cell (*e.g*., mammalian cell), comprising the step of contacting the target animal cell (*e.g.*, mammalian cell) with a composition comprising a synthetic messenger ribonucleic acid (mRNA) encoding an anti-viral polypeptide in an amount effective to be cytostatic and/or cytotoxic to one or more viruses infecting the target animal cell (*e.g.*, mammalian cell). In some embodiments, the composition is effective to be cytostatic and/or cytotoxic to one or more viruses adjacent to the target animal cell (*e.g*., mammalian cell). In some embodiments, the target animal cell (*e.g.,* mammalian cell) is present in an animal subject (*e.g.,* a mammalian subject). In certain embodiments, the subject is a human. In certain embodiments, the subject is a livestock animal. In some embodiments, the composition is administered to the subject by an intravenous route. In certain embodiments, the composition is administered to the subject orally. In certain embodiments, the composition is administered to the subject topically. In certain embodiments, the composition is administered to the subject by inhalation. In certain embodiments, the composition is administered to the subject rectally. In certain embodiments, the composition is administered to the subject vaginally. In certain embodiments, the method further comprises the step of administering an effective amount of an anti-viral agent, e.g., an anti-viral agent described herein, to the subject at the same time or at a different time from the administering the composition, *e.g.,* before or after the administering the composition. In some embodiments, the anti-viral agent is an anti-viral polypeptide, *e.g.,* an anti-viral polypeptide described herein. In some embodiments, the anti-viral agent is a small molecule anti-microbial agent, *e.g.,* a small molecule anti-viral agent described herein. In another embodiment, the administration of the composition described herein may be administered at least once.

Further provided herein are methods for treating and/or preventing a viral infection and/or a disease, disorder, or condition associated with a viral infection, and/or a symptom thereof, in an animal (*e.g.,* a mammalian) subject, comprising contacting a cell of the subject with a nucleic acid described herein, wherein the translatable region of the nucleic acid encodes an anti-viral polypeptide, under conditions such that an effective amount of the anti-viral polypeptide is present in the cell, thereby treating or preventing a microbial infection (*e.g.,* viral infection) and/or a disease, disorder, or condition associated with the viral infection, and/or a symptom thereof, in the subject. In certain embodiments, the cell is an epithelial cell, an endothelial cell, or a mesothelial cell. In certain embodiments, the nucleic acid comprises an RNA molecule formulated for administration by an intravenous route. In certain embodiments, the nucleic acid comprises an RNA molecule formulated for oral administration. In certain embodiments, the nucleic acid comprises an RNA molecule formulated for topical administration. In certain embodiments, the nucleic acid comprises an RNA molecule formulated for administration by inhalation. In certain embodiments, the nucleic acid comprises an RNA molecule formulated for rectal administration. In certain embodiments, the nucleic acid comprises an RNA molecule formulated for vaginal administration.

Further provided herein are methods for inducing *in vivo* translation of a recombinant polypeptide (*e.g.,* an anti-viral polypeptide, *e.g.,* an anti-viral polypeptide described herein) in an animal (*e.g*., a mammalian) subject in need thereof, comprising the step of administering to the subject an effective amount of a composition comprising a nucleic acid comprising: (i) a translatable region encoding the recombinant polypeptide; and (ii) at least one nucleoside modification, under conditions such that the nucleic acid is localized into a cell of the subject and the recombinant polypeptide is capable of being translated in the cell from the nucleic acid. In certain embodiments, the composition comprises mRNA. In certain embodiments, methods are provided, wherein the recombinant polypeptide comprises a functional activity substantially absent in the cell in which the recombinant polypeptide is translated. In certain embodiments, the recombinant polypeptide comprises a polypeptide substantially absent in the cell in the absence of the composition. In certain embodiments, the recombinant polypeptide comprises a polypeptide that antagonizes the activity of an endogenous protein present in, on the surface of, or secreted from the cell. In certain embodiments, the recombinant polypeptide comprises a polypeptide that antagonizes the activity of a biological moiety present in, on the surface of, or secreted from the cell. In certain embodiments, the biological moiety comprises a lipid, a lipoprotein, a nucleic acid, a carbohydrate, or a small molecule toxin. In certain embodiments, the recombinant polypeptide is capable of being secreted from the cell. In certain embodiments, the recombinant polypeptide is capable of being translocated to the plasma membrane of the cell. In certain embodiments, methods are provided, wherein the composition is formulated for administration intramuscularly, transarterially, intraperitoneally, intravenously, intranasally, subcutaneously, endoscopically, transdermally, or intrathecally. In certain embodiments, methods are provided, wherein the composition is formulated for extended release.

Further provided herein are methods for inducing translation of a recombinant polypeptide (*e.g.,* an anti-viral polypeptide, *e.g.,* an anti-viral polypeptide described herein) in a cell population, comprising the step of contacting the cell population with an effective amount of a composition comprising a nucleic acid comprising: (i) a translatable region encoding the recombinant polypeptide; and (ii) at least one nucleoside modification, under conditions such that the nucleic acid is localized into one or more cells of the cell population and the recombinant polypeptide is translated in the cell from the nucleic acid. In certain embodiments, methods are provided, wherein the composition comprises mRNA. In certain embodiments, the composition comprises a cell penetrating compound. In certain embodiments, methods are provided, wherein the step of contacting the cell with the composition is repeated one or more times. In certain embodiments, the step of contacting the cell with the composition is repeated a sufficient number of times such that a predetermined efficiency of protein translation in the cell population.

Further provided herein are methods of reducing the innate immune response of a cell to an exogenous nucleic acid (*e.g.,* a modified mRNA described herein), comprising the steps of: (a) contacting the cell with a first composition comprising a first dose of a first exogenous nucleic acid comprising a translatable region (*e.g.,* encoding an anti-viral polypeptide, *e.g.,* an anti-viral polypeptide described herein) and at least one nucleoside modification; (b) determining the level of the innate immune response of the cell to the first exogenous nucleic acid; (c) contacting the cell with a second composition comprising either: (i) a second dose of the first exogenous nucleic acid, wherein the second dose contains a lesser amount of the first exogenous nucleic acid as compared to the first dose; or (ii) a first dose of a second exogenous nucleic acid, thereby reducing the innate immune response of the cell. In certain embodiments, methods are provided, wherein the step of contacting the cell with the first composition and/or the second composition is repeated one or more times. In certain embodiments, a predetermined efficiency of protein translation in the cell is achieved.

Provided herein are methods of providing a composition (*e.g*., a composition described herein) to a target tissue of a subject (*e.g*., mammalian subject) in need thereof, comprising the step of contacting the target tissue comprising one or more target cells with the composition under conditions such that the composition is substantially retained in the target tissue, and wherein the composition comprises: (a) an effective amount of a ribonucleic acid, wherein the ribonucleic acid is engineered to avoid an innate immune response of a cell into which the ribonucleic acid enters, and wherein the ribonucleic acid comprises a nucleotide sequence encoding a polypeptide of interest, wherein the protein of interest has an anti-viral activity (*e.g*., an anti-viral polypeptide described herein); (b) optionally, a cell penetration agent; and (c) a pharmaceutically acceptable carrier, under conditions such that the polypeptide of interest is produced in at least one target cell.

Further provided herein are isolated polypeptides (*e.g.,* anti-viral polypeptides, *e.g.,* anti-viral polypeptides described herein) produced by translation of the mRNAs described herein.

Further provided herein are isolated complexes comprising a conjugate of a protein and a nucleic acid (*e.g.,* a nucleic acid described herein), comprising (i) an mRNA comprising a translatable region encoding an anti-viral polypeptide, *e.g*., an anti-viral polypeptide described herein, and at least two different nucleoside modifications; and (ii) one or more polypeptides bound to the mRNA in an amount effective to prevent or reduce an innate immune response of a cell into which the complex is introduced.

Further provided herein are libraries comprising a plurality of polynucleotides, wherein the polynucleotides individually comprise: (i) a first nucleic acid sequence encoding a polypeptide (*e.g.,* an anti-viral polypeptide, *e.g*., an anti-viral polypeptide described herein); (ii) at least one nucleoside modification. In certain embodiments, libraries are provided, wherein the polypeptide comprises an antibody or functional portion thereof. In certain embodiments, libraries are provided, wherein the polynucleotides comprise mRNA. In certain embodiments, libraries are provided, wherein the at least one nucleoside modification is selected from the group consisting of pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thiouridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

Further provided herein are methods for enhancing protein (*e.g*., an anti-viral polypeptide, *e.g.*, an anti-viral polypeptide described herein) product yield in a cell culture process, comprising the steps of: (a) providing a cell culture comprising a plurality of host cells; (b) contacting the cell culture with a composition comprising a nucleic acid comprising a translatable region encoding an anti-viral polypeptide, *e.g*., an anti-microbial polypeptide described herein, and at least one nucleoside modification, wherein the nucleic acid exhibits increased protein production efficiency in a cell culture into which the nucleic acid is introduced, relative to a corresponding unmodified nucleic acid. In certain embodiments, methods are provided, wherein the increased protein production efficiency comprises increased cell transfection. In certain embodiments, the increased protein production efficiency comprises increased protein translation from the nucleic acid. In certain embodiments, the increased protein production efficiency comprises decreased nucleic acid degradation. In certain embodiments, the increased protein production efficiency comprises reduced innate immune response of the host cell. In certain embodiments, methods are provided, wherein the cell culture comprises a fed-batch mammalian cell culture process.

Further provided herein are methods for optimizing expression of an engineered protein (*e.g.,* an anti-viral polypeptide, *e.g.,* an anti-viral polypeptide described herein) in a target cell, comprising the steps of: (a) providing a plurality of target cell types; (b) independently contacting with each of the plurality of target cell types an isolated nucleic acid comprising a translatable region encoding an engineered polypeptide and at least one nucleoside modification; and (c) detecting the presence and/or level of the engineered polypeptide in the plurality of target cell types, thereby optimizing expression of an engineered polypeptide in a target cell. In certain embodiments, the engineered polypeptide comprises a post-translational modification. In certain embodiments, the engineered polypeptide comprises a tertiary structure. In certain embodiments, methods are provided, wherein the target cell comprises a mammalian cell line.

Further provided herein are methods of antagonizing a biological pathway in a cell, *e.g.,* a biological pathway associated with a viral infection, comprising the step of contacting the cell with an effective amount of a composition comprising a nucleic acid comprising: (i) a translatable region encoding a recombinant polypeptide (*e.g.,* an anti-viral polypeptide, *e.g.,* an anti-viral polypeptide described herein); and (ii) at least one nucleoside modification, under conditions such that the nucleic acid is localized into the cell and the recombinant polypeptide is capable of being translated in the cell from the nucleic acid, wherein the recombinant polypeptide inhibits the activity of a polypeptide functional in the biological pathway. In certain embodiments, methods are provided, wherein the biological pathway is defective in a cell having a viral infection and/or in a disease, disorder or condition (*e.g*., a disease, disorder, or condition described herein) associated with a viral infection.

Further provided herein are methods of agonizing a biological pathway in a cell, *e.g.* a biological pathway associated with a viral infection, comprising the step of contacting the cell with an effective amount of a composition comprising a nucleic acid comprising: (i) a translatable region encoding a recombinant polypeptide (*e.g.,* an anti-viral polypeptide, *e.g.,* an anti-viral polypeptide described herein); and (ii) at least one nucleoside modification, under conditions such that the nucleic acid is localized into the cell and the recombinant polypeptide is capable of being translated in the cell from the nucleic acid, wherein the recombinant polypeptide induces the activity of a polypeptide functional in the biological pathway. In certain embodiments, the agonized biological pathway modulates an anti-viral activity. In certain embodiments, the biological pathway is reversibly agonized.

Further provided herein are methods for enhancing nucleic acid delivery into a cell population, comprising the steps of: (a) providing a cell culture comprising a plurality of host cells; (b) contacting the cell population with a composition comprising an enhanced nucleic acid comprising a translatable region encoding a polypeptide (*e.g.,* an anti-viral polypeptide, *e.g.,* an anti-viral polypeptide described herein) and at least one nucleoside modification, wherein the enhanced nucleic acid exhibits enhanced retention in the cell population, relative to a corresponding unmodified nucleic acid. In certain embodiments, methods are provided, wherein the retention of the enhanced nucleic acid is at least about 50% greater than the retention of the unmodified nucleic acid. In some embodiments, the retention of the enhanced nucleic acid is at least about 100% greater than the retention of the unmodified nucleic acid. In other embodiments, the retention of the enhanced nucleic acid is at least about 200% greater than the retention of the unmodified nucleic acid. In certain embodiments, methods are provided, wherein the step of contacting the cell with the composition is repeated one or more times.

Further provided herein are methods of nucleic acid co-delivery into a cell population, comprising the steps of: (a) providing a cell culture comprising a plurality of host cells; (b) contacting the cell population with a composition comprising: (i) a first enhanced nucleic acid comprising a translatable region encoding a polypeptide (e.g., an anti-viral polypeptide, e.g., an anti-viral polypeptide described herein) and at least one nucleoside modification; and (ii) a first unmodified nucleic acid, wherein the composition does not substantially induce an innate immune response of the cell population.

Further provided herein are methods of nucleic acid delivery into a cell population, comprising the steps of: (a) providing a cell culture comprising a plurality of host cells; (b) contacting the cell population with a first composition comprising: (i) a first enhanced nucleic acid comprising a translatable region encoding a recombinant polypeptide (*e.g.,* an anti-viral polypeptide, *e.g.,* an anti-viral polypeptide described herein) and at least one nucleoside modification; and (ii) a first unmodified nucleic acid, wherein the composition does not substantially induce an innate immune response of the cell population; and (c) contacting the cell population with a second composition comprising a first unmodified nucleic acid.

Further provided herein are kits comprising a composition (*e.g.,* a pharmaceutical composition) comprising a modified mRNA encoding an anti-viral polypeptide, *e.g.,* an anti-viral polypeptide described herein, in one or more containers, and instructions for use thereof.

Further provided herein are kits for polypeptide production in a subject (*e.g.,* a mammalian subject) suffering from or at risk of developing a viral infection, comprising a first isolated nucleic acid comprising a translatable region and a nucleic acid modification, wherein the nucleic acid is capable of evading an innate immune response of a cell of the non-human vertebrate animal into which the first isolated nucleic acid is introduced, wherein the translatable region encodes a polypeptide comprising an anti-viral activity (*e.g.*, a anti-viral polypeptide described herein), and packaging and instructions therefore. In some embodiments, the instructions comprise instructions for the repeated administration of the first isolated nucleic acid to a cell or a population of cells. In some embodiments, the therapeutic polypeptide is useful in the treatment of an infection in the mammalian subject by a viral pathogen, *e.g.,* a viral pathogen described herein. In some embodiments, the viral pathogen is selected from the group consisting of human immunodeficiency viruses 1 and 2 (HIV-1 and HIV-2), human T-cell leukemia viruses 1 and 2 (HTLV-1 and HTLV-2), respiratory syncytial virus (RSV), human papilloma virus (HPV), adenovirus, hepatitis B virus (HBV), hepatitis C virus (HCV), Epstein-Barr virus (EBV), varicella zoster virus (VZV), cytomegalovirus (CMV), herpes simplex viruses 1 and 2 (HSV-1 and HSV-2), human herpes virus 8 (HHV-8), Yellow Fever virus, Dengue virus, Japanese Encephalitis, and West Nile viruses. In some embodiments, the kit further comprises a second isolated nucleic acid comprising a translatable region. In some embodiments, the translatable region in the second isolated nucleic acid encodes an anti-viral polypeptide, *e.g.,* an anti-viral polypeptide described herein. In some embodiments, the translatable region of the second isolated nucleic acid encodes the same anti-viral polypeptide as the first isolated nucleic acid. In some embodiments, the translatable region of the second isolated nucleic acid encodes a different anti-viral polypeptide than the first isolated nucleic acid. In some embodiments, the second nucleic acid comprises a nucleic acid modification. In some embodiments, the second nucleic acid does not comprise a nucleic acid modification.

Further provided herein are dressings (*e.g.,* wound dressings) or bandages (*e.g.,* adhesive bandages) comprising a pharmaceutical formulation comprising a modified mRNA encoding an anti-viral polypeptide, *e.g.,* an anti-viral polypeptide described herein.

### DETAILED DESCRIPTION OF THE INVENTION

In general, exogenous nucleic acids, particularly viral nucleic acids, introduced into cells induce an innate immune response, resulting in interferon (IFN) production and cell death. However, it is of great interest for therapeutics, diagnostics, reagents and for biological assays to deliver a nucleic acid, *e.g.,* a ribonucleic acid (RNA) inside a cell, either *in vivo* or *ex vivo,* such as to cause intracellular translation of the nucleic acid and production of the encoded protein. Of particular importance is the delivery and function of a non-integrative nucleic acid, as nucleic acids characterized by integration into a target cell are generally imprecise in their expression levels, deleteriously transferable to progeny and neighbor cells, and suffer from the substantial risk of mutation. Provided herein in part are nucleic acids encoding useful polypeptides capable of killing or reducing the growth of viruses and/or modulating a cell's function and/or activity, and methods of making and using these nucleic acids and polypeptides. As described herein, these nucleic acids are capable of reducing the innate immune activity of a population of cells into which they are introduced, thus increasing the efficiency of protein production in that cell population. Further, one or more additional advantageous activities and/or properties of the nucleic acids and proteins of the invention are described.

Provided herein are modified nucleic acids encoding an anti-viral polypeptide, *e.g*., an anti-viral polypeptide described herein, precursors thereof, or partially or fully processed forms of these precursors. In certain embodiments, the modified nucleic acids comprise mRNA. In particular embodiments, the modified mRNA (mmRNA) is derived from cDNA. In certain embodiments, the mmRNA comprises at least two nucleoside modifications. In certain embodiments, these nucleoside modifications comprise 5-methylcytosine and pseudouridine. In some embodiments, around 25%, around 50%, around 75%, or up to and including 100% of cytosine and uridine nucleotides of the modified nucleic acid are modified nucleotides. In certain embodiments, the mmRNA comprises a 5' cap structure and a 3' poly-A tail. In specific embodiments, the 5' cap structure is a Cap 1 structure. In specific embodiments, the poly-A tail comprises at least 10, 20, 30, 40, 50, 60, 70, 80, 90, 100, 110, 120, 130, 140, 150, 160, 170, 180, 190, or 200 nucleotides.

Further, provided herein are compositions (*e.g.*, pharmaceutical compositions) comprising the modified nucleic acids described herein. In certain embodiments, the compositions further comprise a pharmaceutically acceptable carrier. In certain embodiments, the carrier is formulated for systemic or local administration. In certain embodiments, the administration is intravenous. In certain embodiments, the administration is oral. In certain embodiments, the administration is topical. In certain embodiments, the administration is by inhalation. In certain embodiments, the administration is rectal. In certain embodiments, the administration is vaginal. In certain embodiments, the compositions comprise naked modified nucleic acids. In other embodiments, the modified nucleic acid is complexed or encapsulated. For example, the modified nucleic acids may be complexed in liposomal form or may be encapsulated in a nanoparticle. In certain embodiments, the modified nucleic acids, the complex or the nanoparticle further comprise one or more targeting moieties. These moieties can be used to target delivery *in vivo* to certain organs, tissues or cells.

Provided herein are methods of treating a subject having or being suspected of having a viral infection and/or a disease, disorder, or condition associated with a viral infection, the methods comprising administering to a subject in need of such treatment a composition described herein in an amount sufficient to treat the viral infection and/or the disease, disorder, or condition associated with the viral infection. In specific embodiments, the disease, disorder, or condition is associated with one or more cellular and/or molecular changes affecting, for example, the level, activity, and/or localization of an anti-viral polypeptide, *e.g*., an anti-viral polypeptide described herein, precursors thereof, or a partially or fully processed form of these precursors. Cellular and/or molecular changes may affect transcription, translation, posttranslational modification, processing, folding, intra-and/or extracellular trafficking, intra- and/or extracellular stability/turnover, and/or signaling of one or more molecules associated with an anti-viral activity. In certain embodiments, activities associated with an anti-viral polypeptide are compromised, *e.g.* 90%, 80%,70%, 60%, 50%, 40%, 30%, 20%, 10%, 5% or less of wild-type activity. In certain embodiments, the methods of treating a subject having or being suspected of having a viral infection and/or a disease, disorder, or condition associated with a viral infection comprise administering to the subject in need of such treatment a composition comprising a modified nucleic acid described herein in an amount sufficient to kill or inhibit the growth of viruses and/or to treat the disease, disorder, or condition.

A major drawback of many current treatments for diseases described herein is the necessity to produce anti-viral agents as polypeptides. Polypeptides are ordinarily expressed in and isolated from mammalian or bacterial cultures. Bacterial cultures and many cancer-derived cell culture systems do not faithfully recapitulate post-translational modifications, *e.g*., glycosylation and amidation, and protein precursors may not be fully processed. In some instances, the lack of posttranslational modification and processing influences the activity of the final protein product, its localization and/or its target specificity. In other instances, precursors and final cleavage products can have different physiological effects. For production of recombinant proteins, the polypeptide product that is effective for a particular treatment must usually be predetermined because the proteins if administered do not undergo any additional processing. Any modification that is vital for activity must also be present on the recombinant protein because they will not be added by the host when the recombinant proteins are administered. Recombinant protein production and purification is expensive and labor intensive. Protein expression host systems may harbor pathogens (*e.g.* viruses) that may contaminate the purified product. Proteins and particularly protein modifications are inherently unstable und require specific storage conditions and generally have a short shelf life. To be efficacious, recombinant proteins must be further modified, particularly by pegylation to avoid rapid degradation *in vivo.* Still, site-specific pegylation remains difficult because it can lead to loss of activity, loss of target specificity and/or protein aggregation. Veronese et al. Bioconjugate Chem. 18:1824-1830 (2007).

The modified mRNA molecules described herein do not share these problems. In comparison to recombinant proteins, they exhibit increased stability for shipping, handling and storage, are easy to mass produce, and when translated from the modified mRNA, the polypeptide can undergo an array of cell- and/or tissue-specific posttranslational processing, folding and modification.

### Anti-viral polypeptide

Anti-viral polypeptides (AVPs) are small peptides of variable length, sequence and structure with broad spectrum activity against a wide range of viruses. See, *e.g.,* Zaiou, J Mol Med, 2007; 85:317. It has been shown that AVPs have broad-spectrum of rapid onset of killing activities, with potentially low levels of induced resistance and concomitant broad antiinflammatory effects. In some embodiments, the anti-viral polypeptide is under 10kDa, *e.g.,* under 8kDa, 6kDa, 4kDa, 2kDa, or 1kDa. In some embodiments, the anti-viral polypeptide comprises or consists of from about 6 to about 100 amino acids, *e.g.,* from about 6 to about 75 amino acids, about 6 to about 50 amino acids, about 6 to about 25 amino acids, about 25 to about 100 amino acids, about 50 to about 100 amino acids, or about 75 to about 100 amino acids. In certain embodiments, the anti-viral polypeptide comprises or consists of from about 15 to about 45 amino acids. In some embodiments, the anti-viral polypeptide is substantially cationic. In some embodiments, the anti-viral polypeptide is substantially amphipathic. In certain embodiments, the anti-viral polypeptide is substantially cationic and amphipathic. In some embodiments, the anti-viral polypeptide is cytostatic to a virus. In some embodiments, the anti-viral polypeptide is cytotoxic to a virus. In some embodiments, the anti-viral polypeptide is cytostatic and cytotoxic to a virus. In some embodiments, the anti-viral polypeptide is cytostatic to a bacterium, fungus, protozoan, parasite, prion, or a combination thereof. In some embodiments, the anti-viral polypeptide is cytotoxic to a bacterium, fungus, protozoan, parasite, prion or a combination thereof. In certain embodiments, the anti-viral polypeptide is cytostatic and cytotoxic to a bacterium, fungus, protozoan, parasite, prion, or a combination thereof. In some embodiments, the anti-viral polypeptide is cytotoxic to a tumor or cancer cell (*e.g.,* a human cancer cell). In some embodiments, the anti-viral polypeptide is cytostatic to a tumor or cancer cell (*e.g.,* a human cancer cell). In certain embodiments, the anti-viral polypeptide is cytotoxic and cytostatic to a tumor or cancer cell (*e.g.,* a human cancer cell). In some embodiments, the anti-viral polypeptide is a secreted polypeptide.

AVPs have been isolated and described from a wide range of animals: microorganisms, invertebrates, plants, amphibians, birds, fish, and mammals (Wang et al., Nucleic Acids Res. 2009; 37 (Database issue):D933-7). For example, anti-microbial (*e.g*., anti-viral) polypeptides are described in Antimicrobial Peptide Database (http://aps.unmc.edu/AP/main.php; Wang et al., Nucleic Acids Res. 2009; 37 (Database issue): D933-7), CAMP: Collection of Anti-Microbial Peptides (http://www.bicnirrh.res.in/antimicrobial/; Thomas et al., Nucleic Acids Res. 2010; 38 (Database issue):D774-80), US 5221732, US 5447914, US 5519115, US 5607914, US 5714577, US 5734015, US 5798336, US 5821224, US 5849490, US 5856127, US 5905187, US 5994308, US 5998374, US 6107460, US 6191254, US 6211148, US 6300489, US 6329504, US 6399370, US 6476189, US 6478825, US 6492328, US 6514701, US 6573361, US 6573361, US 6576755, US 6605698, US 6624140, US 6638531, US 6642203, US 6653280, US 6696238, US 6727066, US 6730659, US 6743598, US 6743769, US 6747007, US 6790833, US 6794490, US 6818407, US 6835536, US 6835713, US 6838435, US 6872705, US 6875907, US 6884776, US 6887847, US 6906035, US 6911524, US 6936432, US 7001924, US 7071293, US 7078380, US 7091185, US 7094759, US 7166769, US 7244710, US 7314858, and US 7582301, the contents of which are incorporated by references in their entirety.

In certain embodiments, the anti-viral polypeptide is selected from the group consisting of anti-viral polypeptides provided in Table 1. Shown in Table 1, in addition to the name of the anti-viral polypeptide is the definition of the polypeptide and the sequence and SEQ ID NO of the polypeptide.

Exemplary anti-viral polypeptides can also include, but not limited to hBD-2, LL-37, and RNase-7.

The human defensin hBD-2 is expressed throughout human epithelia. The sequence of the precursor peptide consists of 41 residues present in the mature peptide as well as a leader sequence of secreted peptide. Disruption of hBD-2 expression, as in cystic fibrosis, might be associated with recurrent infections of skin and other epithelia.

The anti-microbial peptide, LL-37 is processed from the cathelicidin precursor hCAP18. The inhibition of LL-37 expression by *Shigella* likely causes about 160 million people develop intestinal infections yearly, resulting in over 1 million deaths. It is a multifunctional effector molecule capable of directly killing pathogens, modulating the immune response, stimulating proliferation, angiogenesis, and cellular migration, inhibiting apoptosis, and is associated with inflammation. It may play a part in epithelial cell proliferation as a part in wound closure and that its reduction in chronic wounds impairs re-epithelialization and may contribute to their failure to heal.

RNAse-7 is a potent AMP that was identified in the skin, human kidney and urinary tract. The systemic delivery of this mRNAs will likely allow expression of natural for the body antibiotic polypeptides even in tissues which are not supposed to be under microbial attack at normal physiological stage but have that danger under disease conditions.

In some embodiments, the anti-microbial polypeptide comprises or consists of a defensin. Exemplary defensins include, but not limited to, α-defensins (*e.g*., neutrophil defensin 1, defensin alpha 1, neutrophil defensin 3, neutrophil defensin 4, defensin 5, defensin 6), β-defensins (*e.g*., beta-defensin 1, beta-defensin 2, beta-defensin 103, beta-defensin 107, beta-defensin 110, beta-defensin 136), and θ-defensins. In other embodiments, the anti-microbial polypeptide comprises or consists of a cathelicidin (*e.g*., hCAP18).

The anti-microbial polypeptides described herein may block cell fusion and/or viral entry by one or more enveloped viruses (*e.g*., HIV, HCV). For example, the anti-microbial polypeptide can comprise or consist of a synthetic peptide corresponding to a region, *e.g.,* a consecutive sequence of at least about 5, 10, 15, 20, 25, 30, 35, 40, 45, 50, 55, or 60 amino acids of the transmembrane subunit of a viral envelope protein, *e.g.,* HIV-1 gp120 or gp41. The amino acid and nucleotide sequences of HIV-1 gp120 or gp41 are described in, *e.g.,* Kuiken et al., (2008). "HIV Sequence Compendium", Los Alamos National Laboratory. In some embodiments, the anti-microbial polypeptide has at least about 75%, 80%, 85%, 90%, 95%, 100% sequence homology to the corresponding viral protein sequence. In certain embodiments, the anti-microbial polypeptide comprises or consists of enfuvirtide (FUZEON®): Ac-Tyr-Thr-Ser-Leu-Ile-His-Ser-Leu- Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe-NH₂.

The anti-microbial polypeptides described herein may block viral particle assembly and formation of one or more infective enveloped viruses (*e.g.,* HIV, HCV). For example, the anti-microbial polypeptide can comprise or consist of a synthetic peptide corresponding to a region, *e.g.,* a consecutive sequence of at least about 5, 10, 15, 20, 25, 30, 35, 40, 45, 50, 55, or 60 amino acids of the capsid subunit of a viral capsid protein, *e.g.,* the HIV capsid protein. The amino acid and nucleotide sequences of the HIV-1 capsid protein are described in, *e.g.,* Kuiken et al., (2008). "HIV Sequence Compendium", Los Alamos National Laboratory. In some embodiments, the anti-microbial polypeptide has at least about 75%, 80%, 85%, 90%, 95%, 100% sequence homology to the corresponding viral protein sequence. In other embodiments, the anti-microbial polypeptide comprises or consists of a synthetic peptide corresponding to a region, e.g., a consecutive sequence of at least about 5, 10, 15, 20, 25, 30, 35, 40, 45, 50, 55, or 60 amino acids of the binding domain of a capsid binding protein. In some embodiments, the anti-microbial polypeptide has at least about 75%, 80%, 85%, 90%, 95%, 100% sequence homology to the corresponding sequence of the capsid binding protein.

The anti-microbial polypeptides described herein may block protease dimerization and inhibit cleavage of viral proproteins (*e.g*., HIV Gag-pol processing) into functional proteins thereby preventing release of one or more enveloped viruses (*e.g*., HIV, HCV). For example, the anti-microbial polypeptide can comprise or consist of a synthetic peptide corresponding to a region, *e.g.,* a consecutive sequence of at least about 5, 10, 15, 20, 25, 30, 35, 40, 45, 50, 55, or 60 amino acids of a viral protease, *e.g.*, the HIV-1 protease. The amino acid and nucleotide sequences of the HIV-1 protease are described in, *e.g.,* Kuiken et al., (2008). "HIV Sequence Compendium", Los Alamos National Laboratory. In some embodiments, the anti-microbial polypeptide has at least about 75%, 80%, 85%, 90%, 95%, 100% sequence homology to the corresponding viral protein sequence. In other embodiments, the anti-microbial polypeptide can comprise or consist of a synthetic peptide corresponding to a region, *e.g*., a consecutive sequence of at least about 5, 10, 15, 20, 25, 30, 35, 40, 45, 50, 55, or 60 amino acids of the binding domain of a protease binding protein. In some embodiments, the anti-microbial polypeptide has at least about 75%, 80%, 85%, 90%, 95%, 100% sequence homology to the corresponding sequence of the protease binding protein.

The anti-microbial polypeptides described herein can include a polypeptide corresponding to the inhibitory region of the endogenous human protein TRIM5-α or cyclophilin A (peptidylprolyl isomerase A). The sequences of human TRIM5-α and cyclophilin A are described, *e.g.,* in Stremlau et al., Nature. 2004; 427(6977):848-53 and Takahashi et al., Nature 1989; 337 (6206), 473-475, respectively.

The anti-microbial polypeptides described herein can include an *in vitro*-evolved polypeptide directed against a viral pathogen, *e.g*., a polypeptide identified or selected by the method described in Example 5.

### Modified nucleic acids.

This invention provides nucleic acids, including RNAs such as mRNAs that contain one or more modified nucleosides (termed "modified nucleic acids"), which have useful properties including the lack of a substantial induction of the innate immune response of a cell into which the mRNA is introduced. Because these modified nucleic acids enhance the efficiency of protein production, intracellular retention of nucleic acids, and viability of contacted cells, as well as possess reduced immunogenicity, these nucleic acids having these properties are termed "enhanced nucleic acids" herein.

The term "nucleic acid," in its broadest sense, includes any compound and/or substance that is or can be incorporated into an oligonucleotide chain. Exemplary nucleic acids for use in accordance with the present invention include, but are not limited to, one or more of DNA, RNA, hybrids thereof, RNAi-inducing agents, RNAi agents, siRNAs, shRNAs, miRNAs, antisense RNAs, ribozymes, catalytic DNA, RNAs that induce triple helix formation, aptamers, vectors, *etc.,* described in detail herein.

Provided are modified nucleic acids containing a translatable region encoding an anti-viral polypeptide, *e.g*., an anti-viral polypeptide described herein, and one, two, or more than two different nucleoside modifications. In some embodiments, the modified nucleic acid exhibits reduced degradation in a cell into which the nucleic acid is introduced, relative to a corresponding unmodified nucleic acid. For example, the degradation rate of the nucleic acid is reduced by at least 10%, 20%, 30%, 40%, 50%, 60%, 70%, 80%, or 90%, compared to the degradation rate of the corresponding unmodified nucleic acid. Exemplary nucleic acids include ribonucleic acids (RNAs), deoxyribonucleic acids (DNAs), threose nucleic acids (TNAs), glycol nucleic acids (GNAs), peptide nucleic acids (PNAs), locked nucleic acids (LNAs) or a hybrid thereof. In preferred embodiments, the modified nucleic acid includes messenger RNAs (mRNAs). As described herein, the nucleic acids of the invention do not substantially induce an innate immune response of a cell into which the mRNA is introduced.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1 -methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1 -methyl-1 -deaza-pseudoisocytidine, 1-methyl-1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In certain embodiments it is desirable to intracellularly degrade a modified nucleic acid introduced into the cell, for example if precise timing of protein production is desired. Thus, the invention provides a modified nucleic acid containing a degradation domain, which is capable of being acted on in a directed manner within a cell.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

Other components of nucleic acid are optional, and are beneficial in some embodiments. For example, a 5' untranslated region (UTR) and/or a 3'UTR are provided, wherein either or both may independently contain one or more different nucleoside modifications. In such embodiments, nucleoside modifications may also be present in the translatable region. Also provided are nucleic acids containing a Kozak sequence.

Additionally, nucleic acids encoding anti-viral polypeptides, e.g., anti-viral polypeptides described herein, and containing one or more intronic nucleotide sequences capable of being excised from the nucleic acid are provided herein.

Further, nucleic acids encoding anti-viral polypeptides, e.g., anti-viral polypeptides described herein, and containing an internal ribosome entry site (IRES) are provided herein. An IRES may act as the sole ribosome binding site, or may serve as one of multiple ribosome binding sites of an mRNA. An mRNA containing more than one functional ribosome binding site may encode several peptides or polypeptides that are translated independently by the ribosomes ("multicistronic mRNA"). When nucleic acids are provided with an IRES, further optionally provided is a second translatable region. Examples of IRES sequences that can be used according to the invention include without limitation, those from picornaviruses (e.g. FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SIV) or cricket paralysis viruses (CrPV).

### Prevention or reduction of innate cellular immune response activation using modified nucleic acids.

The term "innate immune response" includes a cellular response to exogenous single stranded nucleic acids, generally of viral or bacterial origin, which involves the induction of cytokine expression and release, particularly the interferons, and cell death. Protein synthesis is also reduced during the innate cellular immune response. While it is advantageous to eliminate the innate immune response in a cell, the invention provides modified mRNAs that substantially reduce the immune response, including interferon signaling, without entirely eliminating such a response. In some embodiments, the immune response is reduced by 10%, 20%, 30%, 40%, 50%, 60%, 70%, 80%, 90%, 95%, 99%, 99.9%, or greater than 99.9% as compared to the immune response induced by a corresponding unmodified nucleic acid. Such a reduction can be measured by expression or activity level of Type 1 interferons or the expression of interferon-regulated genes such as the toll-like receptors (*e.g*., TLR7 and TLR8). Reduction of innate immune response can also be measured by decreased cell death following one or more administrations of modified RNAs to a cell population; *e.g*., cell death is 10%, 25%, 50%, 75%, 85%, 90%, 95%, or over 95% less than the cell death frequency observed with a corresponding unmodified nucleic acid. Moreover, cell death may affect fewer than 50%, 40%, 30%, 20%, 10%, 5%, 1 %, 0.1%, 0.01% or fewer than 0.01% of cells contacted with the modified nucleic acids.

The invention provides for the repeated introduction (*e.g*., transfection) of modified nucleic acids into a target cell population, *e.g*., *in vitro, ex vivo,* or *in vivo.* The step of contacting the cell population may be repeated one or more times (such as two, three, four, five or more than five times). In some embodiments, the step of contacting the cell population with the modified nucleic acids is repeated a number of times sufficient such that a predetermined efficiency of protein translation in the cell population is achieved. Given the reduced cytotoxicity of the target cell population provided by the nucleic acid modifications, such repeated transfections are achievable in a diverse array of cell types.

### Polypeptide variants.

Provided are nucleic acids that encode variant polypeptides, which have a certain identity with a reference polypeptide (*e.g.,* an anti-viral polypeptide, *e.g.,* an anti-viral polypeptide described herein) sequence. The term "identity" as known in the art, refers to a relationship between the sequences of two or more peptides, as determined by comparing the sequences. In the art, "identity" also means the degree of sequence relatedness between peptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related peptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

In some embodiments, the polypeptide variant has the same or a similar activity as the reference polypeptide. Alternatively, the variant has an altered activity (e.g., increased or decreased) relative to a reference polypeptide. Generally, variants of a particular polynucleotide or polypeptide of the invention will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular reference polynucleotide or polypeptide as determined by sequence alignment programs and parameters described herein and known to those skilled in the art.

As recognized by those skilled in the art, protein fragments, functional protein domains, and homologous proteins are also considered to be within the scope of this invention. For example, provided herein is any protein fragment of a reference protein (meaning a polypeptide sequence at least one amino acid residue shorter than a reference polypeptide sequence but otherwise identical) 5, 10, 15, 20, 25, 30, 35, 40, 45, 50, 55, 60, 70, 80, 90, 100 or greater than 100 amino acids in length. In another example, any protein that includes a stretch of about 20, about 30, about 40, about 50, or about 100 amino acids which are about 40%, about 50%, about 60%, about 70%, about 80%, about 90%, about 95%, about 98%, or about 100% identical to any of the sequences described herein can be utilized in accordance with the invention. In certain embodiments, a protein sequence to be utilized in accordance with the invention includes 2, 3, 4, 5, 6, 7, 8, 9, 10, or more mutations as shown in any of the sequences provided or referenced herein.

### Polynucleotide libraries.

Also provided are polynucleotide libraries containing nucleoside modifications, wherein the polynucleotides individually contain a first nucleic acid sequence encoding a polypeptide, such as an anti-viral polypeptide, *e.g.,* an anti-viral polypeptide described herein. Preferably, the polynucleotides are mRNA in a form suitable for direct introduction into a target cell host, which in turn synthesizes the encoded polypeptide.

In certain embodiments, multiple variants of a protein, each with different amino acid modification(s), are produced and tested to determine the best variant in terms of pharmacokinetics, stability, biocompatibility, and/or biological activity, or a biophysical property such as expression level. Such a library may contain 10, 10², 10³, 10⁴, 10⁵, 10⁶, 10⁷, 10⁸, 10⁹, or over 10⁹ possible variants (including substitutions, deletions of one or more residues, and insertion of one or more residues).

### Polypeptide-nucleic acid complexes.

Proper protein translation involves the physical aggregation of a number of polypeptides and nucleic acids associated with the mRNA. Provided by the invention are complexes containing conjugates of protein and nucleic acids, containing a translatable mRNA encoding an anti-viral polypeptide, *e.g.,* an anti-viral polypeptide described herein, and having one or more nucleoside modifications (*e.g.,* at least two different nucleoside modifications) and one or more polypeptides bound to the mRNA. Generally, the proteins are provided in an amount effective to prevent or reduce an innate immune response of a cell into which the complex is introduced.

### Targeting Moieties.

In embodiments of the invention, modified nucleic acids are provided to express a protein-binding partner or a receptor on the surface of the cell, which functions to target the cell to a specific tissue space or to interact with a specific moiety, either *in vivo* or *in vitro.* Suitable protein-binding partners include antibodies and functional fragments thereof, scaffold proteins, or peptides. Additionally, modified nucleic acids can be employed to direct the synthesis and extracellular localization of lipids, carbohydrates, or other biological moieties.

### Untranslatable modified nucleic acids: vaccines.

As described herein, provided are mRNAs having sequences that are substantially not translatable. Such mRNA is effective as a vaccine when administered to a mammalian subject.

Also provided are modified nucleic acids that contain one or more noncoding regions. Such modified nucleic acids are generally not translated, but are capable of binding to and sequestering one or more translational machinery component such as a ribosomal protein or a transfer RNA (tRNA), thereby effectively reducing protein expression in the cell. The modified nucleic acid may contain a small nucleolar RNA (sno-RNA), micro RNA (miRNA), small interfering RNA (siRNA), or Piwi-interacting RNA (piRNA).

Additionally, certain modified nucleosides, or combinations thereof, when introduced into modified nucleic acids activate the innate immune response. Such activating modified nucleic acids, *e.g*., modified RNAs, are useful as adjuvants when combined with polypeptides (*e.g*., anti-viral polypeptides) or other vaccines. In certain embodiments, the activated modified mRNAs contain a translatable region which encodes for a polypeptide (*e.g*., an anti-viral polypeptide (*e.g*., an anti-viral polypeptide described herein)) sequence useful as a vaccine, thus providing the ability to be a self-adjuvant.

### Modified nucleic acid synthesis.

Nucleic acids for use in accordance with the invention may be prepared according to any available technique including, but not limited to chemical synthesis, enzymatic synthesis, which is generally termed *in vitro* transcription, enzymatic or chemical cleavage of a longer precursor, *etc.* Methods of synthesizing RNAs are known in the art (see, *e.g.,* Gait, M.J. (ed.) Oligonucleotide synthesis: a practical approach, Oxford (Oxfordshire), Washington, DC: IRL Press, 1984; and Herdewijn, P. (ed.) Oligonucleotide synthesis: methods and applications, Methods in Molecular Biology, v. 288 (Clifton, N.J.) Totowa, N.J.: Humana Press, 2005; both of which are incorporated herein by reference).

Modified nucleic acids need not be uniformly modified along the entire length of the molecule. Different nucleotide modifications and/or backbone structures may exist at various positions in the nucleic acid. One of ordinary skill in the art will appreciate that the nucleotide analogs or other modification(s) may be located at any position(s) of a nucleic acid such that the function of the nucleic acid is not substantially decreased. A modification may also be a 5' or 3' terminal modification. The nucleic acids may contain at a minimum one and at maximum 100% modified nucleotides, or any intervening percentage, such as at least 50% modified nucleotides, at least 80% modified nucleotides, or at least 90% modified nucleotides.

Generally, the length of a modified mRNA of the present invention is greater than 30 nucleotides in length. In another embodiment, the RNA molecule is greater than 35, 40, 45, 50, 60, 75, 100, 125, 150, 175, 200, 250, 300, 350, 400, 450, 500, 600, 700, 800, 900, 1000, 1100, 1200, 1300, 1400, 1500, 1800, 2000, 3000, 4000, or 5000 nucleotides, or greater than 5000 nucleotides.

### Uses of modified nucleic acids.

### Therapeutic Agents.

The modified nucleic acids described herein can be used as therapeutic agents to treat or prevent viral infections and/or diseases, disorders, or conditions associated with viral infections. Provided herein are compositions (*e.g*., pharmaceutical compositions), formulations, methods, kits, dressings (*e.g*., wound dressings), bandages (adhesive bandages), and reagents for treatment or prevention of diseases, disorders, or conditions, *e.g*., diseases, disorders, or conditions associated with viral infections, in humans and other animals (*e.g*., mammals). The active therapeutic agents of the invention include modified nucleic acids, cells containing modified nucleic acids or polypeptides translated from the modified nucleic acids, polypeptides translated from modified nucleic acids, and cells contacted with cells containing modified nucleic acids or polypeptides translated from the modified nucleic acids.

Provided are methods of inducing translation of a recombinant polypeptide (*e.g*., an anti-viral polypeptide described herein) in a cell population using the modified nucleic acids described herein. Such translation can be *in vivo, ex vivo, in culture,* or *in vitro.* The cell population is contacted with an effective amount of a composition containing a nucleic acid that has at least one nucleoside modification, and a translatable region encoding the recombinant polypeptide. The population is contacted under conditions such that the nucleic acid is localized into one or more cells of the cell population and the recombinant polypeptide is translated in the cell from the nucleic acid.

An effective amount of the composition is provided based, at least in part, on the target tissue, target cell type, means of administration, physical characteristics of the nucleic acid (*e.g.,* size, and extent of modified nucleosides), and other determinants. In general, an effective amount of the composition provides efficient protein production in the cell, preferably more efficient than a composition containing a corresponding unmodified nucleic acid. Increased efficiency may be demonstrated by increased cell transfection (i.e., the percentage of cells transfected with the nucleic acid), increased protein translation from the nucleic acid, decreased nucleic acid degradation (as demonstrated, *e.g.*, by increased duration of protein translation from a modified nucleic acid), or reduced innate immune response of the host cell.

Aspects of the disclosures are directed to methods of inducing *in vivo* translation of a recombinant polypeptide (*e.g*., an anti-viral polypeptide described herein) in a human or animal (*e.g*., mammalian) subject in need thereof. Therein, an effective amount of a composition containing a nucleic acid that has at least one nucleoside modification and a translatable region encoding the recombinant polypeptide (*e.g.,* an anti-viral polypeptide described herein) is administered to the subject using the delivery methods described herein. The nucleic acid is provided in an amount and under other conditions such that the nucleic acid is localized into a cell of the subject and the recombinant polypeptide is translated in the cell from the nucleic acid. The cell in which the nucleic acid is localized, or the tissue in which the cell is present, may be targeted with one or more than one rounds of nucleic acid administration.

Other aspects of the disclosures relate to transplantation of cells containing modified nucleic acids to a human or animal (*e.g.,* mammalian) subject. Administration of cells to human or animal (*e.g.,* mammalian) subjects is known to those of ordinary skill in the art, such as local implantation (*e.g.,* topical or subcutaneous administration), organ delivery or systemic injection (*e.g.,* intravenous injection or inhalation), as is the formulation of cells in pharmaceutically acceptable carrier. Compositions containing modified nucleic acids are formulated for administration intramuscularly, transarterially, intraocularly, vaginally, rectally, intraperitoneally, intravenously, intranasally, subcutaneously, endoscopically, transdermally, or intrathecally. In some embodiments, the composition is formulated for extended release.

The subject to whom the therapeutic agent is administered suffers from or is at risk of developing a disease, disorder, or deleterious condition. Provided are methods of identifying, diagnosing, and classifying subjects on these bases, which may include clinical diagnosis, biomarker levels, genome-wide association studies (GWAS), and other methods known in the art.

In certain embodiments, nucleic acids encoding an anti-viral polypeptide, *e.g.,* an anti-viral polypeptide described herein, are administered to subjects in need of anti-viral polypeptide administration.

In certain embodiments, the administered modified nucleic acid directs production of one or more recombinant polypeptides that provide a functional activity which is substantially absent in the cell in which the recombinant polypeptide is translated. For example, the missing functional activity may be enzymatic, structural, or gene regulatory in nature. In related embodiments, the administered modified nucleic acid directs production of one or more recombinant polypeptides that increases (*e.g.,* synergistically) a functional activity which is present but substantially deficient in the cell in which the recombinant polypeptide is translated.

In other embodiments, the administered modified nucleic acid directs production of one or more recombinant polypeptides that replace a polypeptide (or multiple polypeptides) that is substantially absent in the cell in which the recombinant polypeptide is translated. Such absence may be due to genetic mutation of the encoding gene or regulatory pathway thereof. In some embodiments, the recombinant polypeptide increases the level of an endogenous protein in the cell to a desirable level; such an increase may bring the level of the endogenous protein from a subnormal level to a normal level, or from a normal level to a super-normal level.

Alternatively, the recombinant polypeptide functions to antagonize the activity of an endogenous protein present in, on the surface of, or secreted from the cell. Usually, the activity of the endogenous protein is deleterious to the subject, for example, due to mutation of the endogenous protein resulting in altered activity or localization. Additionally, the recombinant polypeptide antagonizes, directly or indirectly, the activity of a biological moiety present in, on the surface of, or secreted from the cell. Examples of antagonized biological moieties include lipids (*e.g.,* cholesterol), a lipoprotein (*e.g.,* low density lipoprotein), a nucleic acid, a carbohydrate, a protein toxin such as shiga and tetanus toxins, or a small molecule toxin such as botulinum, cholera, and diphtheria toxins. Additionally, the antagonized biological molecule may be an endogenous protein that exhibits an undesirable activity, such as a cytotoxic or cytostatic activity.

The recombinant proteins described herein are engineered for localization within the cell, potentially within a specific compartment such as the nucleus, or are engineered for secretion from the cell or translocation to the plasma membrane of the cell.

As described herein, a useful feature of the modified nucleic acids of the invention is the capacity to reduce the innate immune response of a cell to an exogenous nucleic acid. Provided are methods for performing the titration, reduction or elimination of the immune response in a cell or a population of cells. In some embodiments, the cell is contacted with a first composition that contains a first dose of a first exogenous nucleic acid including a translatable region and at least one nucleoside modification, and the level of the innate immune response of the cell to the first exogenous nucleic acid is determined. Subsequently, the cell is contacted with a second composition, which includes a second dose of the first exogenous nucleic acid, the second dose containing a lesser amount of the first exogenous nucleic acid as compared to the first dose. Alternatively, the cell is contacted with a first dose of a second exogenous nucleic acid. The second exogenous nucleic acid may contain one or more modified nucleosides, which may be the same or different from the first exogenous nucleic acid or, alternatively, the second exogenous nucleic acid may not contain modified nucleosides. The steps of contacting the cell with the first composition and/or the second composition may be repeated one or more times. Additionally, efficiency of protein production (*e.g*., protein translation) in the cell is optionally determined, and the cell may be re-transfected with the first and/or second composition repeatedly until a target protein production efficiency is achieved.

### Topical delivery applied to the skin.

The skin is a desirable target site for nucleic acid delivery. It is readily accessible, and gene expression may be restricted not only to the skin, potentially avoiding nonspecific toxicity, but also to specific layers and cell types within the skin. The site of cutaneous expression of the delivered nucleic acid will depend on the route of nucleic acid delivery. Three routes are commonly considered to deliver nucleic acids to the skin: (i) topical application (*e.g.* for local/regional treatment); (ii) intradermal injection (*e.g.* for local/regional treatment); and (iii) systemic delivery (*e.g.,* for treatment of dermatologic diseases that affect both cutaneous and extracutaneous regions). Nucleic acids can be delivered to the skin by several different approaches. Most have been shown to work for DNA, such as, topical application of non-cationic liposome-DNA complex, cationic liposome-DNA complex, particle-mediated (gene gun), puncture-mediated gene transfections, and viral delivery approaches. After gene delivery, gene products have been detected in a number of skin cell types, including but not limited to basal keratinocytes, sebaceous gland cells, dermal fibroblasts and dermal macrophages.

In certain embodiments, dressing formulations comprising a modified nucleic acid encoding for an anti-viral polypeptide, *e.g.,* an anti-viral polypeptide described herein, precursor or a partially or fully processed form are provided herein.

In certain embodiments, the composition described herein is formulated for administration via a bandage (*e.g.,* adhesive bandage).

The modified nucleic acids encoding for an anti-viral polypeptide, *e.g.,* an anti-viral polypeptide described herein, precursor or a partially or fully processed form described herein may be intermixed with the dressing formulations or may be applied separately, *e.g.* by soaking or spraying.

### Targeting Moieties.

In embodiments of the invention, modified nucleic acids are provided to express a protein-binding partner or a receptor on the surface of the cell, which functions to target the cell to a specific tissue space or to interact with a specific moiety, either *in vivo* or *in vitro.* Suitable protein-binding partners include antibodies and functional fragments thereof, scaffold proteins, or peptides. Additionally, modified nucleic acids can be employed to direct the synthesis and extracellular localization of lipids, carbohydrates, or other biological moieties.

### Methods of treating diseases and conditions.

Provided are methods for treating or preventing a viral infection and/or a disease, disorder, or condition associated with a viral infection, and/or a symptom thereof, by providing an anti-viral activity. Because of the rapid initiation of protein production following introduction of modified mRNAs, as compared to viral DNA vectors, the compounds of the present invention are particularly advantageous in treating acute or chronic diseases such as microbial infections and sepsis. Moreover, the lack of transcriptional regulation of the modified mRNAs of the invention is advantageous in that accurate titration of protein production is achievable. In some embodiments, modified mRNAs and their encoded polypeptides in accordance with the present invention may be used for therapeutic purposes.

In some embodiments, modified mRNAs and their encoded polypeptides in accordance with the present invention may be used for treatment of viral infections and/or any of a variety of diseases, disorders, and/or conditions associated with viral infections.

In one embodiment, modified mRNAs and their encoded polypeptides in accordance with the present disclosure may be useful in the treatment of inflammatory disorders coincident with or resulting from infection.

Exemplary diseases, disorders, or conditions associated with viral infections include, but not limited to, acute febrile pharyngitis, pharyngoconjunctival fever, epidemic keratoconjunctivitis, infantile gastroenteritis, Coxsackie infections, infectious mononucleosis, Burkitt lymphoma, acute hepatitis, chronic hepatitis, hepatic cirrhosis, hepatocellular carcinoma, primary HSV-1 infection (*e.g.,* gingivostomatitis in children, tonsillitis and pharyngitis in adults, keratoconjunctivitis), latent HSV-1 infection (*e.g*., herpes labialis and cold sores), primary HSV-2 infection, latent HSV-2 infection, aseptic meningitis, infectious mononucleosis, Cytomegalic inclusion disease, Kaposi sarcoma, multicentric Castleman disease, primary effusion lymphoma, AIDS, influenza, Reye syndrome, measles, postinfectious encephalomyelitis, Mumps, hyperplastic epithelial lesions (*e.g*., common, flat, plantar and anogenital warts, laryngeal papillomas, epidermodysplasia verruciformis), cervical carcinoma, squamous cell carcinomas, croup, pneumonia, bronchiolitis, common cold, Poliomyelitis, Rabies, bronchiolitis, pneumonia, influenza-like syndrome, severe bronchiolitis with pneumonia, German measles, congenital rubella, Varicella, and herpes zoster.

Exemplary viral pathogens include, but not limited to, adenovirus, coxsackievirus, dengue virus, encephalitis virus, Epstein-Barr virus, hepatitis A virus, hepatitis B virus, hepatitis C virus, herpes simplex virus type 1, herpes simplex virus type 2, cytomegalovirus, human herpesvirus type 8, human immunodeficiency virus, influenza virus, measles virus, mumps virus, human papillomavirus, parainfluenza virus, poliovirus, rabies virus, respiratory syncytial virus, rubella virus, varicella-zoster virus, West Nile virus, and yellow fever virus. Viral pathogens may also include viruses that cause resistant viral infections.

Provided herein, are methods to prevent infection and/or sepsis in a subject at risk of developing infection and/or sepsis, the method comprising administering to a subject in need of such prevention a composition comprising a modified nucleic acid precursor encoding an anti-viral polypeptide, *e.g*., an anti-viral polypeptide described herein, or a partially or fully processed form thereof in an amount sufficient to prevent infection and/or sepsis. In certain embodiments, the subject at risk of developing infection and/or sepsis is a cancer patient. In certain embodiments, the cancer patient has undergone a conditioning regimen. In some embodiments, the conditioning regiment comprises chemotherapy, irradiation or both.

As a non-limiting example, sepsis may be treated using the modified mRNAs described herein encoding Protein C, its zymogen or prepro-protein, the active form of Protein C (APC), variants of Protein C which are known in the art, or the Protein C like molecules, variants and derivatives taught in US Patents 7,226,999, 7,498,305, 6,630,138; each of which is incorporated herein by reference in its entirety.

In one embodiment, the modified mRNAs of the present invention may be administered in conjunction with one or more antibiotics. These include, but are not limited to Aknilox , Ambisome, Amoxycillin, Ampicillin, Augmentin, Avelox, Azithromycin, Bactroban, Betadine, Betnovate, Blephamide, Cefaclor, Cefadroxil, Cefdinir, Cefepime, Cefix, Cefixime, Cefoxitin, Cefpodoxime, Cefprozil, Cefuroxime, Cefzil, Cephalexin, Cephazolin, Ceptaz, Chloramphenicol, Chlorhexidine, Chloromycetin, Chlorsig, Ciprofloxacin, Clarithromycin, Clindagel, Clindamycin, Clindatech, Cloxacillin, Colistin, Co-trimoxazole, Demeclocycline, Diclocil, Dicloxacillin, Doxycycline, Duricef, Erythromycin, Flamazine, Floxin, Framycetin, Fucidin, Furadantin, Fusidic, Gatifloxacin, Gemifloxacin, Gemifloxacin, Ilosone, Iodine, Levaquin, Levofloxacin, Lomefloxacin, Maxaquin, Mefoxin, Meronem, Minocycline, Moxifloxacin, Myambutol, Mycostatin, Neosporin, Netromycin, Nitrofurantoin, Norfloxacin, Norilet, Ofloxacin, Omnicef, Ospamox, Oxytetracycline, Paraxin, Penicillin, Pneumovax, Polyfax, Povidone, Rifadin, Rifampin, Rifaximin, Rifinah, Rimactane, Rocephin, Roxithromycin, Seromycin, Soframycin, Sparfloxacin, Staphlex, Targocid, Tetracycline, Tetradox, Tetralysal, tobramycin, Tobramycin, Trecator, Tygacil, Vancocin, Velosef, Vibramycin, Xifaxan, Zagam, Zitrotek, Zoderm, Zymar, and Zyvox.

Further provided herein, are methods to treat infection and/or sepsis in a subject, the method comprising administering to a subject in need of such treatment a composition comprising a modified nucleic acid precursor encoding an anti-viral polypeptide, e.g., an anti-viral polypeptide described herein, or a partially or fully processed form thereof in an amount sufficient to treat an infection and/or sepsis. In certain embodiments, the subject in need of treatment is a cancer patient. In certain embodiments, the cancer patient has undergone a conditioning regimen. In some embodiments, the conditioning regiment comprises chemotherapy, irradiation or both.

In certain embodiments, the subject exhibits active or chronic viral infections. In certain embodiments, the subject has received or is receiving a therapy. In certain embodiments, the therapy is radiotherapy, chemotherapy, steroids, or ultraviolet radiation. In certain embodiments, the patient suffers from a microvascular disorder. In some embodiments, the microvascular disorder is diabetes. In some embodiments, the wound is an ulcer. In a specific embodiment, the wound is a diabetic foot ulcer. In certain embodiments, the subject has one or more burn wounds. In certain embodiments, the administration is local or systemic. In certain embodiments, the administration is subcutaneous. In certain embodiments, the administration is intravenous. In certain embodiments, the administration is oral. In certain embodiments, the administration is topical. In certain embodiments, the administration is by inhalation. In certain embodiments, the administration is rectal. In certain embodiments, the administration is vaginal.

### Combination Therapy

Provided are methods for treating or preventing a viral infection and/or a disease, disorder, or condition associated with a viral infection, or a symptom thereof, in a subject, by administering a modified nucleic acid encoding an anti-viral polypeptide, *e.g.,* an anti-viral polypeptide described herein in combination with an anti-viral agent, *e.g.,* an anti-viral polypeptide or a small molecule anti-viral agent described herein.

The agents can be administered simultaneously, for example in a combined unit dose (e.g., providing simultaneous delivery of both agents). Alternatively, the agents can be administered at a specified time interval, for example, an interval of minutes, hours, days or weeks. Generally, the agents are concurrently bioavailable, *e.g.,* detectable, in the subject. In some embodiments, the agents are administered essentially simultaneously, for example two unit dosages administered at the same time, or a combined unit dosage of the two agents. In other embodiments, the agents are delivered in separate unit dosages. The agents can be administered in any order, or as one or more preparations that includes two or more agents. In a preferred embodiment, at least one administration of one of the agents, *e.g*., the first agent, is made within minutes, one, two, three, or four hours, or even within one or two days of the other agent, *e.g*., the second agent. In some embodiments, combinations can achieve synergistic results, *e.g.,* greater than additive results, *e.g.,* at least 25, 50, 75, 100, 200, 300, 400, or 500% greater than additive results.

Exemplary anti-viral agents include, but not limited to, abacavir (ZIAGEN®), abacavir/lamivudine/zidovudine (trizivir®), aciclovir or acyclovir (CYCLOVIR®, HERPEX®, ACIVIR®, ACIVIRAX®, ZOVIRAX®, ZOVIR®), adefovir (Preveon®, Hepsera®), amantadine (SYMMETREL®), amprenavir (AGENERASE®), ampligen, arbidol, atazanavir (REYATAZ®), boceprevir, cidofovir, darunavir (PREZISTA®), delavirdine (RESCRIPTOR®), didanosine (VIDEX®), docosanol (ABREVA®), edoxudine, efavirenz (SUSTIVA®, STOCRIN®), emtricitabine (EMTRIVA®), emtricitabine/tenofovir/efavirenz (ATRIPLA®), enfuvirtide (FUZEON®), entecavir (BARACLUDE®, ENTAVIR®), famciclovir (FAMVIR®), fomivirsen (VITRAVENE®), fosamprenavir (LEXIVA®, TELZIR®), foscarnet (FOSCAVIR®), fosfonet, ganciclovir (CYTOVENE®, CYMEVENE®, VITRASERT®), GS 9137 (ELVITEGRAVIR®), imiquimod (ALDARA®, ZYCLARA®, BESELNA®), indinavir (CRIXIVAN®), inosine, inosine pranobex (IMUNOVIR®), interferon type I, interferon type II, interferon type III, kutapressin (NEXAVIR®), lamivudine (ZEFFIX®, HEPTOVIR®, EPIVIR®), lamivudine/zidovudine (COMBIVIR®), lopinavir, loviride, maraviroc (SELZENTRY®, CELSENTRI®), methisazone, MK-2048, moroxydine, nelfinavir (VIRACEPT®), nevirapine (VIRAMUNE®), oseltamivir (TAMIFLU®), peginterferon alfa-2a (PEGASYS®), penciclovir (DENAVIR®), peramivir, pleconaril, podophyllotoxin (CONDYLOX®), raltegravir (ISENTRESS®), ribavirin (COPEGUs®, REBETOL®, RIBASPHERE®, VILONA® AND VIRAZOLE®), rimantadine (FLUMADINE®), ritonavir (NORVIR®), pyramidine, saquinavir (INVIRASE®, FORTOVASE®), stavudine, tea tree oil (melaleuca oil), tenofovir (VIREAD®), tenofovir/emtricitabine (TRUVADA®), tipranavir (APTIVUS®), trifluridine (VIROPTIC®), tromantadine (VIRU-MERZ®), valaciclovir (VALTREX®), valganciclovir (VALCYTE®), vicriviroc, vidarabine, viramidine, zalcitabine, zanamivir (RELENZA®), and zidovudine (azidothymidine (AZT), RETROVIR®, RETROVIS®).

### Targeting of pathogenic organisms: purification of biological materials.

Provided herein are methods for targeting viruses, using modified mRNAs that encode cytostatic or cytotoxic polypeptides. Preferably the mRNA introduced to the target virus contains modified nucleosides or other nucleic acid sequence modifications that the mRNA is translated exclusively, or preferentially, in the target virus, to reduce possible off-target effects of the therapeutic. Such methods are useful for removing viruses from biological material, including blood, semen, eggs, and transplant materials including embryos, tissues, and organs.

### Targeting of diseased cells.

Provided herein are methods for targeting pathogenic or diseased cells, particularly cells that are infected with one or more viruses, using modified mRNAs that encode cytostatic and/or cytotoxic polypeptides. Preferably the mRNA introduced into the target pathogenic cell contains modified nucleosides or other nucleic acid sequence modifications that the mRNA is translated exclusively, or preferentially, in the target pathogenic cell, to reduce possible off-target effects of the therapeutic. Alternatively, the invention provides targeting moieties that are capable of targeting the modified mRNAs to preferentially bind to and enter the target pathogenic cell.

### Methods of protein production.

The methods provided herein are useful for enhancing protein (*e.g.,* an anti-viral polypeptide described herein) product yield in a cell culture process. In a cell culture containing a plurality of host cells, introduction of the modified mRNAs described herein results in increased protein production efficiency relative to a corresponding unmodified nucleic acid. Such increased protein production efficiency can be demonstrated, *e.g.,* by showing increased cell transfection, increased protein translation from the nucleic acid, decreased nucleic acid degradation, and/or reduced innate immune response of the host cell. Protein production can be measured by ELISA, and protein activity can be measured by various functional assays known in the art. The protein production may be generated in a continuous or a fed-batch mammalian process.

Additionally, it is useful to optimize the expression of a specific polypeptide (*e.g.,* an anti-viral polypeptide described herein) in a cell line or collection of cell lines of potential interest, particularly an engineered protein such as a protein variant of a reference protein having a known activity. In one embodiment, provided is a method of optimizing expression of an engineered protein in a target cell, by providing a plurality of target cell types, and independently contacting with each of the plurality of target cell types a modified mRNA encoding an engineered polypeptide. Additionally, culture conditions may be altered to increase protein production efficiency. Subsequently, the presence and/or level of the engineered polypeptide in the plurality of target cell types is detected and/or quantitated, allowing for the optimization of an engineered polypeptide's expression by selection of an efficient target cell and cell culture conditions relating thereto. Such methods are particularly useful when the engineered polypeptide contains one or more post-translational modifications or has substantial tertiary structure, situations which often complicate efficient protein production.

### Modulation of biological pathways.

The rapid translation of modified mRNAs introduced into cells provides a desirable mechanism of modulating target biological pathways, *e.g.,* biological pathways associated with viral infections and/or diseases, disorders or conditions associated with viral infections. Such modulation includes antagonism or agonism of a given pathway. In one embodiment, a method is provided for antagonizing a biological pathway in a cell by contacting the cell with an effective amount of a composition comprising a modified nucleic acid encoding a recombinant polypeptide, under conditions such that the nucleic acid is localized into the cell and the recombinant polypeptide is capable of being translated in the cell from the nucleic acid, wherein the recombinant polypeptide inhibits the activity of a polypeptide functional in the biological pathway.

Alternatively, provided are methods of agonizing a biological pathway in a cell by contacting the cell with an effective amount of a modified nucleic acid encoding a recombinant polypeptide under conditions such that the nucleic acid is localized into the cell and the recombinant polypeptide is capable of being translated in the cell from the nucleic acid, and the recombinant polypeptide induces the activity of a polypeptide functional in the biological pathway. Exemplary agonized biological pathways include pathways that modulate anti-viral activity. Such agonization is reversible or, alternatively, irreversible.

### Methods of cellular nucleic acid delivery.

Methods of the present invention enhance nucleic acid delivery into a cell population, *in vivo, ex vivo,* or *in culture.* For example, a cell culture containing a plurality of host cells (*e.g.,* eukaryotic cells such as yeast or mammalian cells) is contacted with a composition that contains an enhanced nucleic acid having at least one nucleoside modification and, optionally, a translatable region encoding an anti-viral polypeptide, *e.g.,* an anti-viral polypeptide described herein. The composition also generally contains a transfection reagent or other compound that increases the efficiency of enhanced nucleic acid uptake into the host cells. The enhanced nucleic acid exhibits enhanced retention in the cell population, relative to a corresponding unmodified nucleic acid. The retention of the enhanced nucleic acid is greater than the retention of the unmodified nucleic acid. In some embodiments, it is at least about 50%, 75%, 90%, 95%, 100%, 150%, 200% or more than 200% greater than the retention of the unmodified nucleic acid. Such retention advantage may be achieved by one round of transfection with the enhanced nucleic acid, or may be obtained following repeated rounds of transfection.

In some embodiments, the enhanced nucleic acid is delivered to a target cell population with one or more additional nucleic acids. Such delivery may be at the same time, or the enhanced nucleic acid is delivered prior to delivery of the one or more additional nucleic acids. The additional one or more nucleic acids may be modified nucleic acids or unmodified nucleic acids. It is understood that the initial presence of the enhanced nucleic acids does not substantially induce an innate immune response of the cell population and, moreover, that the innate immune response will not be activated by the later presence of the unmodified nucleic acids. In this regard, the enhanced nucleic acid may not itself contain a translatable region, if the protein desired to be present in the target cell population is translated from the unmodified nucleic acids.

### Pharmaceutical Compositions

The present invention provides enhanced nucleic acids (*e.g.,* nucleic acids described herein), and complexes containing enhanced nucleic acids associated with other deliverable moieties. Thus, the present invention provides pharmaceutical compositions comprising one or more enhanced nucleic acids, or one or more such complexes, and one or more pharmaceutically acceptable excipients. Pharmaceutical compositions may optionally comprise one or more additional therapeutically active substances. In some embodiments, compositions are administered to humans. For the purposes of the present disclosure, the phrase "active ingredient" generally refers to an enhanced nucleic acid to be delivered as described herein.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other animals (*e.g.,* primates, mammals), including commercially relevant mammals such as cattle, pigs, horses, sheep, cats, dogs, mice, and/or rats; and/or birds, including commercially relevant birds such as chickens, ducks, geese, and/or turkeys.

Formulations of the pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, shaping and/or packaging the product into a desired single- or multi-dose unit.

A pharmaceutical composition in accordance with the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition in accordance with the invention will vary, depending upon the identity, size, and/or condition of the subject treated and further depending upon the route by which the composition is to be administered. By way of example, the composition may comprise between 0.1% and 100% (w/w) active ingredient.

Pharmaceutical formulations may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006; incorporated herein by reference) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

In some embodiments, a pharmaceutically acceptable excipient is at least 95%, at least 96%, at least 97%, at least 98%, at least 99%, or 100% pure. In some embodiments, an excipient is approved for use in humans and for veterinary use. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, *etc.,* and/or combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, *etc.,* and/or combinations thereof.

Exemplary surface active agents and/or emulsifiers include, but are not limited to, natural emulsifiers (*e.g*. acacia, agar, alginic acid, sodium alginate, tragacanth, chondrux, cholesterol, xanthan, pectin, gelatin, egg yolk, casein, wool fat, cholesterol, wax, and lecithin), colloidal clays (*e.g*. bentonite [aluminum silicate] and Veegum® [magnesium aluminum silicate]), long chain amino acid derivatives, high molecular weight alcohols (*e.g*. stearyl alcohol, cetyl alcohol, oleyl alcohol, triacetin monostearate, ethylene glycol distearate, glyceryl monostearate, and propylene glycol monostearate, polyvinyl alcohol), carbomers (*e.g.* carboxy polymethylene, polyacrylic acid, acrylic acid polymer, and carboxyvinyl polymer), carrageenan, cellulosic derivatives (*e.g.* carboxymethylcellulose sodium, powdered cellulose, hydroxymethyl cellulose, hydroxypropyl cellulose, hydroxypropyl methylcellulose, methylcellulose), sorbitan fatty acid esters (e.g. polyoxyethylene sorbitan monolaurate [Tween®20], polyoxyethylene sorbitan [Tween®60], polyoxyethylene sorbitan monooleate [Tween®80], sorbitan monopalmitate [Span®40], sorbitan monostearate [Span®60], sorbitan tristearate [Span®65], glyceryl monooleate, sorbitan monooleate [Span®80]), polyoxyethylene esters (*e.g.* polyoxyethylene monostearate [Myrj®45], polyoxyethylene hydrogenated castor oil, polyethoxylated castor oil, polyoxymethylene stearate, and Solutol®), sucrose fatty acid esters, polyethylene glycol fatty acid esters (*e.g.* Cremophor®), polyoxyethylene ethers, (*e.g.* polyoxyethylene lauryl ether [Brij®30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic®F 68, Poloxamer®188, cetrimonium bromide, cetylpyridinium chloride, benzalkonium chloride, docusate sodium, *etc.* and/or combinations thereof.

Exemplary binding agents include, but are not limited to, starch (*e.g.* cornstarch and starch paste); gelatin; sugars (*e.g.* sucrose, glucose, dextrose, dextrin, molasses, lactose, lactitol, mannitol,); natural and synthetic gums (*e.g.* acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (Veegum®), and larch arabogalactan); alginates; polyethylene oxide; polyethylene glycol; inorganic calcium salts; silicic acid; polymethacrylates; waxes; water; alcohol; *etc*.; and combinations thereof.

Exemplary preservatives may include, but are not limited to, antioxidants, chelating agents, antimicrobial preservatives, antifungal preservatives, alcohol preservatives, acidic preservatives, and/or other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and/or sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and/or trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and/or thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and/or sorbic acid. Exemplary alcohol preservatives include, but are not limited to, ethanol, polyethylene glycol, phenol, phenolic compounds, bisphenol, chlorobutanol, hydroxybenzoate, and/or phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and/or phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus®, Phenonip®, methylparaben, Germall®115, Germaben®II, Neolone™, Kathon™, and/or Euxyl®.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, *etc.,* and/or combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, *etc.,* and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and/or combinations thereof.

Liquid dosage forms for oral and parenteral administration include, but are not limited to, pharmaceutically acceptable emulsions, microemulsions, solutions, suspensions, syrups, and/or elixirs. In addition to active ingredients, liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and/or perfuming agents. In certain embodiments for parenteral administration, compositions are mixed with solubilizing agents such as Cremophor®, alcohols, oils, modified oils, glycols, polysorbates, cyclodextrins, polymers, and/or combinations thereof.

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables.

Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of an active ingredient, it is often desirable to slow the absorption of the active ingredient from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are prepared by entrapping the drug in liposomes or microemulsions which are compatible with body tissues.

Compositions for rectal or vaginal administration are typically suppositories which can be prepared by mixing compositions with suitable non-irritating excipients such as cocoa butter, polyethylene glycol or a suppository wax which are solid at ambient temperature but liquid at body temperature and therefore melt in the rectum or vaginal cavity and release the active ingredient. Solid dosage forms for oral administration include capsules, tablets, pills, powders, and granules. In such solid dosage forms, an active ingredient is mixed with at least one inert, pharmaceutically acceptable excipient such as sodium citrate or dicalcium phosphate and/or fillers or extenders (*e.g.* starches, lactose, sucrose, glucose, mannitol, and silicic acid), binders (*e.g.* carboxymethylcellulose, alginates, gelatin, polyvinylpyrrolidinone, sucrose, and acacia), humectants (*e*.*g.* glycerol), disintegrating agents (*e.g.* agar, calcium carbonate, potato or tapioca starch, alginic acid, certain silicates, and sodium carbonate), solution retarding agents (*e.g.* paraffin), absorption accelerators (*e.g.* quaternary ammonium compounds), wetting agents (*e.g.* cetyl alcohol and glycerol monostearate), absorbents (*e.g.* kaolin and bentonite clay), and lubricants (*e.g*. talc, calcium stearate, magnesium stearate, solid polyethylene glycols, sodium lauryl sulfate), and mixtures thereof. In the case of capsules, tablets and pills, the dosage form may comprise buffering agents.

Solid compositions of a similar type may be employed as fillers in soft and hard-filled gelatin capsules using such excipients as lactose or milk sugar as well as high molecular weight polyethylene glycols and the like. Solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polyethylene glycols and the like.

Dosage forms for topical and/or transdermal administration of a composition may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required. Additionally, the present invention contemplates the use of transdermal patches, which often have the added advantage of providing controlled delivery of a compound to the body. Such dosage forms may be prepared, for example, by dissolving and/or dispensing the compound in the proper medium. Alternatively or additionally, rate may be controlled by either providing a rate controlling membrane and/or by dispersing the compound in a polymer matrix and/or gel.

Suitable devices for use in delivering intradermal pharmaceutical compositions described herein include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver liquid compositions to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate vaccine in powder form through the outer layers of the skin to the dermis are suitable. Alternatively or additionally, conventional syringes may be used in the classical mantoux method of intradermal administration.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1% to about 10% (w/w) active ingredient, although the concentration of active ingredient may be as high as the solubility limit of the active ingredient in the solvent. Formulations for topical administration may further comprise one or more of the additional ingredients described herein.

A pharmaceutical composition may be prepared, packaged, and/or sold in a formulation suitable for pulmonary administration via the buccal cavity. Such a formulation may comprise dry particles which comprise the active ingredient and which have a diameter in the range from about 0.5 nm to about 7 nm or from about 1 nm to about 6 nm. Such compositions are suitably in the form of dry powders for administration using a device comprising a dry powder reservoir to which a stream of propellant may be directed to disperse the powder and/or using a self propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 nm and at least 95% of the particles by number have a diameter less than 7 nm. Alternatively, at least 95% of the particles by weight have a diameter greater than 1 nm and at least 90% of the particles by number have a diameter less than 6 nm. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50% to 99.9% (w/w) of the composition, and active ingredient may constitute 0.1% to 20% (w/w) of the composition. A propellant may further comprise additional ingredients such as a liquid nonionic and/or solid anionic surfactant and/or a solid diluent (which may have a particle size of the same order as particles comprising the active ingredient).

Pharmaceutical compositions formulated for pulmonary delivery may provide an active ingredient in the form of droplets of a solution and/or suspension. Such formulations may be prepared, packaged, and/or sold as aqueous and/or dilute alcoholic solutions and/or suspensions, optionally sterile, comprising active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. Droplets provided by this route of administration may have an average diameter in the range from about 0.1 nm to about 200 nm.

Formulations described herein as being useful for pulmonary delivery are useful for intranasal delivery of a pharmaceutical composition. Another formulation suitable for intranasal administration is a coarse powder comprising the active ingredient and having an average particle from about 0.2 µm to 500 µm. Such a formulation is administered in the manner in which snuff is taken, *i.e.* by rapid inhalation through the nasal passage from a container of the powder held close to the nose.

Formulations suitable for nasal administration may, for example, comprise from about as little as 0.1% (w/w) and as much as 100% (w/w) of active ingredient, and may comprise one or more of the additional ingredients described herein. A pharmaceutical composition may be prepared, packaged, and/or sold in a formulation suitable for buccal administration. Such formulations may, for example, be in the form of tablets and/or lozenges made using conventional methods, and may have, for example, 0.1% to 20% (w/w) active ingredient, the balance comprising an orally dissolvable and/or degradable composition and, optionally, one or more of the additional ingredients described herein. Alternately, formulations suitable for buccal administration may comprise a powder and/or an aerosolized and/or atomized solution and/or suspension comprising active ingredient. Such powdered, aerosolized, and/or aerosolized formulations, when dispersed, may have an average particle and/or droplet size in the range from about 0.1 nm to about 200 nm, and may further comprise one or more of any additional ingredients described herein.

A pharmaceutical composition may be prepared, packaged, and/or sold in a formulation suitable for ophthalmic administration. Such formulations may, for example, be in the form of eye drops including, for example, a 0.1/1.0% (w/w) solution and/or suspension of the active ingredient in an aqueous or oily liquid excipient. Such drops may further comprise buffering agents, salts, and/or one or more other of any additional ingredients described herein. Other opthalmically-administrable formulations which are useful include those which comprise the active ingredient in microcrystalline form and/or in a liposomal preparation. Ear drops and/or eye drops are contemplated as being within the scope of this invention.

General considerations in the formulation and/or manufacture of pharmaceutical agents may be found, for example, in Remington: The Science and Practice of Pharmacy 21st ed., Lippincott Williams & Wilkins, 2005 (incorporated herein by reference).

The present invention provides methods comprising administering modified mRNAs and their encoded proteins or complexes in accordance with the invention to a subject in need thereof. Nucleic acids, proteins or complexes, or pharmaceutical, imaging, diagnostic, or prophylactic compositions thereof, may be administered to a subject using any amount and any route of administration effective for preventing, treating, diagnosing, or imaging a disease, disorder, and/or condition (*e.g*., a disease, disorder, and/or condition relating to viral infections). The exact amount required will vary from subject to subject, depending on the species, age, and general condition of the subject, the severity of the disease, the particular composition, its mode of administration, its mode of activity, and the like. Compositions in accordance with the invention are typically formulated in dosage unit form for ease of administration and uniformity of dosage. It will be understood, however, that the total daily usage of the compositions of the present invention will be decided by the attending physician within the scope of sound medical judgment. The specific therapeutically effective, prophylactially effective, or appropriate imaging dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

Devices may also be used in conjunction with the present invention. In one embodiment, a device is used to assess levels of a protein which has been administered in the form of a modified mRNA. The device may comprise a blood, urine or other biofluidic test. It may be as large as to include an automated central lab platform or a small decentralized bench top device.

### Kits.

The invention provides a variety of kits for conveniently and/or effectively carrying out methods of the present invention. Typically kits will comprise sufficient amounts and/or numbers of components to allow a user to perform multiple treatments of a subject(s) and/or to perform multiple experiments.

In one embodiment, the levels of a modified mRNA of the present invention may be measured by immunoassay. In this embodiment, the assay may be used to assess levels of modified mRNA or its activated form or a variant delivered as or in response to the administration of the modified mRNA.

### Dressings and bandages.

The invention provides a variety of dressings (*e.g*., wound dressings) or bandages (*e.g*., adhesive bandages) for conveniently and/or effectively carrying out methods of the present invention. Typically dressings or bandages will comprise sufficient amounts of pharmaceutical compositions and/or modified nucleic acids described herein to allow a user to perform multiple treatments of a subject(s).

### Animal models.

Anti-viral agents can be tested in healthy animals (*e.g*., mice) exposed to specific viral pathogens. Anti-viral agents can also be tested in immunodeficient animal (*e.g*., mouse) models to test infection process without interference from other immune mechanisms except innate immunity.

Severe Combined Immunodeficiency (SCID) is a severe immunodeficiency genetic disorder that is characterized by the complete inability of the adaptive immune system to mount, coordinate, and sustain an appropriate immune response, usually due to absent or atypical T and B lymphocytes. *Scid* mice are important tools for researching hematopoiesis, innate and adaptive immunity, autoimmunity, infectious diseases, cancer, vaccine development, and regenerative medicine *in vivo.*

Strain NOD.Cg*-Prkdc^{scid} Il2rg^{tm1Wjl}l*SzJ (005557 Jacson Lab), commonly known as NOD *scid* gamma (NSG), is the latest breakthrough in the development of immunodeficient models. It combines the innate immunity deficiencies of the NOD/ShiLtJ background, the *scid* mutation, and IL2 receptor gamma chain (*Il2rg*) deficiency. The latter two deficiencies combine to eliminate mature T cells, B cells, and NK cells. Because the *Il2rg* knockout prevents the development of lymphoma, NSG mice survive longer than other *scid* strains, enabling long-term experiments.

The B6 *scid* - strain B6.CB17*-Prkdc^{scid}*/SzJ (001913, Jacson Lab), B6 *scid mice* lack most B and T cells. B6 *scid* is more severely immunodeficient and supports better engraftment of allogeneic and xenogeneic cells, tissues, and tumors than *Foxnl^{nu}* mutant strains.

The humanized mouse model of HIV infection to investigate mechanisms of viral dissemination, of HIV-induced immune activation, and of HIV-induced immune dysfunction can be used too MGH. Another mouse model - EcoHIV infected about 75 percent of the mice tested, an efficiency rate comparable with that of HIV in humans. The EcoHIV infection was present in immune cells and white blood cells, the spleen, abdominal cavity and brain.

C57BL/6-*Btk^{tmlArte}* 9723-F- mouse model for Bruton's disease. Bruton's tyrosine kinase (Btk) is a member of the Tec kinase family and has been implicated in the primary immunodeficiency X-linked agammaglobulinemia. Btk is thought to play multiple roles in the haematopoietic system, including B-cell development, stimulation of mast cells and the onset of autoimmune diseases. The Btk (Bruton's tyrosine kinase) KinaseSwitch mouse strain carries point mutations at the genomic level at positions T474A/S538A in the ATP binding pocket of the Btk kinase domain (BtkT474A /S538A).

### Definitions

*Therapeutic Agent:* The term "therapeutic agent" refers to any agent that, when administered to a subject, has a therapeutic, diagnostic, and/or prophylactic effect and/or elicits a desired biological and/or pharmacological effect.
*Administered in combination:* As used herein, the term "administered in combination" or "combined administration" means that two or more agents (*e.g.,* a modified nucleic acid encoding an anti-viral polypeptide, *e.g.,* an anti-viral polypeptide described herein, and an anti-viral agent (*e.g.,* an anti-viral polypeptide or a small molecule anti-viral compound described herein)) are administered to a subject at the same time or within an interval such that there is overlap of an effect of each agent on the patient. In some embodiments, they are administered within about 60, 30, 15, 10, 5, or 1 minute of one another. In some embodiments, the administrations of the agents are spaced sufficiently close together such that a combinatorial (*e.g.,* a synergistic) effect is achieved.
*Animal:* As used herein, the term "animal" refers to any member of the animal kingdom. In some embodiments, "animal" refers to humans at any stage of development. In some embodiments, "animal" refers to non-human animals at any stage of development. In certain embodiments, the non-human animal is a mammal (*e.g.,* a rodent, a mouse, a rat, a rabbit, a monkey, a dog, a cat, a sheep, cattle, a primate, or a pig). In some embodiments, animals include, but are not limited to, mammals, birds, reptiles, amphibians, fish, and worms. In some embodiments, the animal is a transgenic animal, genetically-engineered animal, or a clone.
*Approximately:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).
*Associated with:* As used herein, the terms "associated with," "conjugated," "linked," "attached," and "tethered," when used with respect to two or more moieties, means that the moieties are physically associated or connected with one another, either directly or via one or more additional moieties that serves as a linking agent, to form a structure that is sufficiently stable so that the moieties remain physically associated under the conditions in which the structure is used, *e.g.,* physiological conditions. As used herein, the terms "associated with," when used with respect to a virus and a disease, disorder, or condition, means the virus is found more frequently (*e.g.,* at least 10%, 25%, 50%, 75%, 100%, 200%, 500%, 1000% more frequently) in patients with the disease, disorder, or condition than in healthy controls and/or there is a frequent co-occurrence of the virus in the disease, disorder, or condition. In some embodiments, the virus can be a direct and/or singular cause of the disease, disorder, or condition. In some embodiments, the virus can be a necessary, but not sufficient, cause of the disease, disorder, or condition (*e.g.,* only causes the disease, disorder or condition in combination with one or more other causal factors (*e.g.,* genetic factors, or toxin exposure)). In some embodiments, the virus can predispose to the development of or increase the risk of getting the disease, disorder, or condition. In some embodiments, the virus can also be an "innocent bystander" that plays no significant role in the etiology of the disease, disorder, or condition but is more prevalent in patients with the disease, disorder, or condition for some reason such as the compromised immune response caused by the disease, disorder, or condition.
*Biologically active:* As used herein, the phrase "biologically active" refers to a characteristic of any substance that has activity in a biological system and/or organism. For instance, a substance that, when administered to an organism, has a biological effect on that organism, is considered to be biologically active. In particular embodiments, where a nucleic acid is biologically active, a portion of that nucleic acid that shares at least one biological activity of the whole nucleic acid is typically referred to as a "biologically active" portion.
*Conserved*: As used herein, the term "conserved" refers to nucleotides or amino acid residues of a polynucleotide sequence or amino acid sequence, respectively, that are those that occur unaltered in the same position of two or more related sequences being compared. Nucleotides or amino acids that are relatively conserved are those that are conserved amongst more related sequences than nucleotides or amino acids appearing elsewhere in the sequences. In some embodiments, two or more sequences are said to be "completely conserved" if they are 100% identical to one another. In some embodiments, two or more sequences are said to be "highly conserved" if they are at least 70% identical, at least 80% identical, at least 90% identical, or at least 95% identical to one another. In some embodiments, two or more sequences are said to be "highly conserved" if they are about 70% identical, about 80% identical, about 90% identical, about 95%, about 98%, or about 99% identical to one another. In some embodiments, two or more sequences are said to be "conserved" if they are at least 30% identical, at least 40% identical, at least 50% identical, at least 60% identical, at least 70% identical, at least 80% identical, at least 90% identical, or at least 95% identical to one another. In some embodiments, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another.
*Cytostatic:* As used herein, "cytostatic" refers to inhibiting, reducing, suppressing the growth, division, or multiplication of a cell (*e.g.,* a mammalian cell (*e.g.,* a human cell)), bacterium, virus, fungus, protozoan, parasite, prion, or a combination thereof.
*Cytotoxic:* As used herein, "cytotoxic" refers to killing or causing injurous, toxic, or deadly effect on a cell (*e.g.,* a mammalian cell (*e.g.,* a human cell)), bacterium, virus, fungus, protozoan, parasite, prion, or a combination thereof.
*Expression:* As used herein, "expression" of a nucleic acid sequence refers to one or more of the following events: (1) production of an RNA template from a DNA sequence (*e.g.,* by transcription); (2) processing of an RNA transcript (*e.g.,* by splicing, editing, 5' cap formation, and/or 3' end processing); (3) translation of an RNA into a polypeptide or protein; and (4) post-translational modification of a polypeptide or protein.
*Functional*: As used herein, a "functional" biological molecule is a biological molecule in a form in which it exhibits a property and/or activity by which it is characterized.
*Homology:* As used herein, the term "homology" refers to the overall relatedness between polymeric molecules, *e.g.* between nucleic acid molecules (*e.g.* DNA molecules and/or RNA molecules) and/or between polypeptide molecules. In some embodiments, polymeric molecules are considered to be "homologous" to one another if their sequences are at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% identical. In some embodiments, polymeric molecules are considered to be "homologous" to one another if their sequences are at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% similar. The term "homologous" necessarily refers to a comparison between at least two sequences (nucleotides sequences or amino acid sequences). In accordance with the invention, two nucleotide sequences are considered to be homologous if the polypeptides they encode are at least about 50% identical, at least about 60% identical, at least about 70% identical, at least about 80% identical, or at least about 90% identical for at least one stretch of at least about 20 amino acids. In some embodiments, homologous nucleotide sequences are characterized by the ability to encode a stretch of at least 4-5 uniquely specified amino acids. Both the identity and the approximate spacing of these amino acids relative to one another must be considered for nucleotide sequences to be considered homologous. For nucleotide sequences less than 60 nucleotides in length, homology is determined by the ability to encode a stretch of at least 4-5 uniquely specified amino acids. In accordance with the invention, two protein sequences are considered to be homologous if the proteins are at least about 50% identical, at least about 60% identical, at least about 70% identical, at least about 80% identical, or at least about 90% identical for at least one stretch of at least about 20 amino acids.
*Identity:* As used herein, the term "identity" refers to the overall relatedness between polymeric molecules, *e.g.,* between nucleic acid molecules (*e.g.* DNA molecules and/or RNA molecules) and/or between polypeptide molecules. Calculation of the percent identity of two nucleic acid sequences, for example, can be performed by aligning the two sequences for optimal comparison purposes (*e.g.,* gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991; each of which is incorporated herein by reference. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988); incorporated herein by reference. Techniques for determining identity are codified in publicly available computer programs. Exemplary computer software to determine homology between two sequences include, but are not limited to, GCG program package, Devereux, J., et al., Nucleic Acids Research, 12(1), 387 (1984)), BLASTP, BLASTN, and FASTA Atschul, S. F. et al., J. Molec. Biol., 215, 403 (1990)).
*Inhibit expression of a gene:* As used herein, the phrase "inhibit expression of a gene" means to cause a reduction in the amount of an expression product of the gene. The expression product can be an RNA transcribed from the gene (*e.g.,* an mRNA) or a polypeptide translated from an mRNA transcribed from the gene. Typically a reduction in the level of an mRNA results in a reduction in the level of a polypeptide translated therefrom. The level of expression may be determined using standard techniques for measuring mRNA or protein.
*In vitro:* As used herein, the term "*in vitro*" refers to events that occur in an artificial environment, *e.g*., in a test tube or reaction vessel, in cell culture, in a Petri dish, *etc.,* rather than within an organism (*e.g.,* animal, plant, or microbe).
*In vivo:* As used herein, the term "*in vivo*" refers to events that occur within an organism (*e.g.,* animal, plant, or microbe).
*Isolated:* As used herein, the term "isolated" refers to a substance or entity that has been (1) separated from at least some of the components with which it was associated when initially produced (whether in nature or in an experimental setting), and/or (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.
*Preventing:* As used herein, the term "preventing" refers to partially or completely delaying onset of a viral infection; partially or completely delaying onset of one or more symptoms, features, or clinical manifestations of a particular disease, disorder, and/or condition associated with a viral infection; partially or completely delaying onset of one or more symptoms, features, or manifestations of a particular disease, disorder, and/or condition prior to an identifiable viral infection; partially or completely delaying progression from an latent viral infection to an active viral infection or a particular disease, disorder and/or condition; and/or decreasing the risk of developing pathology associated with the viral infection or the disease, disorder, and/or condition.
*Similarity*: As used herein, the term "similarity" refers to the overall relatedness between polymeric molecules, *e.g.* between nucleic acid molecules (*e.g.* DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art.
*Subject:* As used herein, the term "subject" or "patient" refers to any organism to which a composition in accordance with the invention may be administered, *e.g.,* for experimental, diagnostic, prophylactic, and/or therapeutic purposes. Typical subjects include animals (*e.g.,* mammals such as mice, rats, rabbits, non-human primates, and humans) and/or plants.
*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.
*Suffering from*: An individual who is "suffering from" a disease, disorder, and/or condition has been diagnosed with or displays one or more symptoms of a disease, disorder, and/or condition.
*Susceptible to:* An individual who is "susceptible to" a disease, disorder, and/or condition has not been diagnosed with and/or may not exhibit symptoms of the disease, disorder, and/or condition. In some embodiments, an individual who is susceptible to a disease, disorder, and/or condition (for example, cancer) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein and/or nucleic acid associated with the disease, disorder, and/or condition; (4) habits and/or lifestyles associated with development of the disease, disorder, and/or condition; (5) a family history of the disease, disorder, and/or condition; and (6) exposure to and/or infection with a microbe associated with development of the disease, disorder, and/or condition. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.
*Therapeutically effective amount:* As used herein, the term "therapeutically effective amount" means an amount of an agent to be delivered (*e.g.,* nucleic acid, drug, therapeutic agent, diagnostic agent, prophylactic agent, *etc.*) that is sufficient, when administered to a subject suffering from or susceptible to a disease, disorder, and/or condition, to treat, improve symptoms of, diagnose, prevent, and/or delay the onset of the disease, disorder, and/or condition.
*Transcription factor:* As used herein, the term "transcription factor" refers to a DNA-binding protein that regulates transcription of DNA into RNA, for example, by activation or repression of transcription. Some transcription factors effect regulation of transcription alone, while others act in concert with other proteins. Some transcription factor can both activate and repress transcription under certain conditions. In general, transcription factors bind a specific target sequence or sequences highly similar to a specific consensus sequence in a regulatory region of a target gene. Transcription factors may regulate transcription of a target gene alone or in a complex with other molecules.
*Treating*: As used herein, the term "treating" refers to partially or completely alleviating, ameliorating, improving, relieving, delaying onset of, inhibiting progression of, reducing severity of, and/or reducing incidence of one or more symptoms features or clinical manifestations of a particular disease, disorder, and/or condition. For example, "treating" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.
*Unmodified:* As used herein, "unmodified" refers to the protein or agent prior to being modified.

### Equivalents and Scope

Those skilled in the art will recognize, or be able to ascertain using no more than routine experimentation, many equivalents to the specific embodiments, described herein. The scope of the present invention is not intended to be limited to the above Description, but rather is as set forth in the appended claims.

Those skilled in the art will recognize, or be able to ascertain using no more than routine experimentation, many equivalents to the specific embodiments in accordance with the invention described herein. The scope of the present invention is not intended to be limited to the above Description, but rather is as set forth in the appended claims.

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process. Furthermore, it is to be understood that the invention encompasses all variations, combinations, and permutations in which one or more limitations, elements, clauses, descriptive terms, *etc.,* from one or more of the listed claims is introduced into another claim. For example, any claim that is dependent on another claim can be modified to include one or more limitations found in any other claim that is dependent on the same base claim. Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where elements are presented as lists, *e.g.,* in Markush group format, it is to be understood that each subgroup of the elements is also disclosed, and any element(s) can be removed from the group. It should it be understood that, in general, where the invention, or aspects of the invention, is/are referred to as comprising particular elements, features, *etc.,* certain embodiments of the invention or aspects of the invention consist, or consist essentially of, such elements, features, *etc.* For purposes of simplicity those embodiments have not been specifically set forth *in haec verba* herein. It is also noted that the term "comprising" is intended to be open and permits the inclusion of additional elements or steps.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

In addition, it is to be understood that any particular embodiment of the present invention that falls within the prior art may be explicitly excluded from any one or more of the claims. Since such embodiments are deemed to be known to one of ordinary skill in the art, they may be excluded even if the exclusion is not set forth explicitly herein. Any particular embodiment of the compositions of the invention (*e.g.,* any nucleic acid or protein encoded thereby; any method of production; any method of use; *etc.*) can be excluded from any one or more claims, for any reason, whether or not related to the existence of prior art.

All cited sources, for example, references, publications, databases, database entries, and art cited herein, are incorporated into this application by reference, even if not expressly stated in the citation. In case of conflicting statements of a cited source and the instant application, the statement in the instant application shall control.

### EXAMPLES

Modified mRNAs (mmRNAs) according to the invention can be made using standard laboratory methods and materials. The open reading frame (ORF) of the gene of interest is flanked by a 5' untranslated region (UTR) containing a strong Kozak translational initiation signal and a 3' UTR (*e.g.,* an alpha-globin 3' UTR) terminating with an oligo(dT) sequence for templated addition of a polyA tail. The mmRNAs can be modified with pseudouridine (ψ) and 5-methyl-cytidine (5meC) to reduce the cellular innate immune response. Kariko K et al. Immunity 23:165-75 (2005), Kariko K et al. Mol Ther 16:1833-40 (2008), Anderson BR et al. NAR (2010).

The cloning, gene synthesis and vector sequencing can be performed by DNA2.0 Inc. (Menlo Park, CA). The ORFs can be restriction digested and used for cDNA synthesis using tailed-PCR. This tailed-PCR cDNA product can be used as the template for the modified mRNA synthesis reaction using 25mM each modified nucleotide mix (modified U/C was manufactured by TriLink Biotech, San Diego, CA, unmodifed A/G was purchased from Epicenter Biotechnologies, Madison, WI) and CellScript MegaScript™ (Epicenter Biotechnologies, Madison, WI) complete mRNA synthesis kit. The *in vitro* transcription reaction can be run for 3-4 hours at 37°C. PCR reaction can use HiFi PCR 2X Master Mix™ (Kapa Biosystems, Woburn, MA). The *in vitro* transcribed mRNA product can be run on an agarose gel and visualized. mRNA can be purified with Ambion/Applied Biosystems (Austin, TX) MEGAClear RNA™ purification kit. PCR reaction can be purified using PureLink™ PCR purification kit (Invitrogen, Carlsbad, CA) or PCR cleanup kit (Qiagen, Valencia, CA). The product can be quantified on Nanodrop™ UV Absorbance (ThermoFisher, Waltham, MA). Quality, UV absorbance quality and visualization of the product can be performed on a 1.2% agarose gel. The product can be resuspended in TE buffer.

When transfected into mammalian cells, the modified mRNAs may have a stability of between 12-18 hours.

For animal experiments, the IV delivery solution can be 150mM NaCl, 2 mM CaCl2, 2 mM Na+-phosphate, and 0.5 mM EDTA, pH 6.5 and 10µl lipofectamine (RNAiMax™, Invitrogen, Carlsbad, CA).

### Example 1: Use of synthetic modified mRNAs to produce functional anti-microbial peptides and proteins by human cells

The goal of this example is to express several functional anti-microbial polypeptides (AMPs) (*e.g.,* anti-viral polypeptides) from modified RNA in several human cell lines to test antimicrobial effect of AMPs with distinct patterns of natural distribution and activities.

Each AMP (hBD-2, LL-37, or RNAse-7) is cloned into propagation plasmid in connection with sequences required for efficient translation and prolonged life of mRNA in cell with globin 5' and 3' UTRs and polyA tail. The mRNAs containing modified nucleotides and/or backbone modifications are transcribed using a standard T7 RNA polymerase-dependent transcription system from plasmid templates. Those mRNAs are transfected into a panel of primary human cell lines including keratinocytes and fibroblasts using a lipophilic carrier. The intensive optimization of expression is performed in matrix-type experiments focusing on dose, media and delivery reagents selection. Then a dose titration curve of AMP expression can be established in a repeat administration protocol. As a positive transfection control, each construct encodes the EGFP gene for visualization. The expressed and secreted polypeptides are detected by corresponded antibodies by ELISA and Western blots. The specific antimicrobial activity is tested in corresponded microbiological plate assays or neutralization assays required for the selection of targeted microorganisms. The strain collection can be tested for sensitivity to AMPs by determining their minimal inhibitory concentration (MIC) using those methods. Apoptosis is monitored using FACS with Annexin VCy5.5 and DAPI staining. Apoptotic DNA fragmentation can also be observed by agarose gel electrophoresis. Interferon production is assayed from the cell supernatant using standard ELISA techniques and qPCR of inflammatory gene products. Experiments can be carried out with a collection of different viral pathogens of different origins (food processing environment, food products, and human clinical isolates).

### Example 2: The modified mRNA technology as a tool for developing novel antibiotic activity

The goal of this example is to develop efficient protocol for discovery, validation and development of new AMPs (*e.g.,* AVPs).

The AMP validation protocol in high throughput manner can be developed. There have been many new AMPs recently discovered, but their mechanisms of action and utility for therapeutic applications remain unknown. Modified RNA technology allows for the simultaneous testing of new AMPs for human cell toxicity and antimicrobial activities. The sequence of newly discovered candidates can be cloned for *in vitro* RNA synthesis and testing in high throughput screens without actual peptide expression. Following by the optimal protocol for modified mRNA transfection, several new AMPs expressed in human cells against a panel of microorganisms can be tested. The AMP improvement protocol can be developed. 2-3 known AMPs are selected and a systematic walkthrough mutagenesis by PCR and clone resulting constructs in plasmid vectors are performed. The library of those mutants can be tested one-by-one in a high throughput screen according to developed protocols in comparison to wild type peptides. Functional domains in testing proteins and peptides associated with human cytotoxicity and domains linked to certain mechanisms of antimicrobial activities can be identified. The results of those scanning efforts can allow engineering AMPs with optimal non-toxic but rapid bacteriostatic activities.

### Example 3: The effect of synthetic modified mRNAs coding intracellular communication factors on the expression of AMPs in human cells

The goal of this example is to use modified mRNAs coding intracellular communication factors to induce innate immune system including expression of AMPs (e.g., AVPs).

The expression of AMP genes in a variety of epithelial cells can be enhanced using specific nutrients, vitamins (D) and other short chain fatty acids as therapeutic treatment. The opportunity for more specific signal for expression of AMP can be investigated. hBD-2 messenger RNA expression in foreskin-derived keratinocytes was greatly up-regulated with TNF-α within 1 h of stimulation and persisted for more than 48 h. The TNF-α gene can be used for synthesis of modified mRNA and transfected into a panel of primary human cell lines including keratinocytes and fibroblasts using a lipophilic carrier. It can be used to test expression of several AMPs including hBD-2 in human cells. The expressed TNF-α and secreted AMPs can be detected by corresponded antibodies by ELISA and Western blots. The specific anti-microbial activity can be tested in corresponded microbiological plate assays or anti-microbial neutralization assays required for the selection of targeted microorganisms. Apoptosis can be monitored using FACS with Annexin VCy5.5 and DAPI staining. Apoptotic DNA fragmentation can also be observed by agarose gel electrophoresis. Interferon production can be assayed from the cell supernatant using standard ELISA techniques and qPCR of inflammatory gene products.

### Example 4: Use of synthetic modified mRNAs to produce functional antimicrobial peptides and proteins by animal cells for development of antibiotics for agriculture industry

The goal of this example is to express several functional AMPs (*e.g.,* AVPs) from modified RNA in several animal cell lines to test antimicrobial effect of AMPs with distinct patterns of natural distribution and activities to test possibility to use modified RNAs as antibiotics in agriculture.

Each AMP (hBD-2, LL-37, and RNAse-7) can be cloned into propagation plasmid in connection with sequences required for efficient translation and prolonged life of mRNA in cell with globin 5' and 3' UTRs and polyA tail. The mRNAs containing modified nucleotides and/or backbone modifications can be transcribed using a standard T7 RNA polymerase-dependent transcription system from plasmid templates. Those mRNAs are transfected into a panel of primary human cell lines including keratinocytes and fibroblasts using a lipophilic carrier. The intensive optimization of expression can be performed in matrix-type experiments focusing on dose, media and delivery reagents selection. A dose titration curve of AMP expression can be established in a repeat administration protocol. As a positive transfection control, each construct encodes the EGFP gene for visualization. The expressed and secreted polypeptides can be detected by corresponded antibodies by ELISA and Western blots. The specific antimicrobial activity can be tested in corresponded microbiological plate assays or antimicrobial neutralization assays required for the selection of targeted microorganisms. Apoptosis is monitored using FACS with Annexin VCy5.5 and DAPI staining. Apoptotic DNA fragmentation can also be observed by agarose gel electrophoresis. Interferon production can be assayed from the cell supernatant using standard ELISA techniques and qPCR of inflammatory gene products.

### Example 5: In vitro selection of anti-viral inhibitory peptides encoded by synthetic modified mRNA

The viral lifecycle may be inhibited by antibody mimetic anti-viral peptides at a number of points. Viral entry into the host cell can be prevented by inhibitory peptides that ameliorate the proper folding of the viral hairpin fusion complex. Alternatively, intracellular viral propagation may be inhibited by antiviral peptides directed against viral capsid assembly thereby preventing the formation of functional infectious viral particles. The goal of this example is to identify anti-viral peptides using mRNA-display technology directed against specific viral capsid proteins or viral envelope proteins from HIV, herpes or influenza viruses. The mRNA display *in vitro* selection can be performed similar to previously described methods (Wilson et al., PNAS USA, 2001, 98(7):375). Briefly, a synthetic oligonucleotide library is constructed containing ∼10¹³ unique sequences in a 30-nt randomized region for selection of a 10aa antiviral peptide. The oligonucleotide library is synthesized containing a 3'-puromycin nucleotide analog used to covently attach the nascent peptide chain to its encoded mRNA during the *in vitro* translation step in rabbit reticulocyte lysate. A pre-selection round can filter the mRNA peptide-display library over a ligand-free column to remove non-specific binding partners from the pool. The selection rounds can then proceed through passage and incubation over a target viral-protein functionalized selection column followed by a wash through selection buffer (20 mM Tris-HCl, pH7.5; 100 mM NaCl). The bound peptides are eluted with an alkaline elution buffer (0.1M KOH) and the sequence information in the peptide is recovered through RT-PCR of the attached mRNA. Mutagenic PCR may be performed between selection rounds to further optimized binding affinity and peptide stability. Based on previous mRNA-display selections (Wilson et al., PNAS USA, 2001, 98(7):375), this selection is expected to recover high affinity (K_{d} ∼50pM-50nM) anti-viral peptides after 15-20 rounds of selection. To test *in vivo* functionality of the anti-viral peptide, synthetic modified mRNAs encoding the anti-viral peptide are transfected into target cells. Post-transfection culture transduction with infectious virus or mock-virus are performed and viral propagation can be monitored through standard pfu counts and qPCR of viral genomic material. Cells transfected with synthetic mRNAs encoding the appropriate anti-viral peptide inhibitor are expected to reduce viral propagation, display reduced pfu counts, reduced viral RNA or DNA in culture, and increase cell survival. *In vivo* efficacy, PK and toxicology can be studied in appropriate animal models.

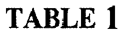

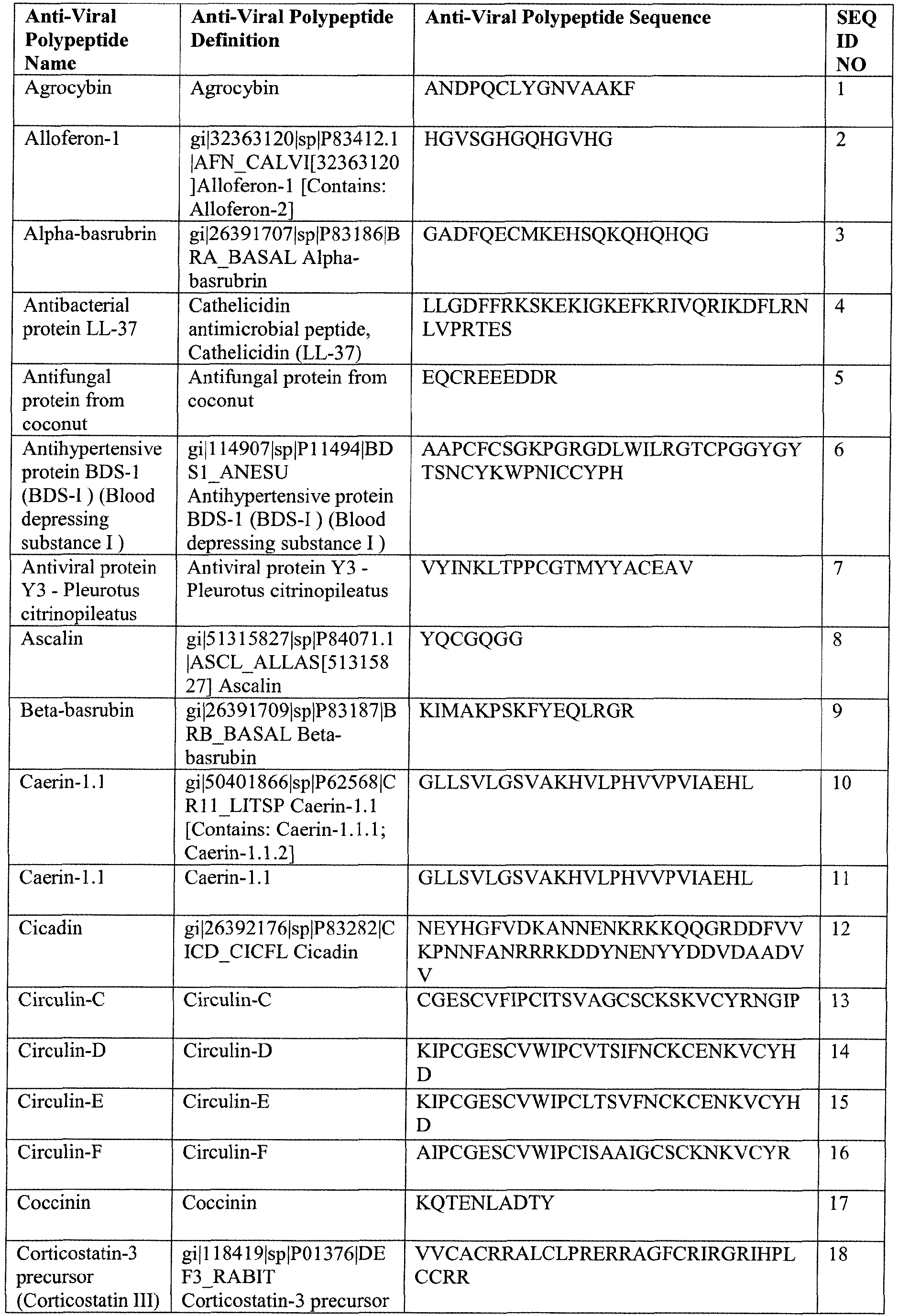

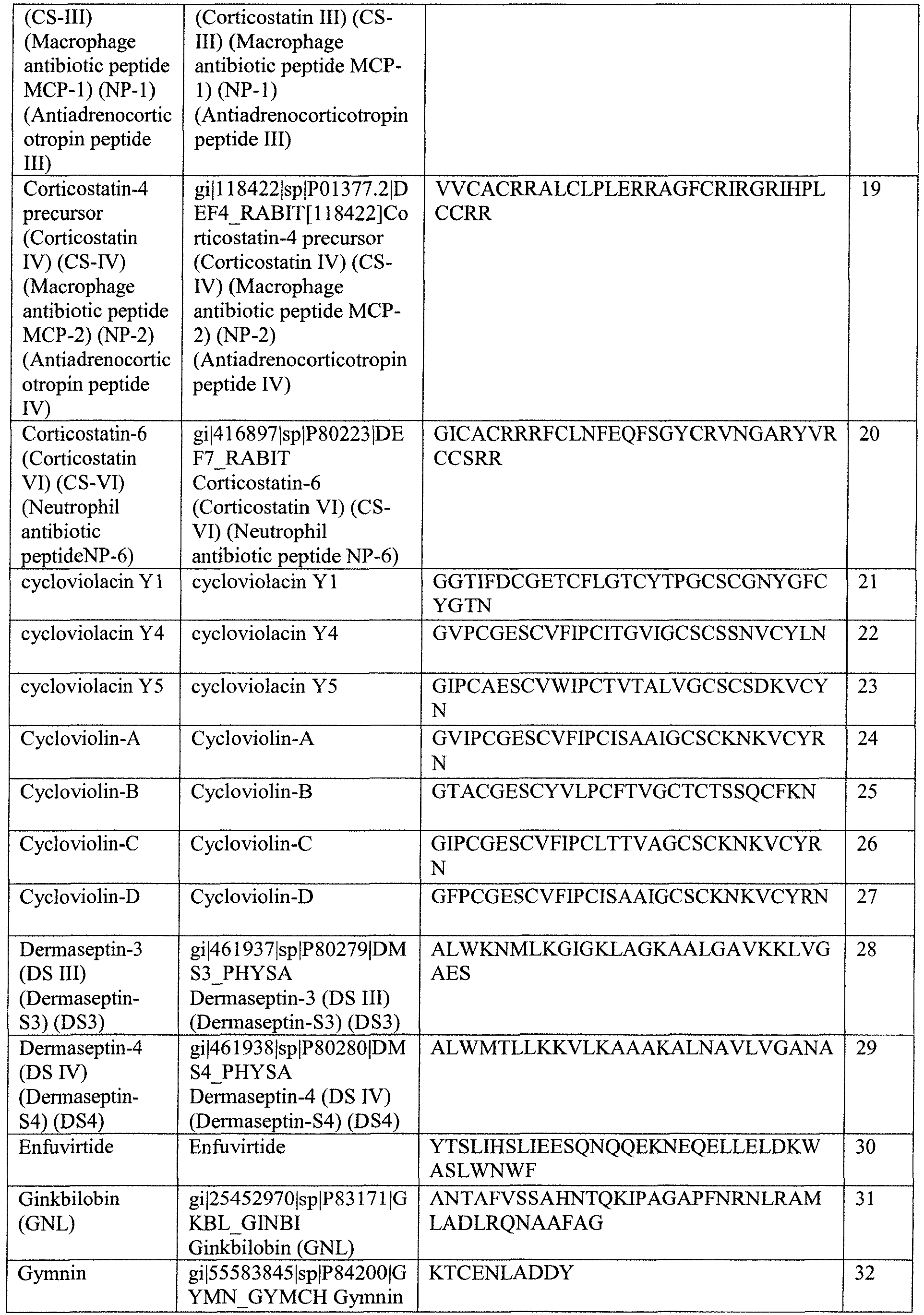

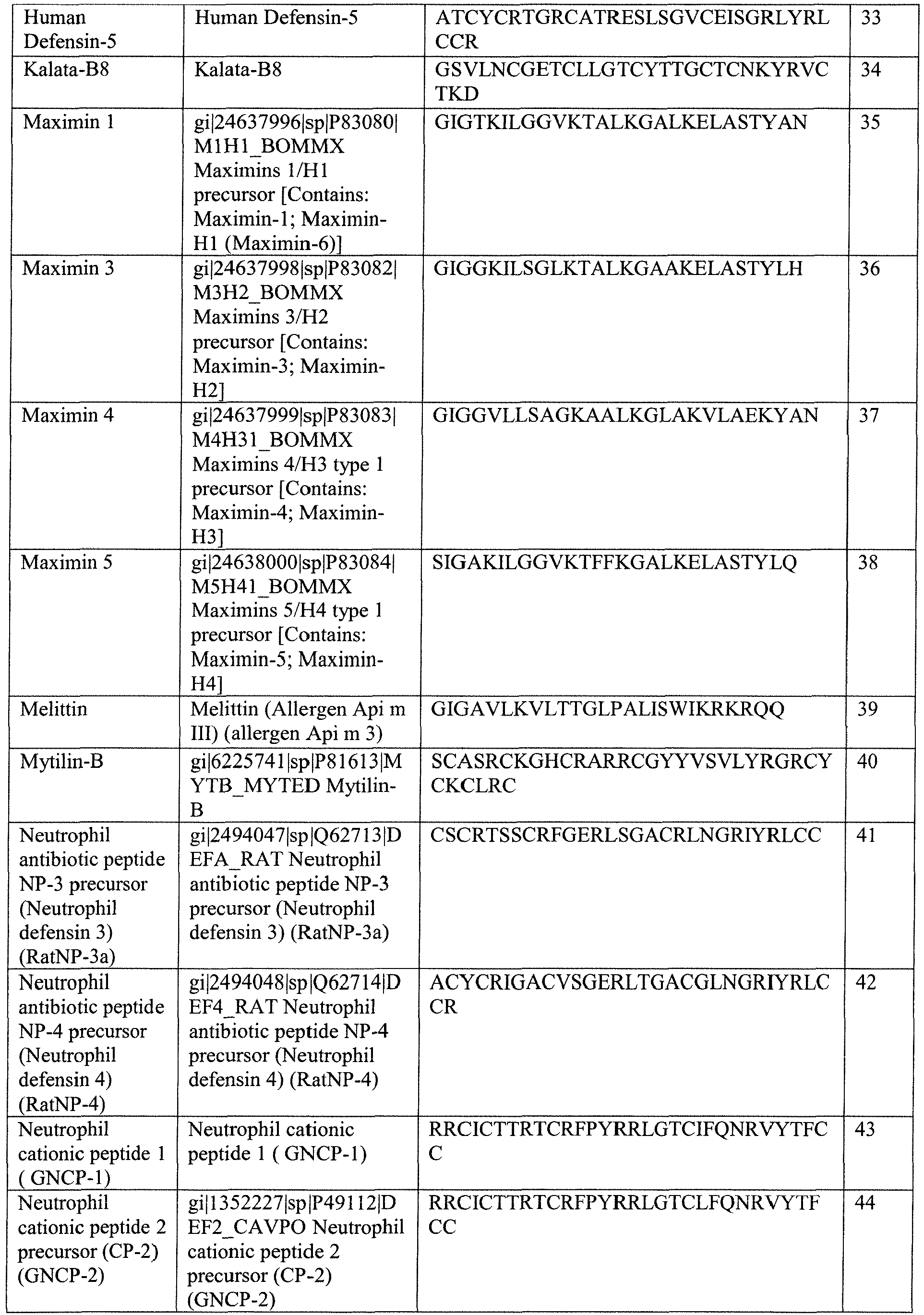

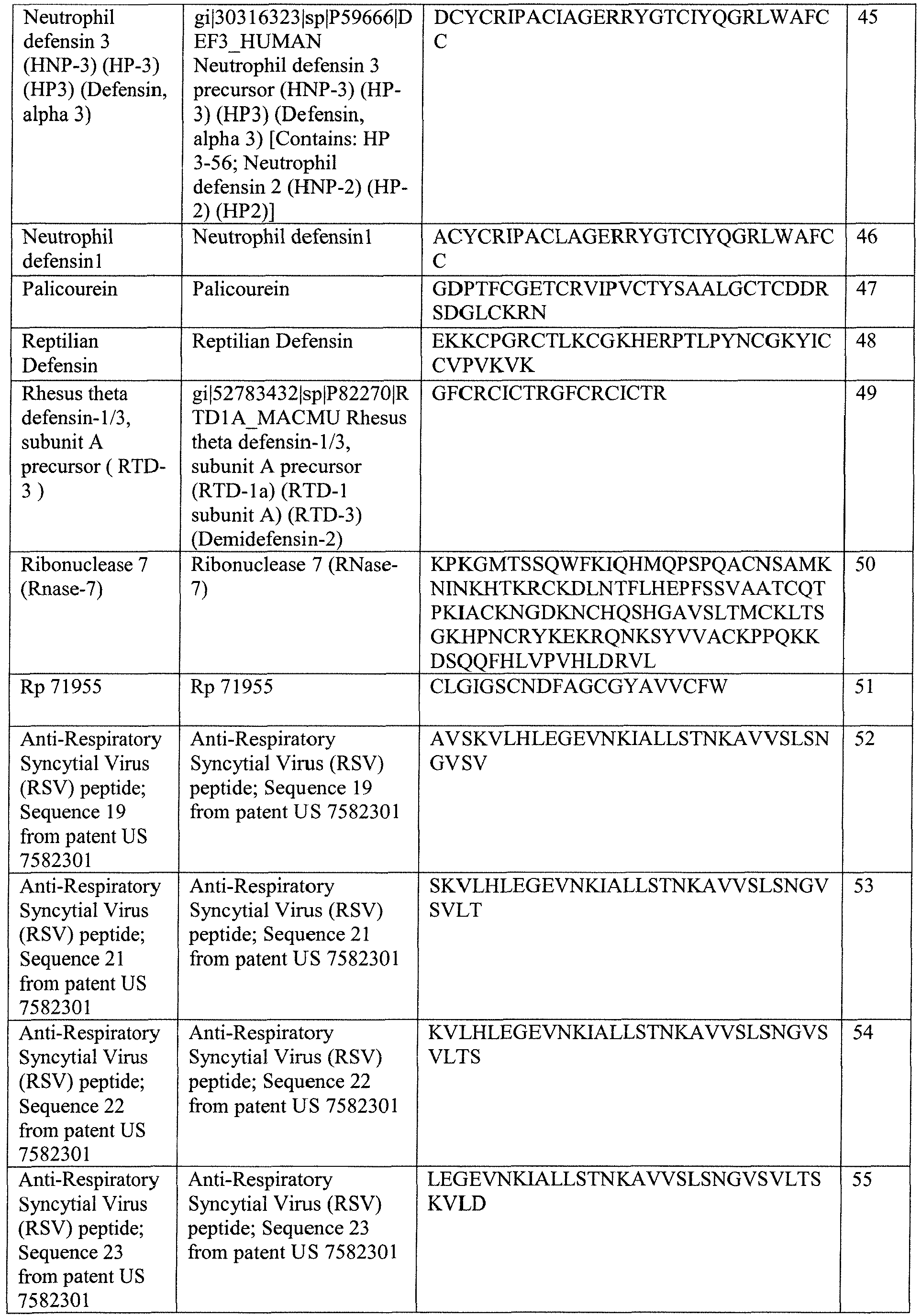

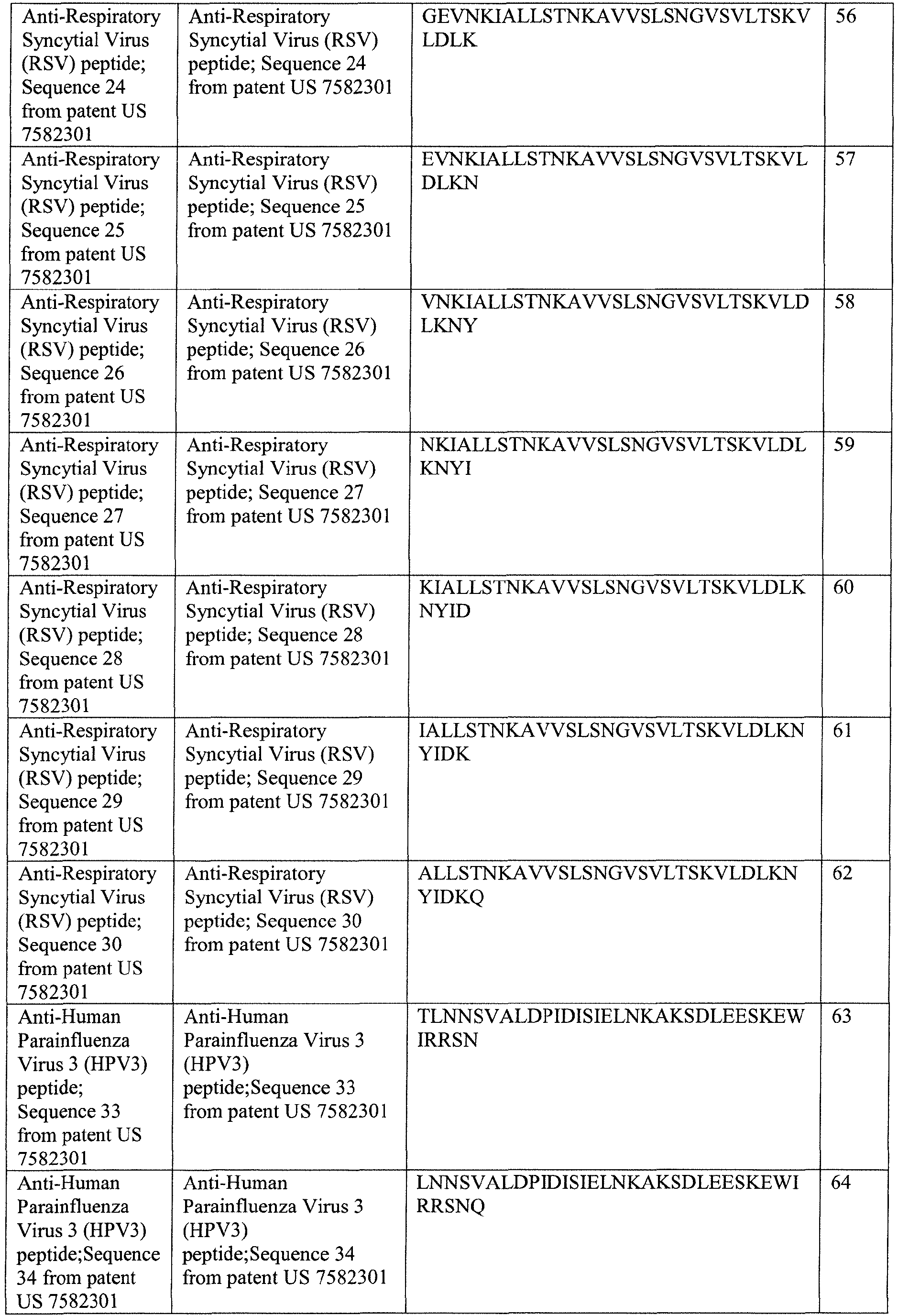

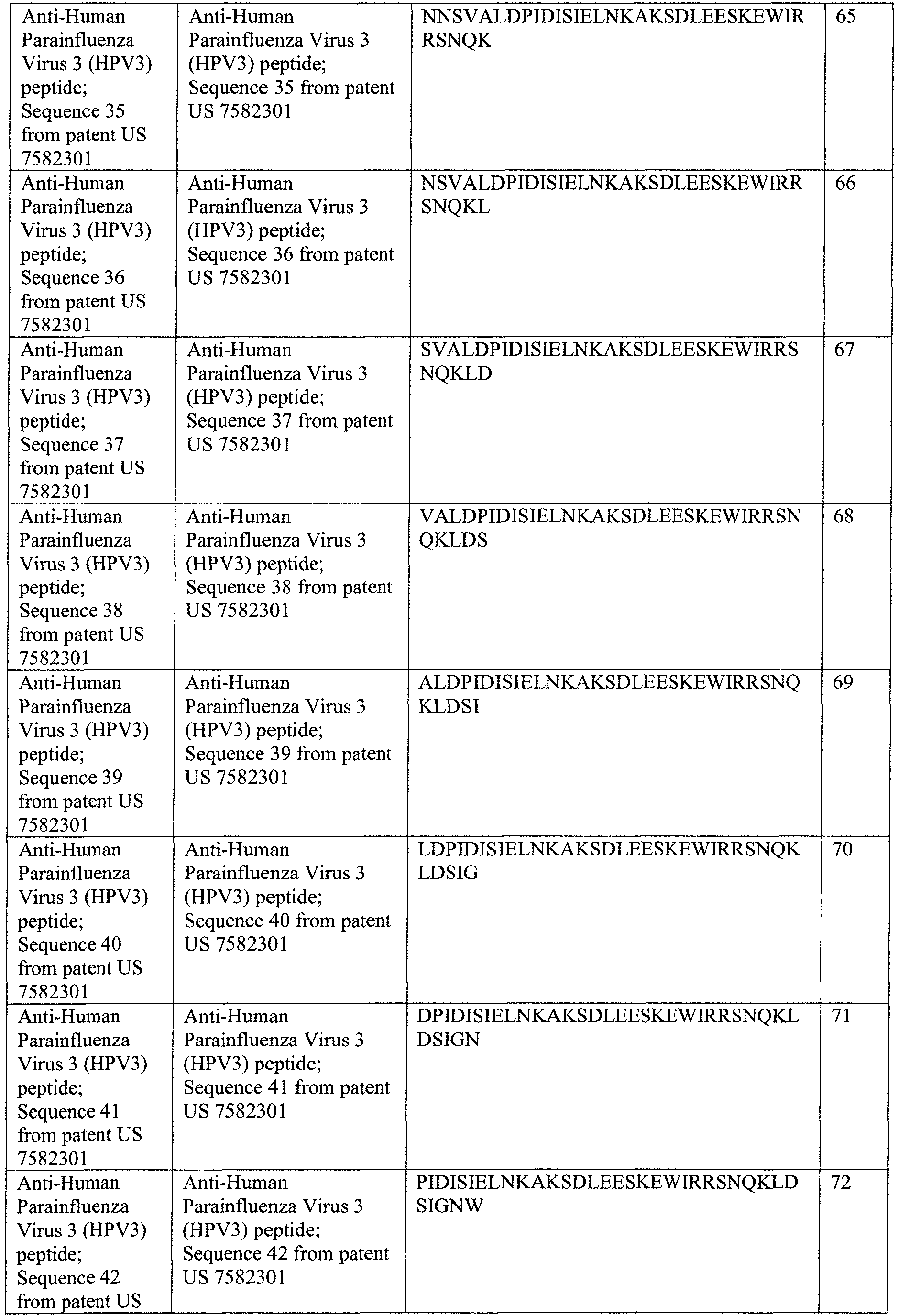

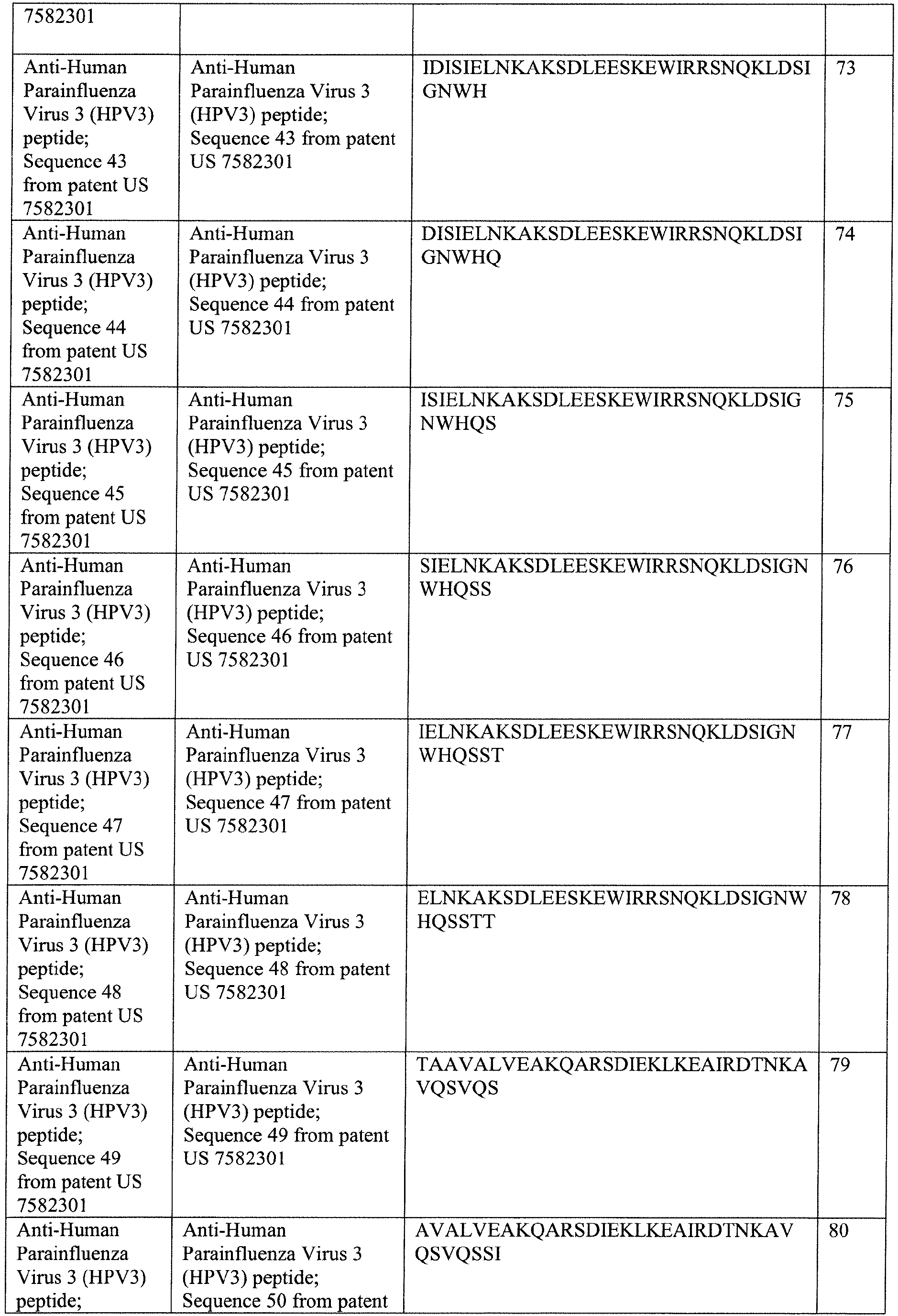

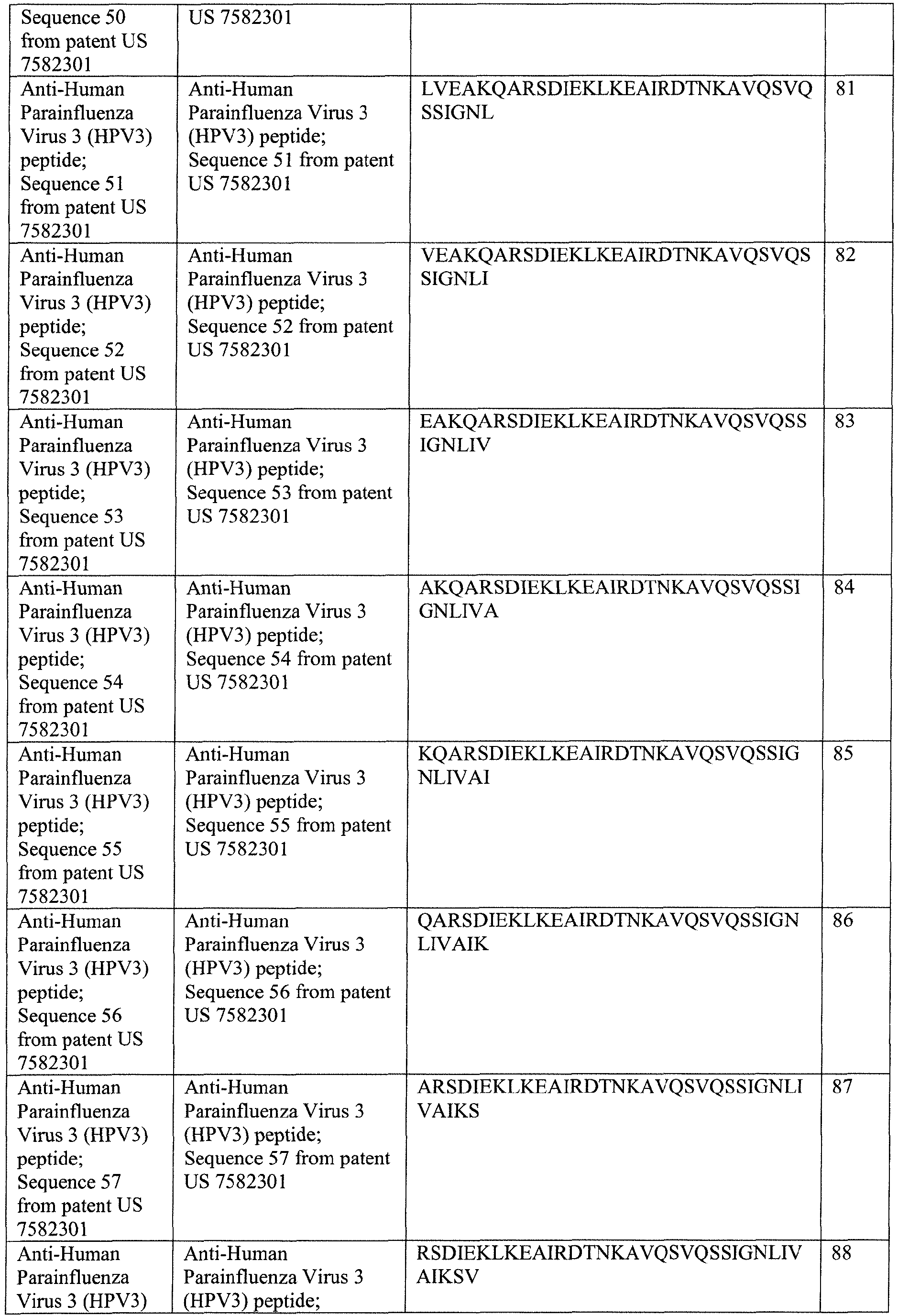

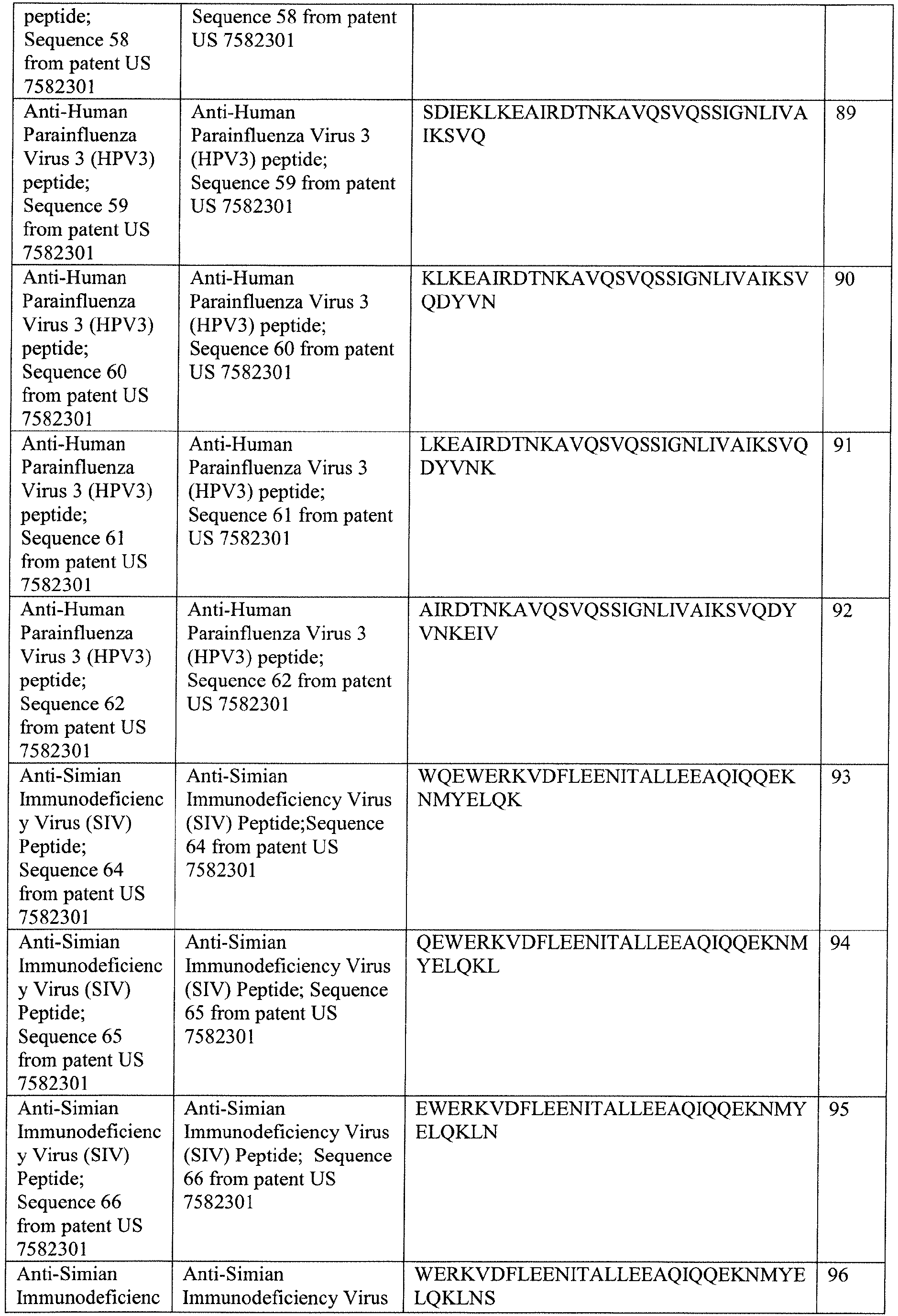

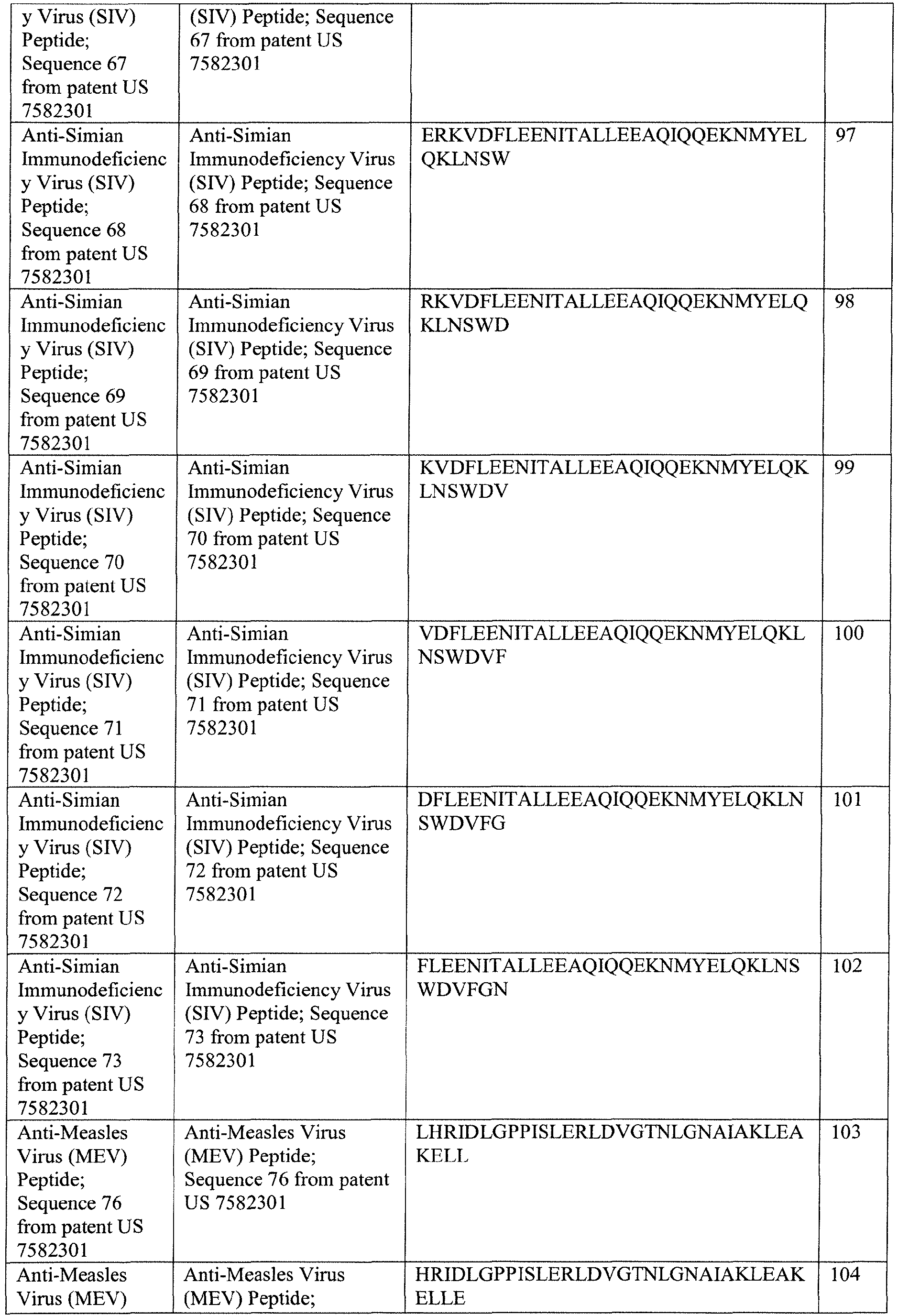

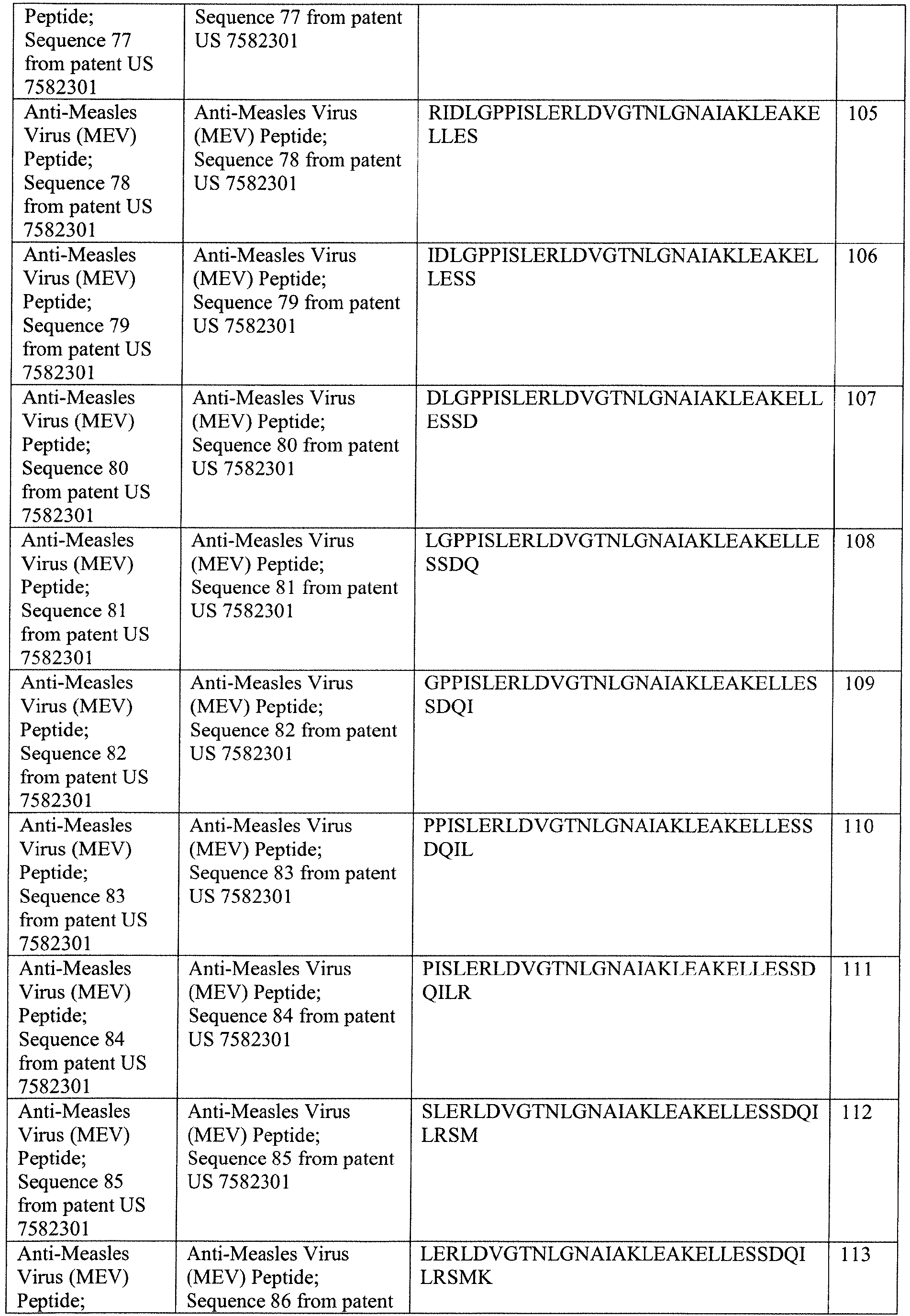

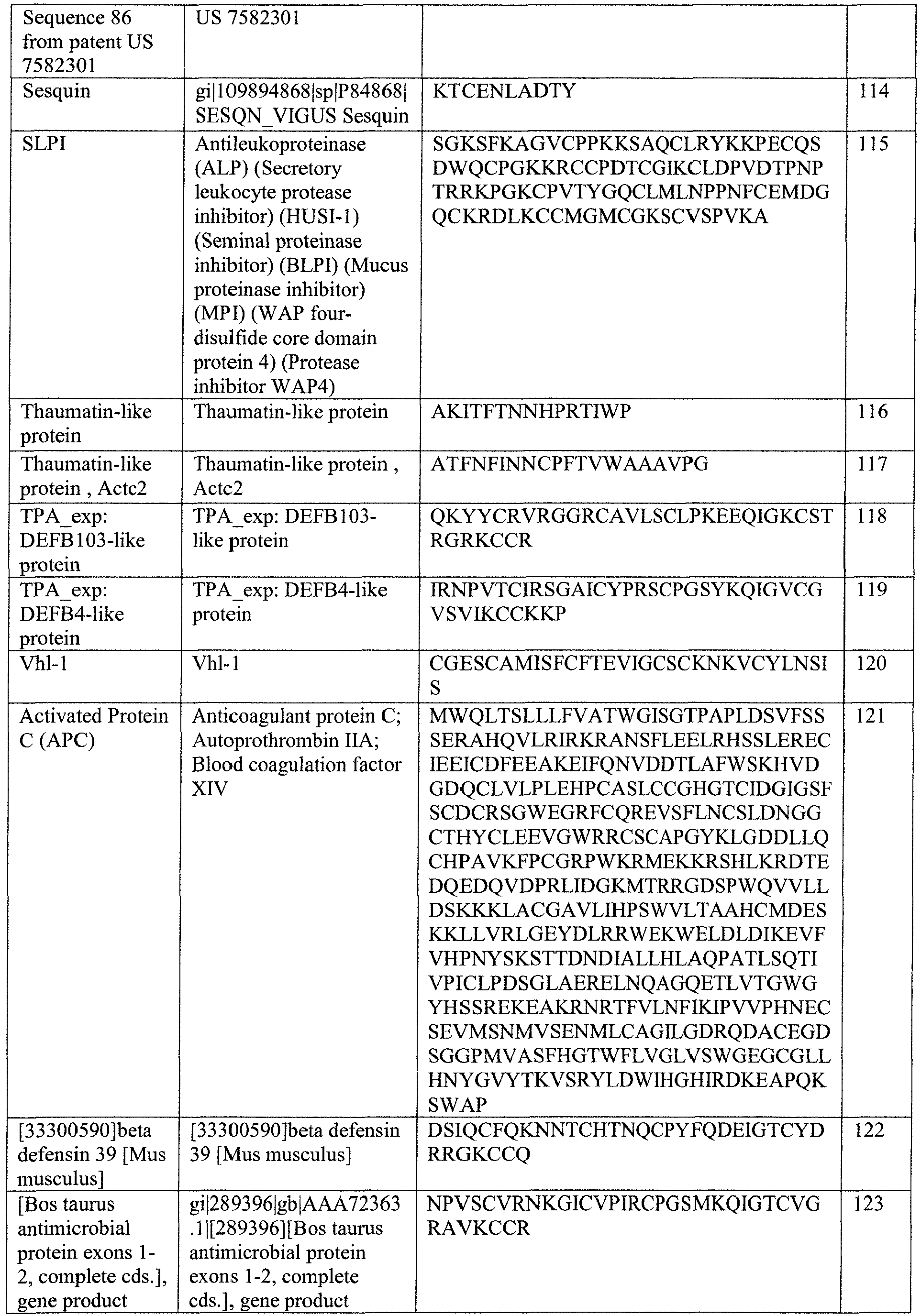

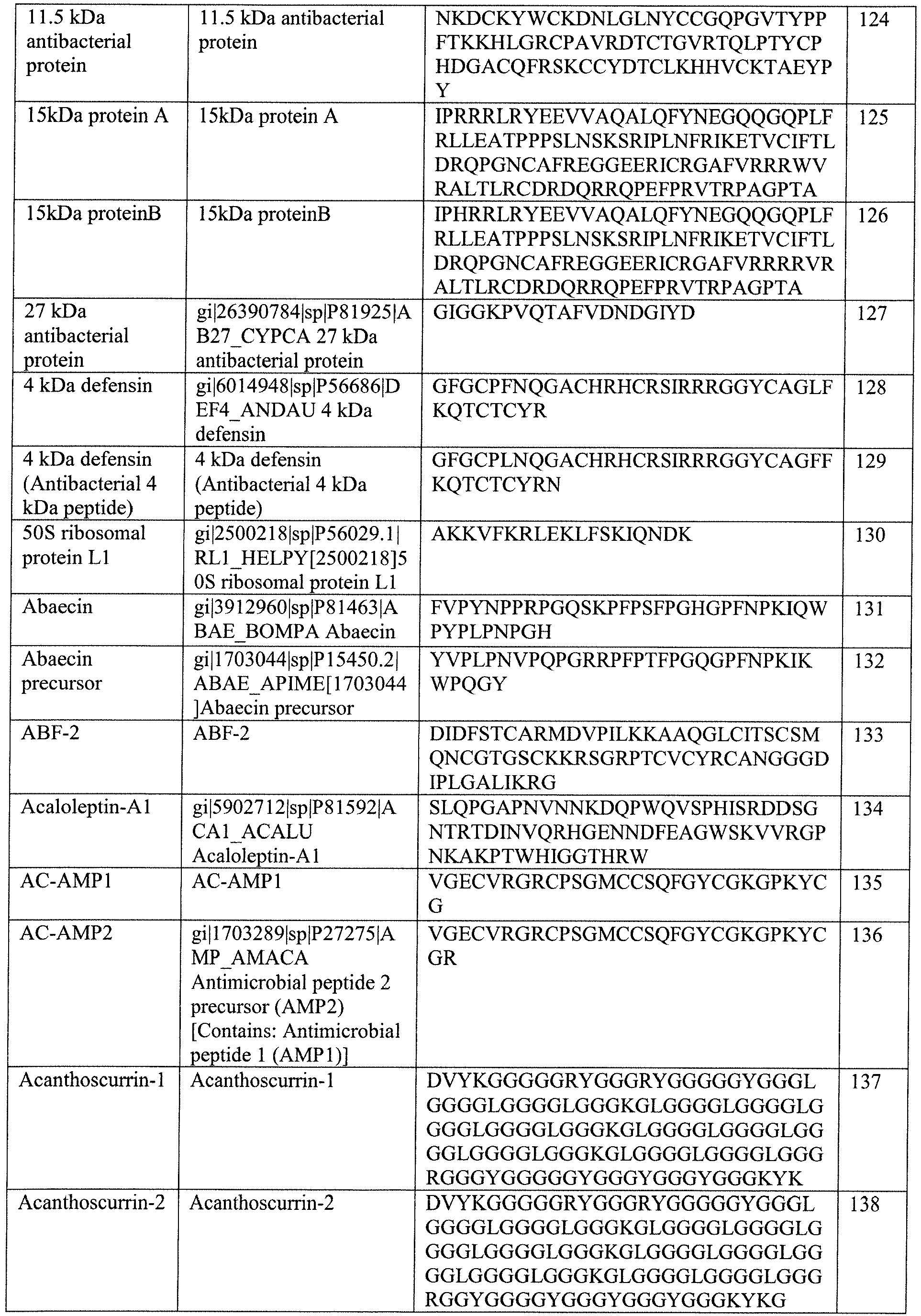

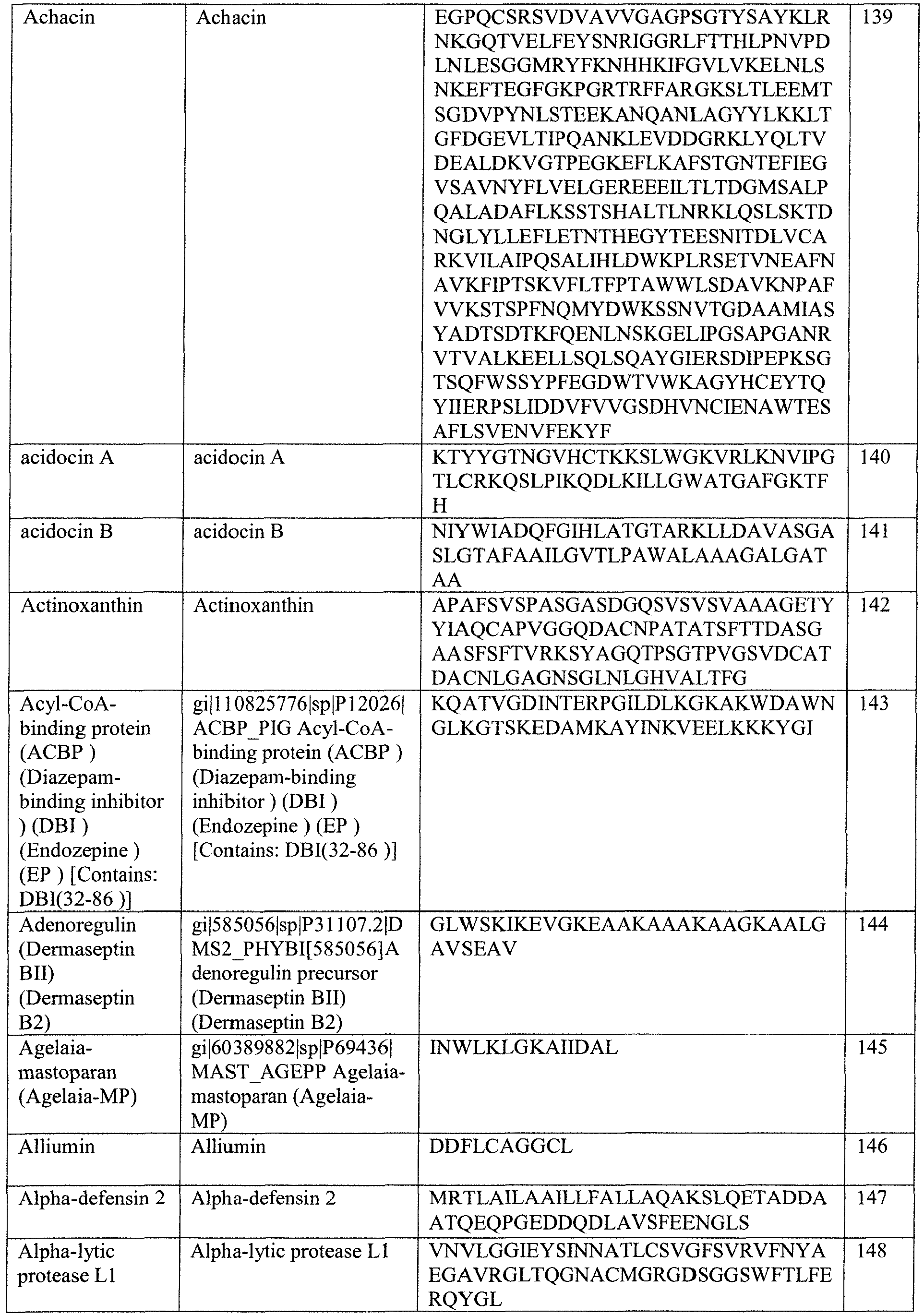

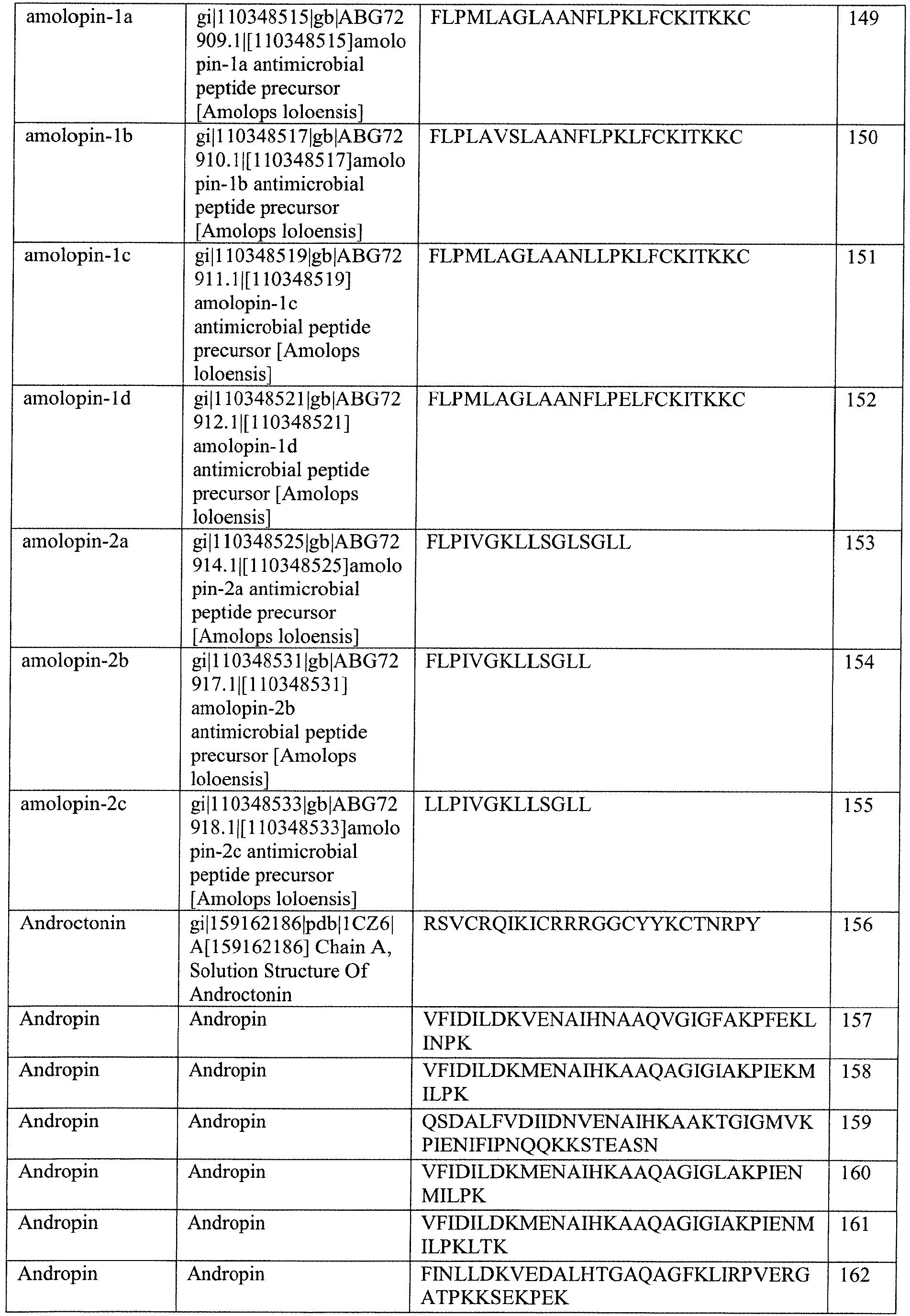

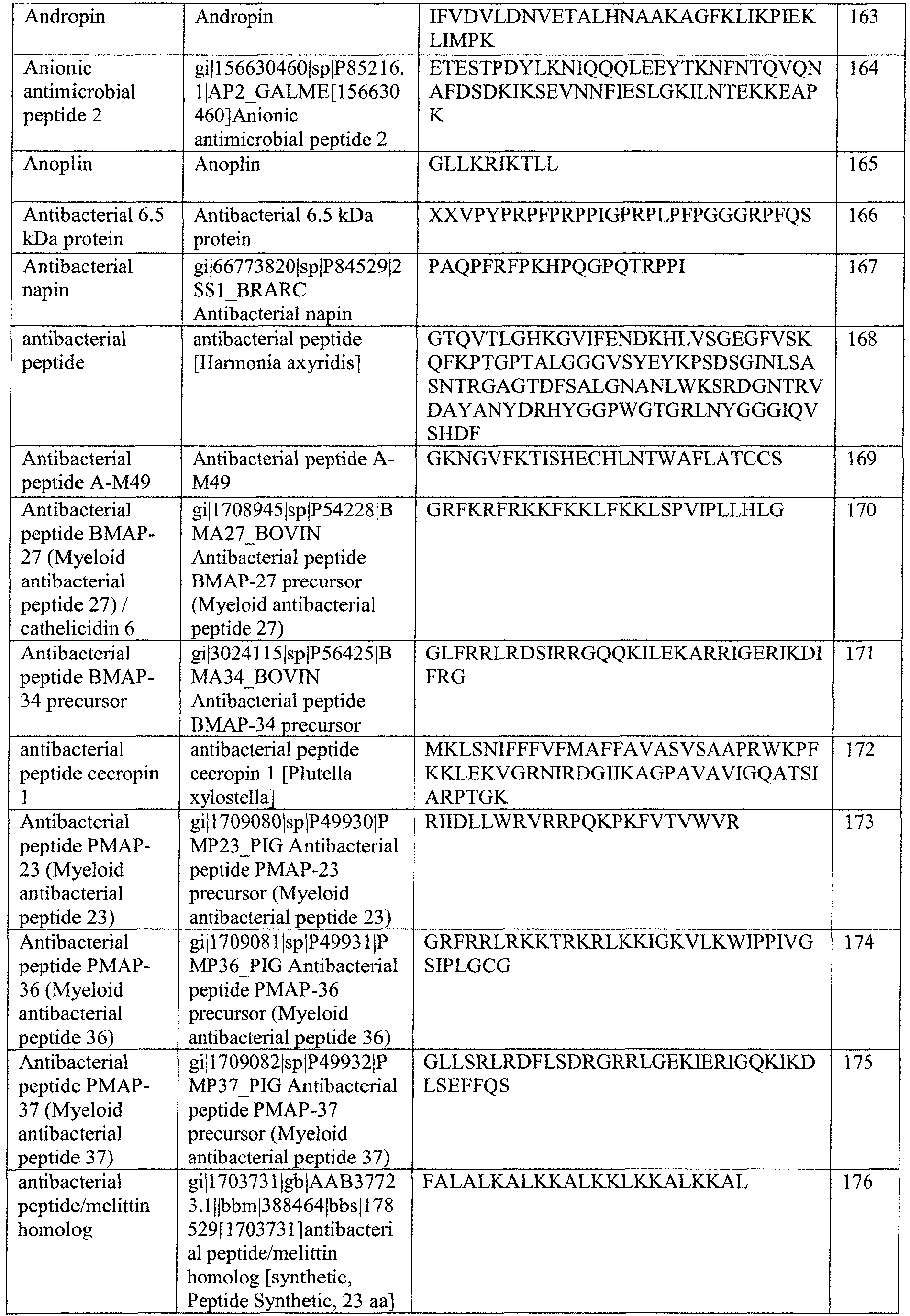

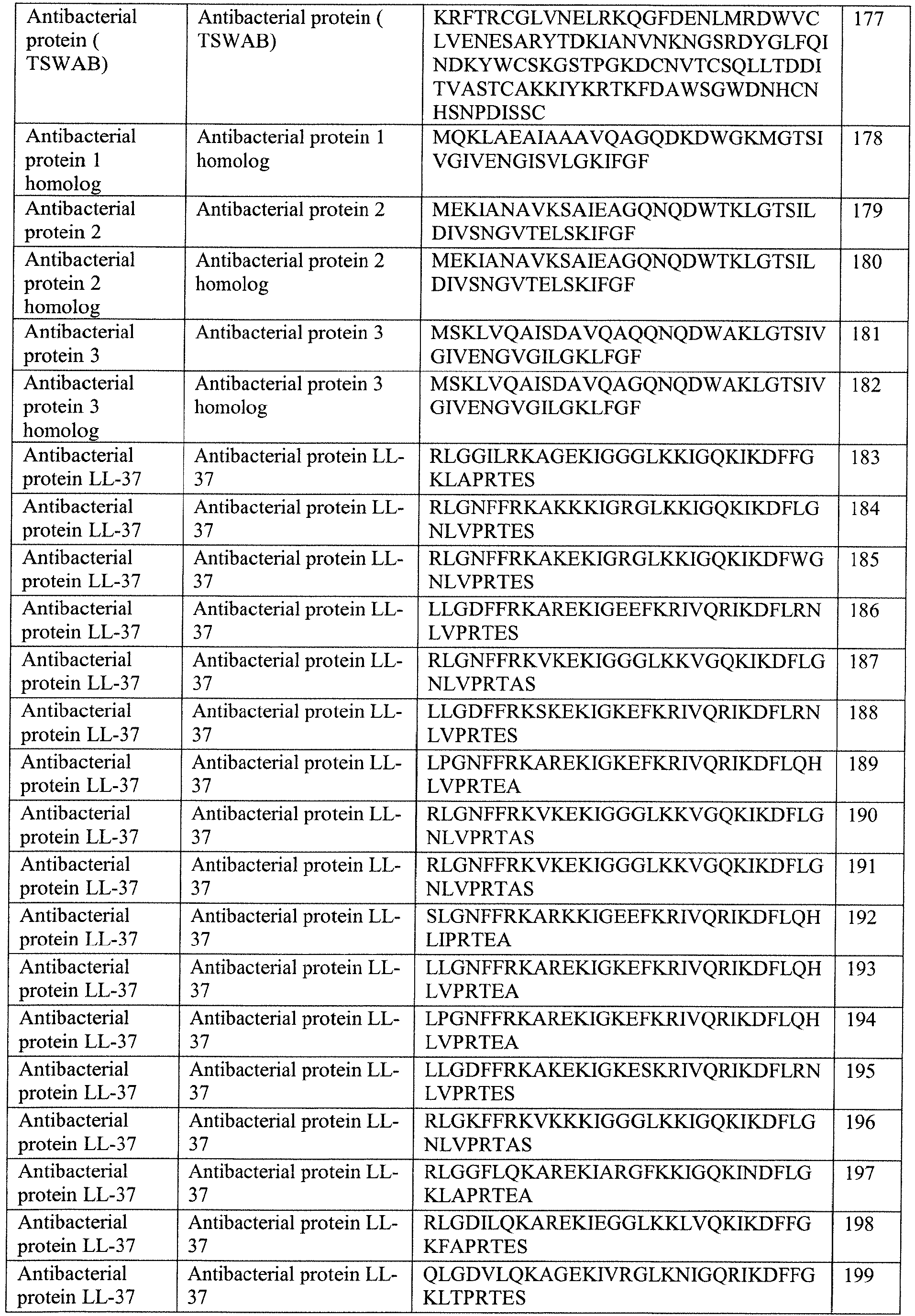

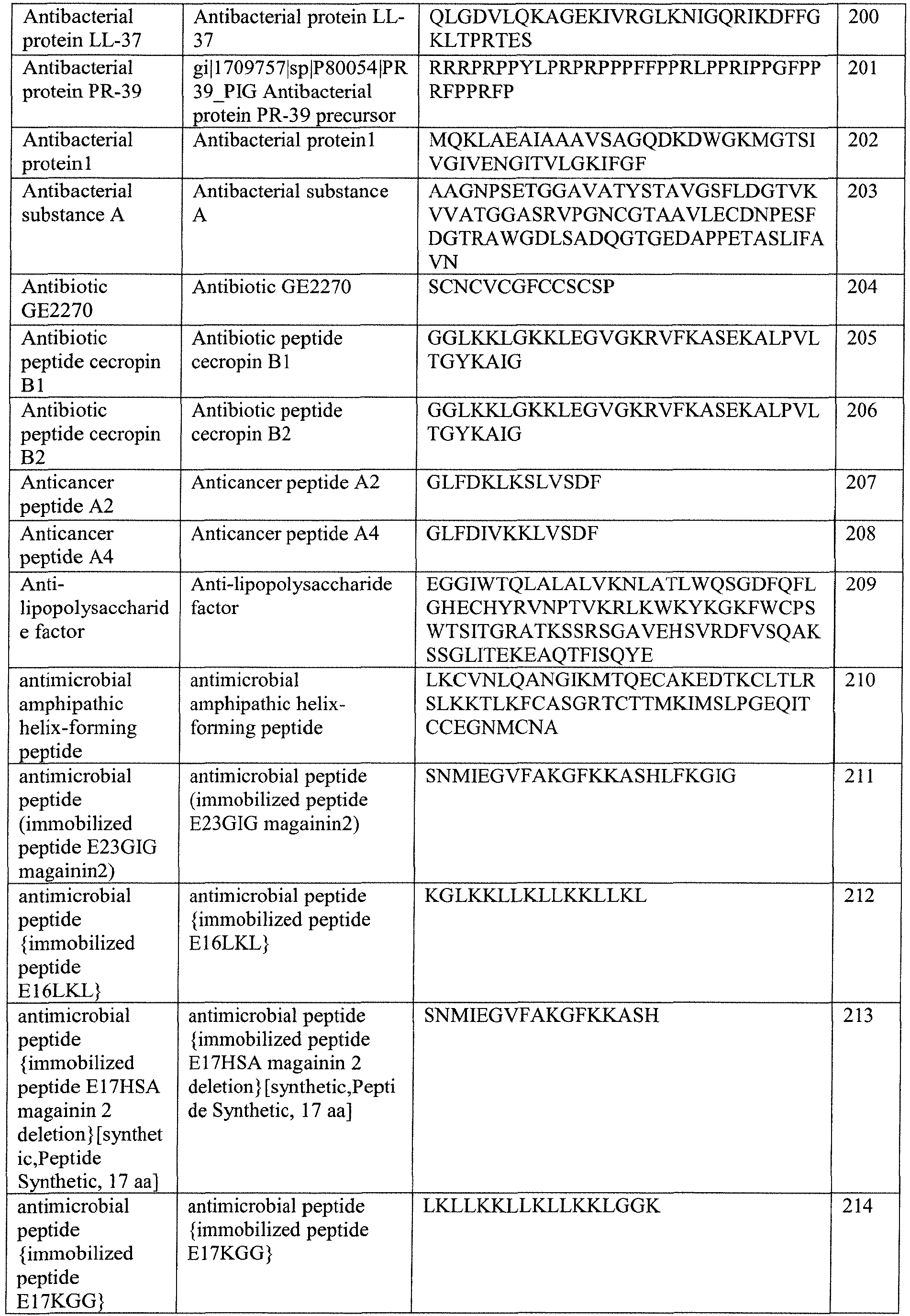

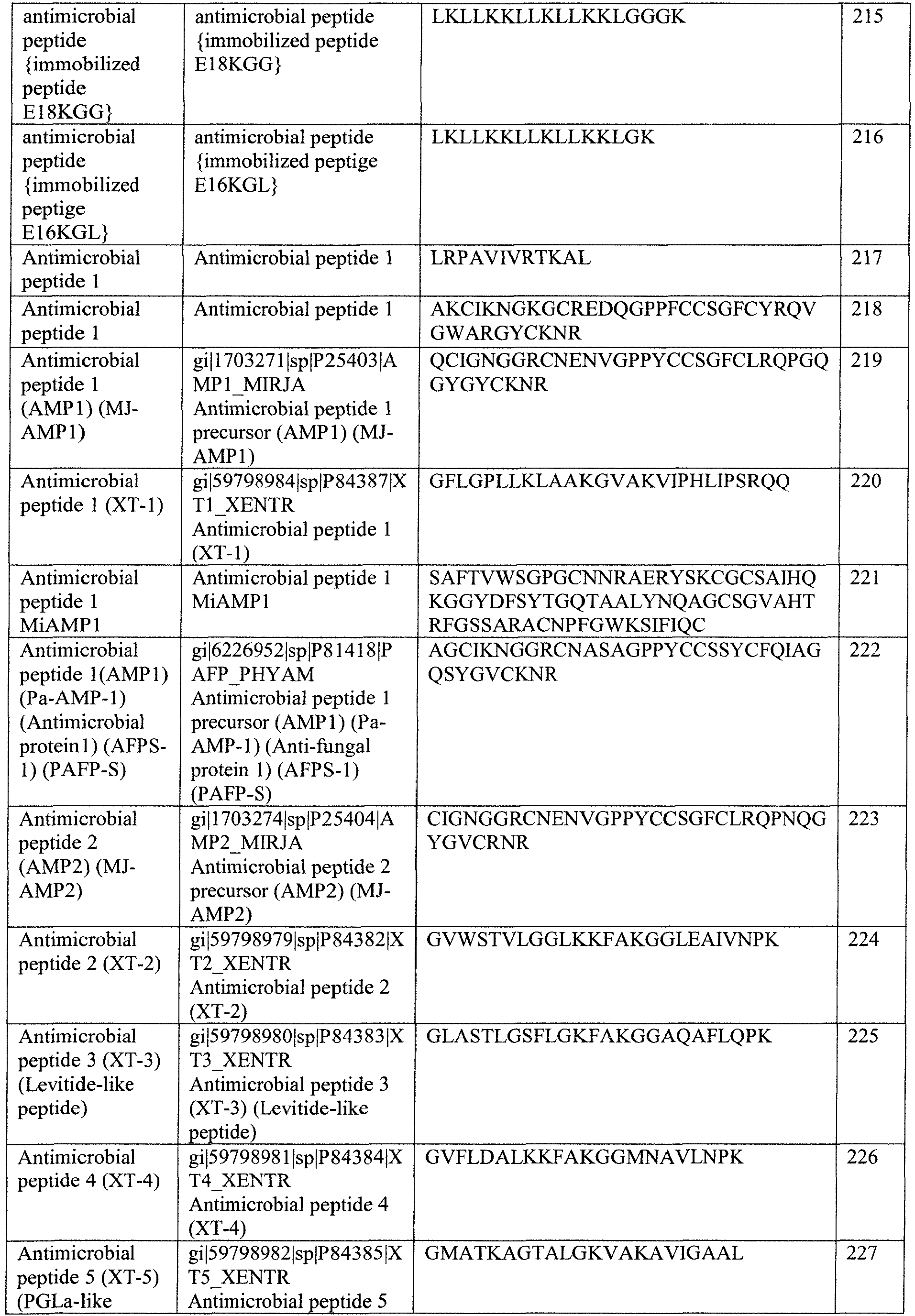

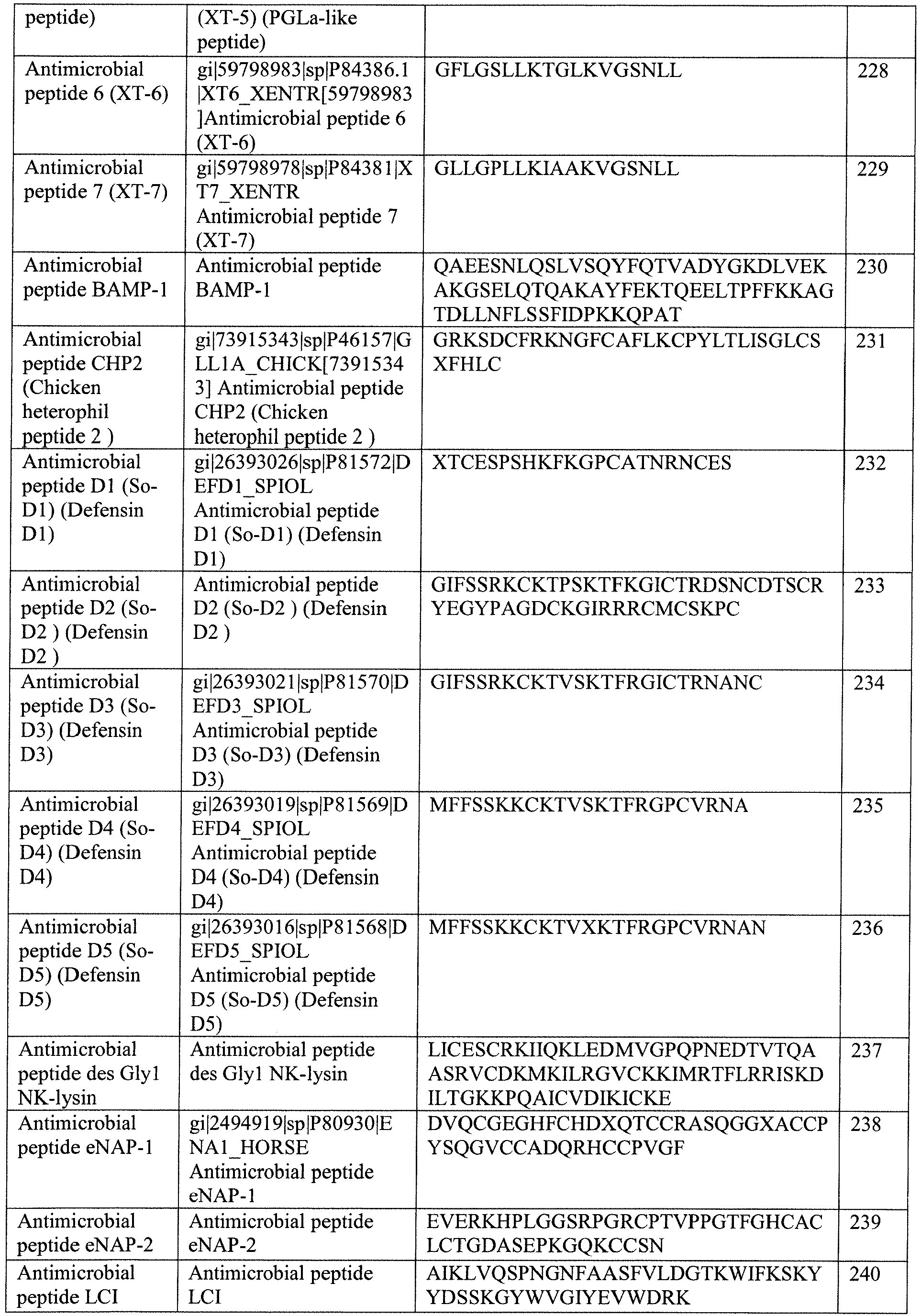

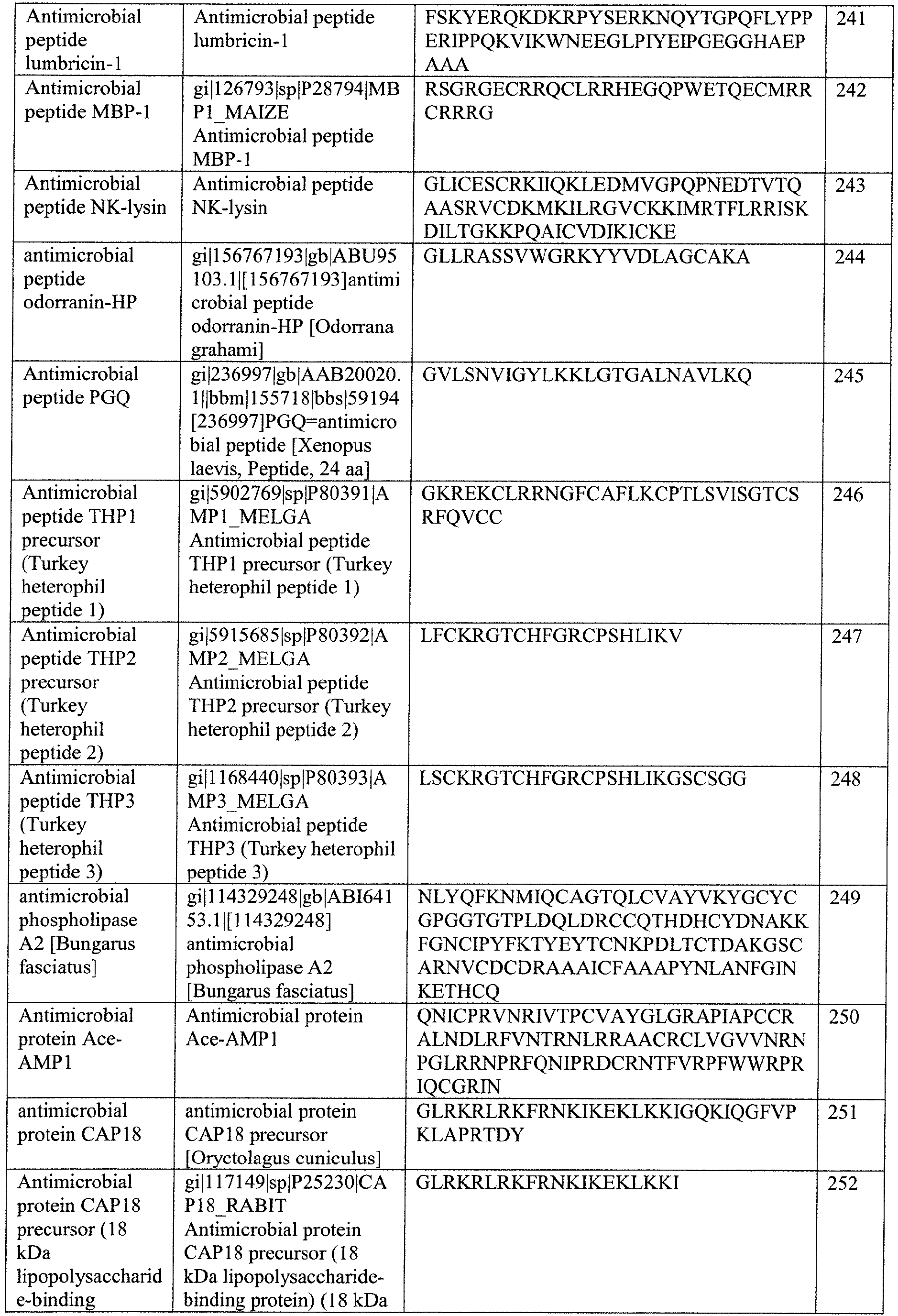

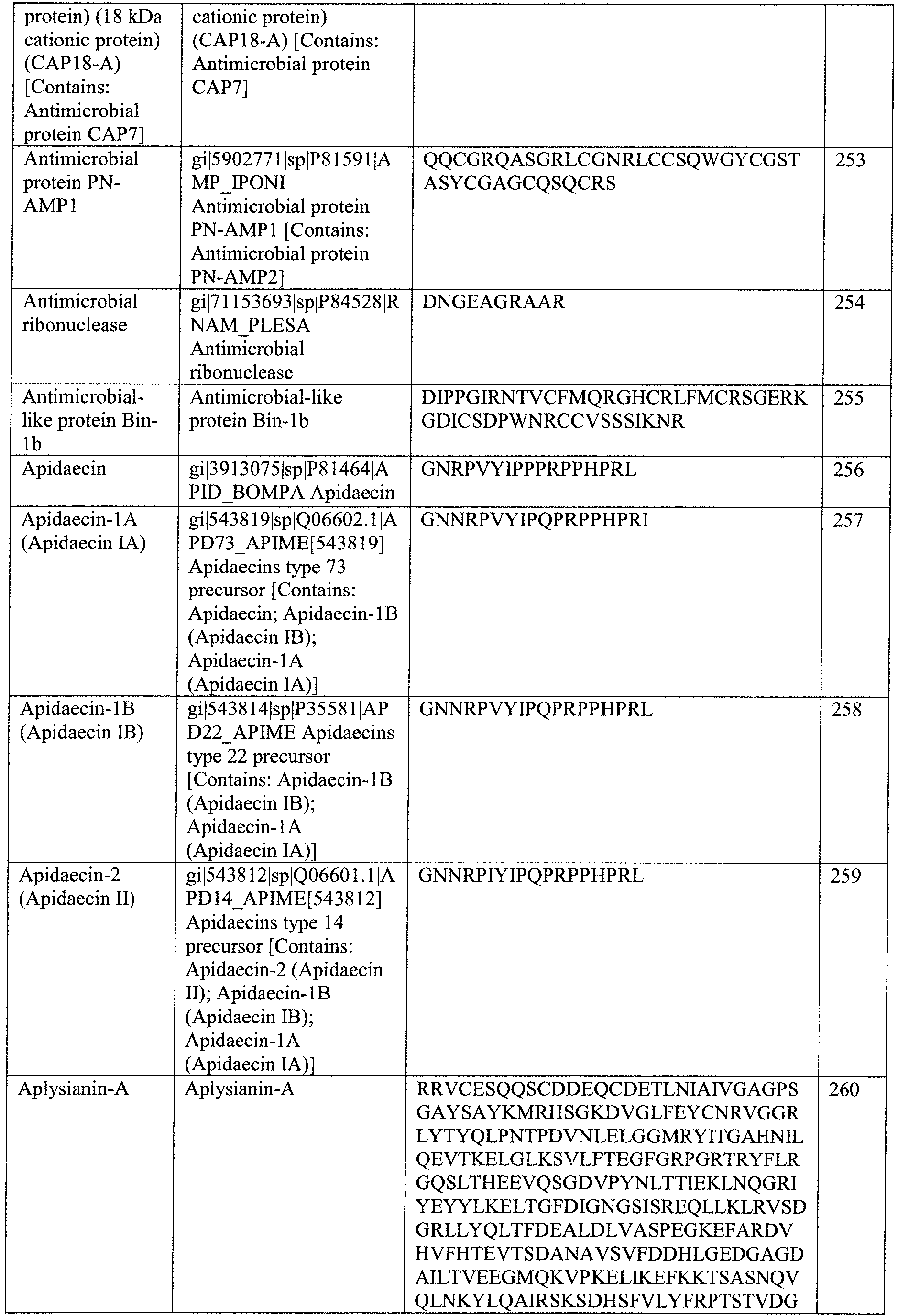

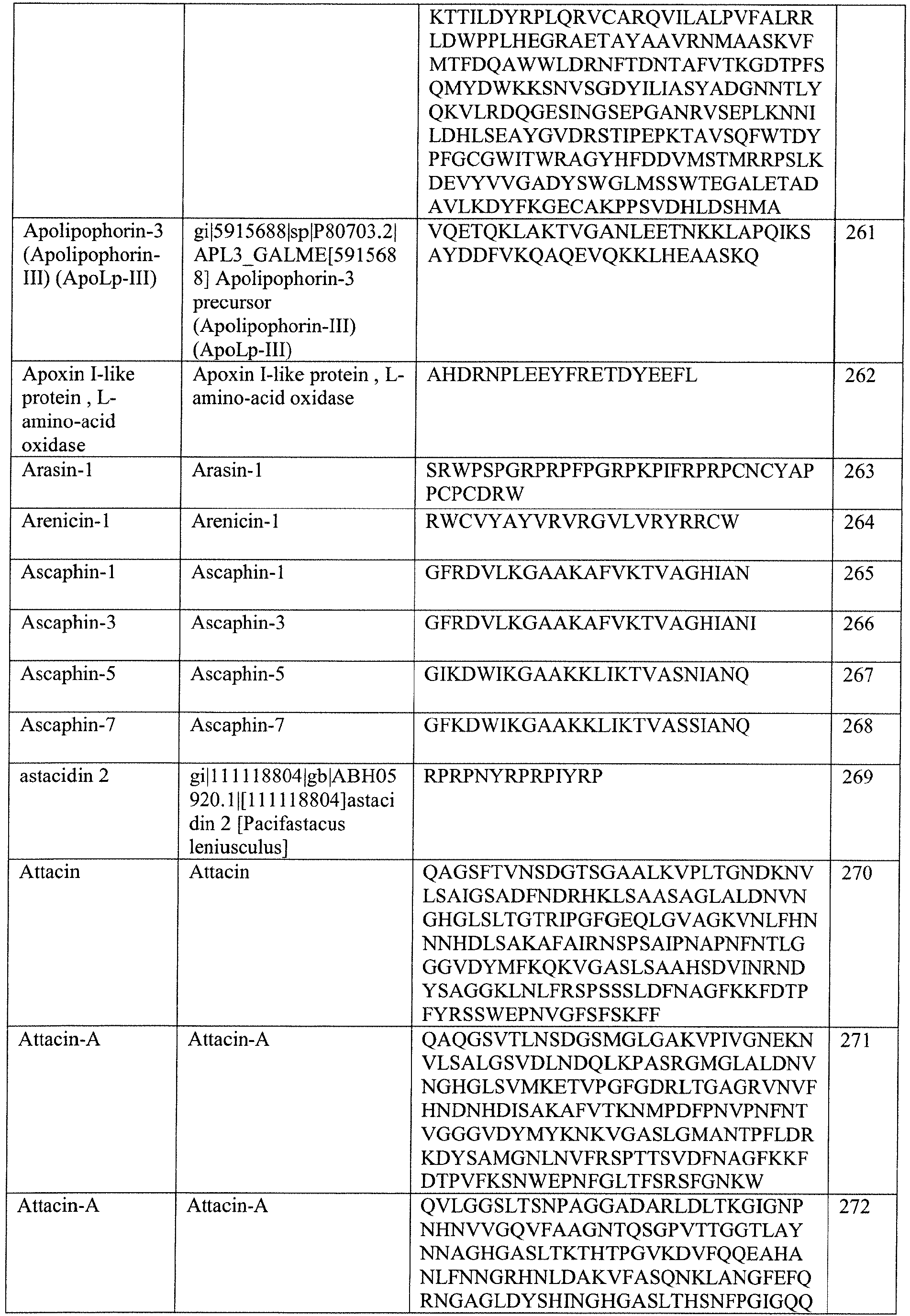

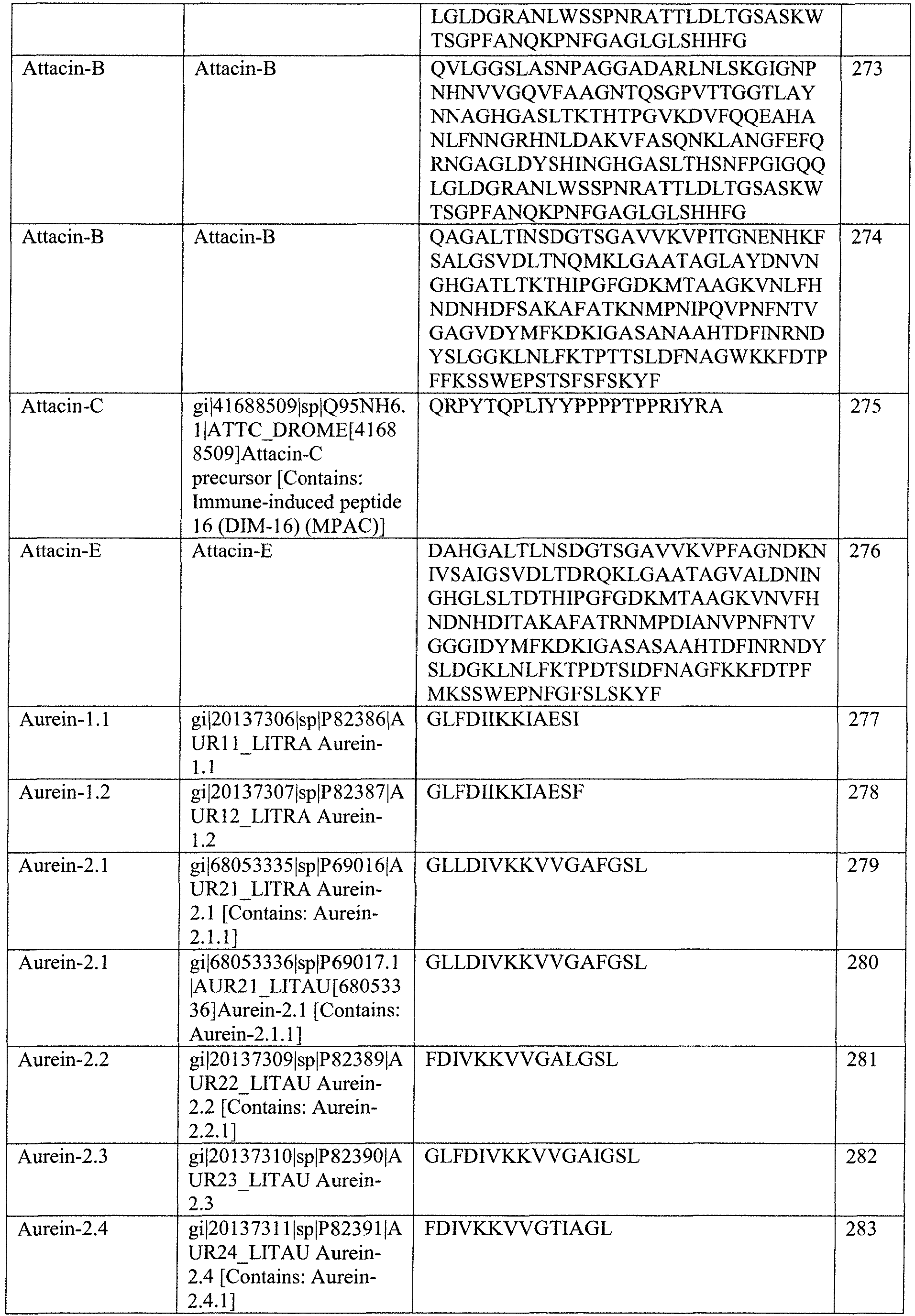

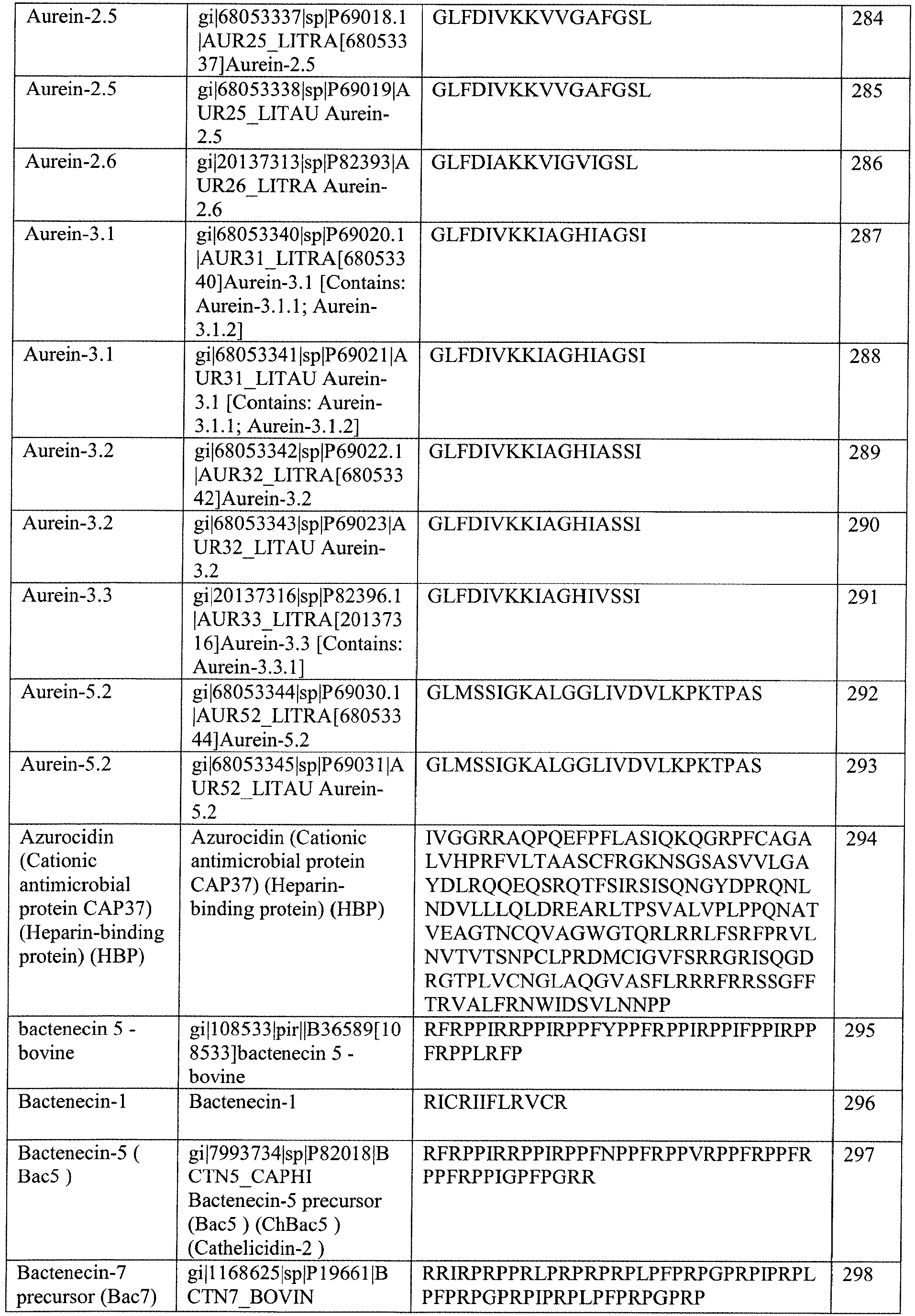

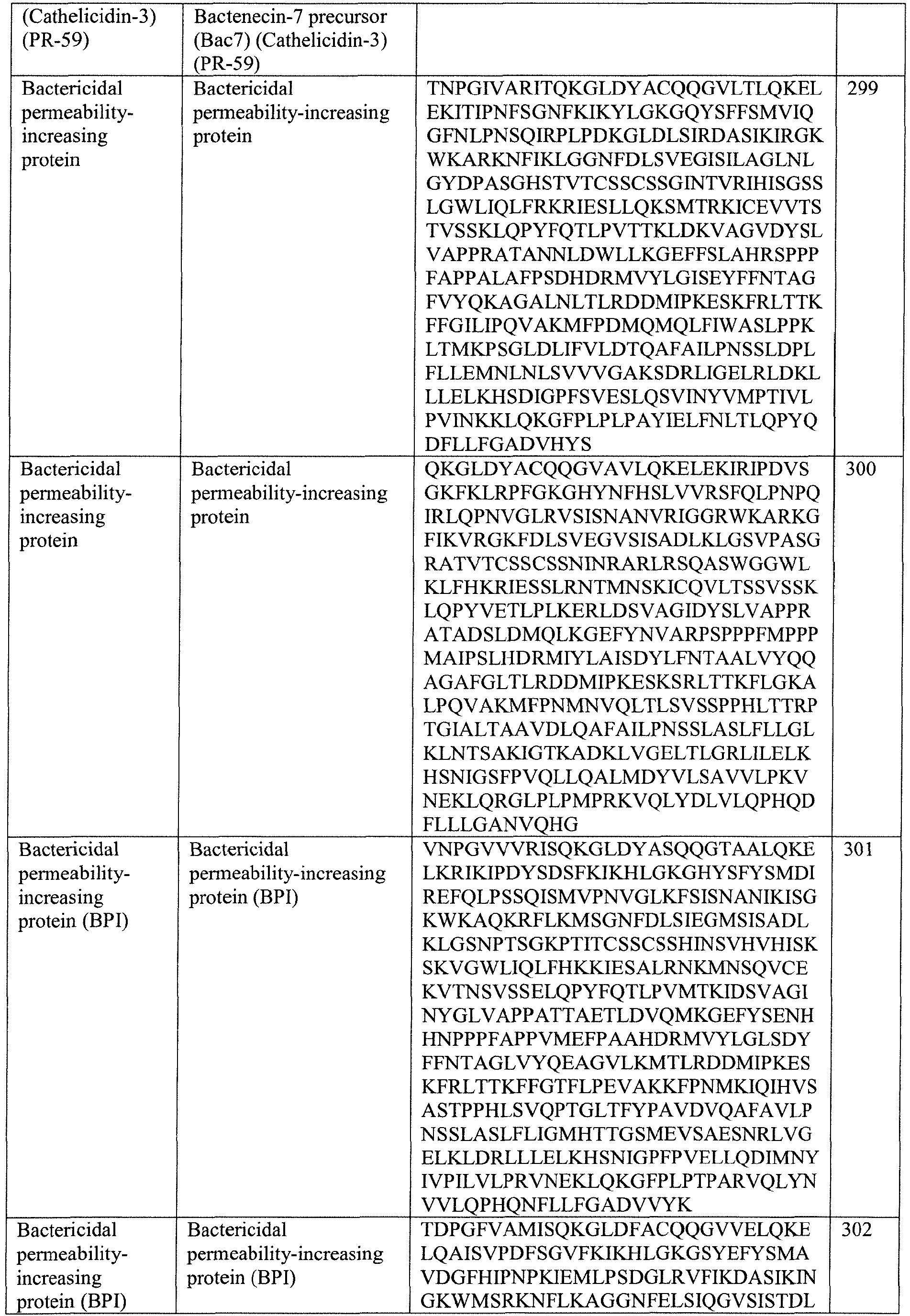

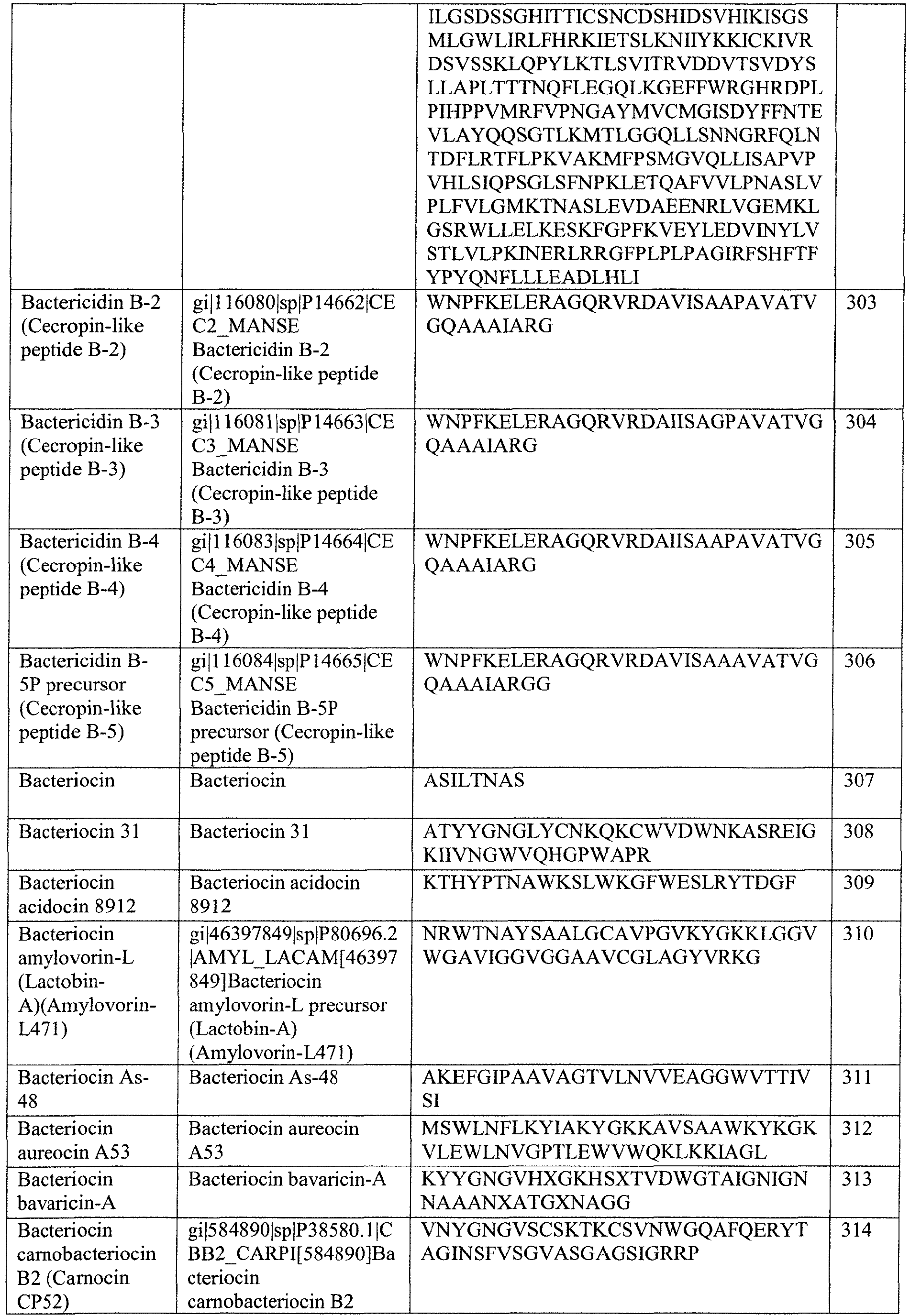

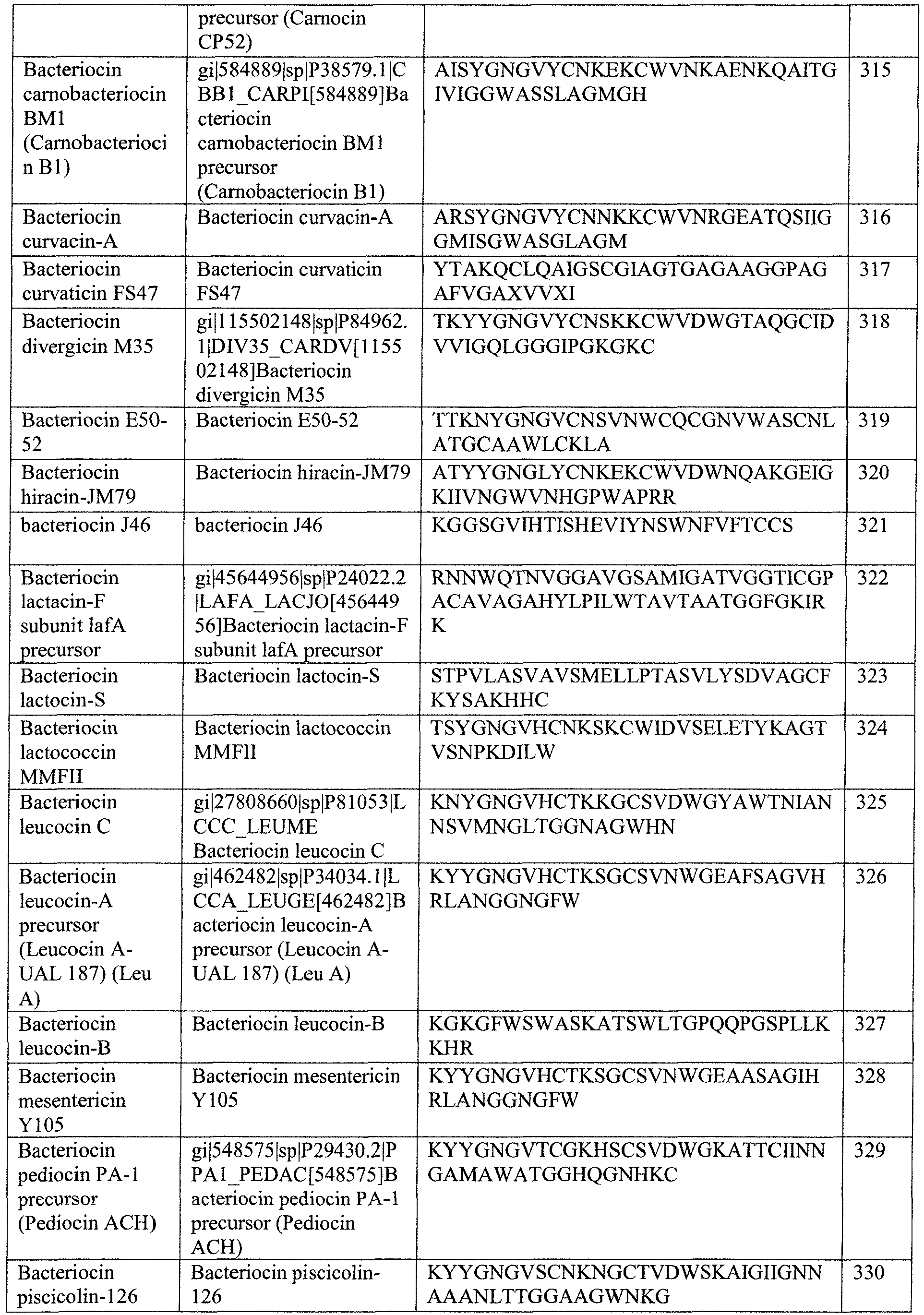

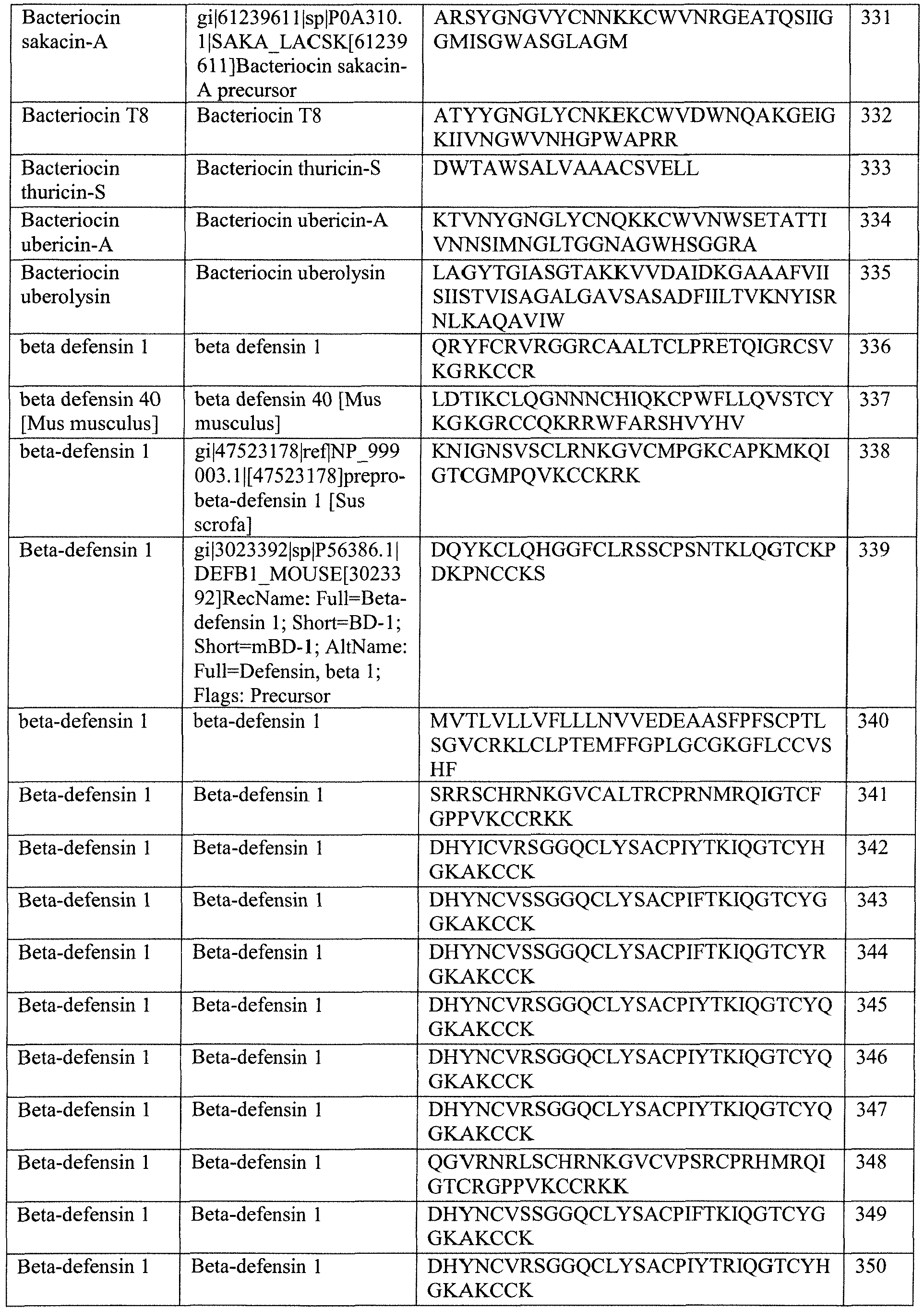

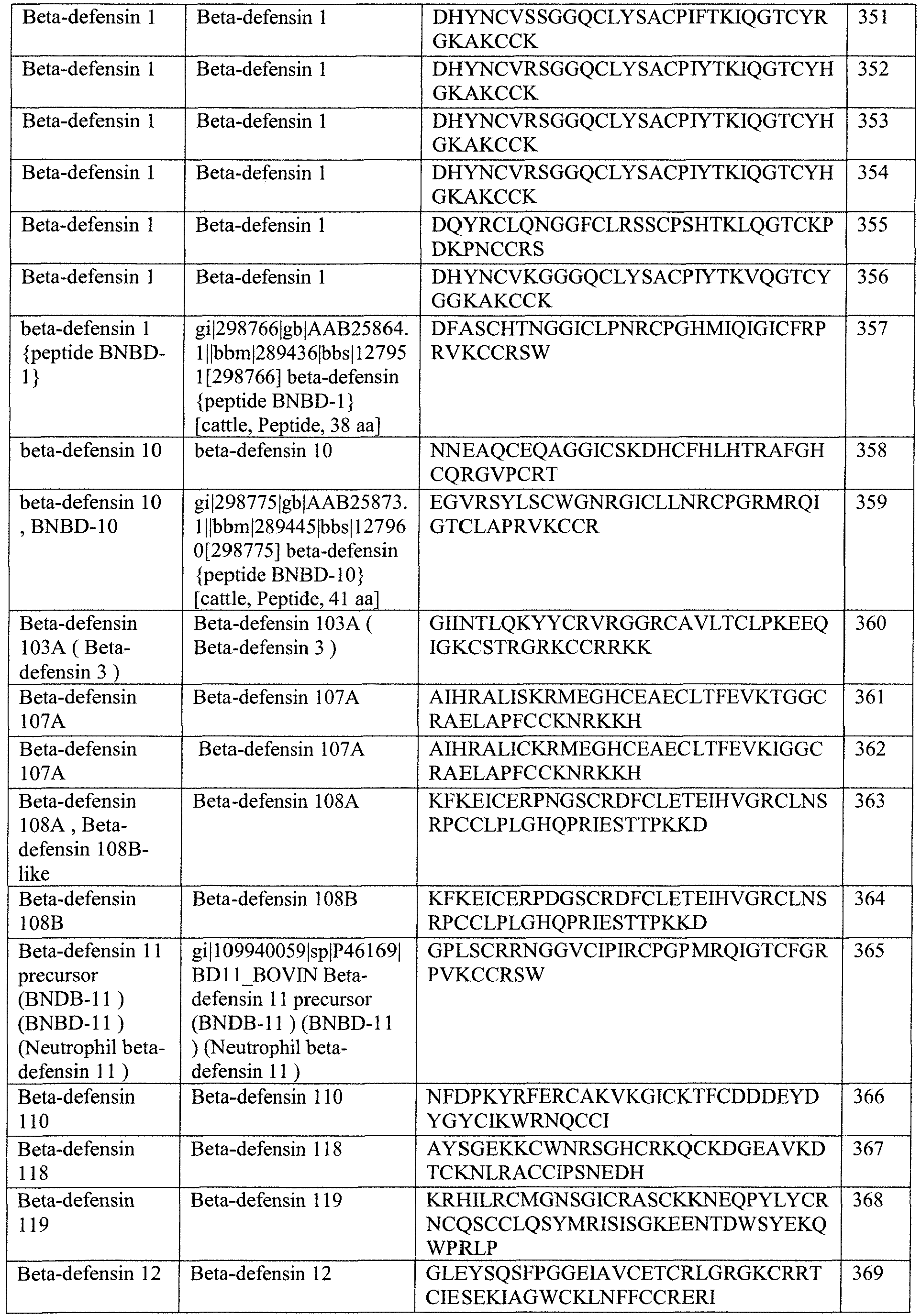

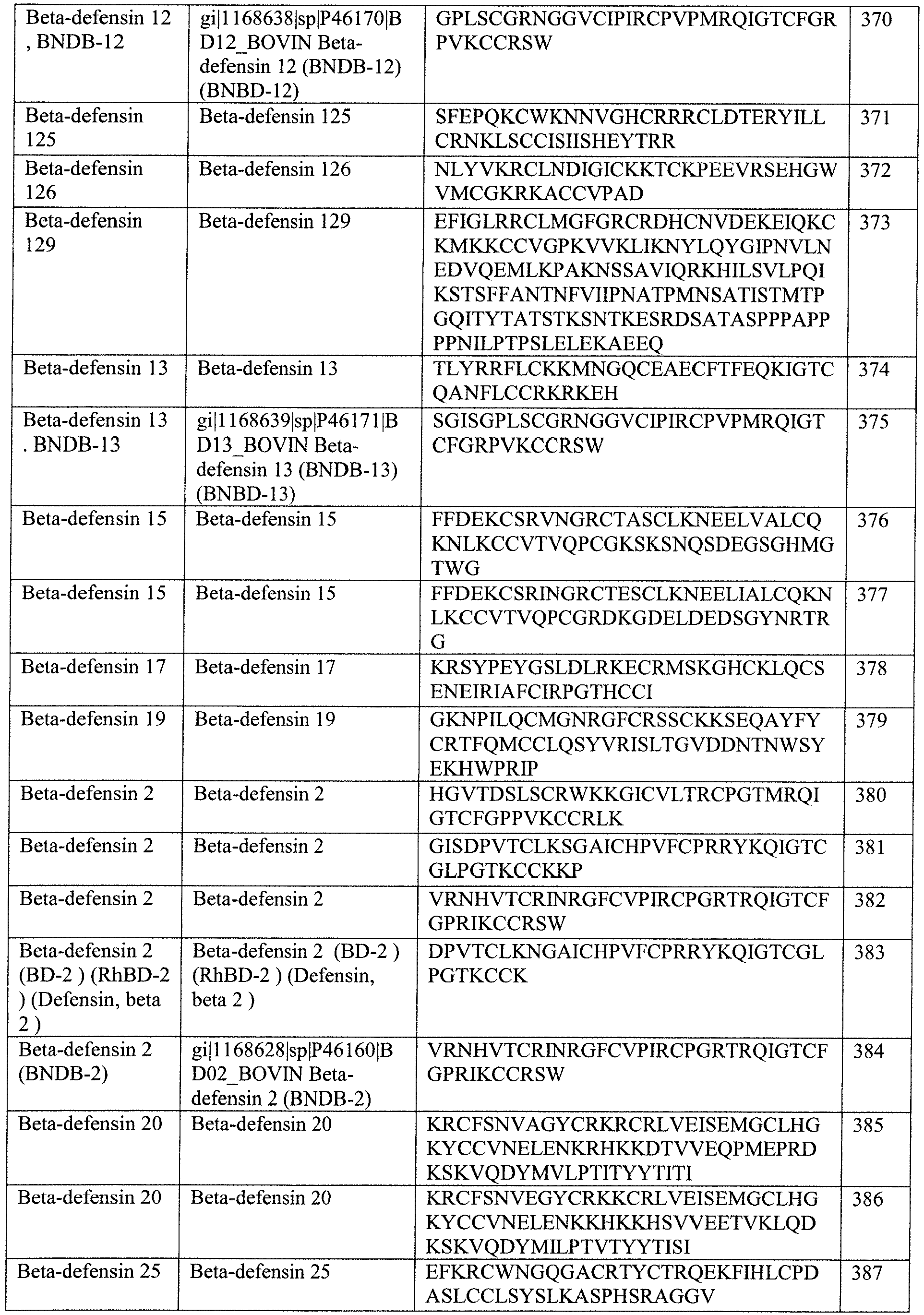

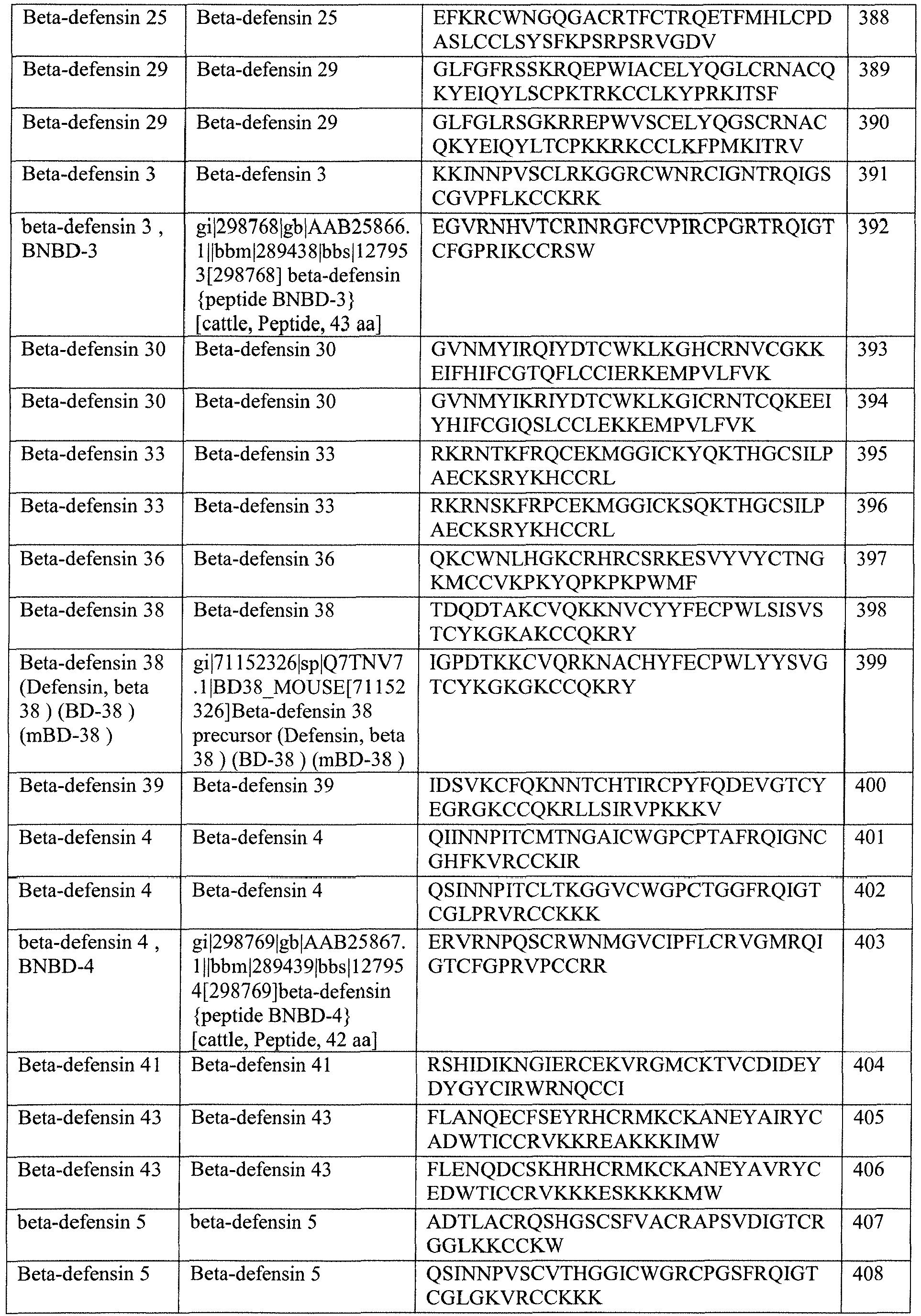

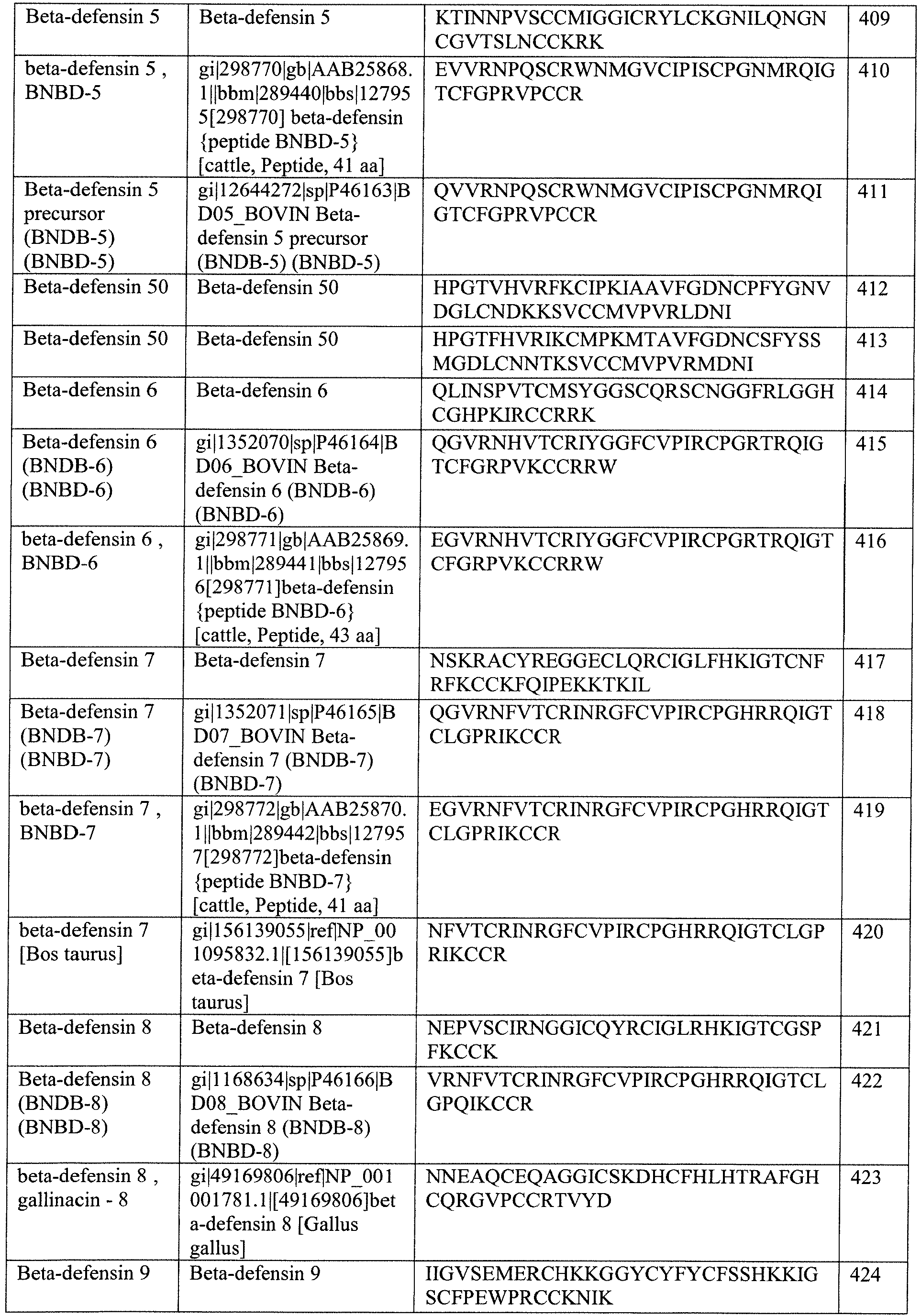

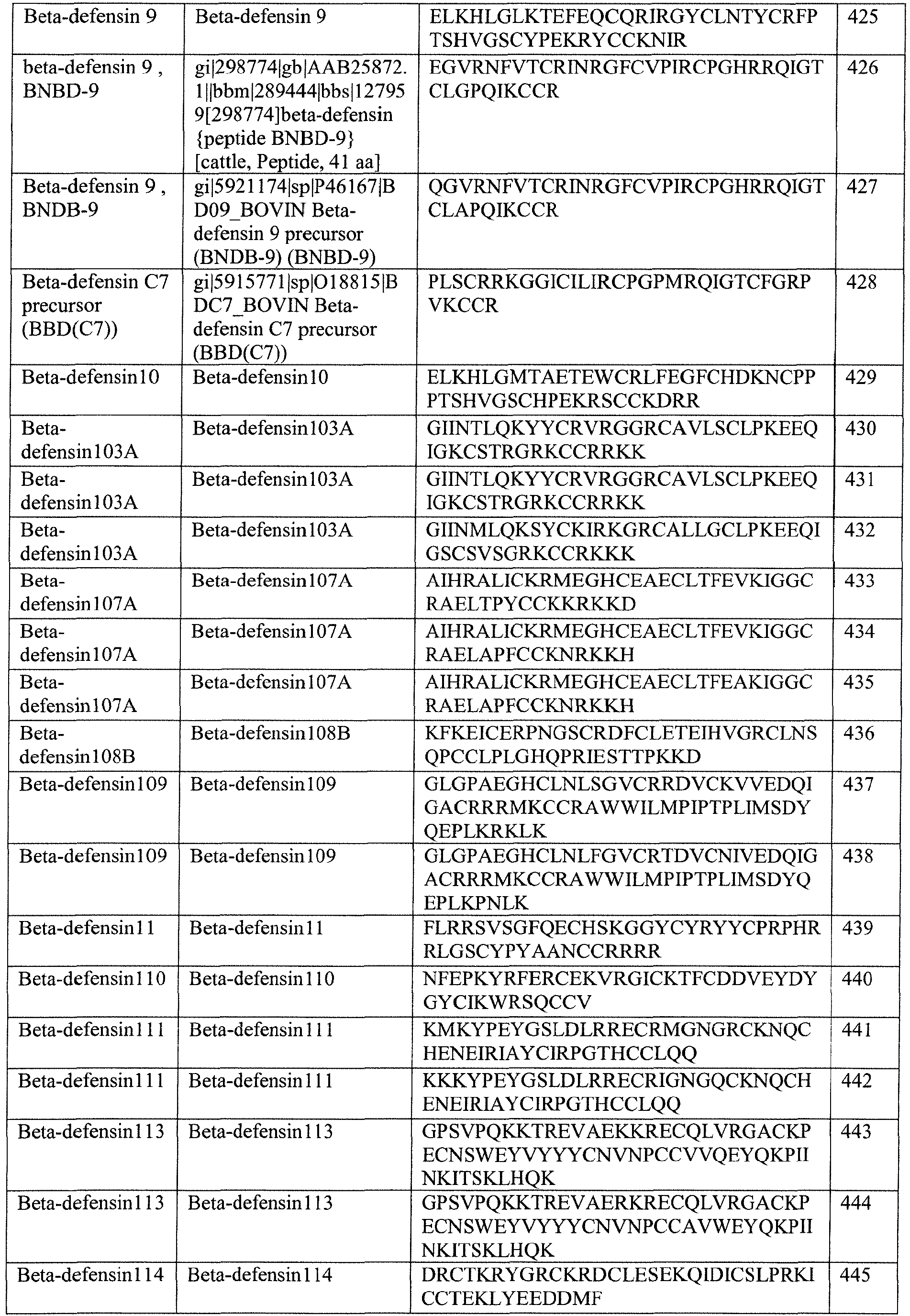

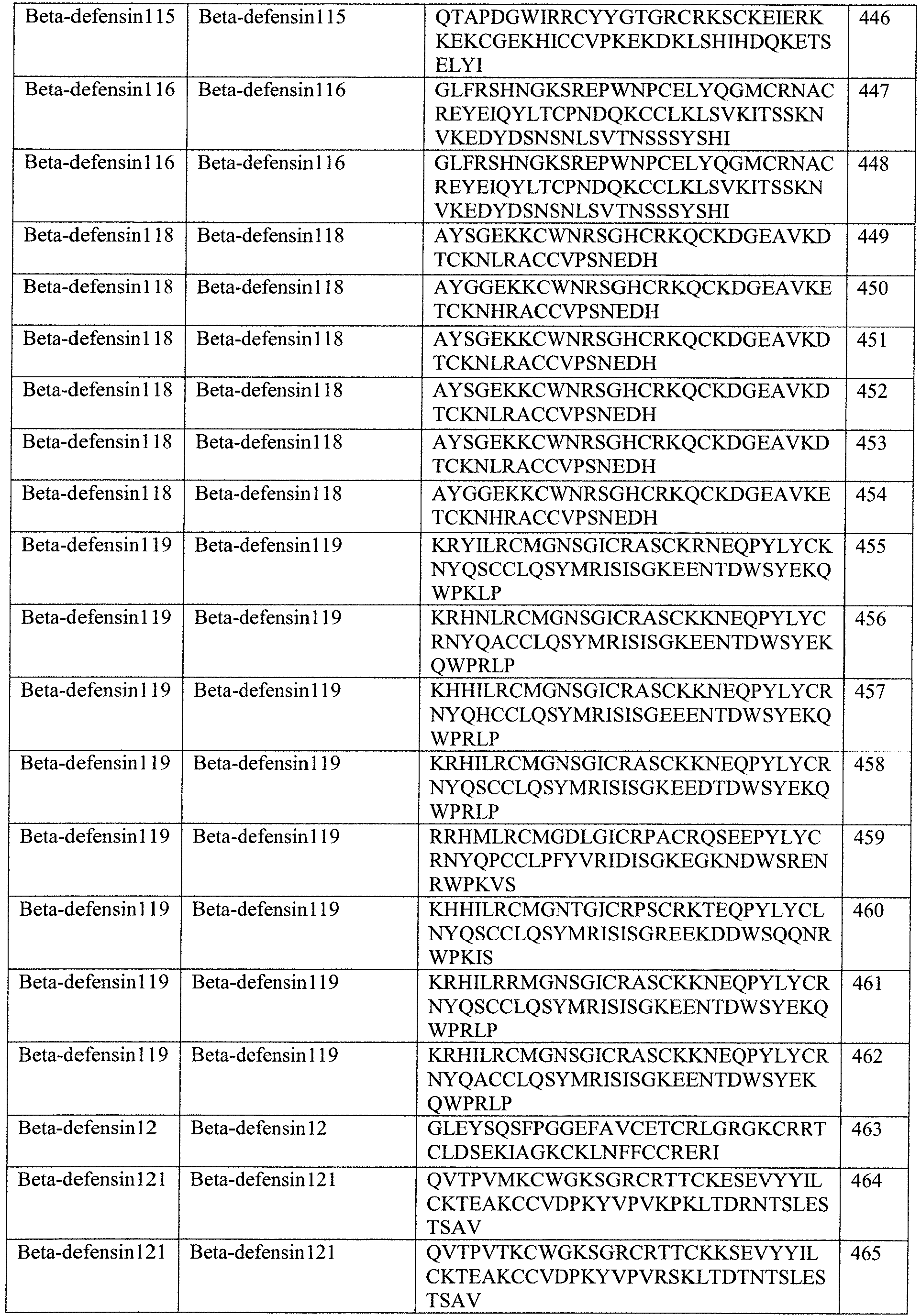

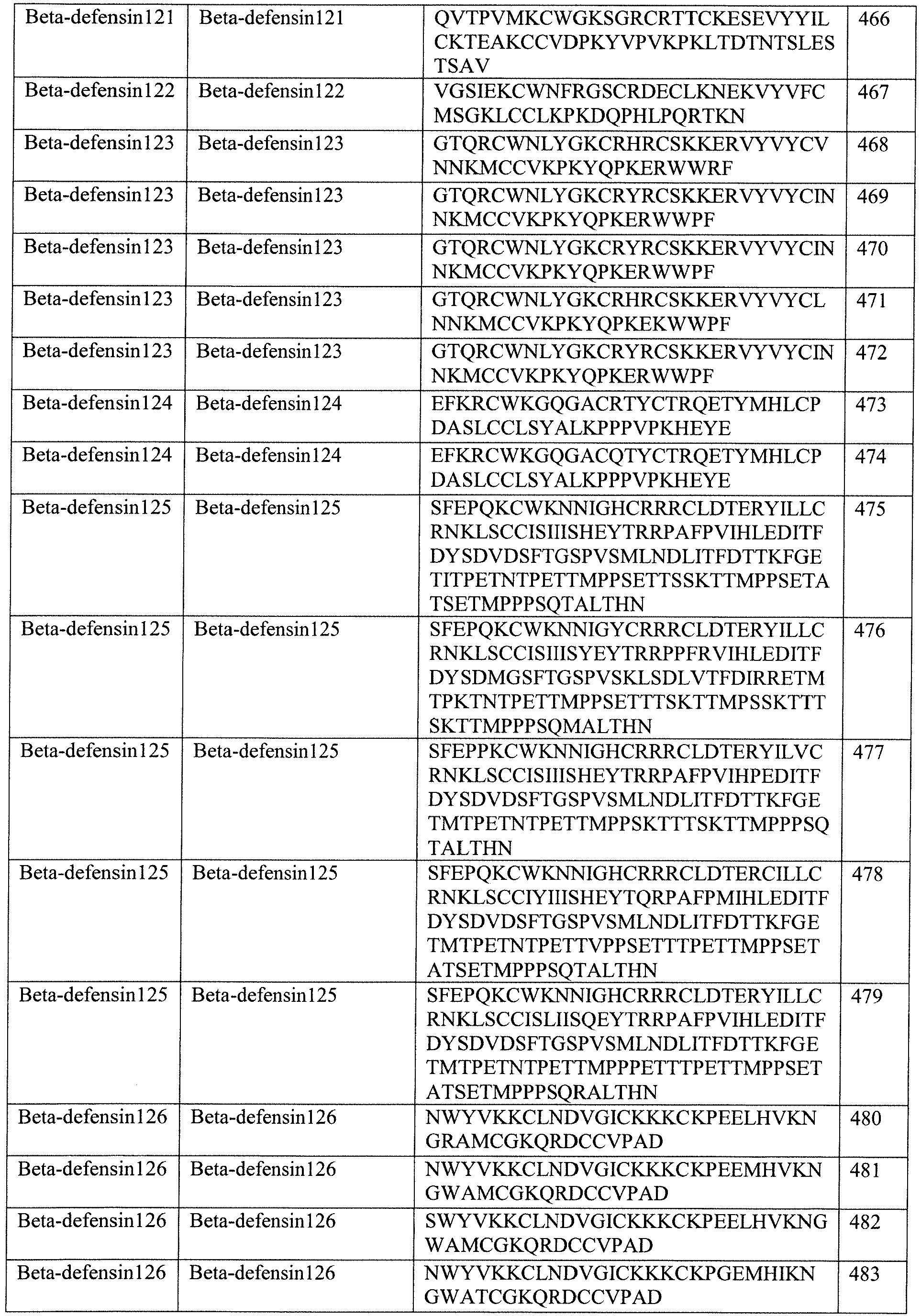

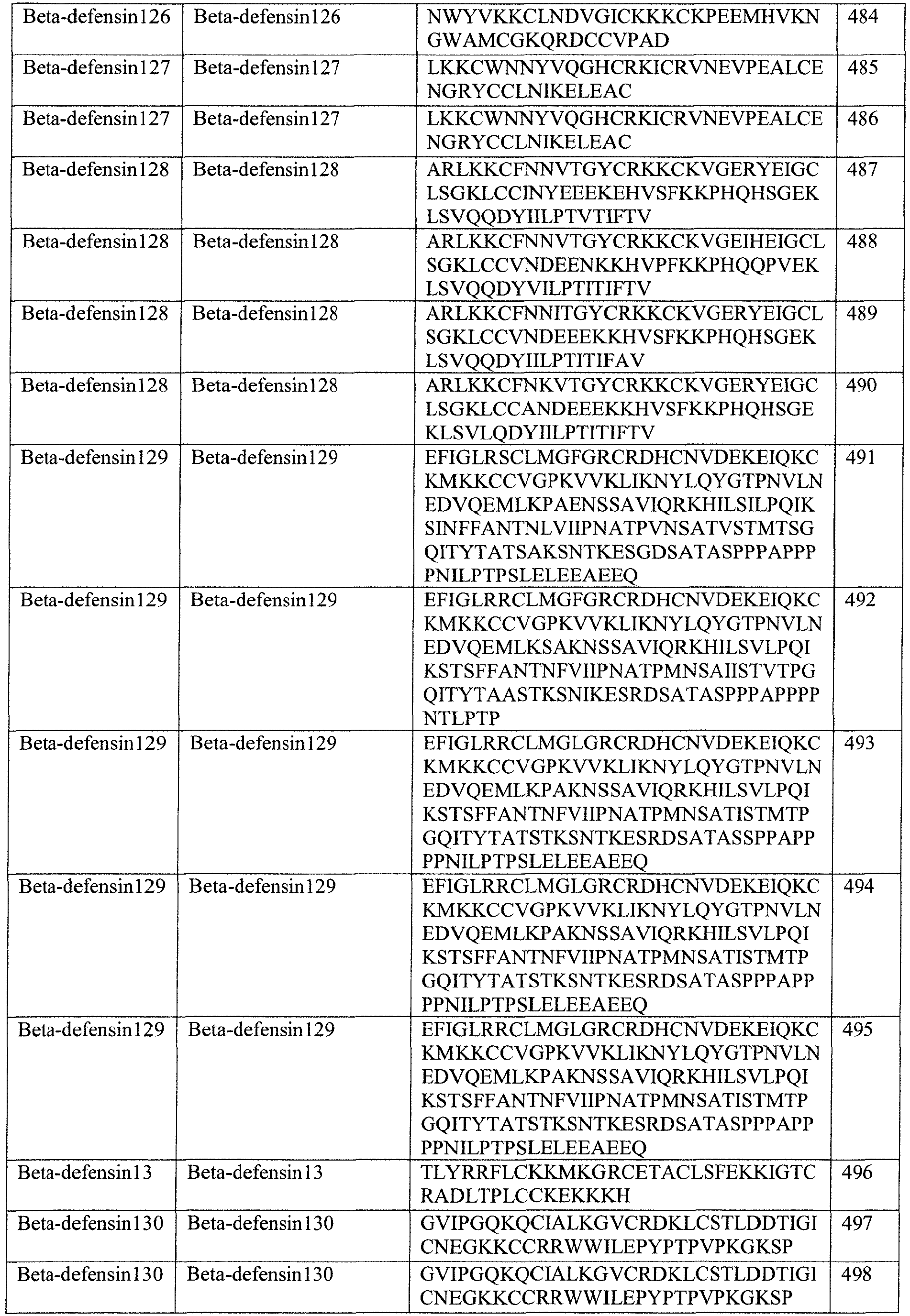

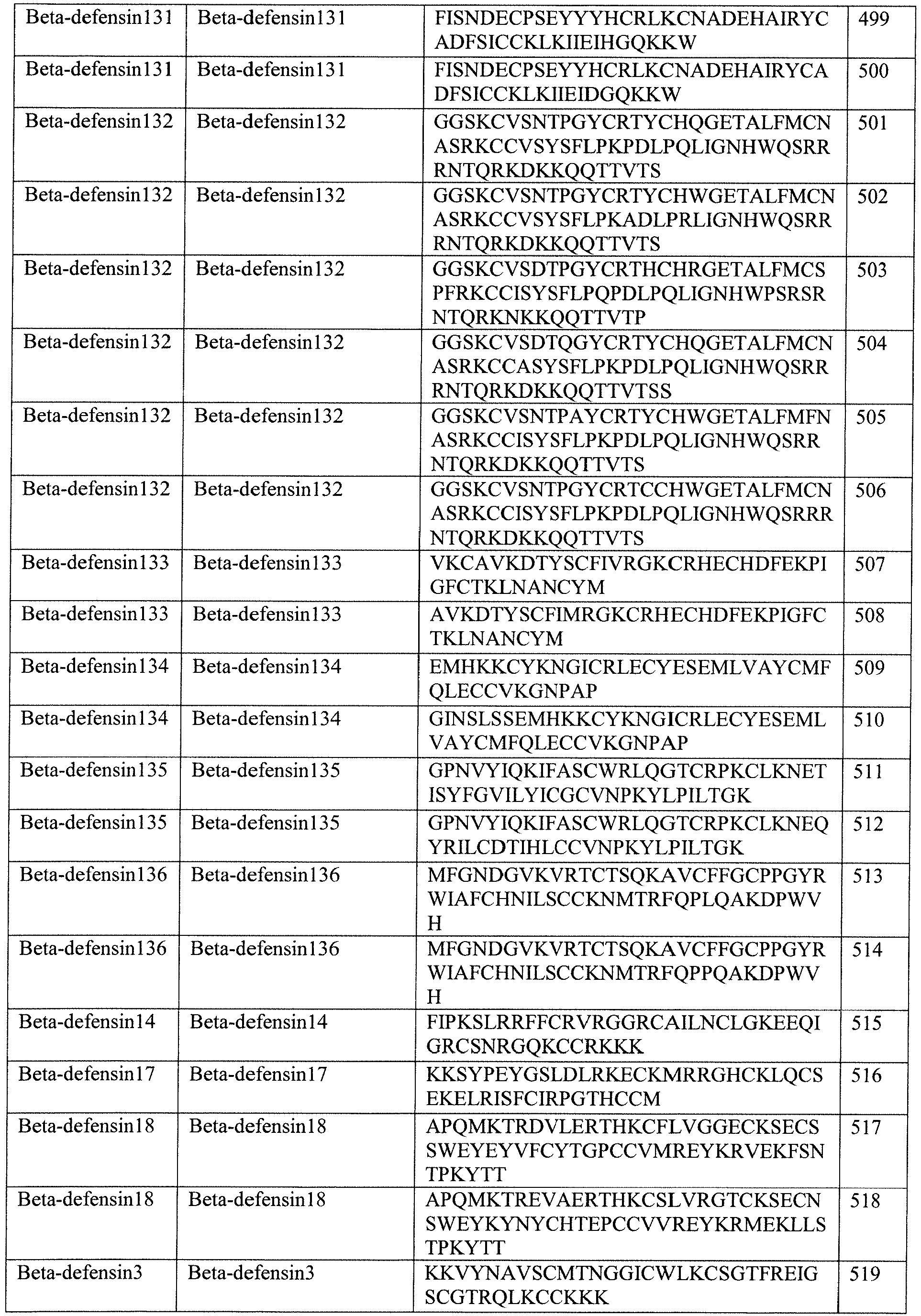

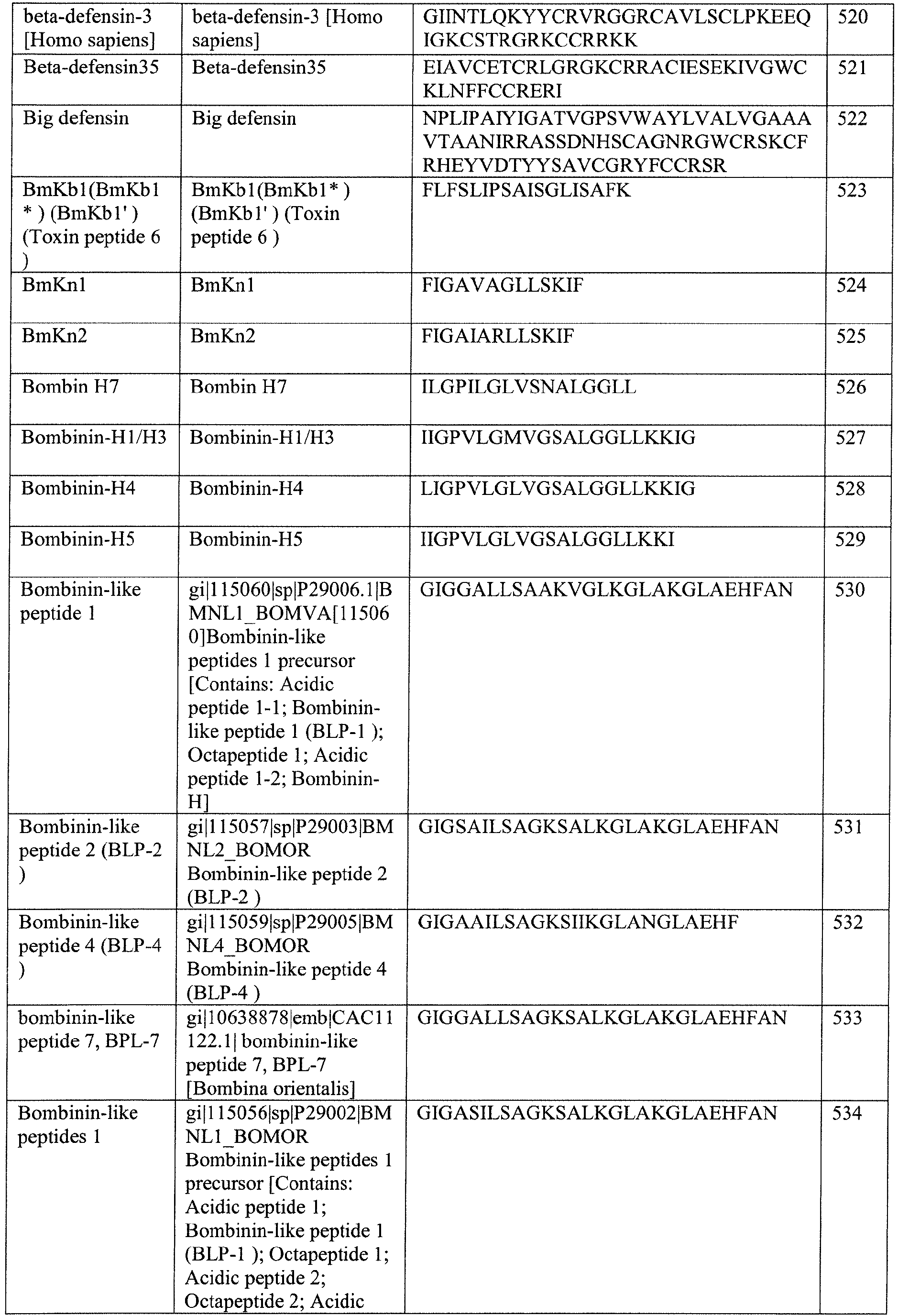

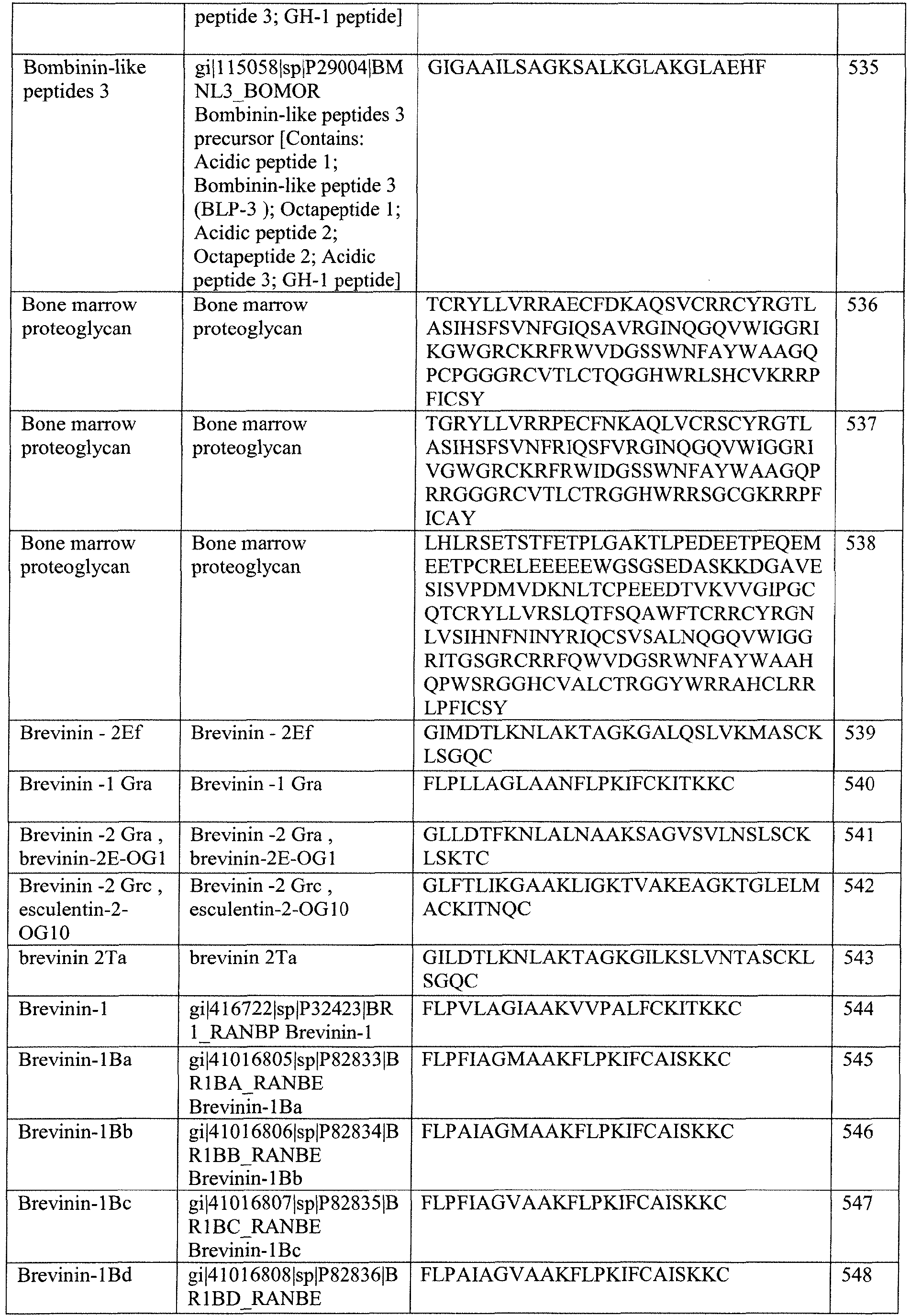

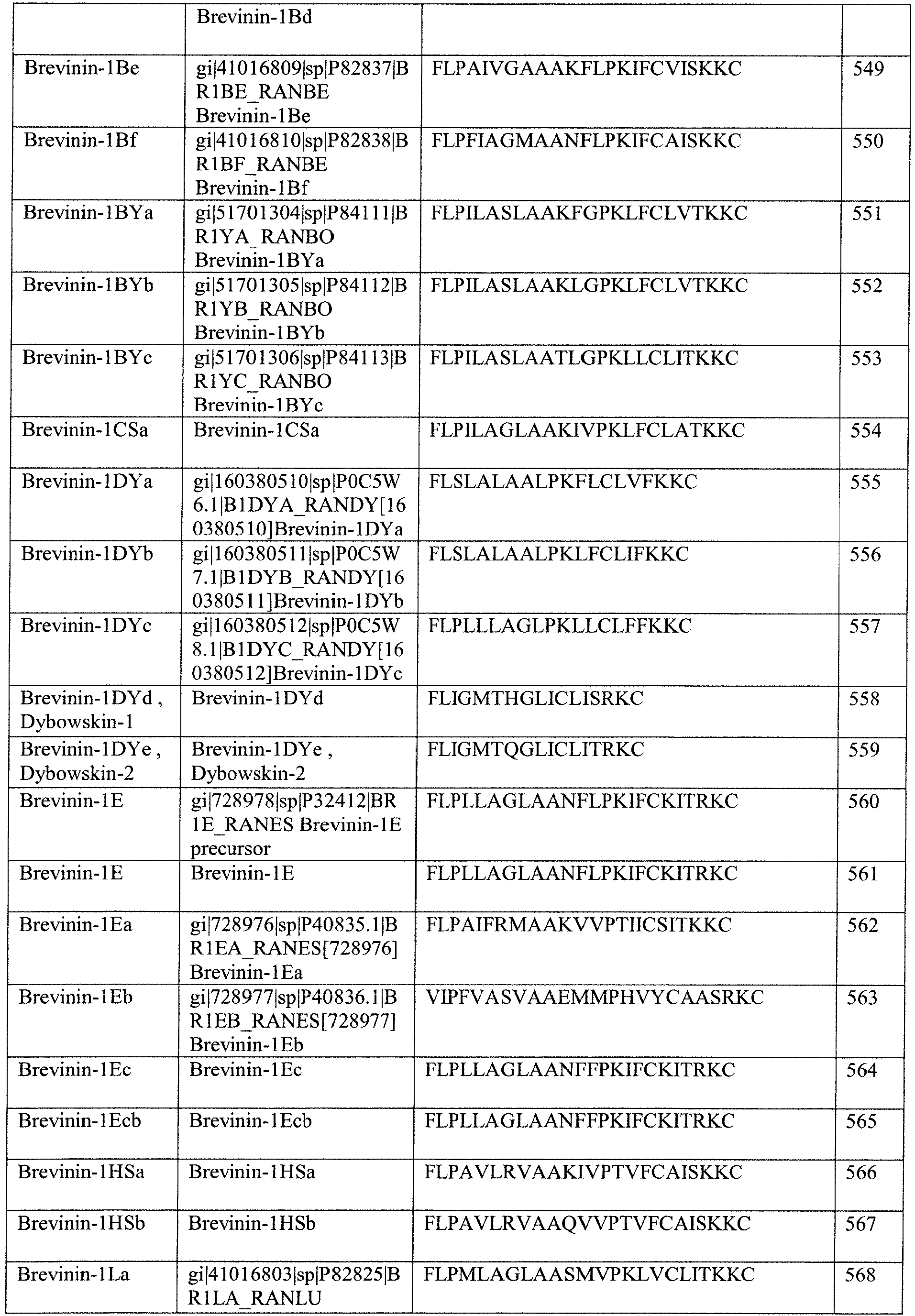

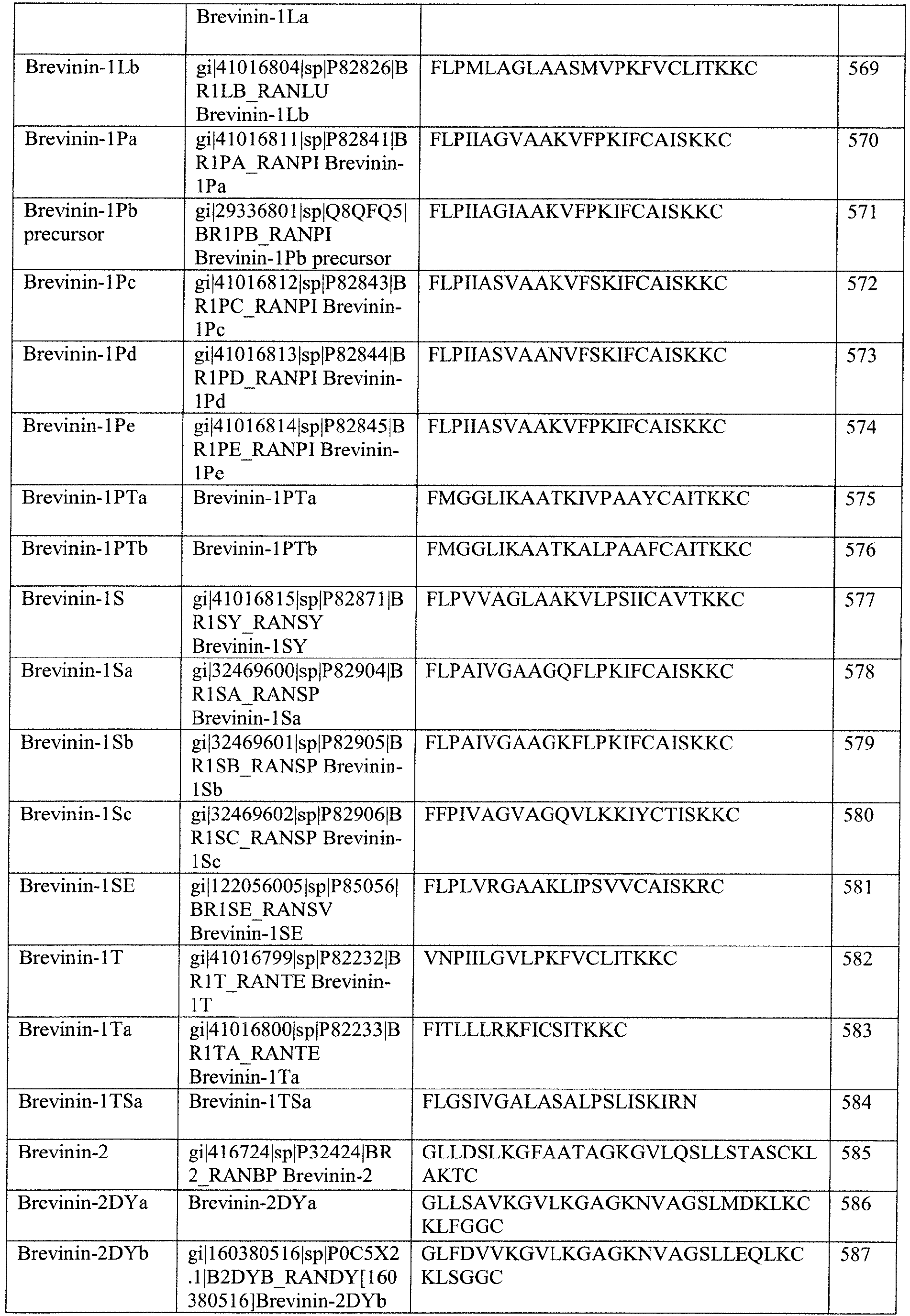

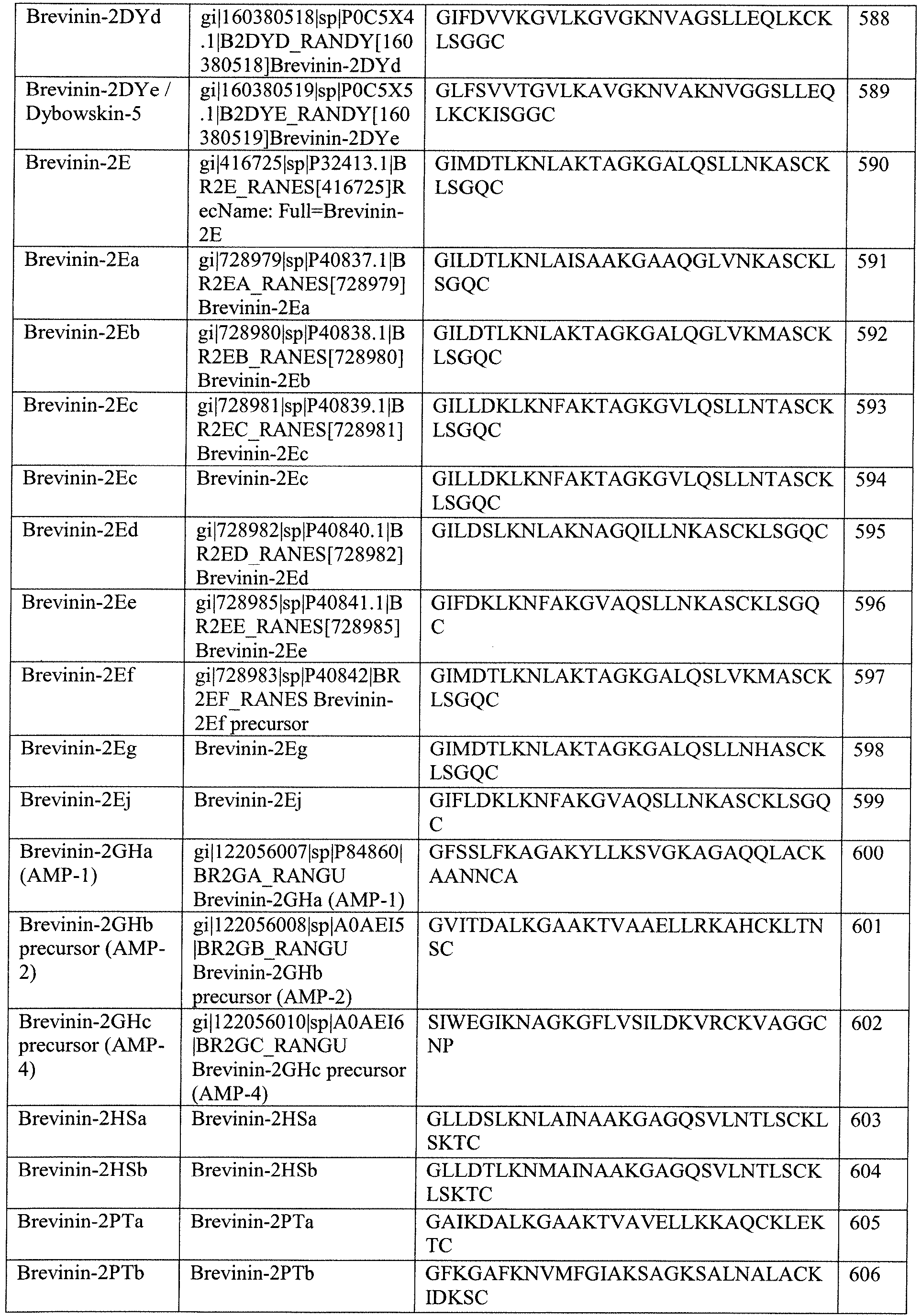

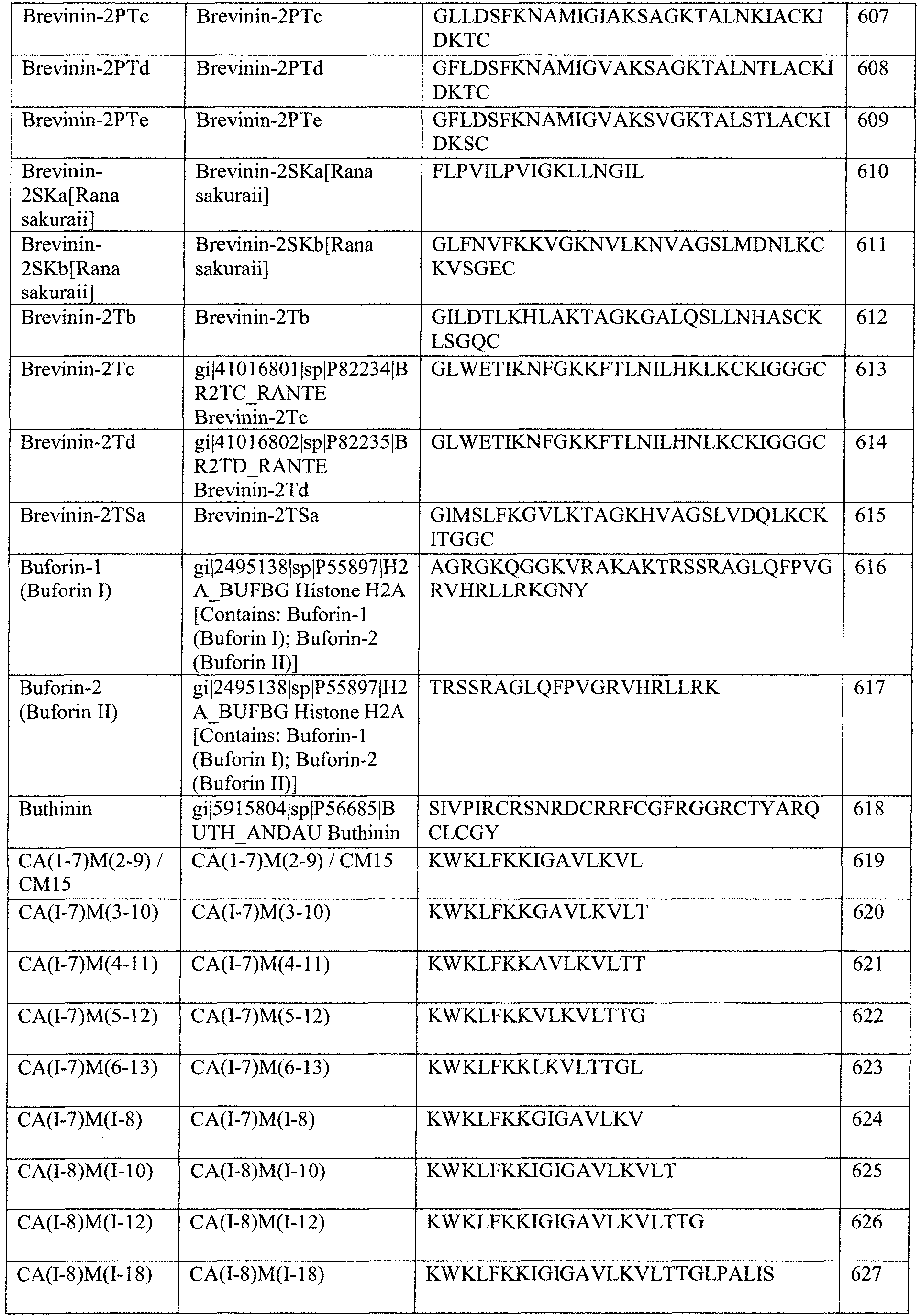

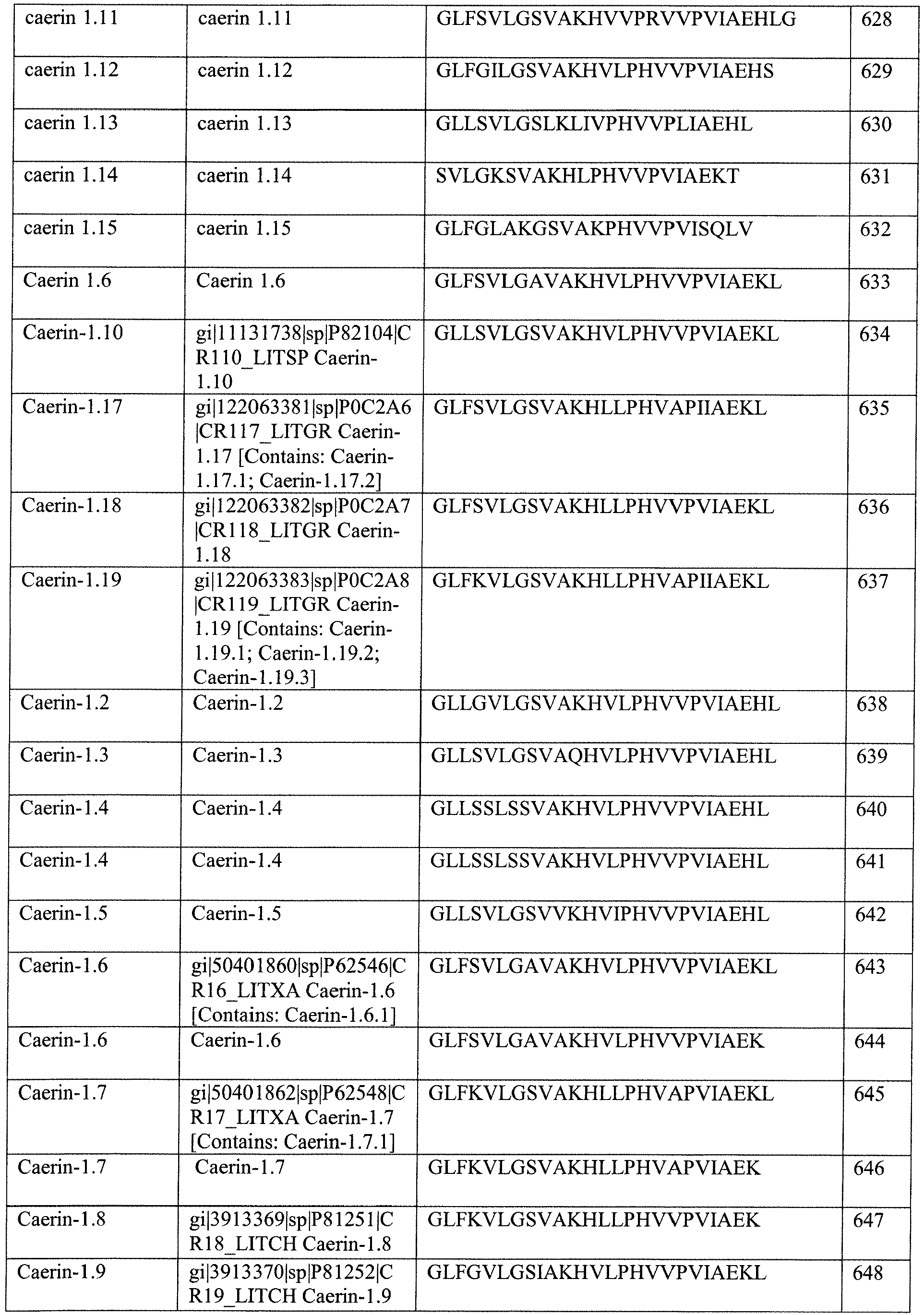

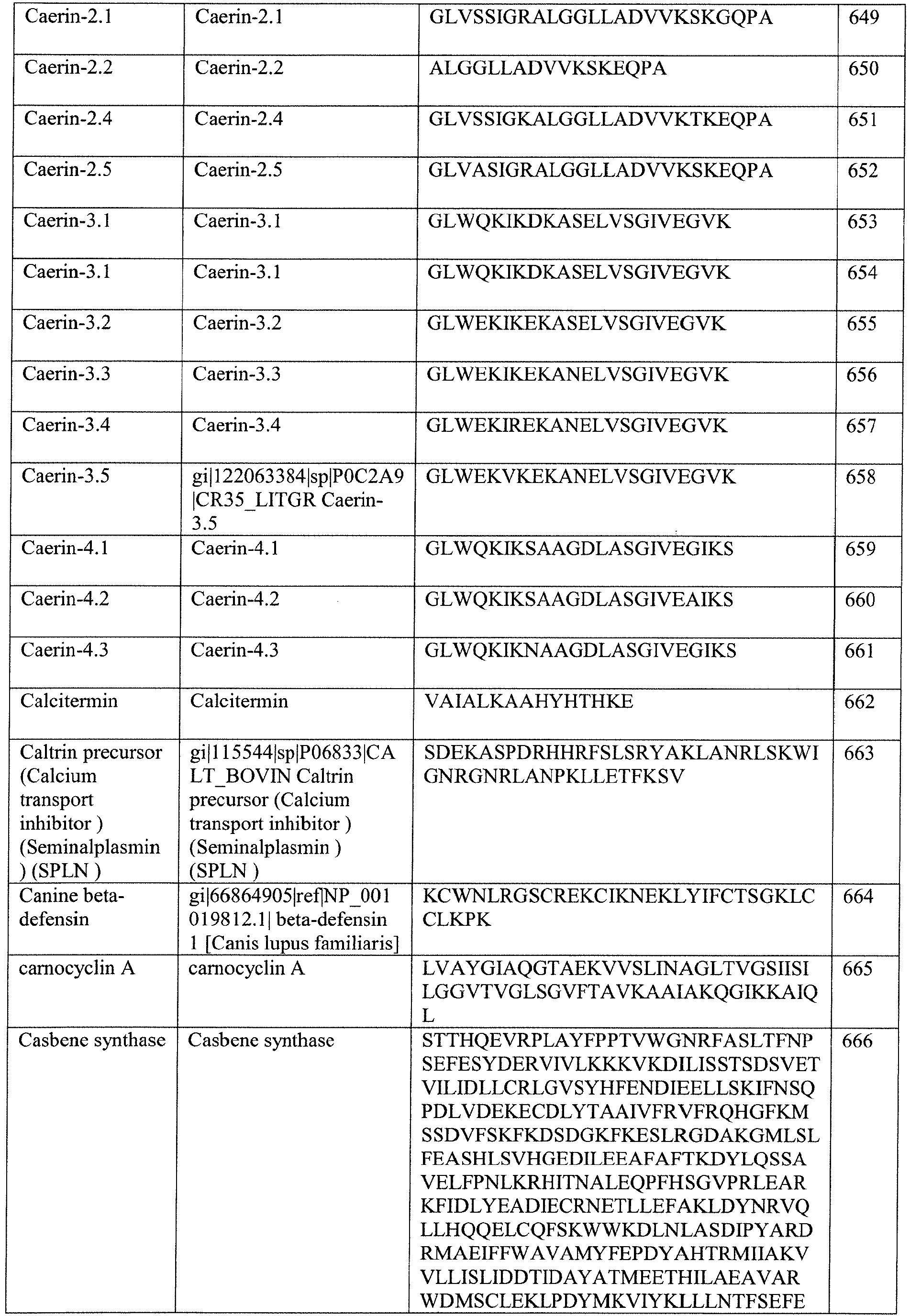

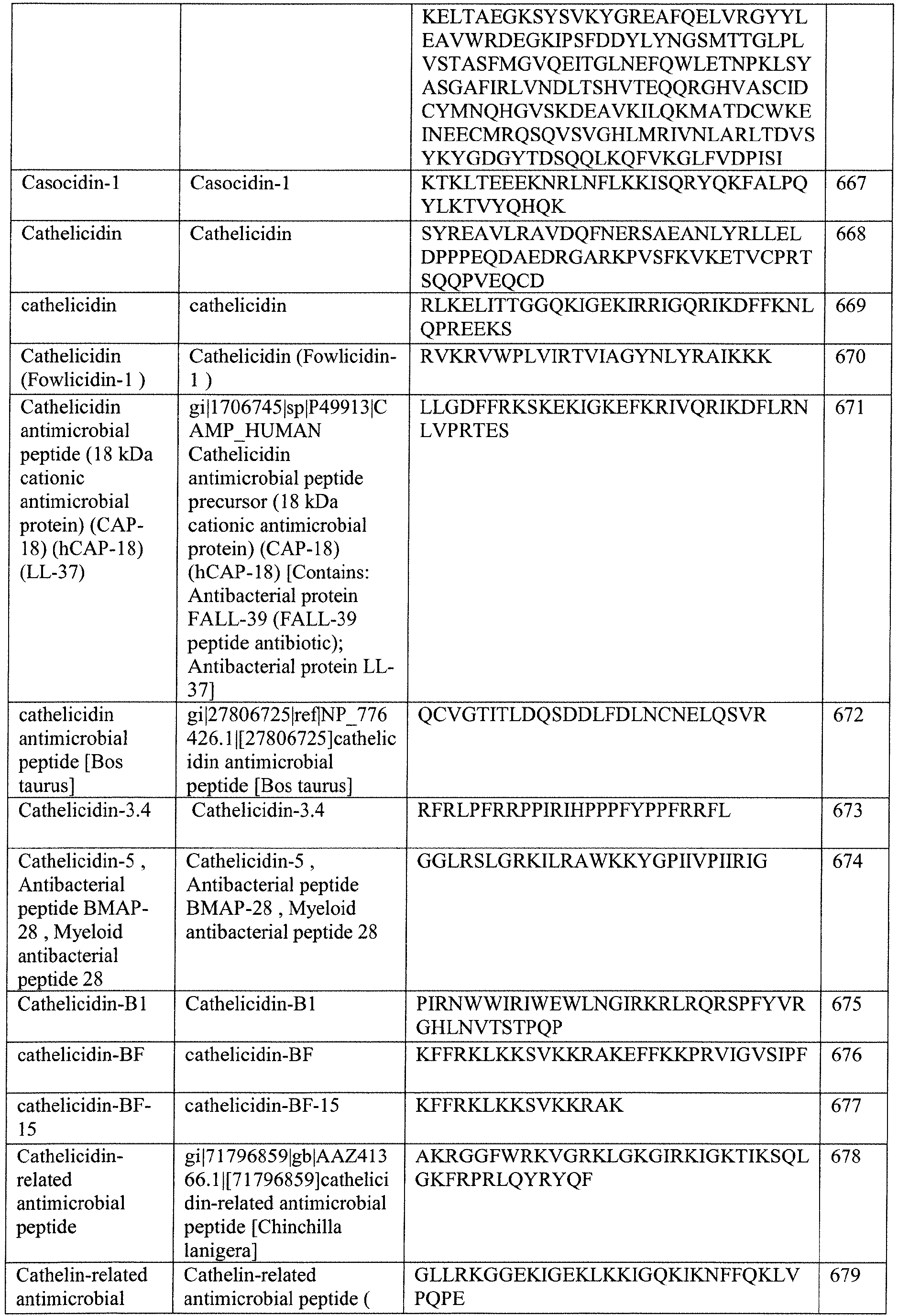

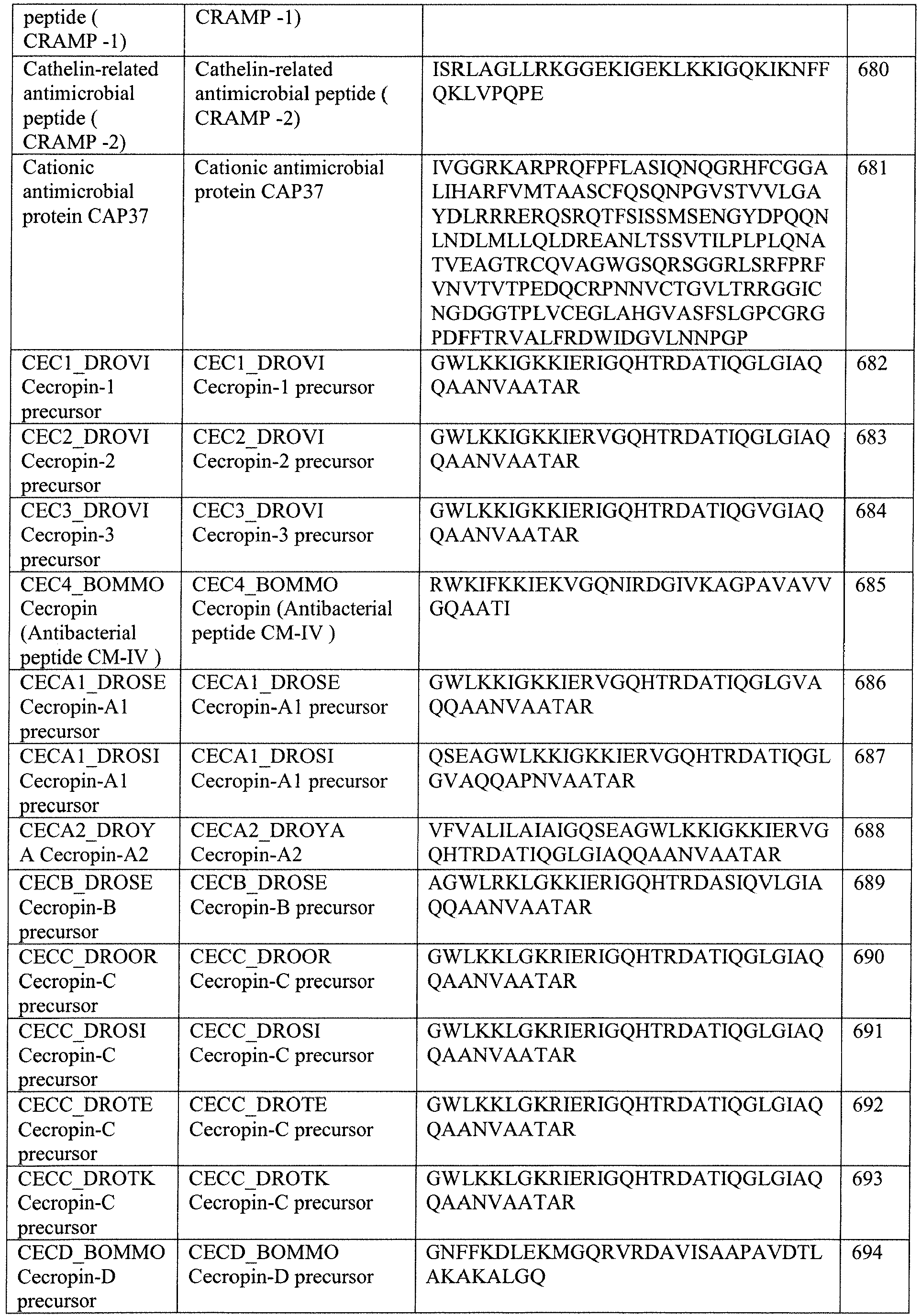

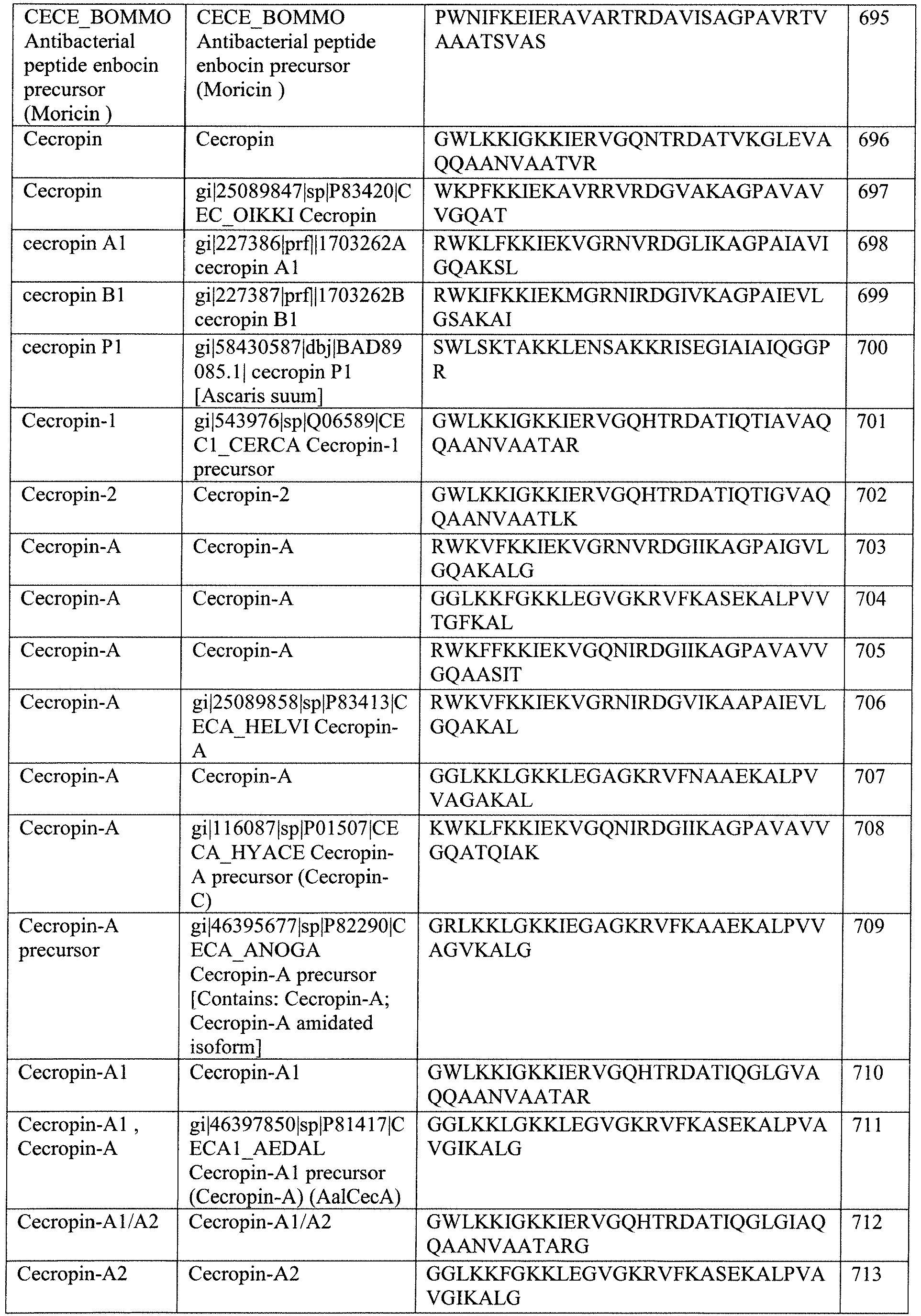

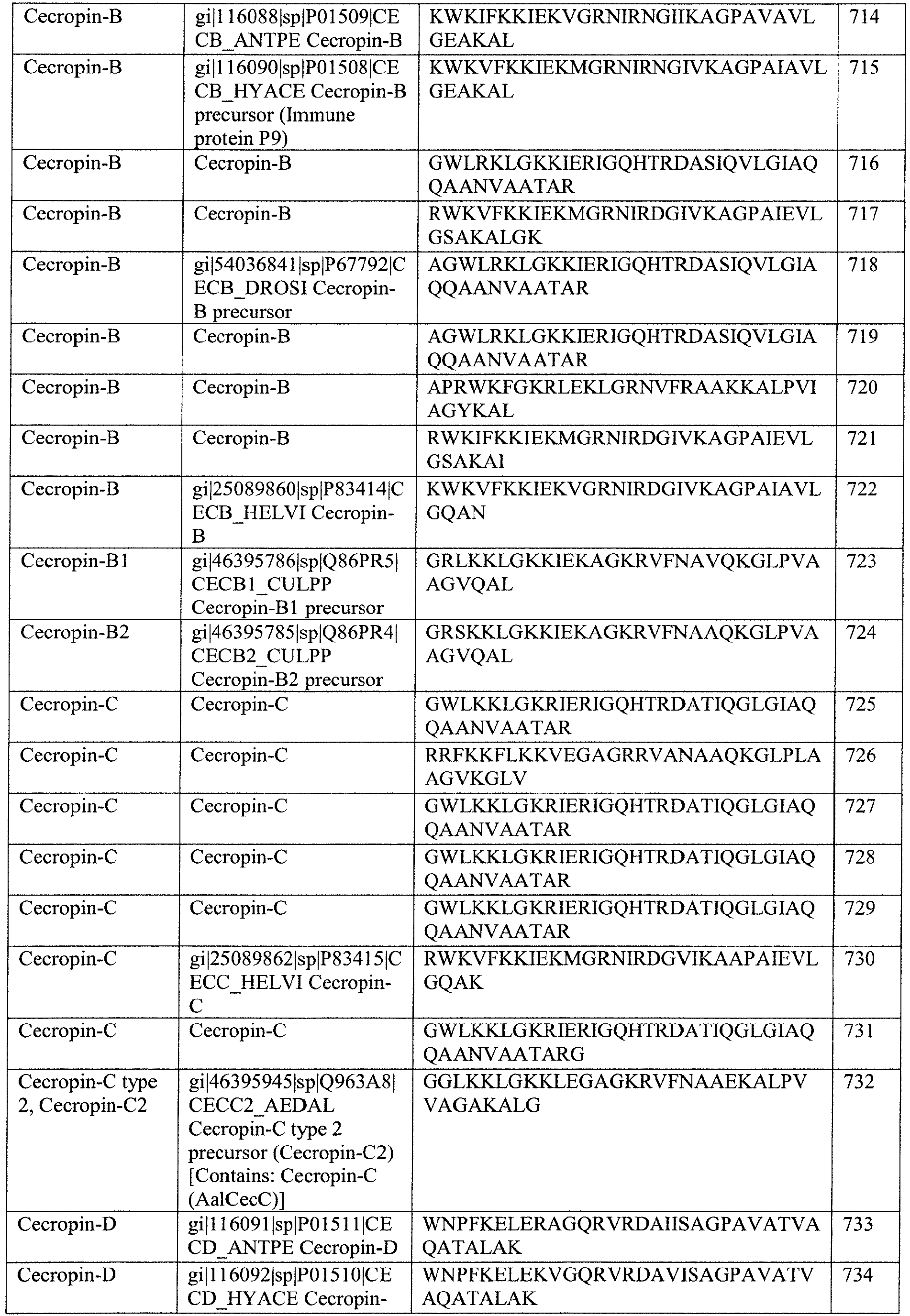

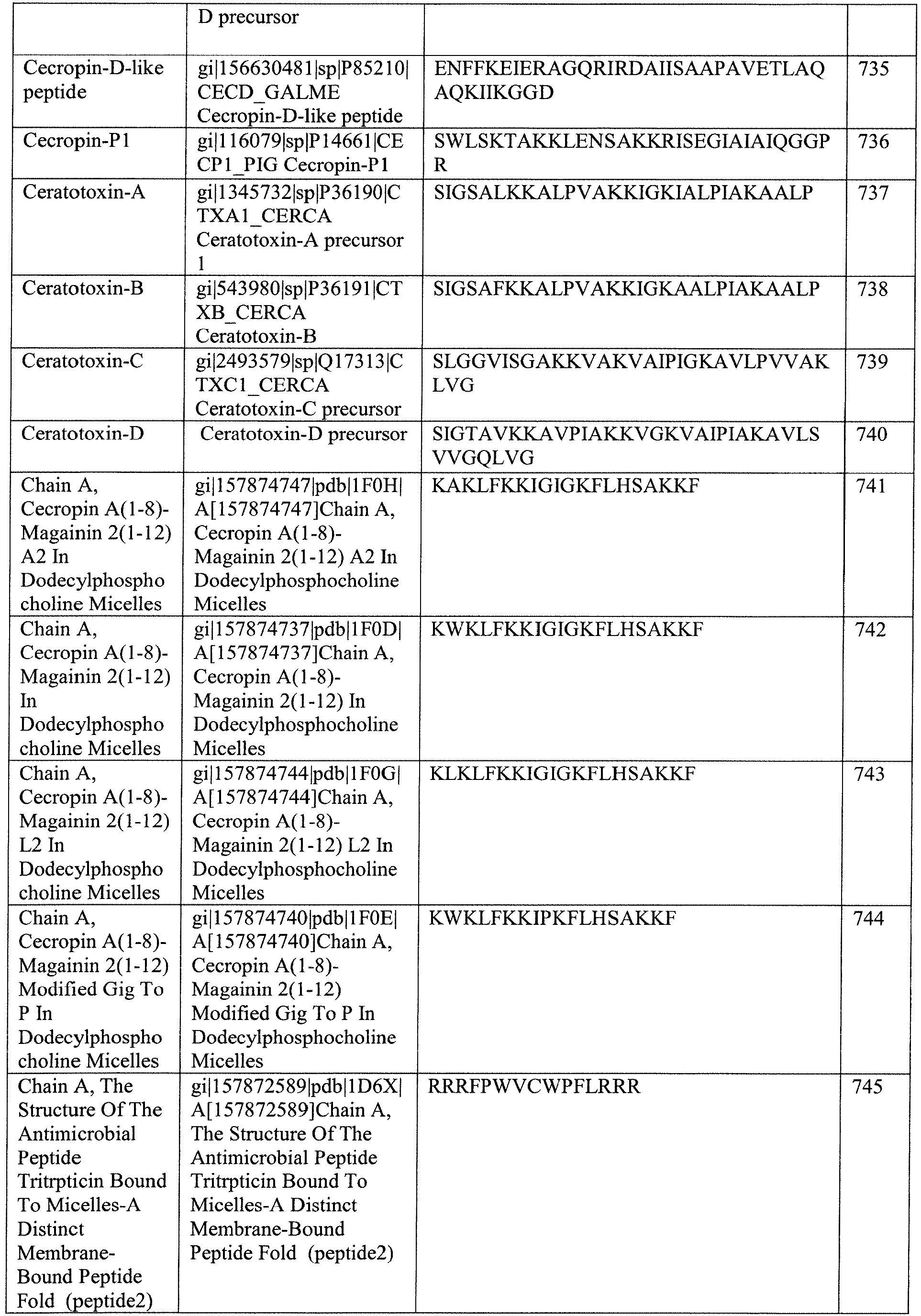

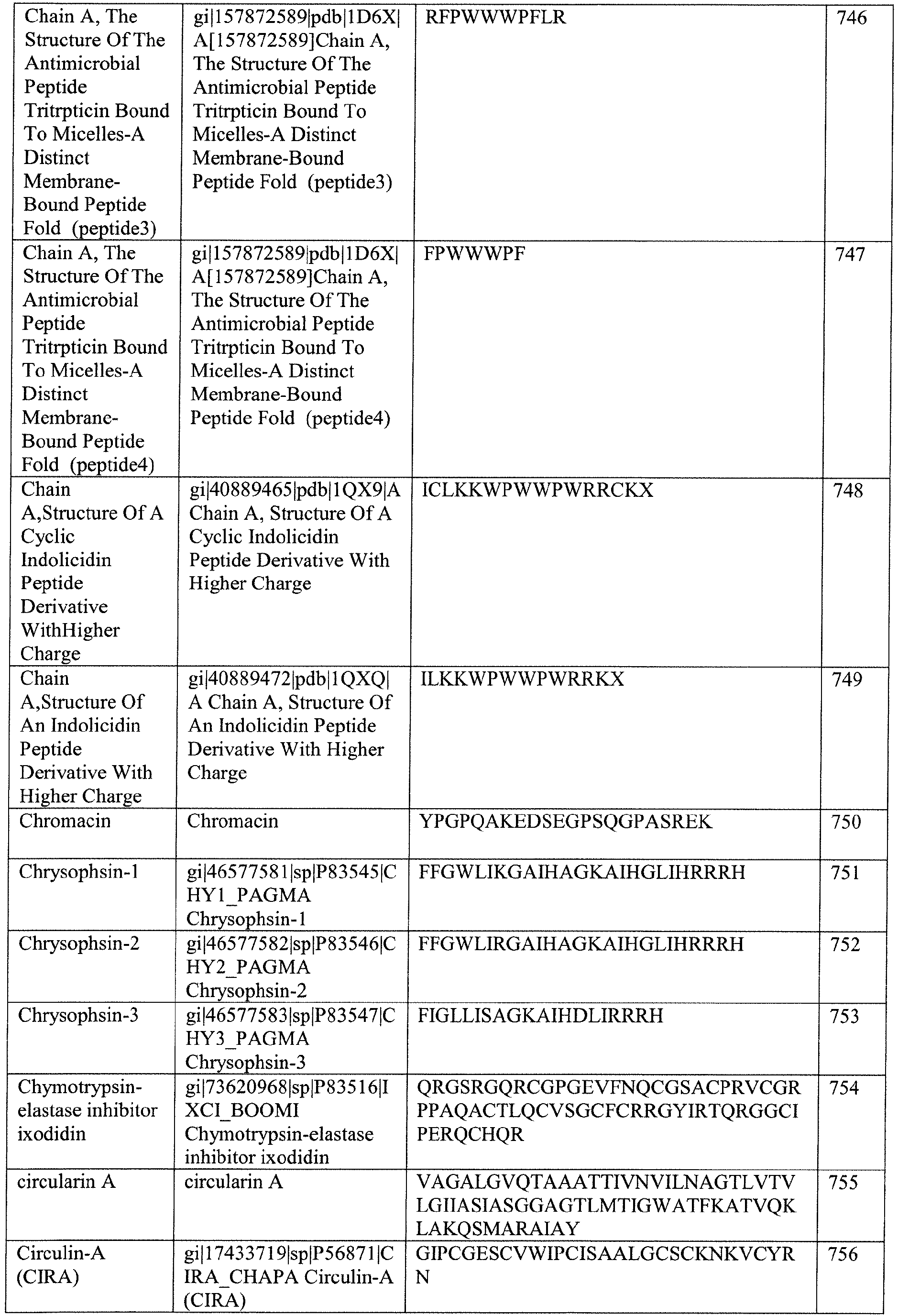

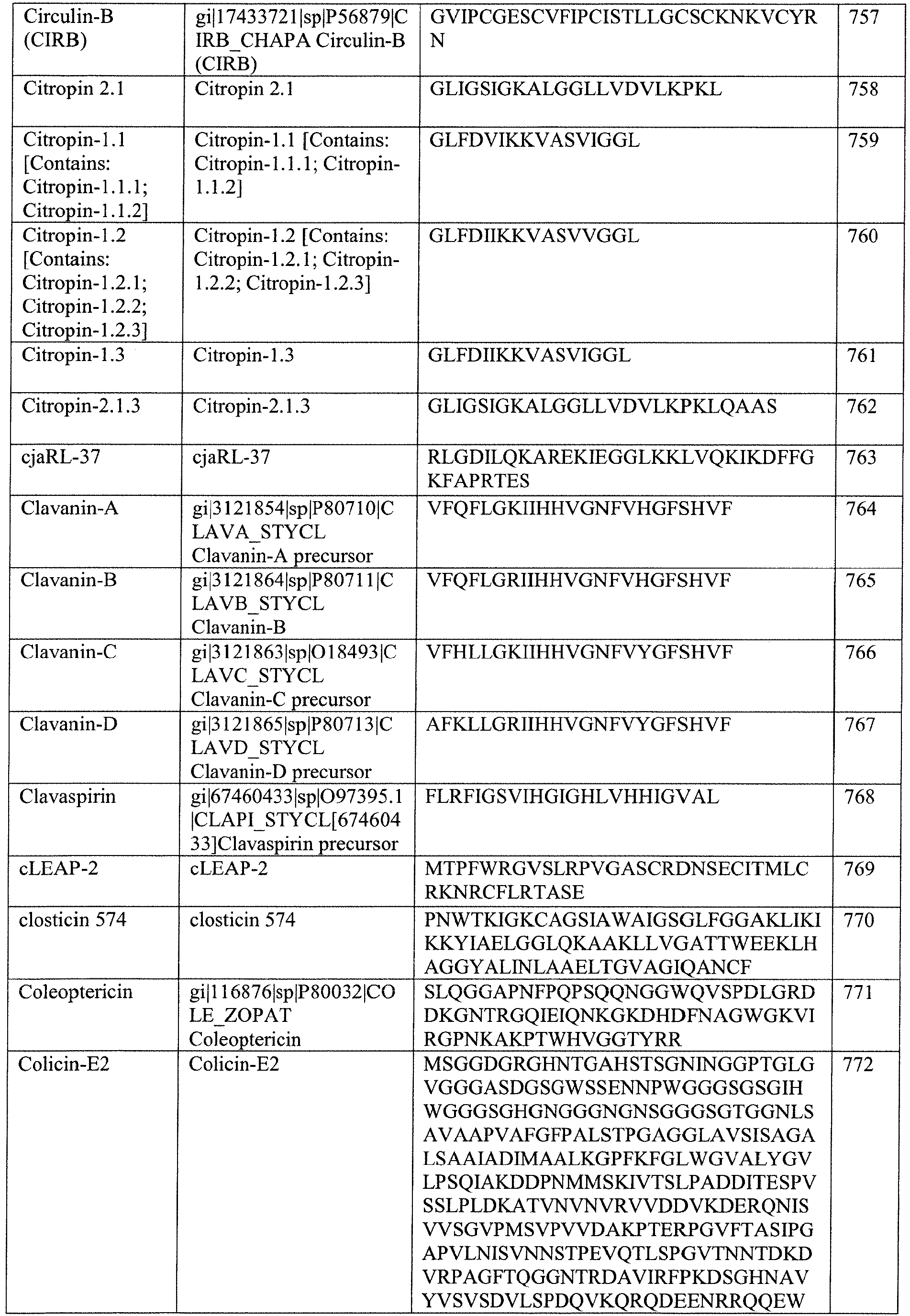

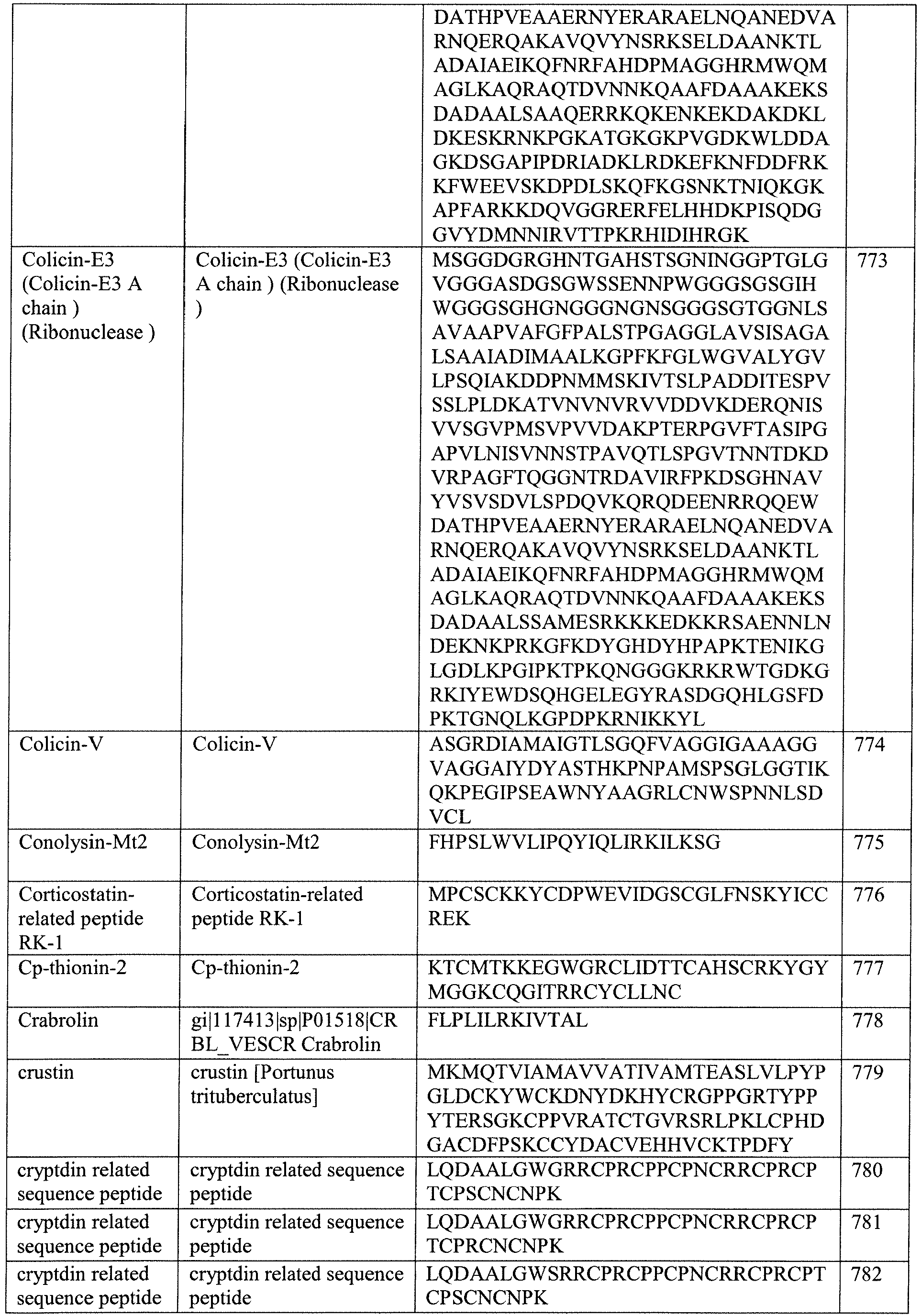

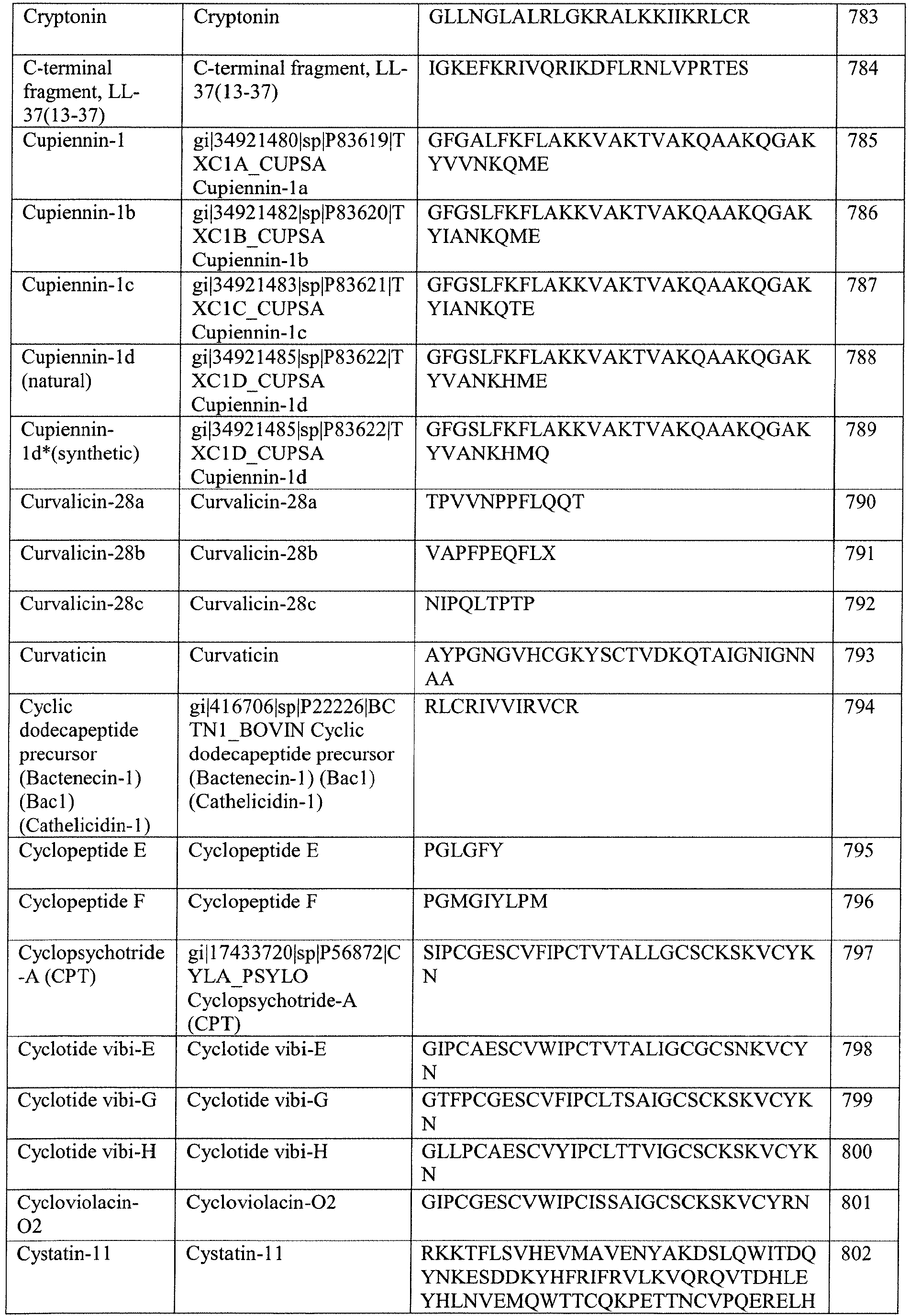

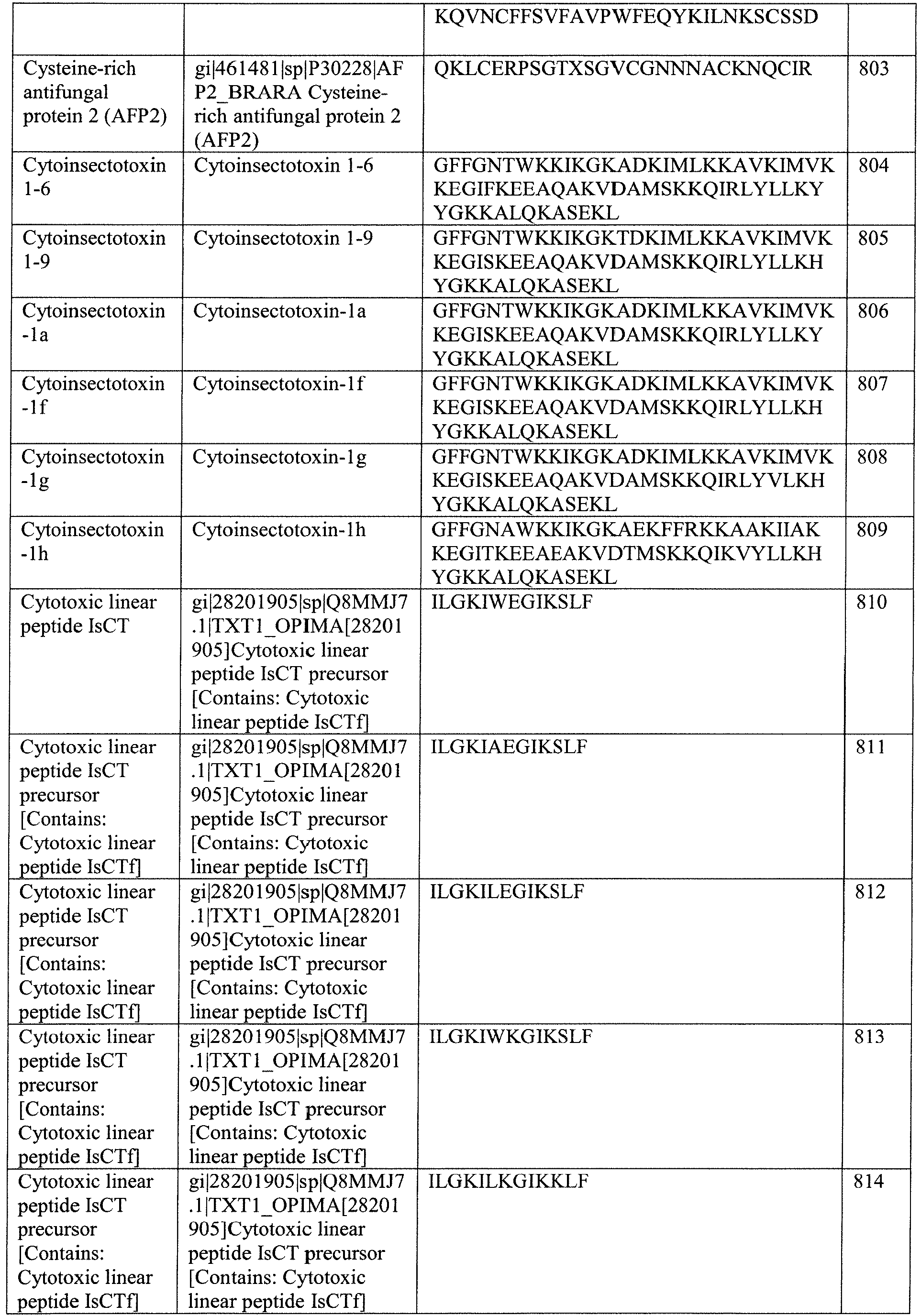

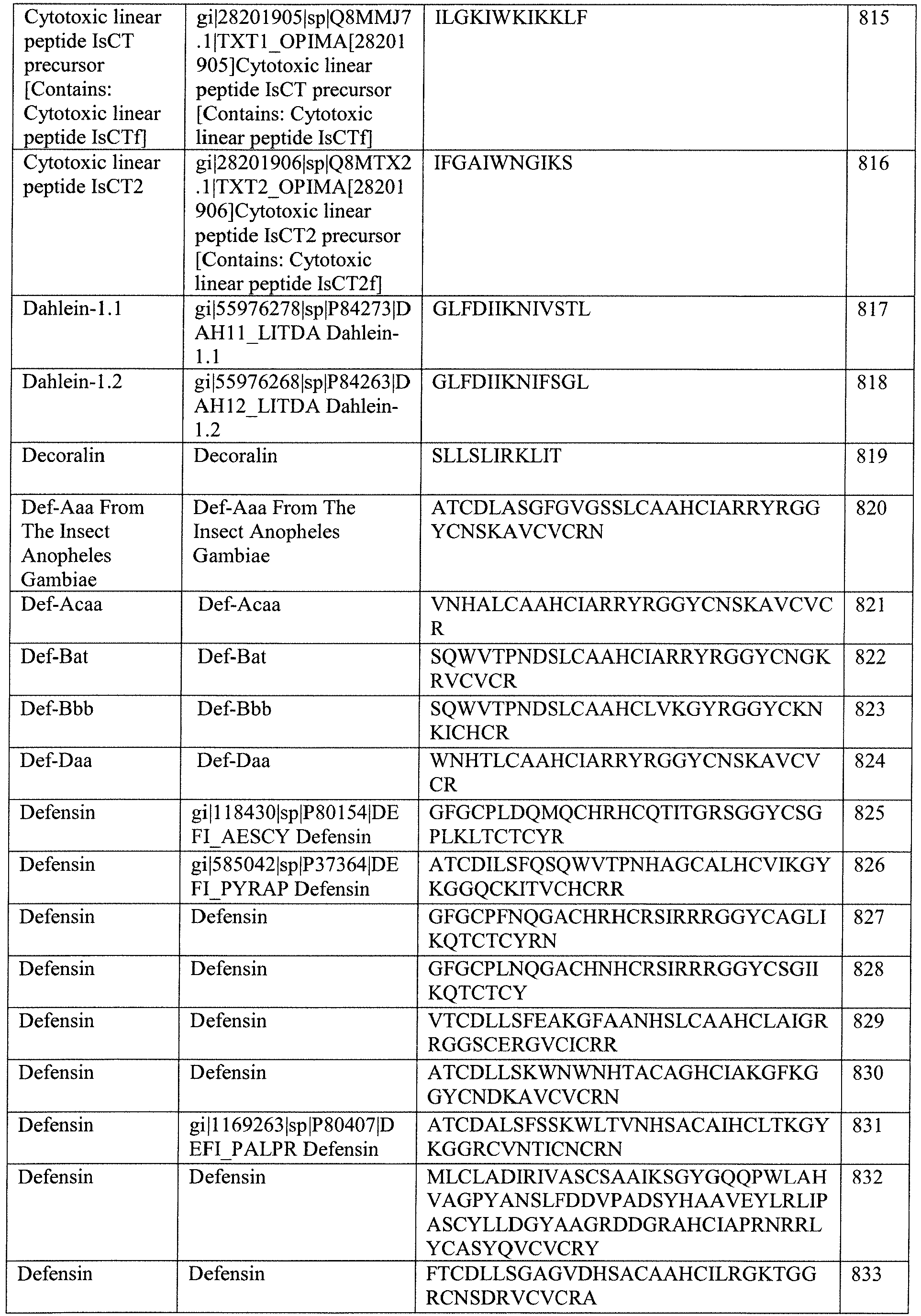

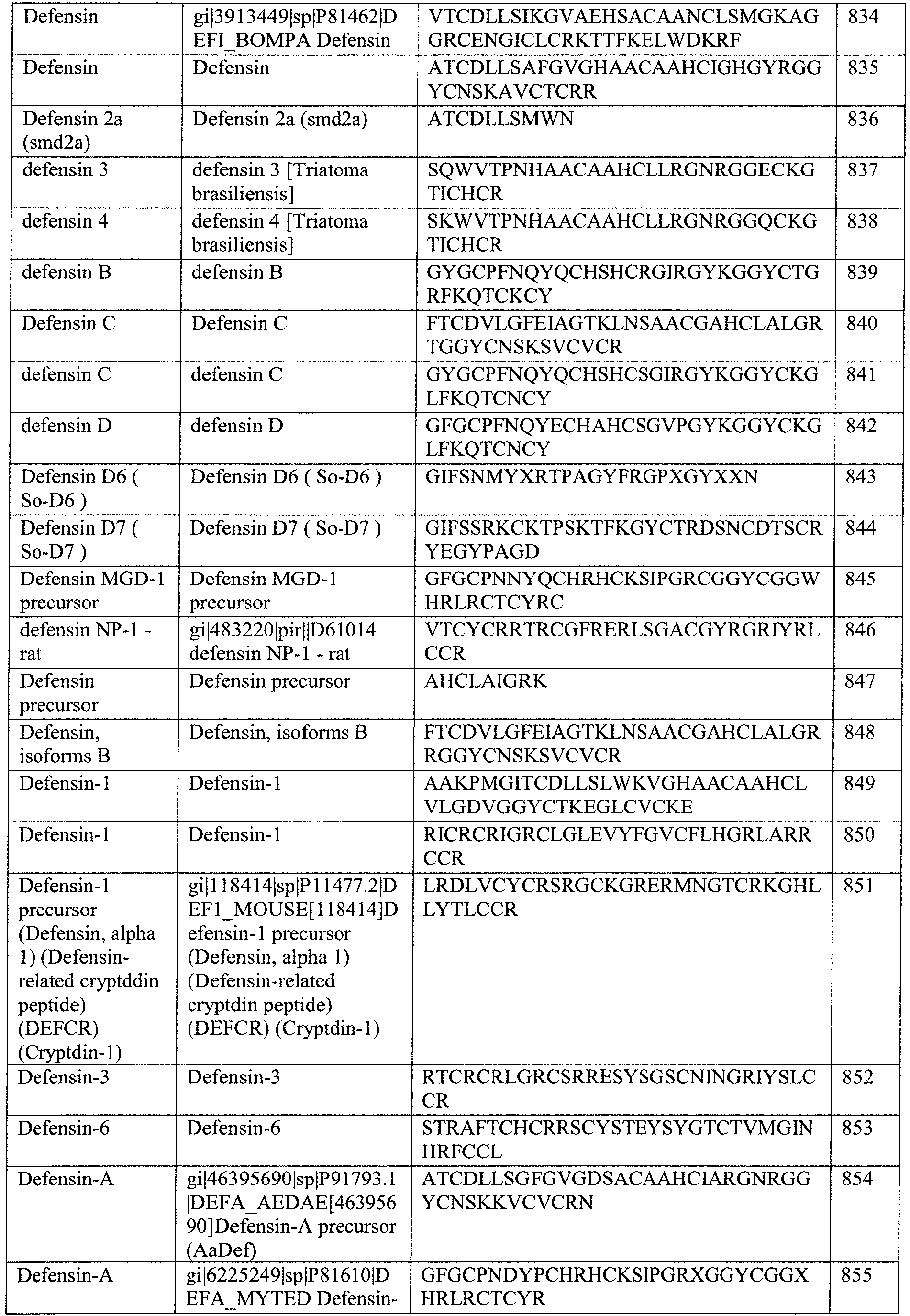

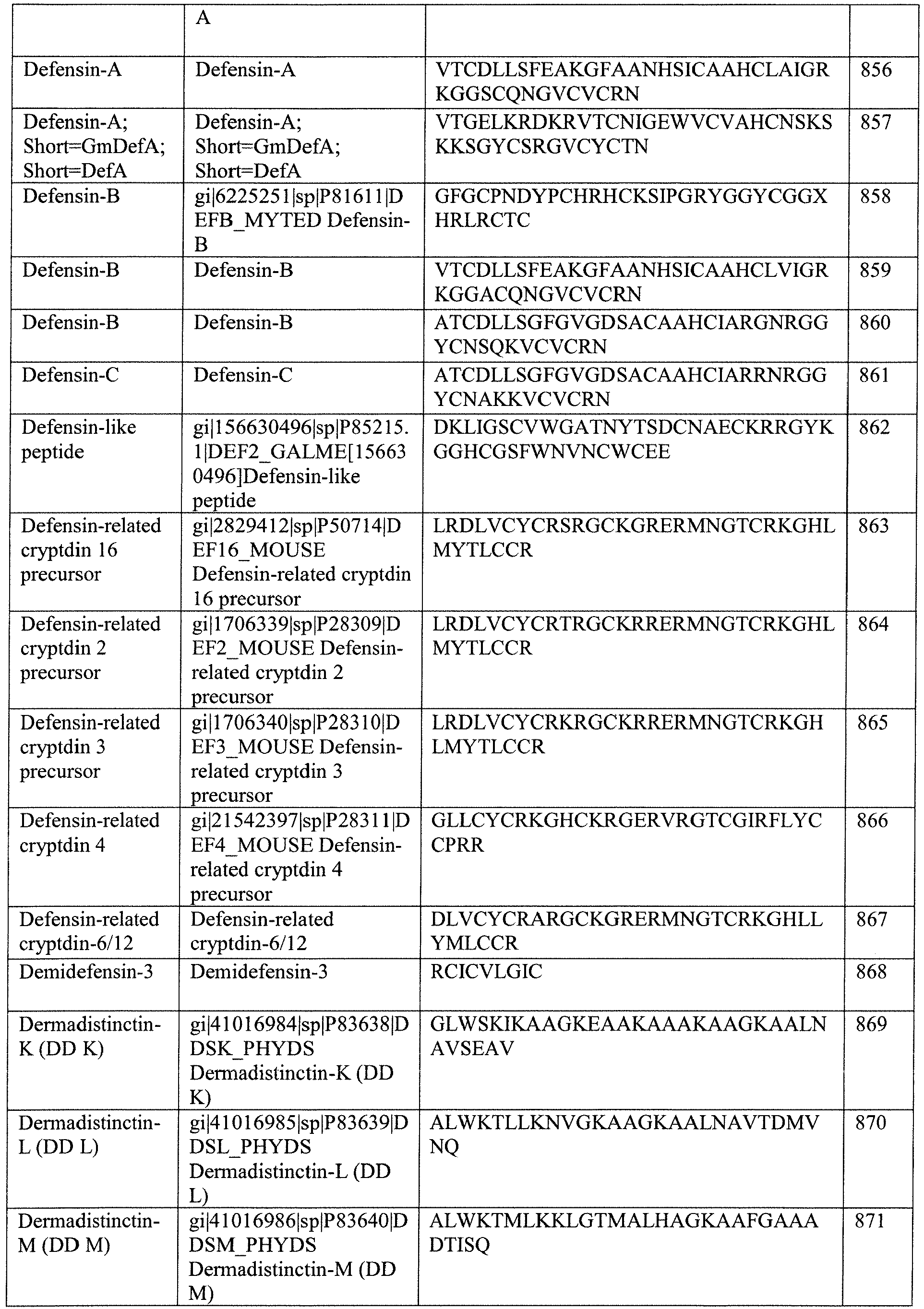

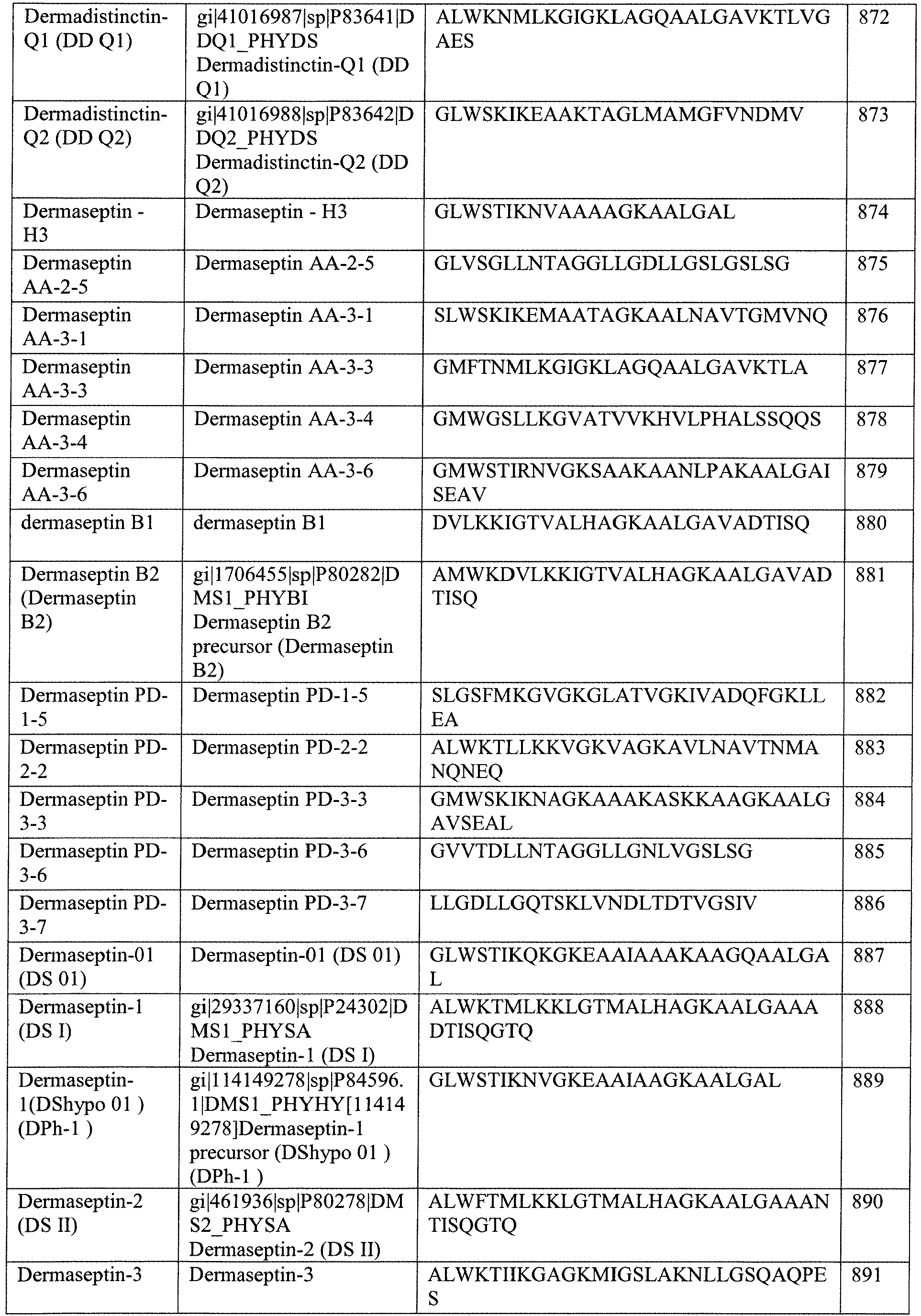

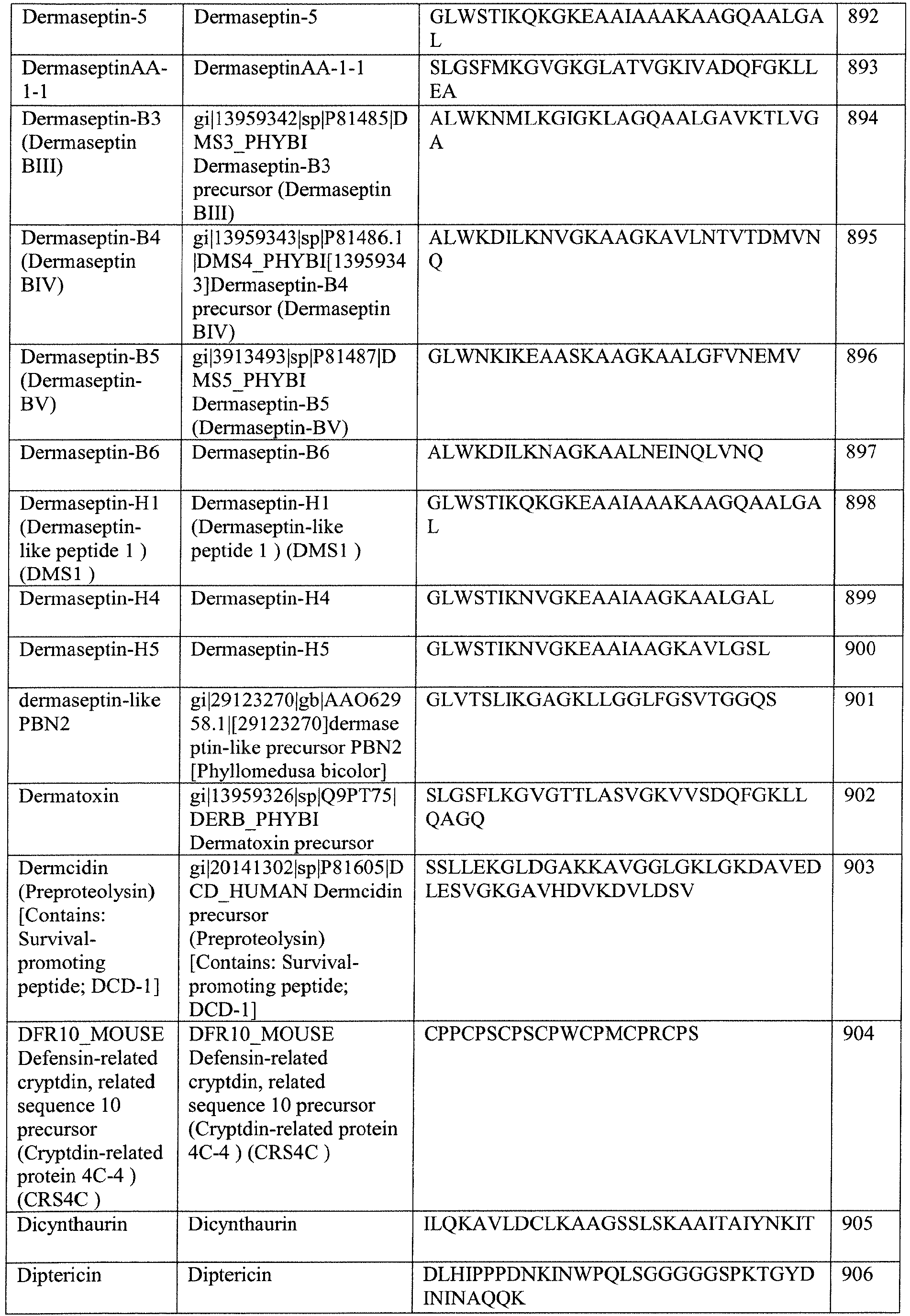

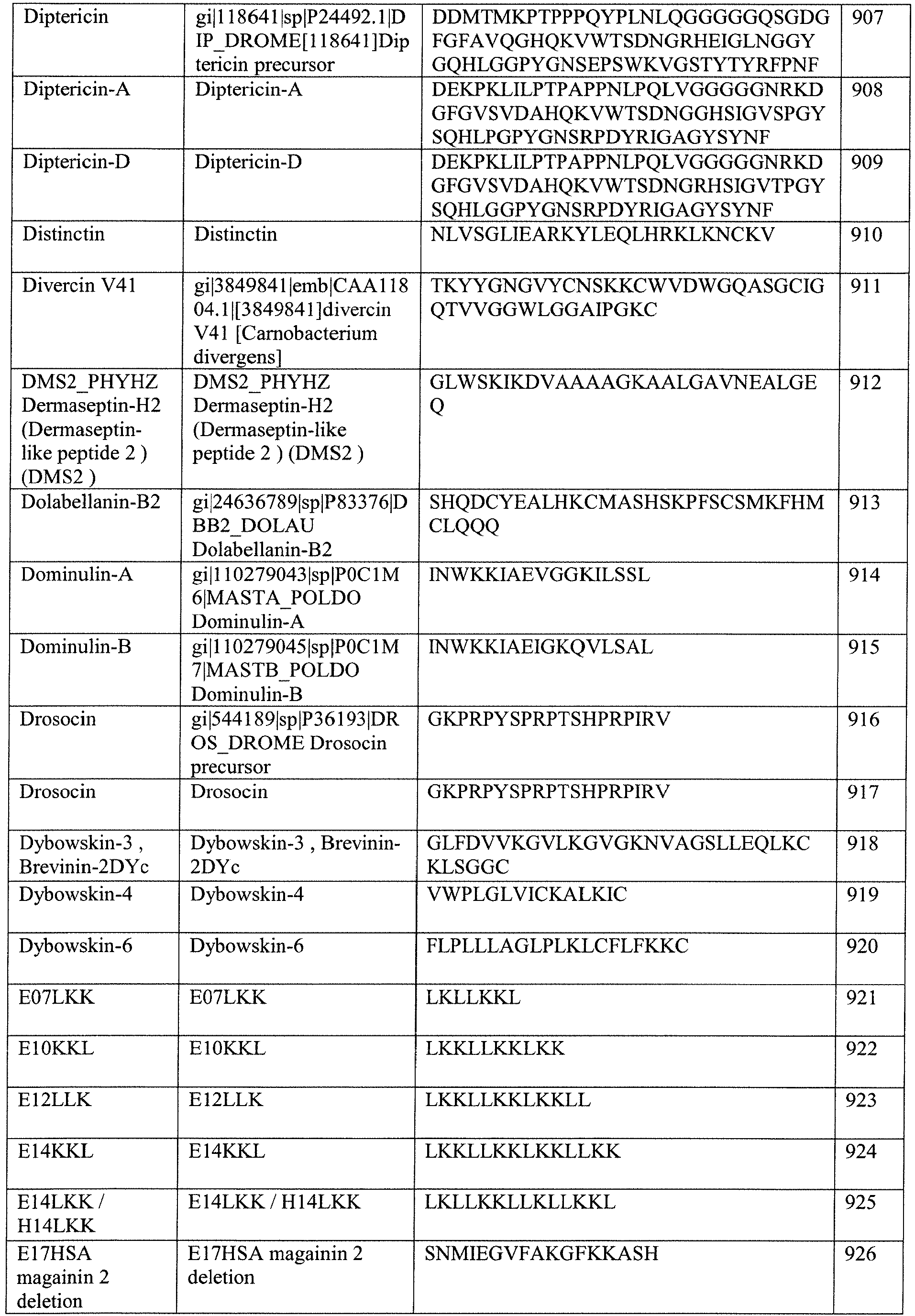

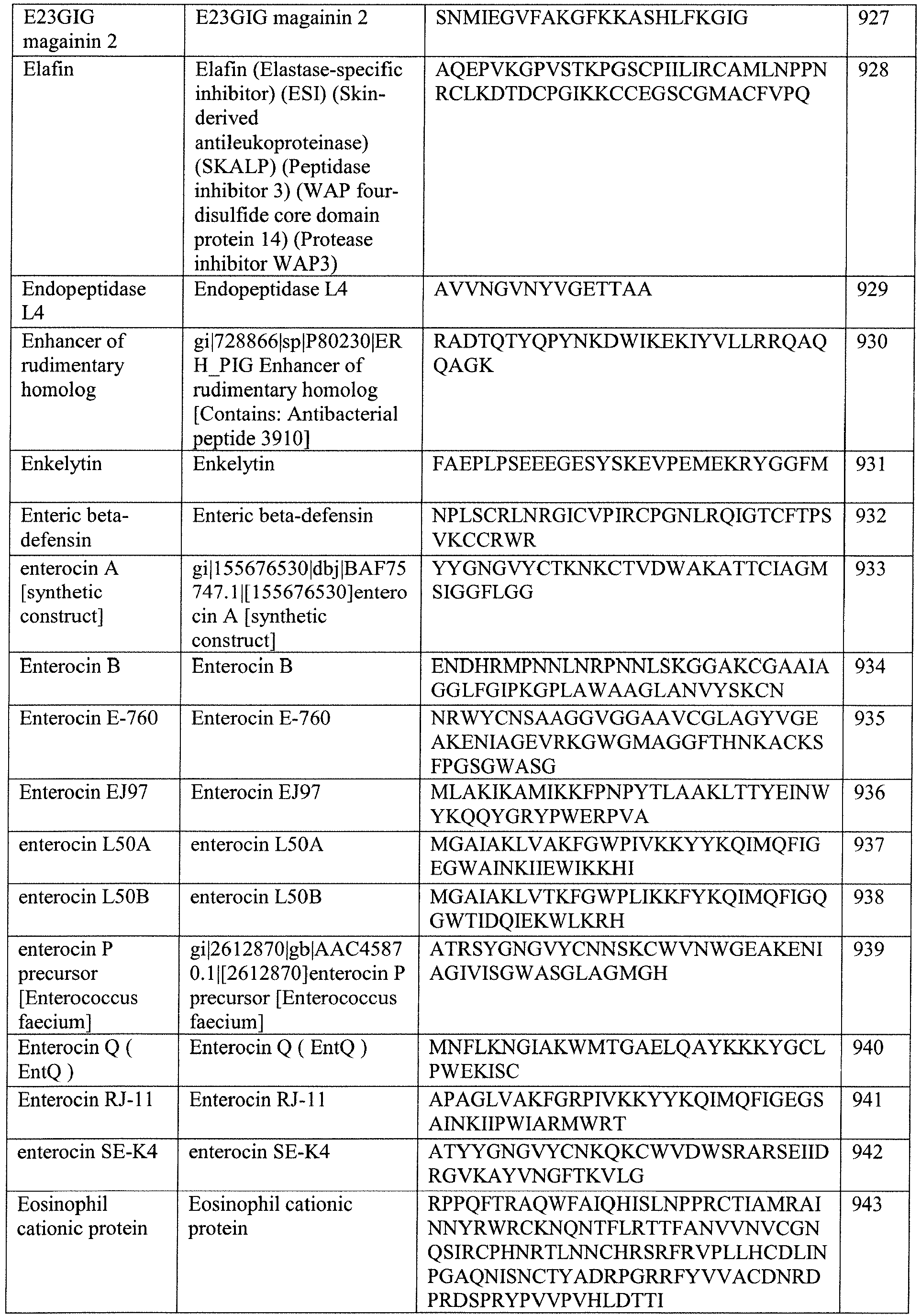

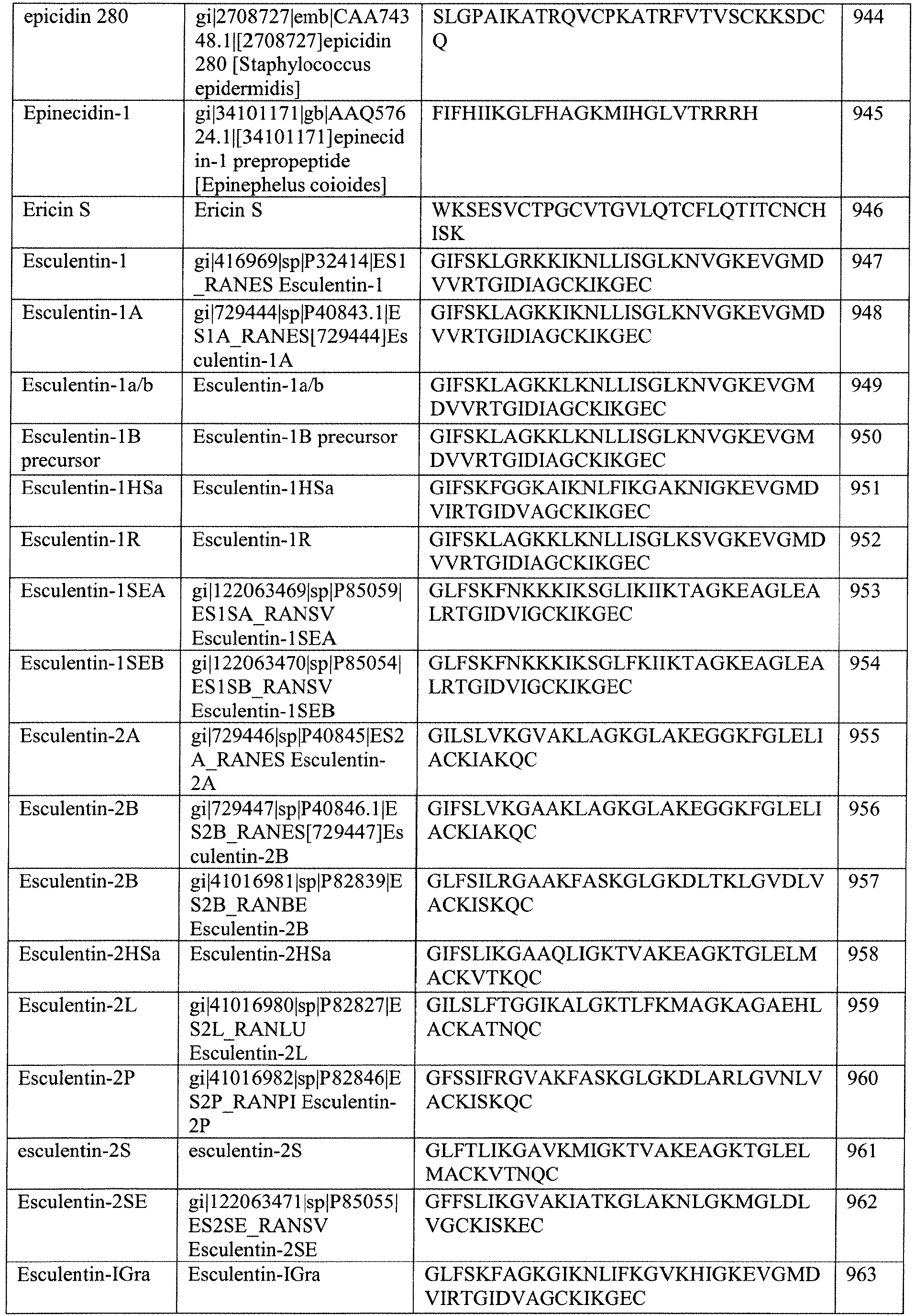

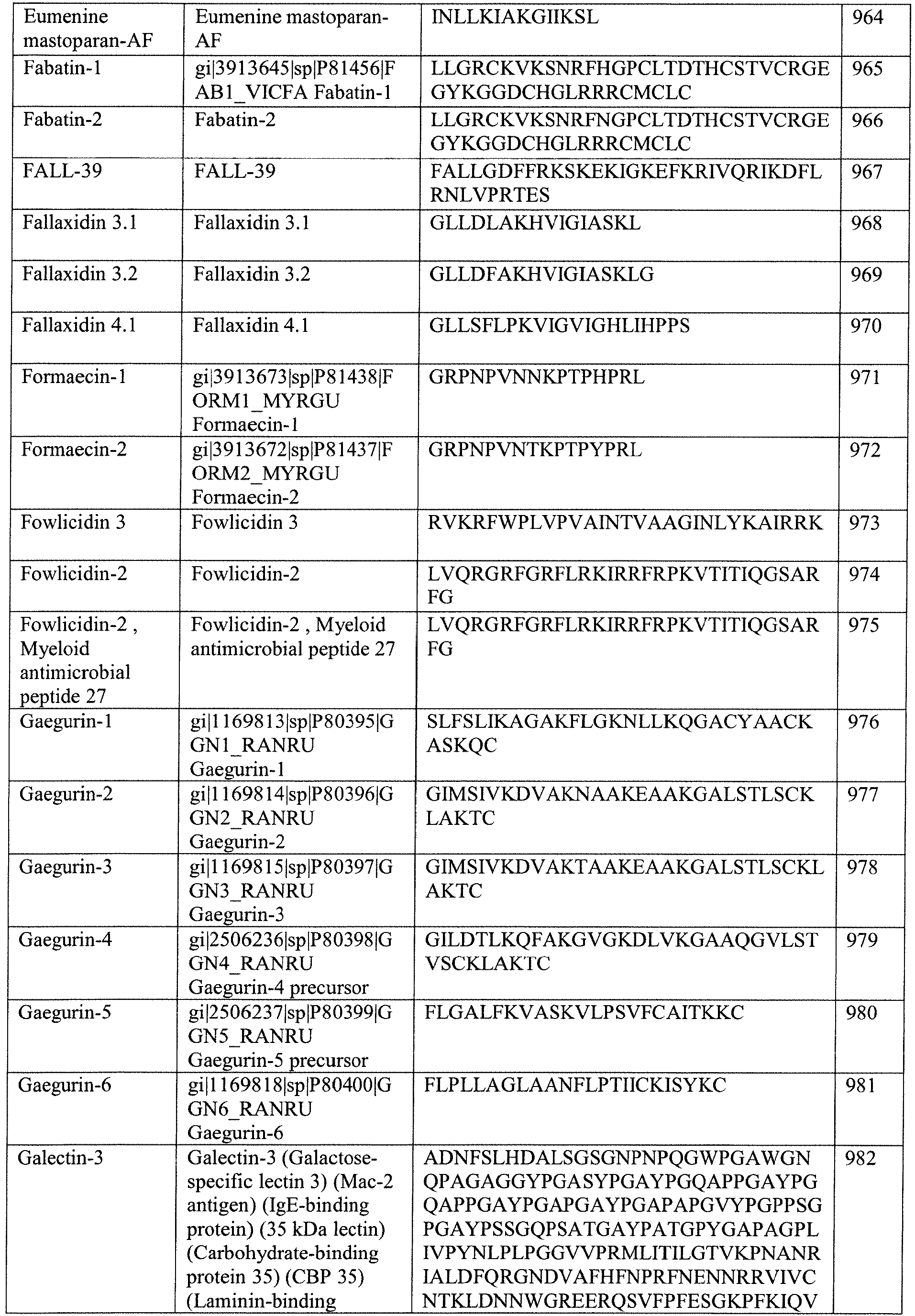

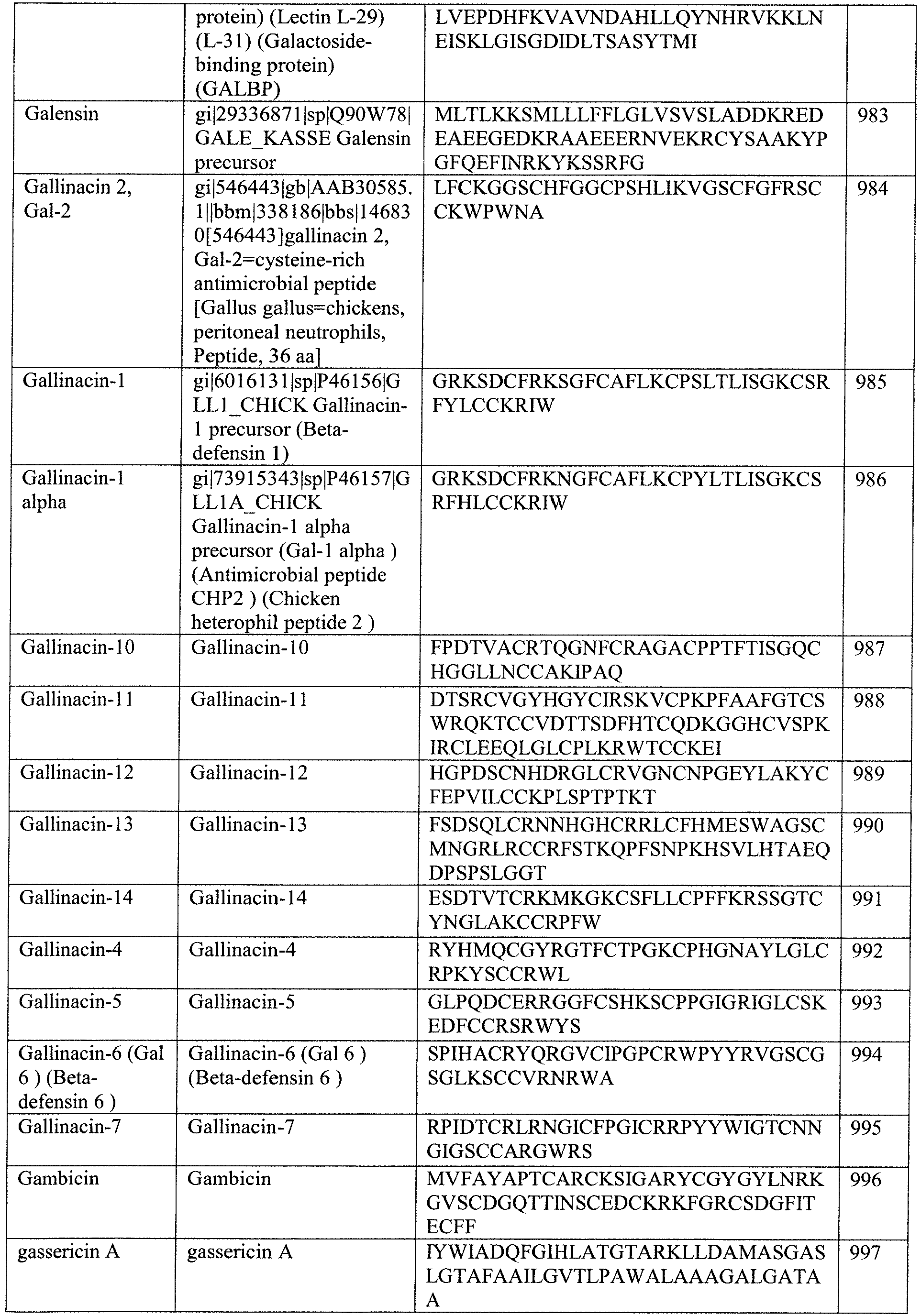

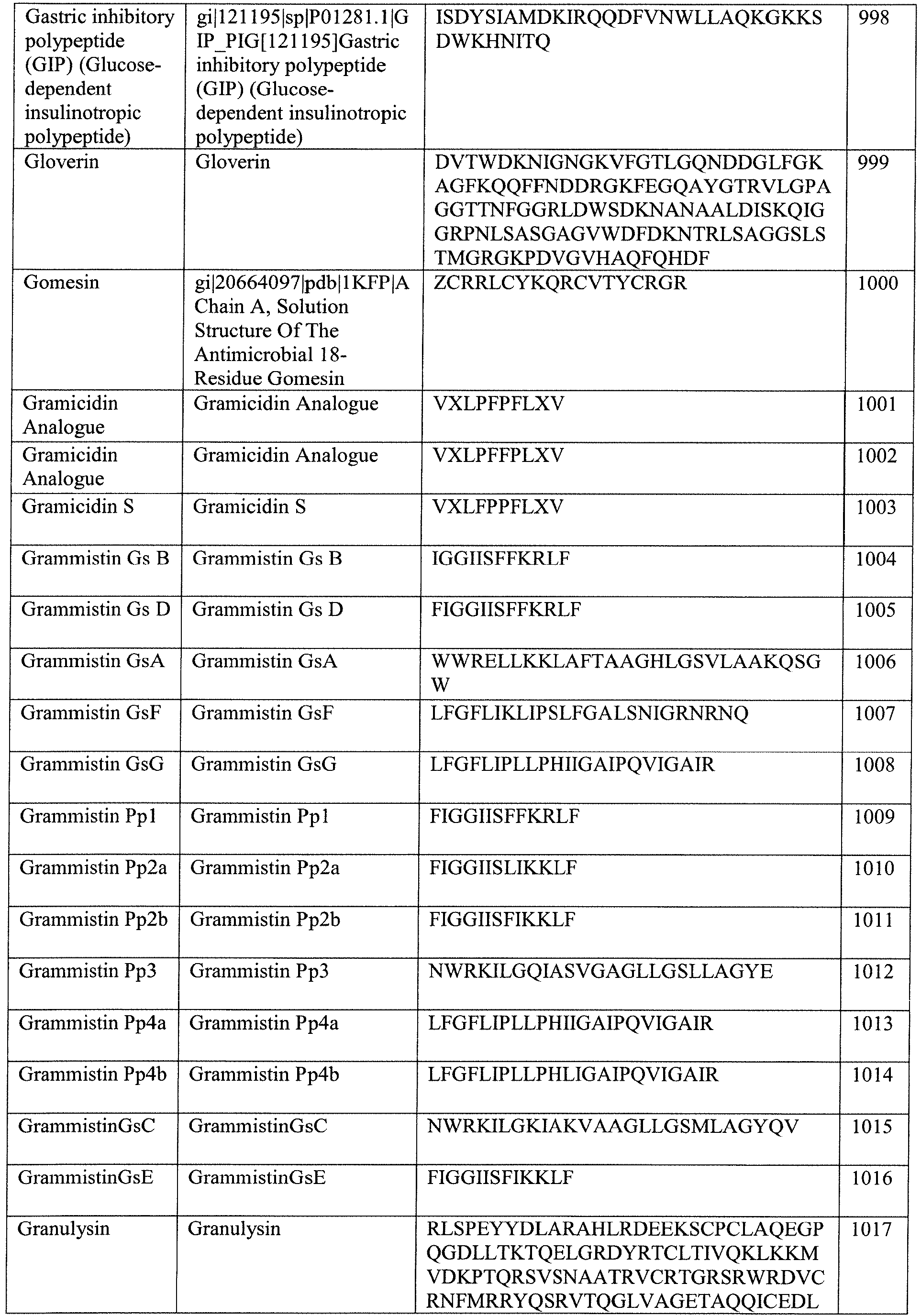

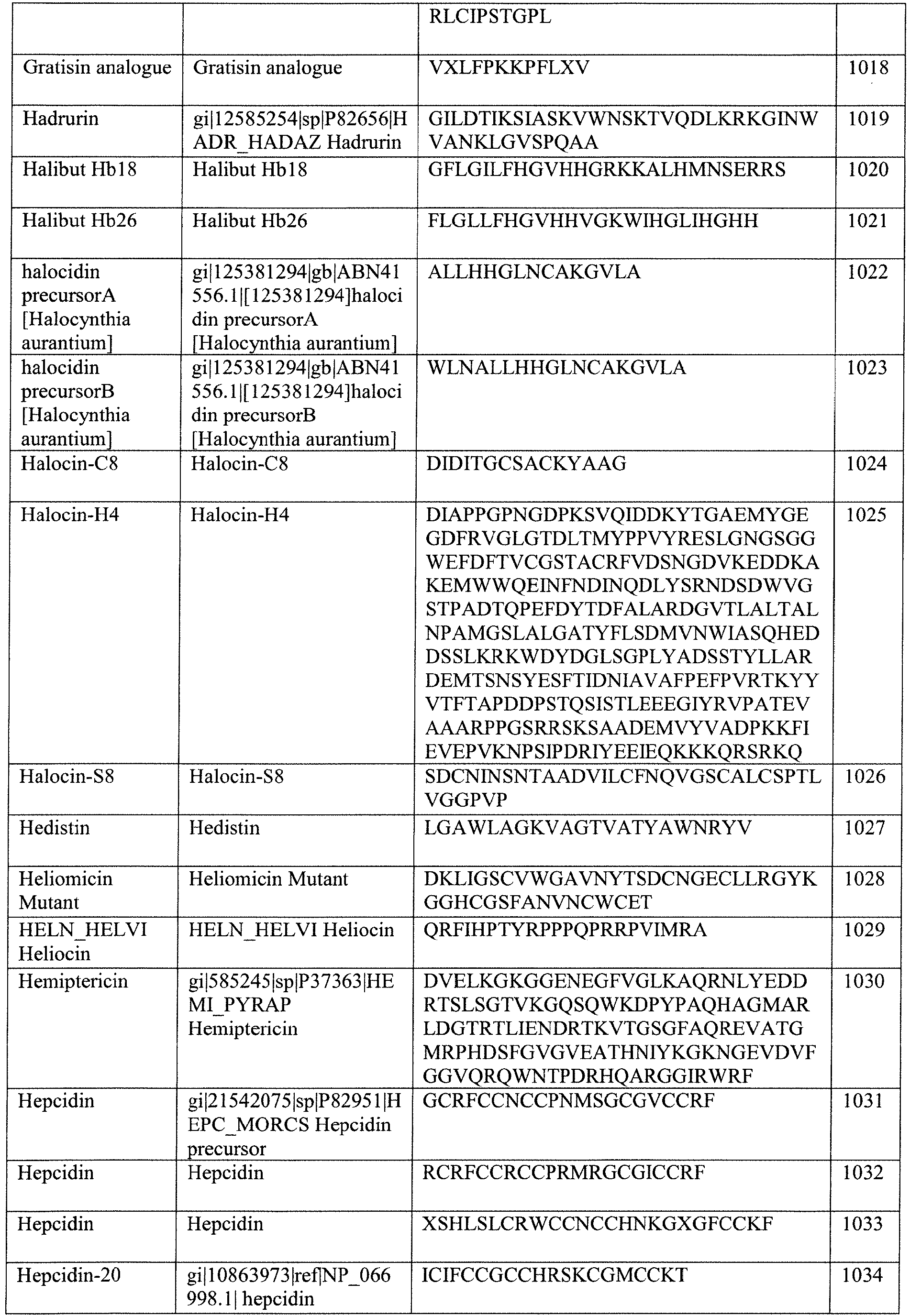

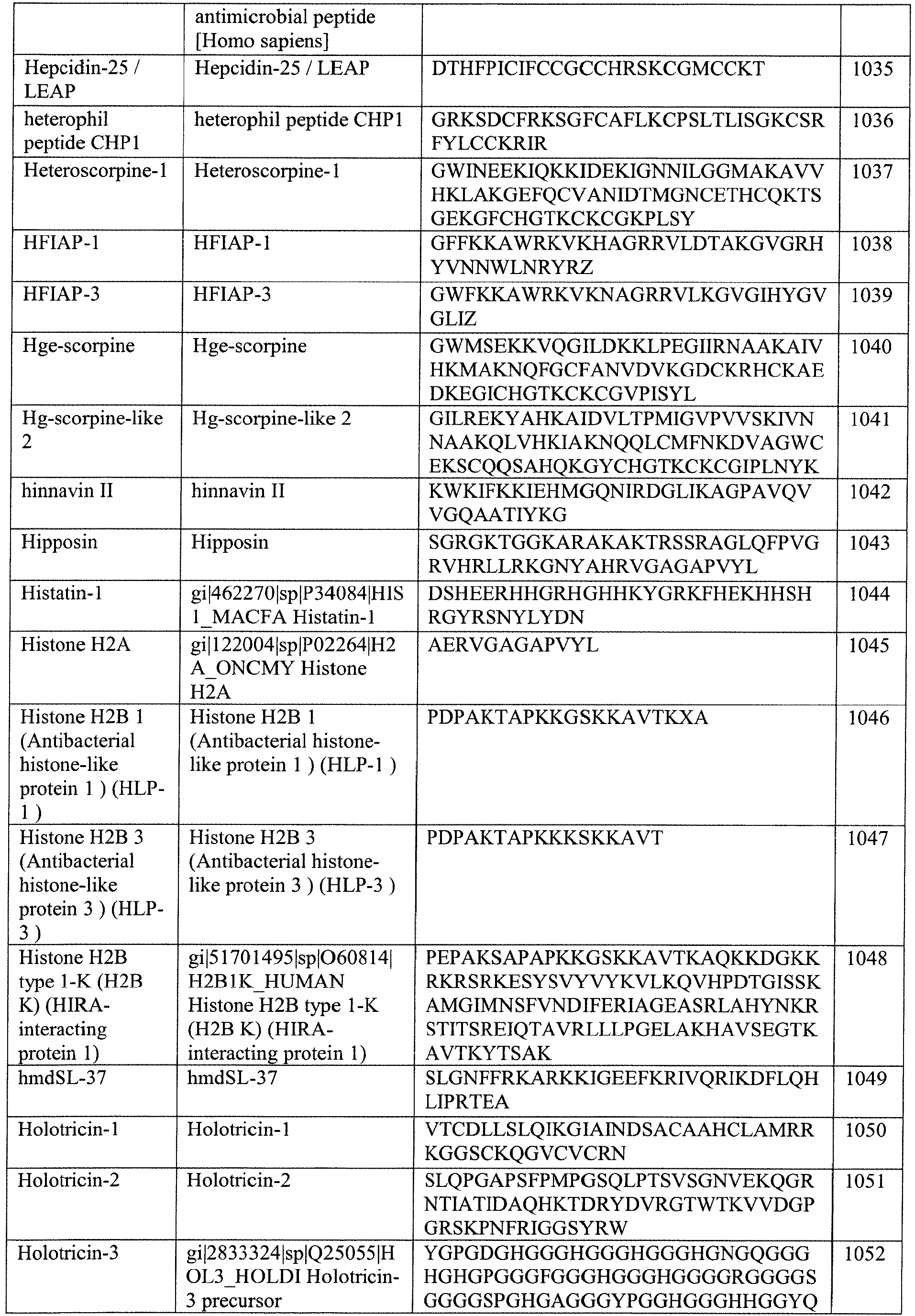

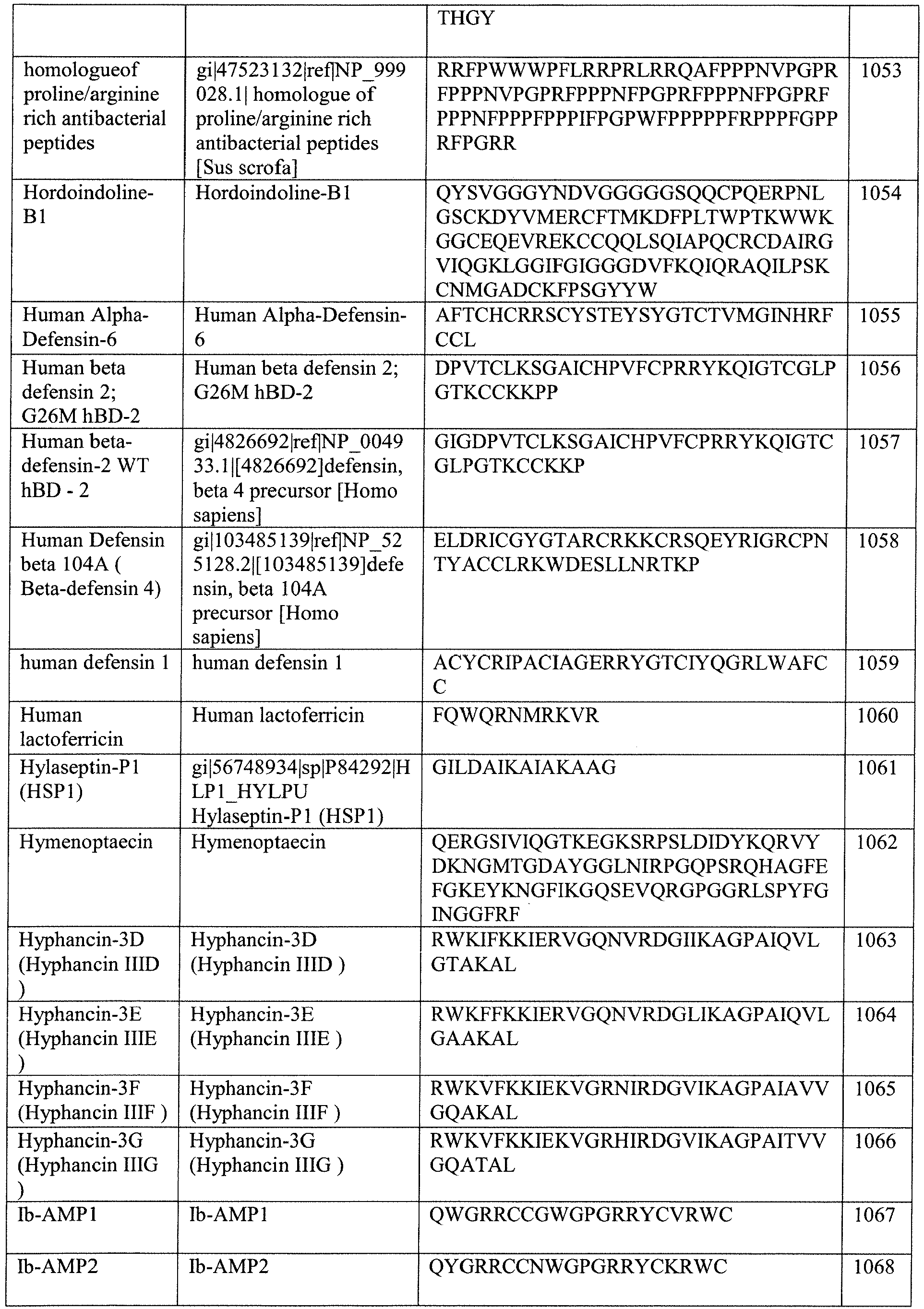

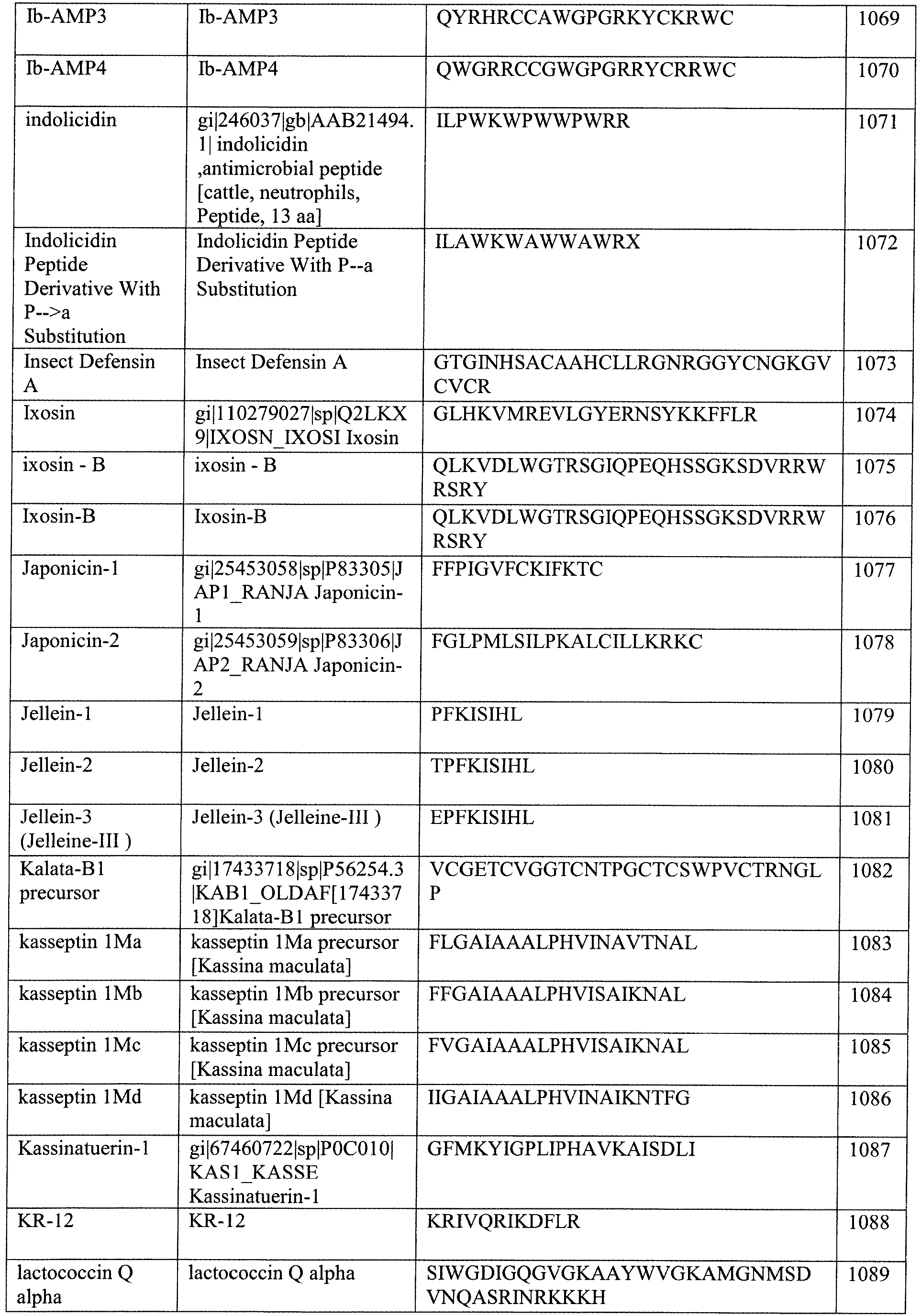

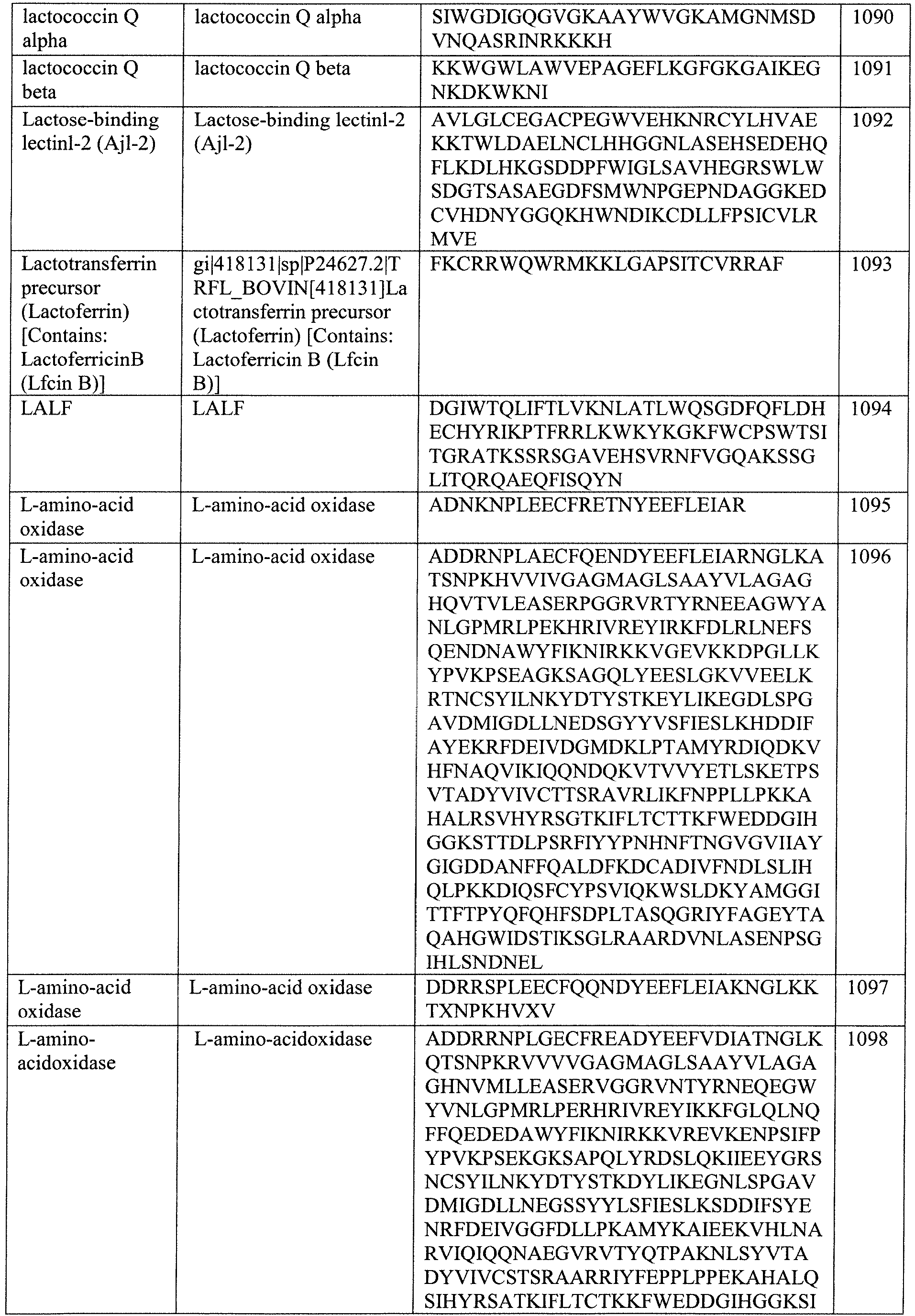

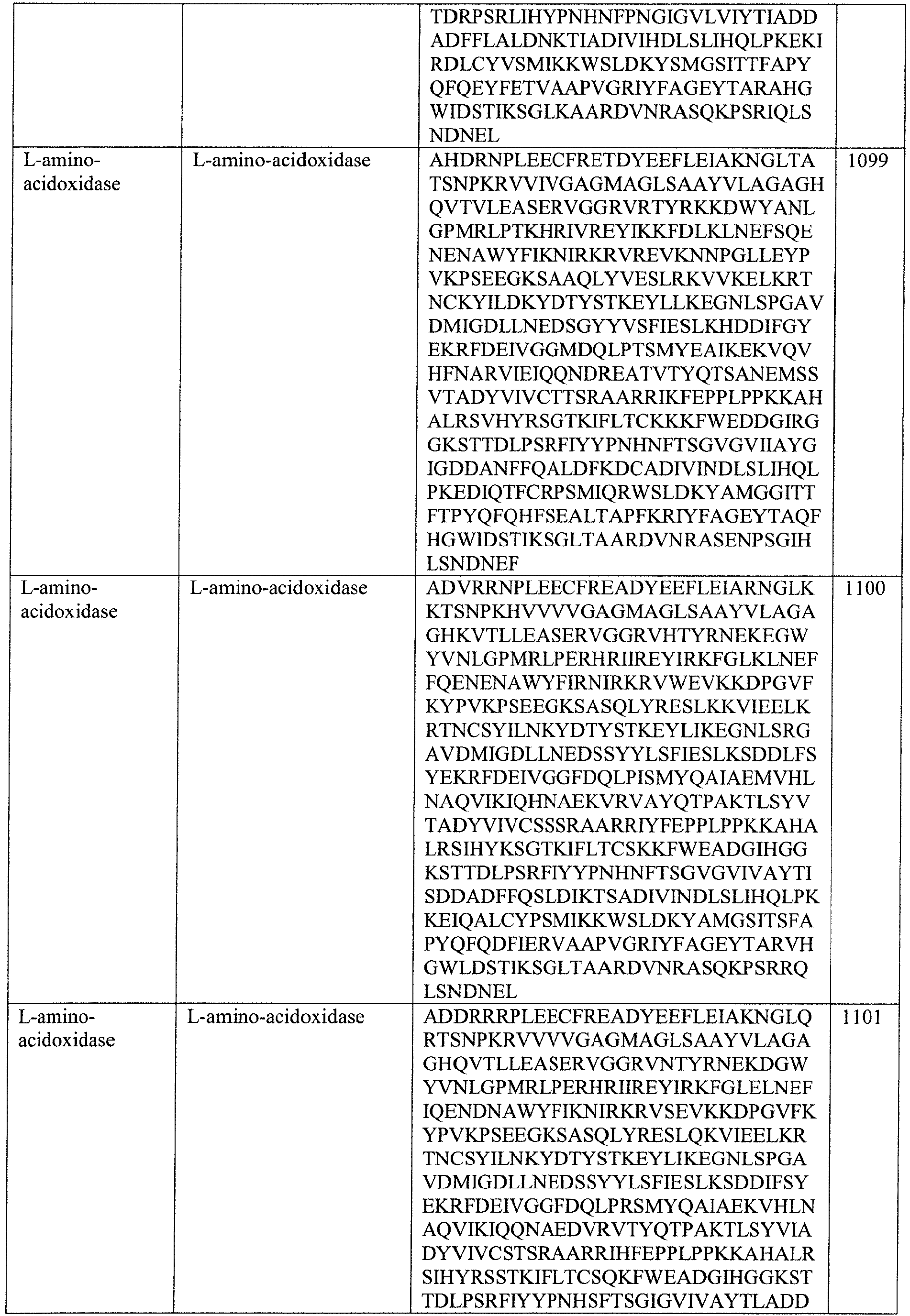

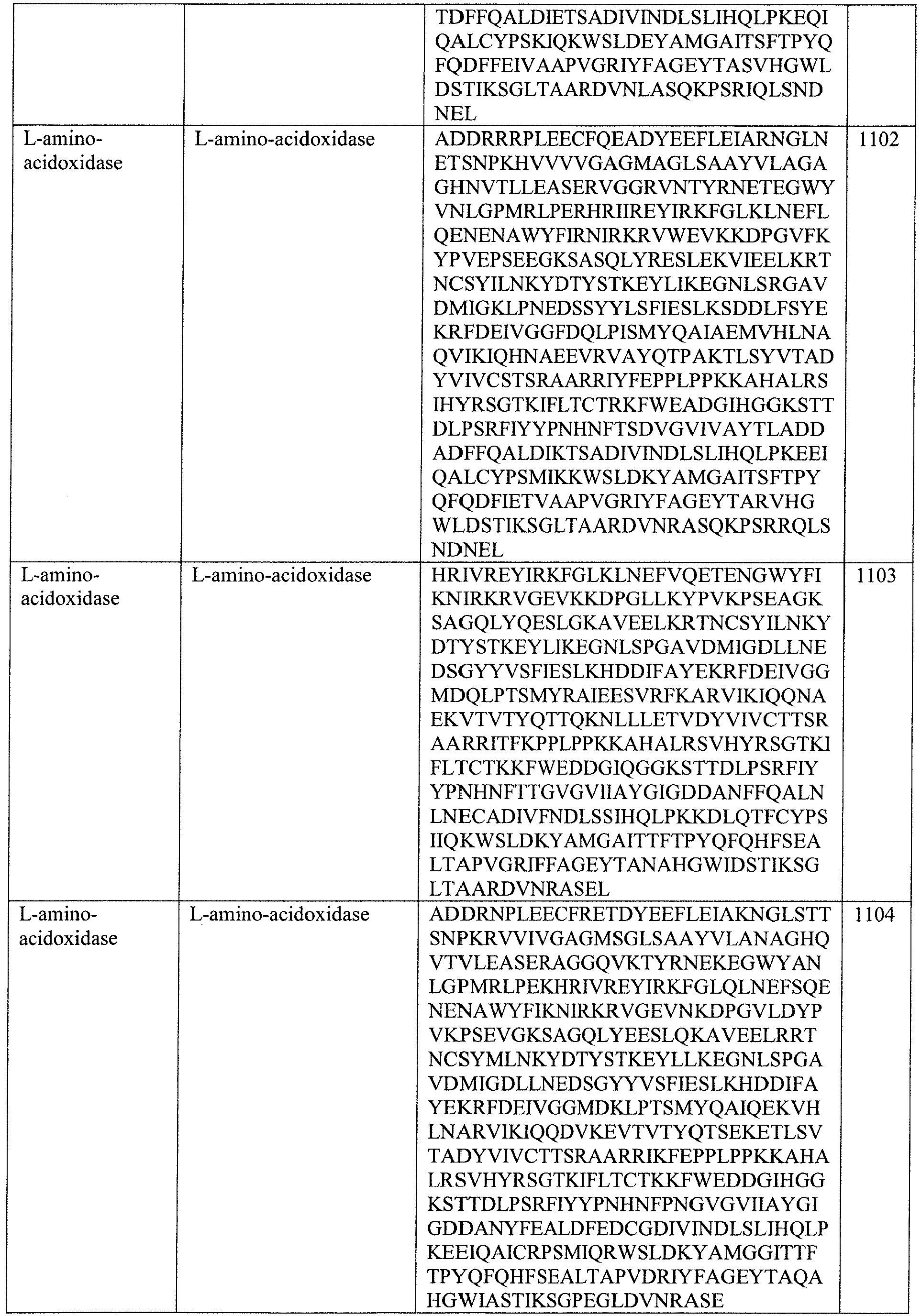

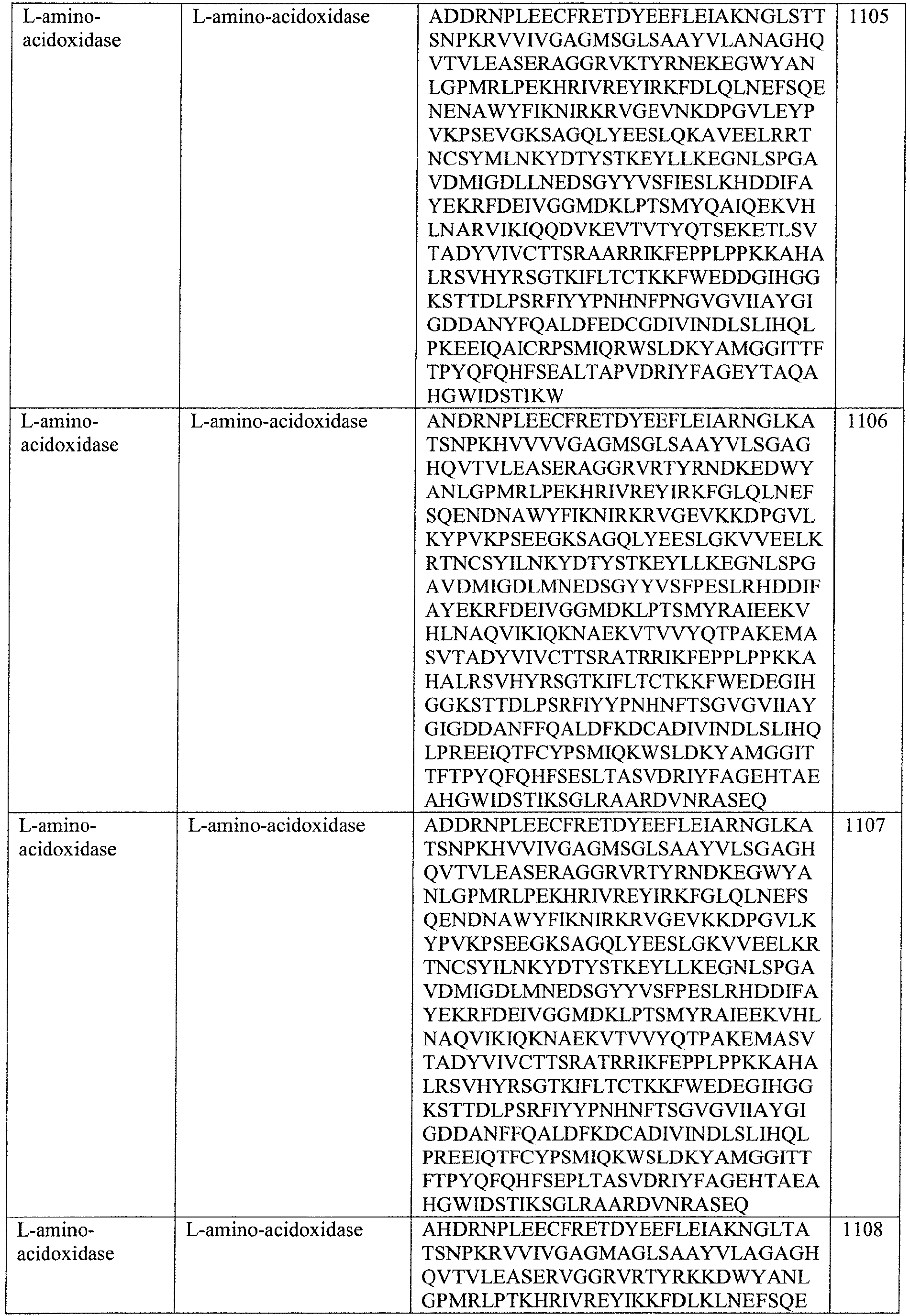

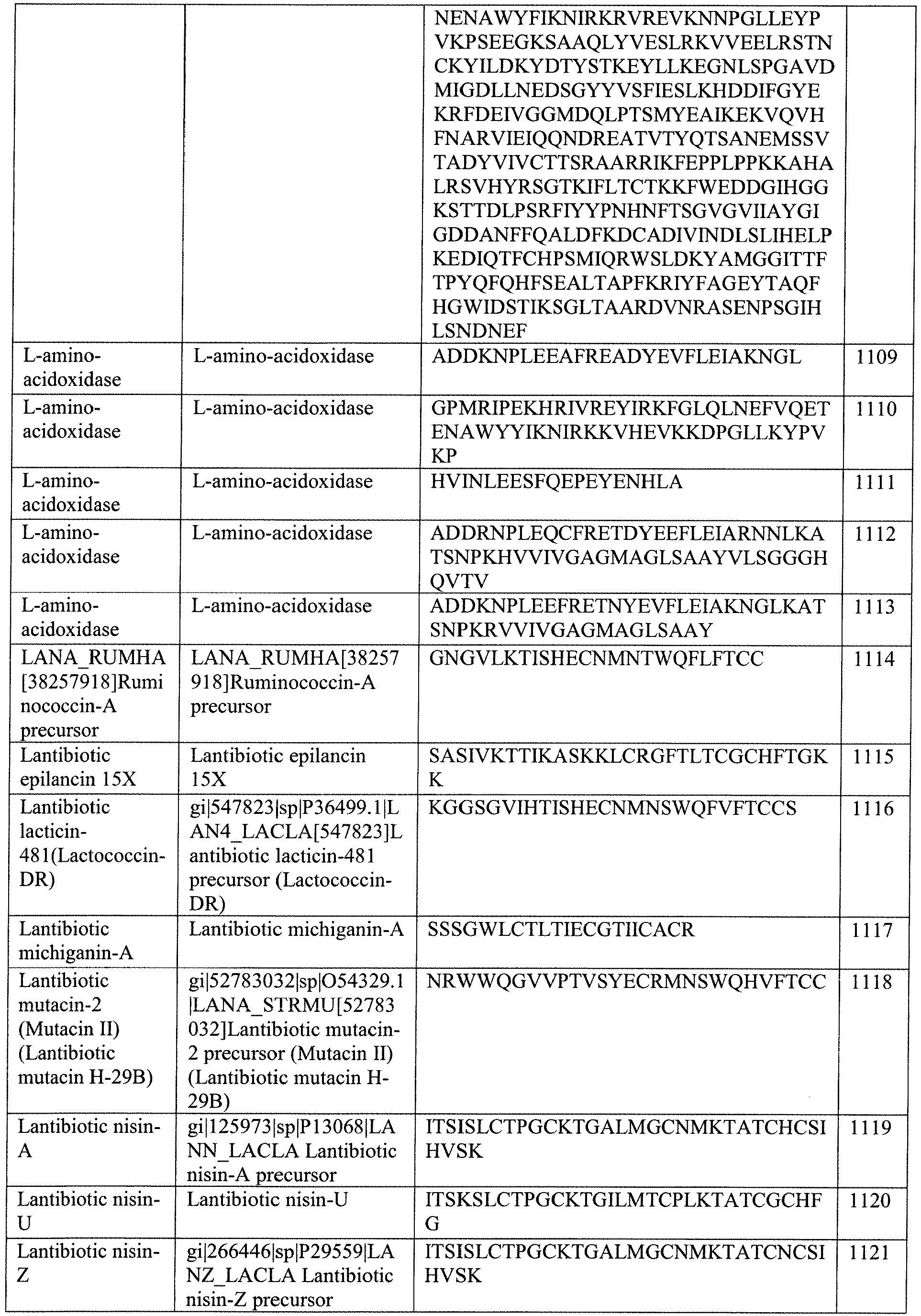

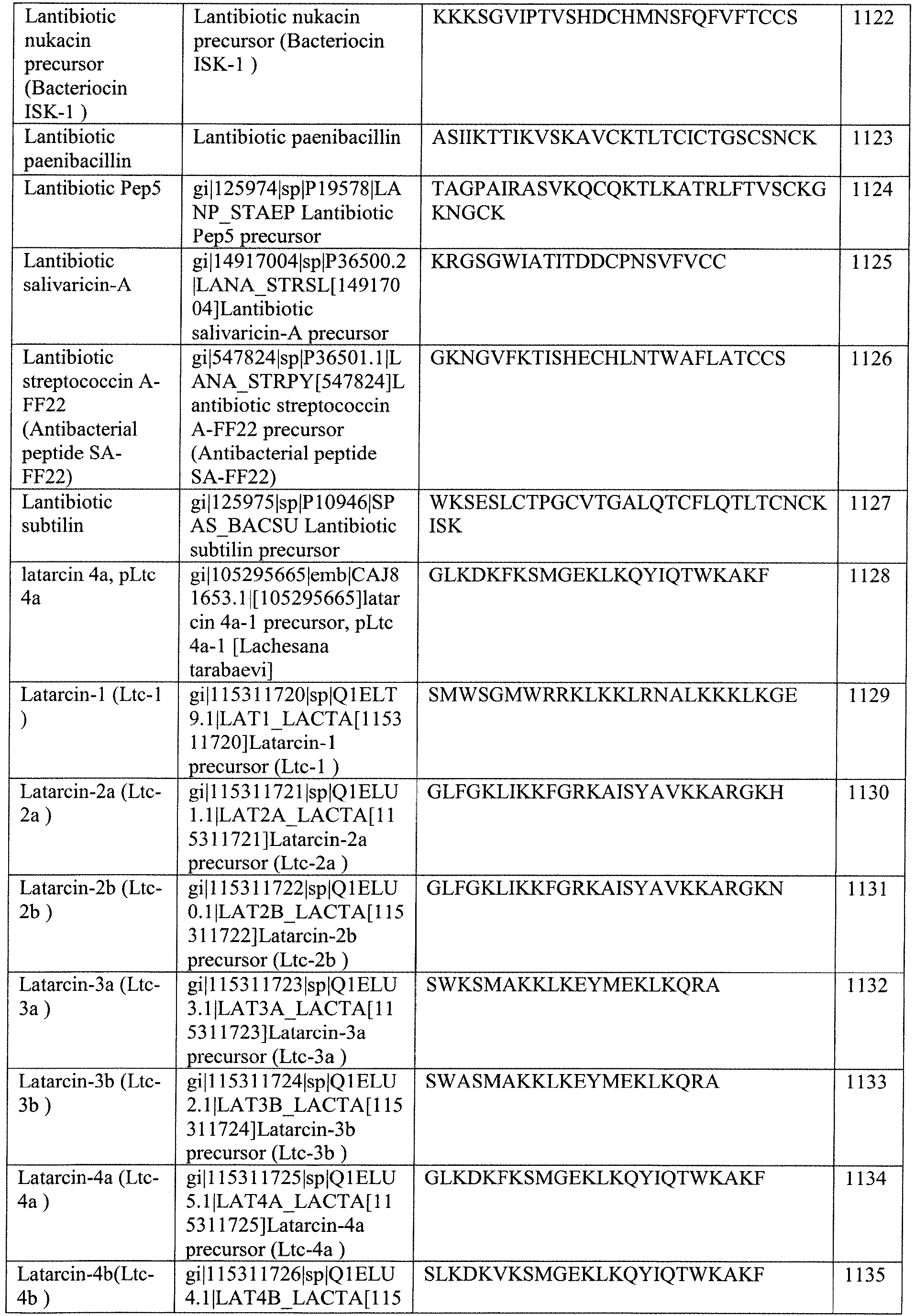

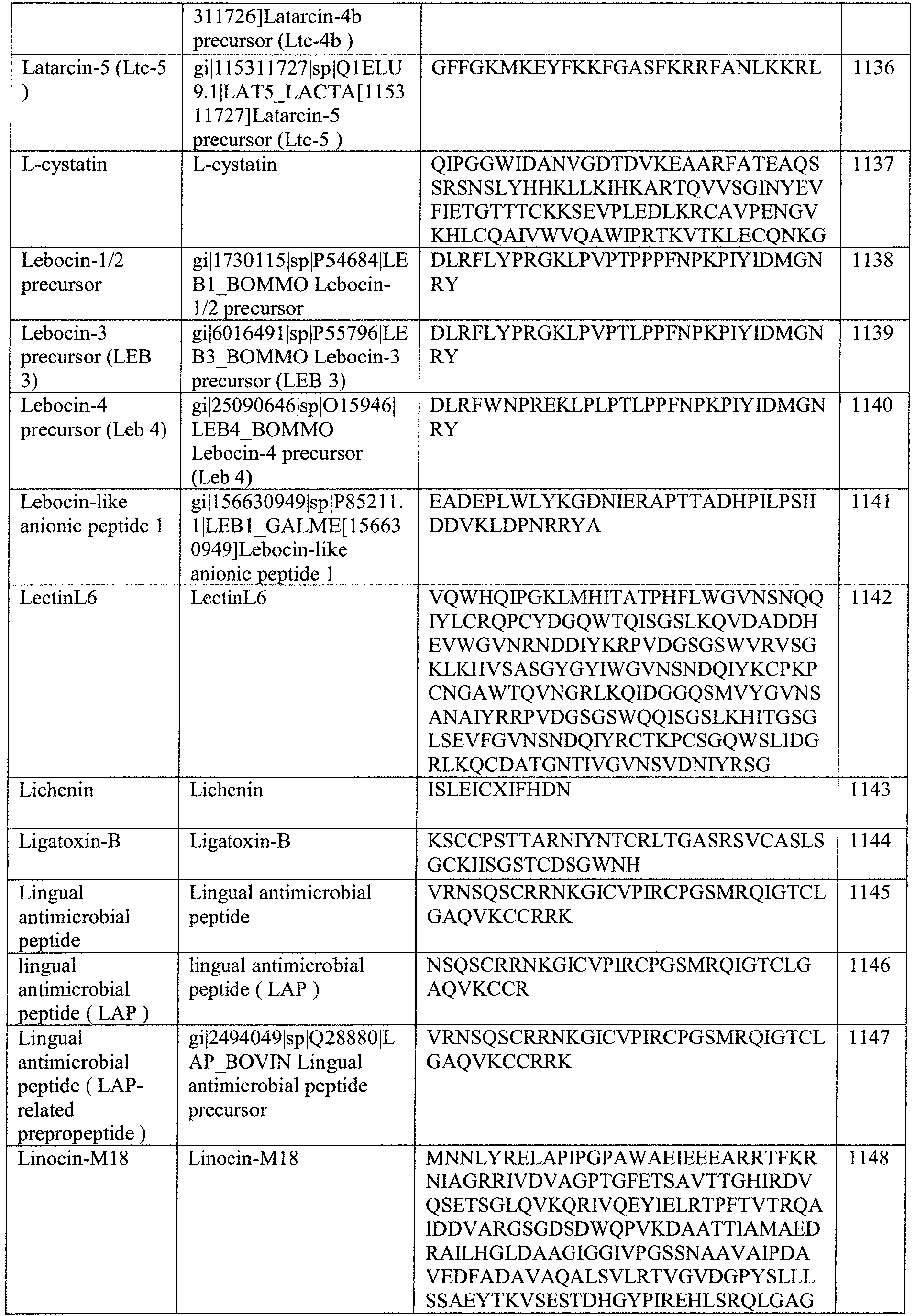

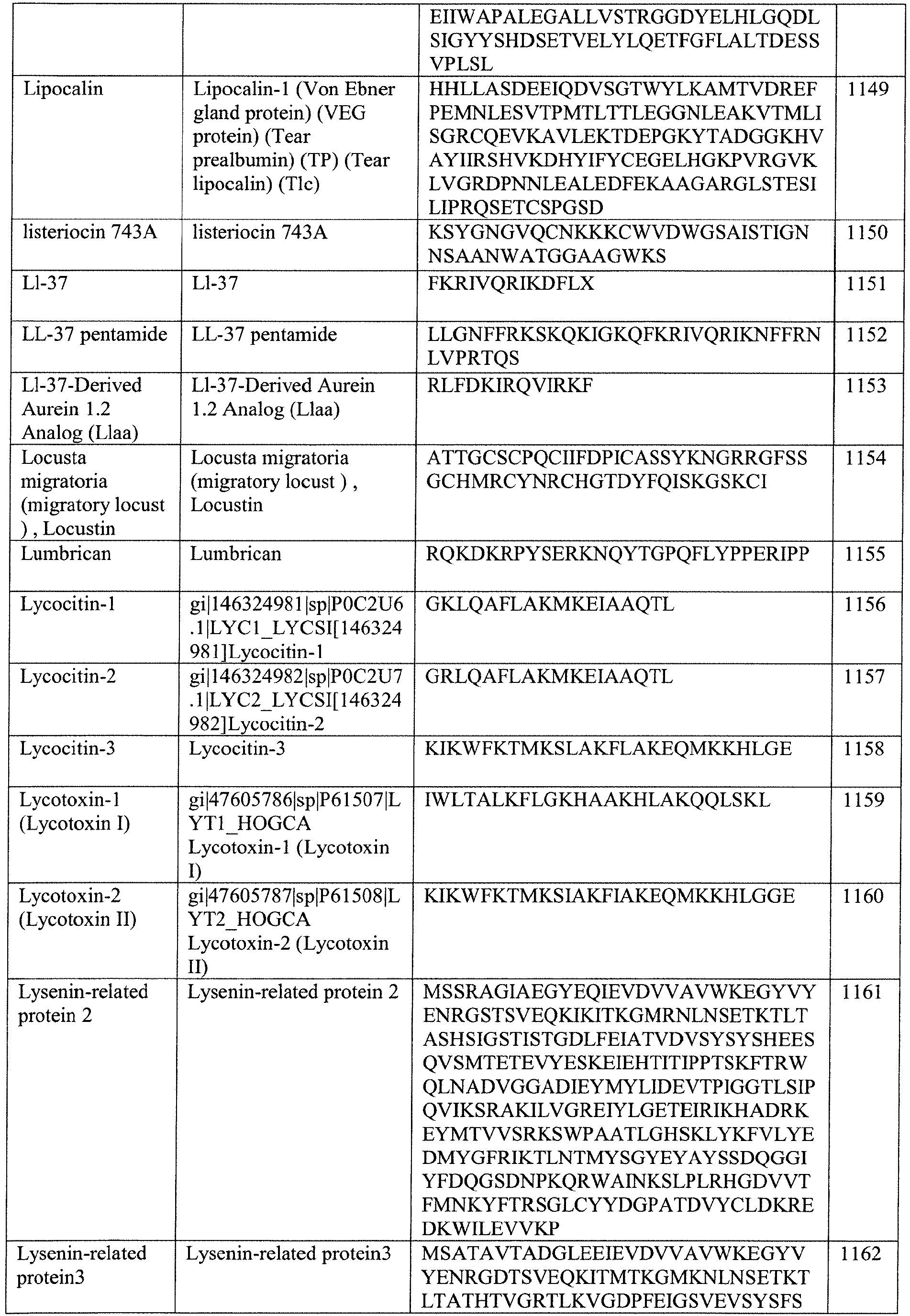

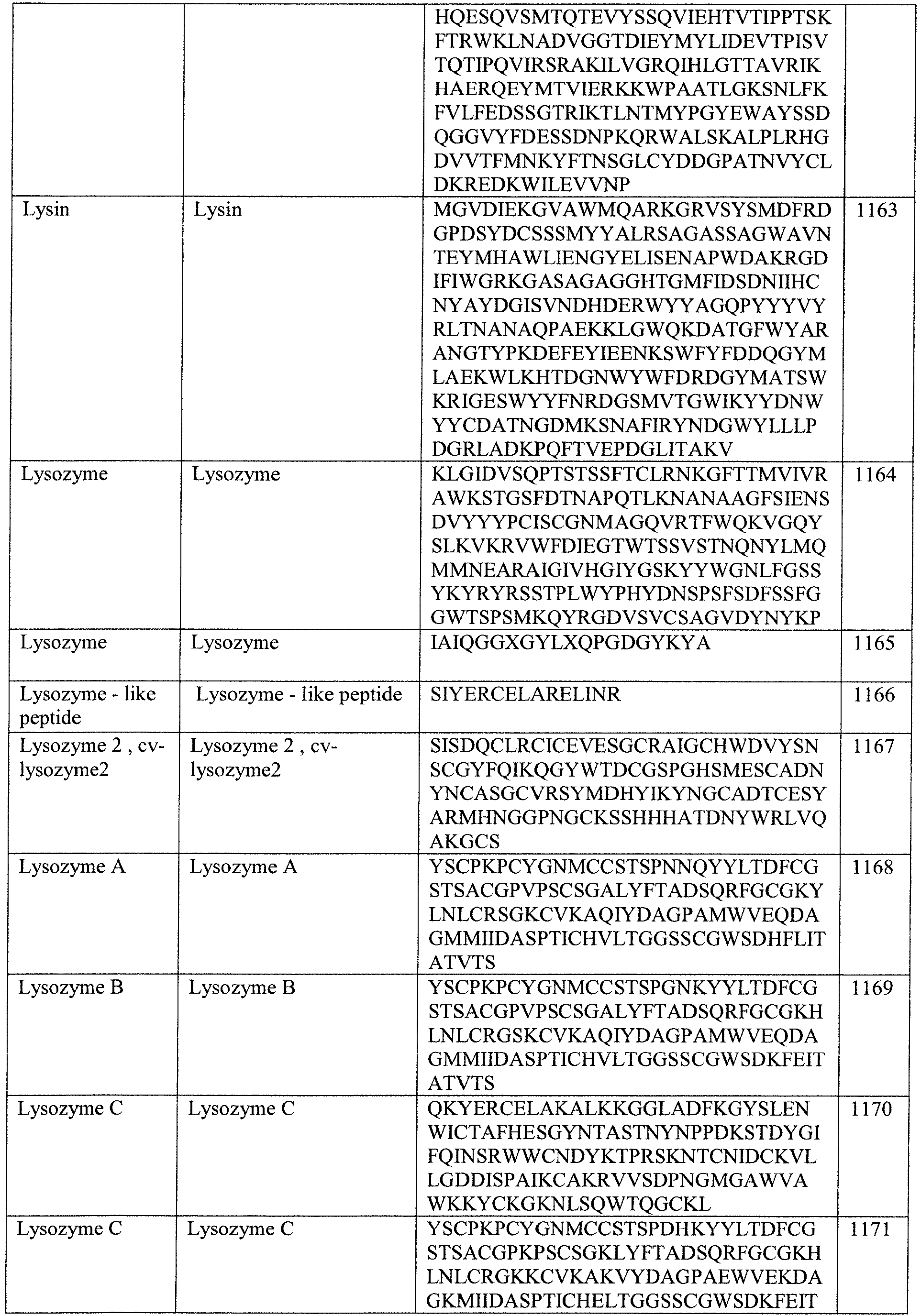

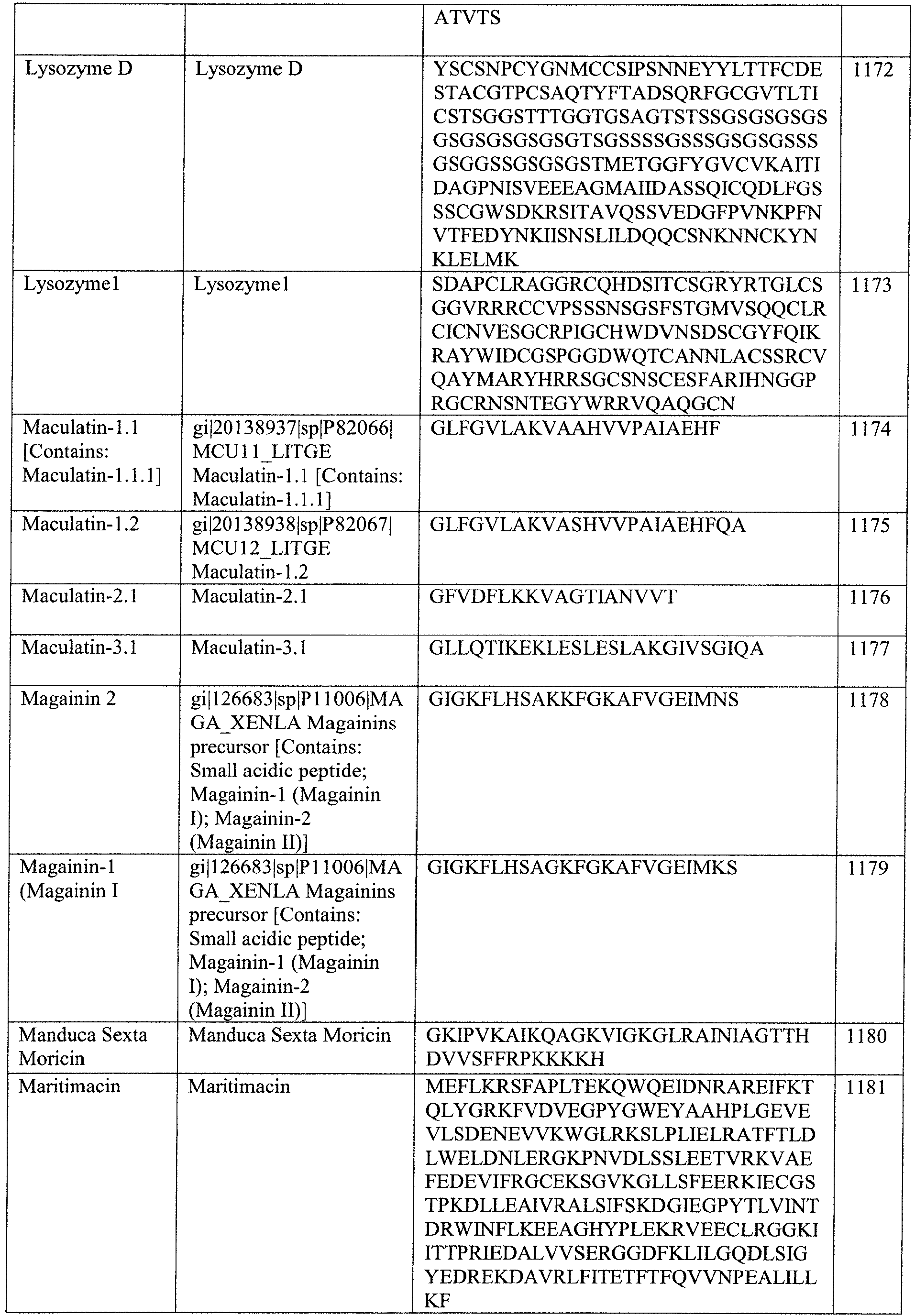

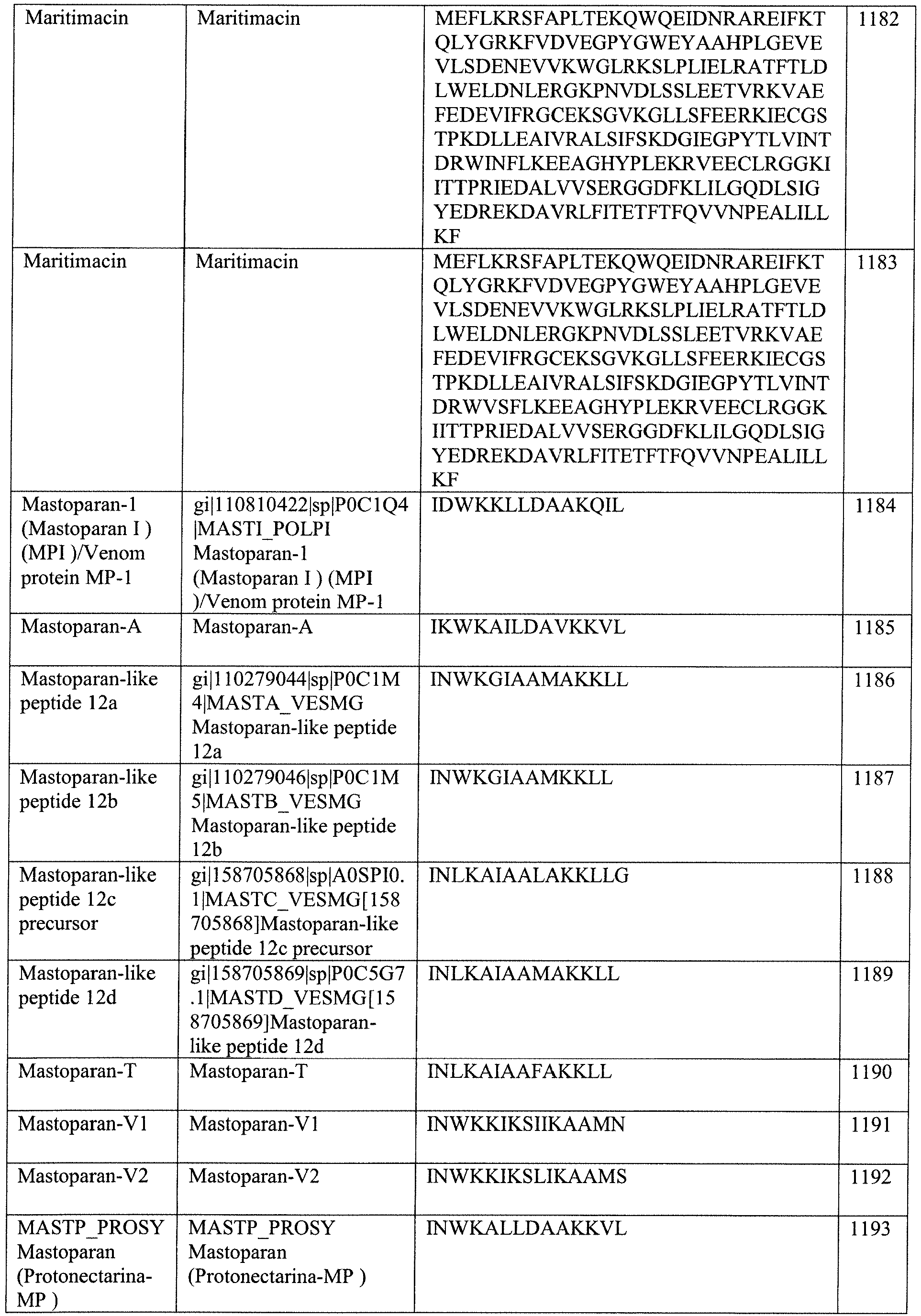

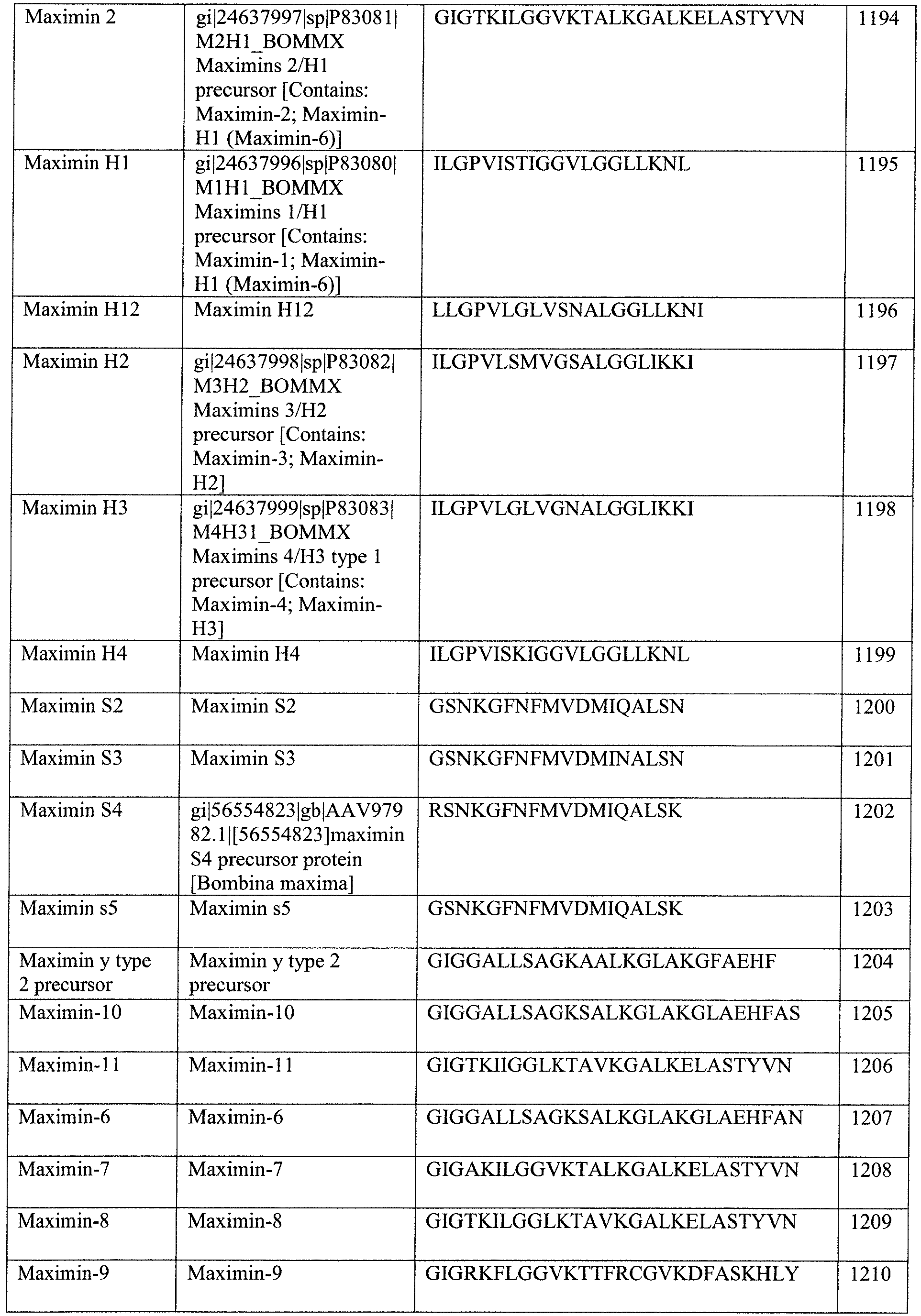

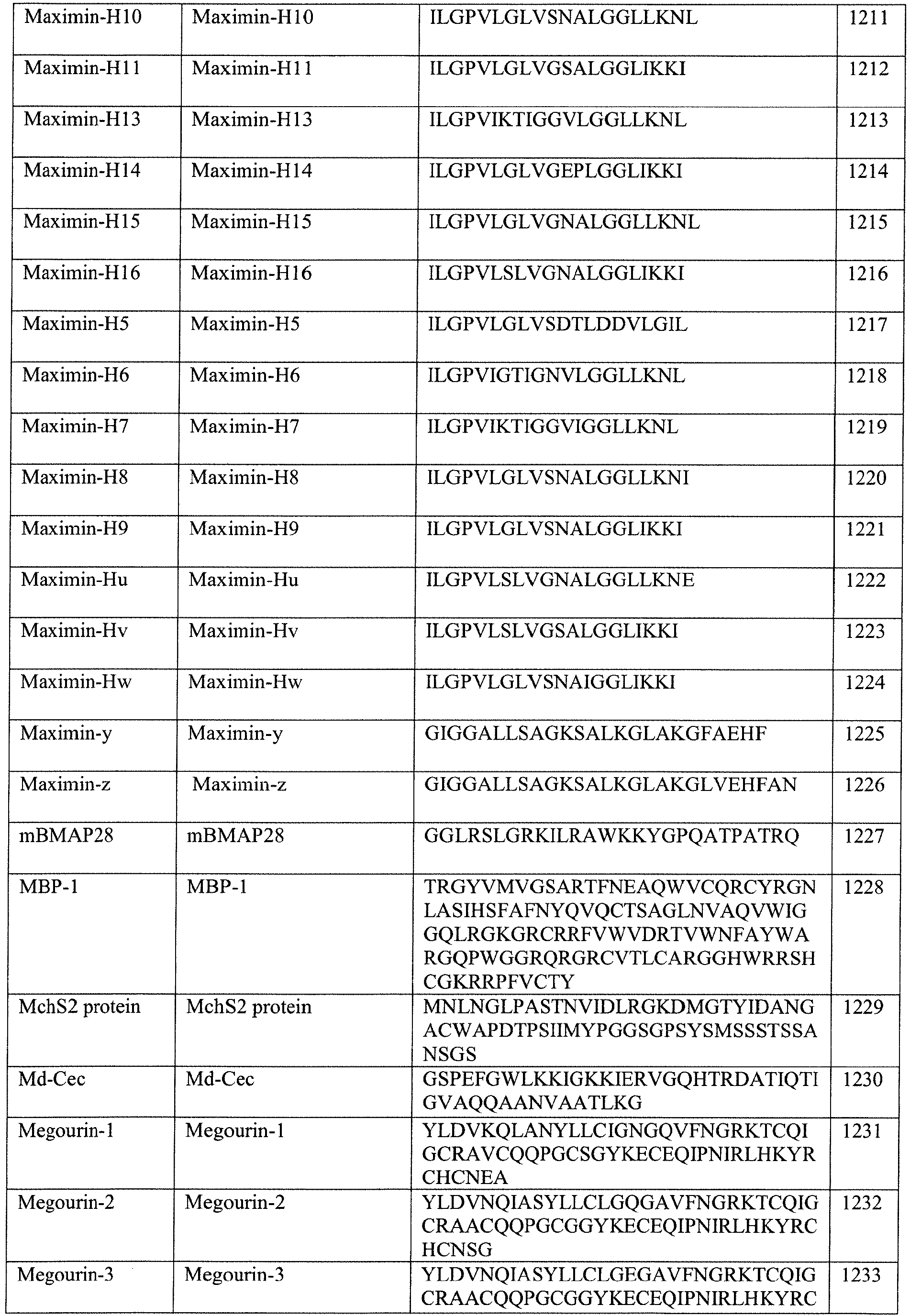

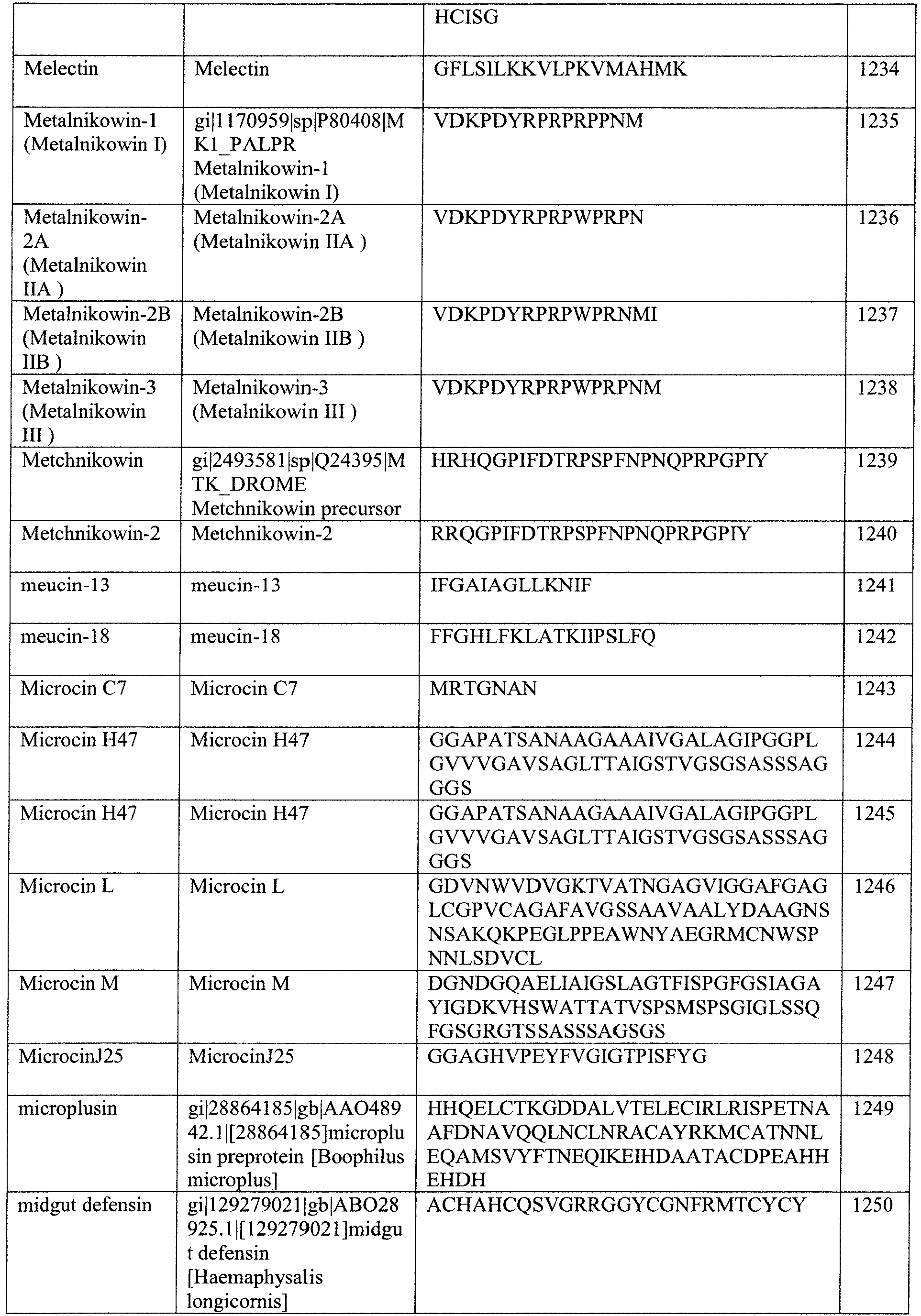

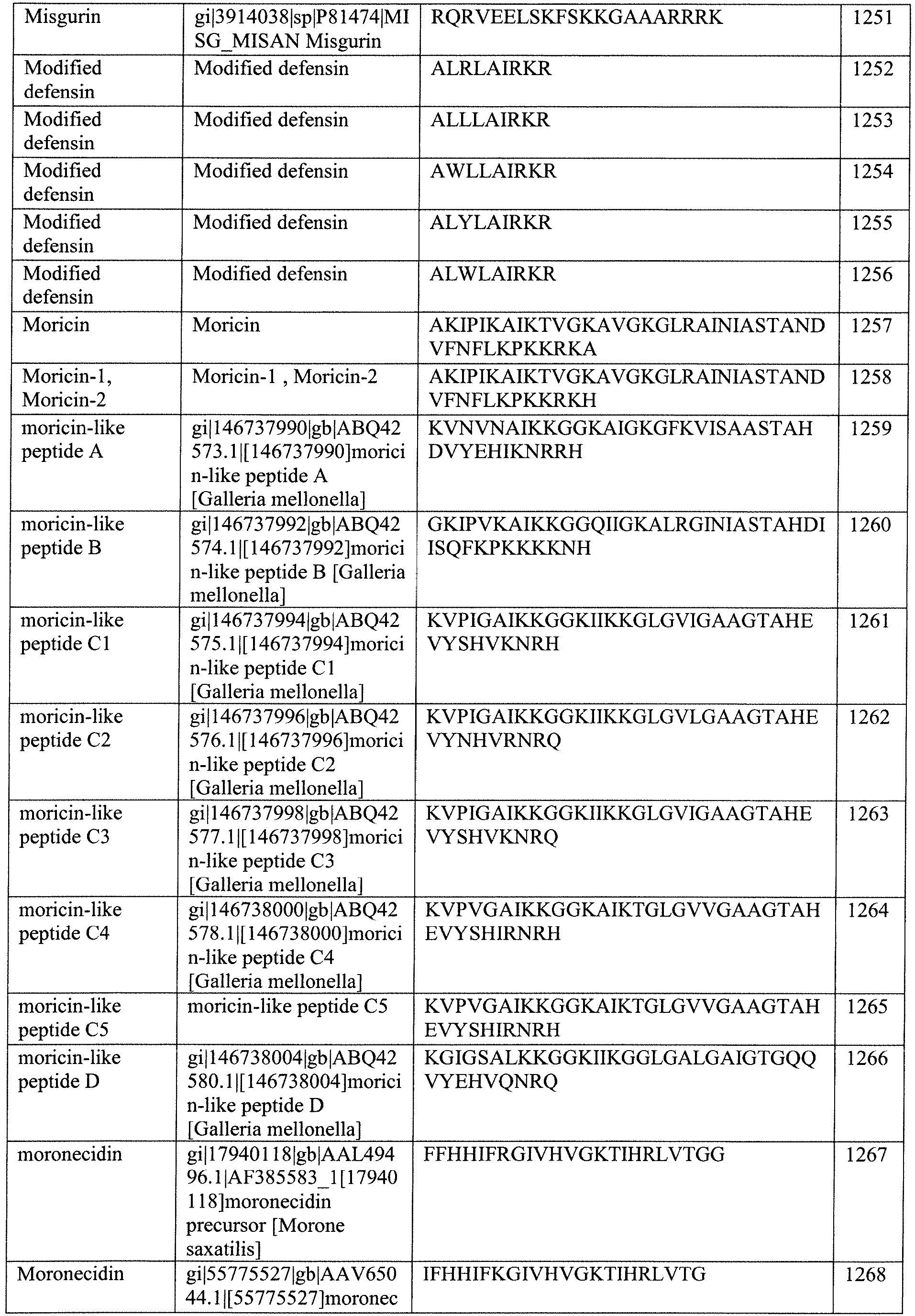

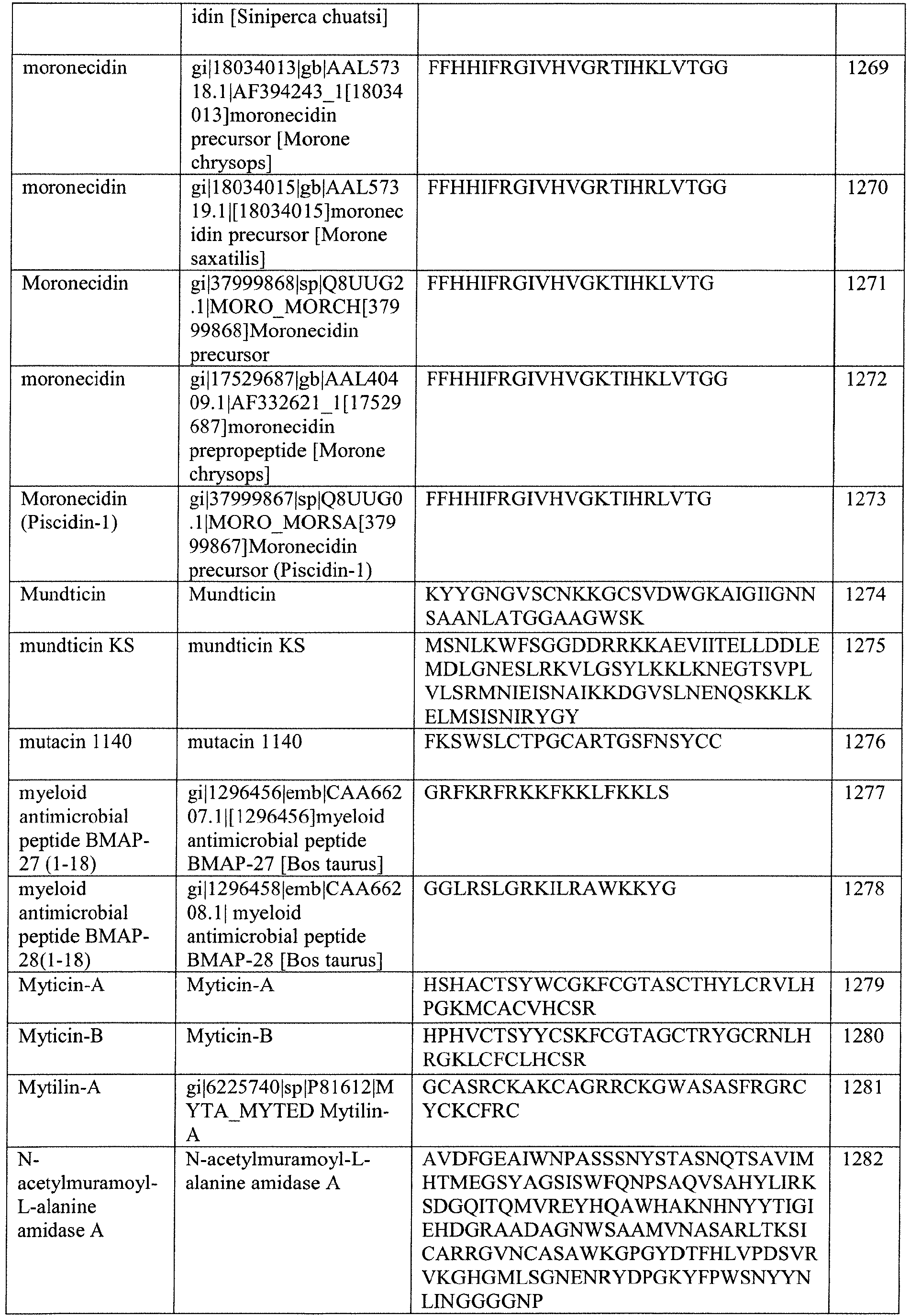

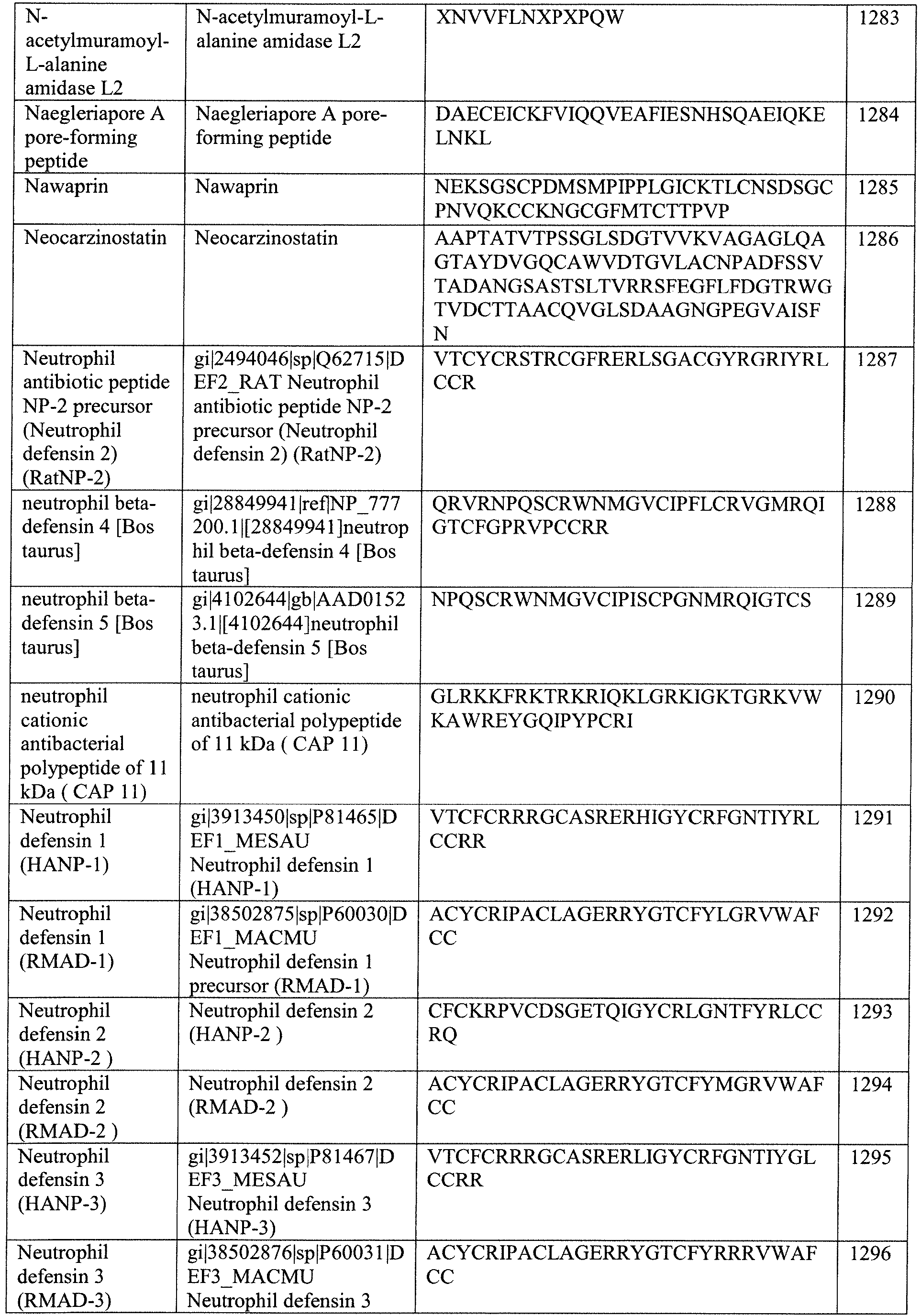

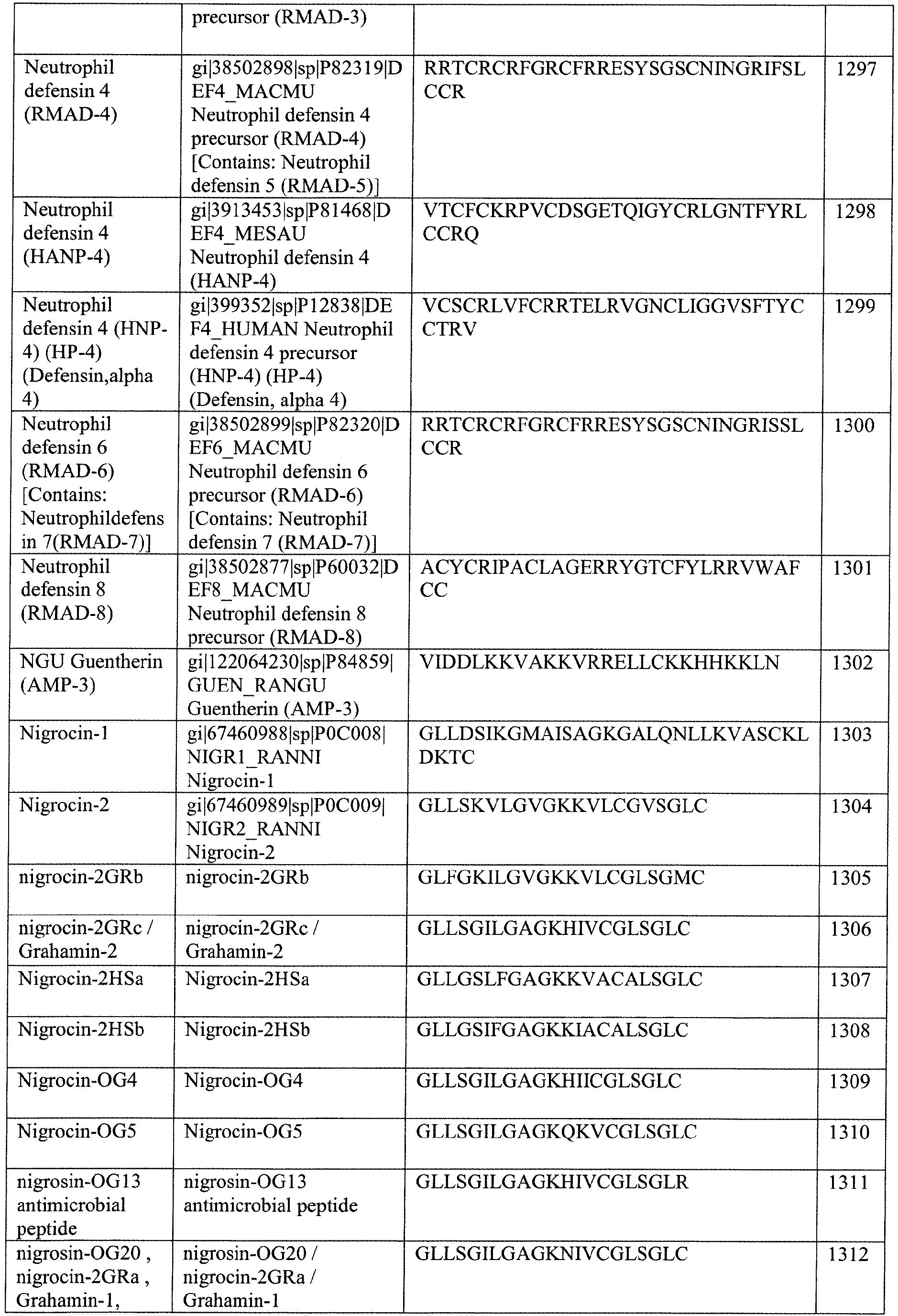

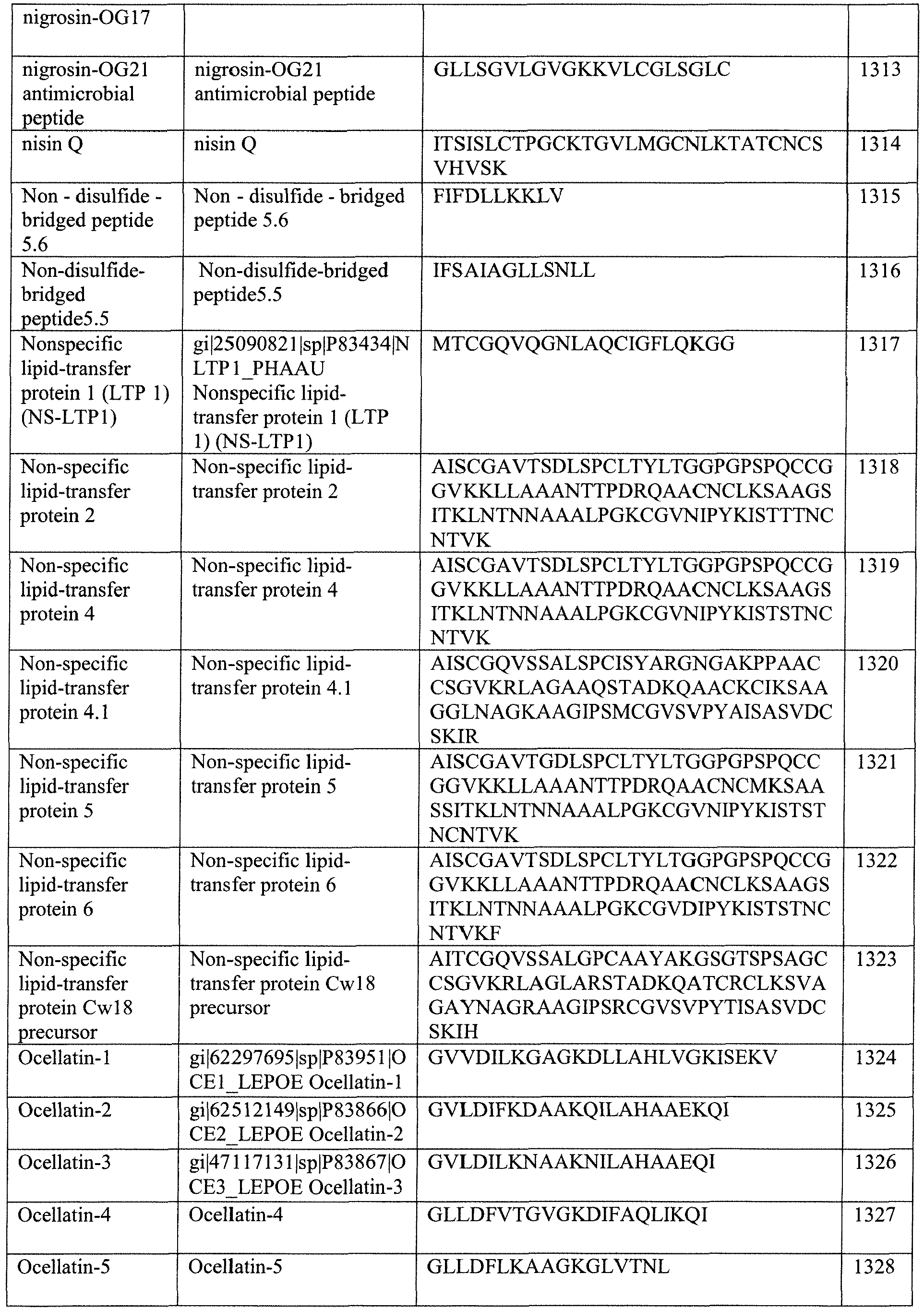

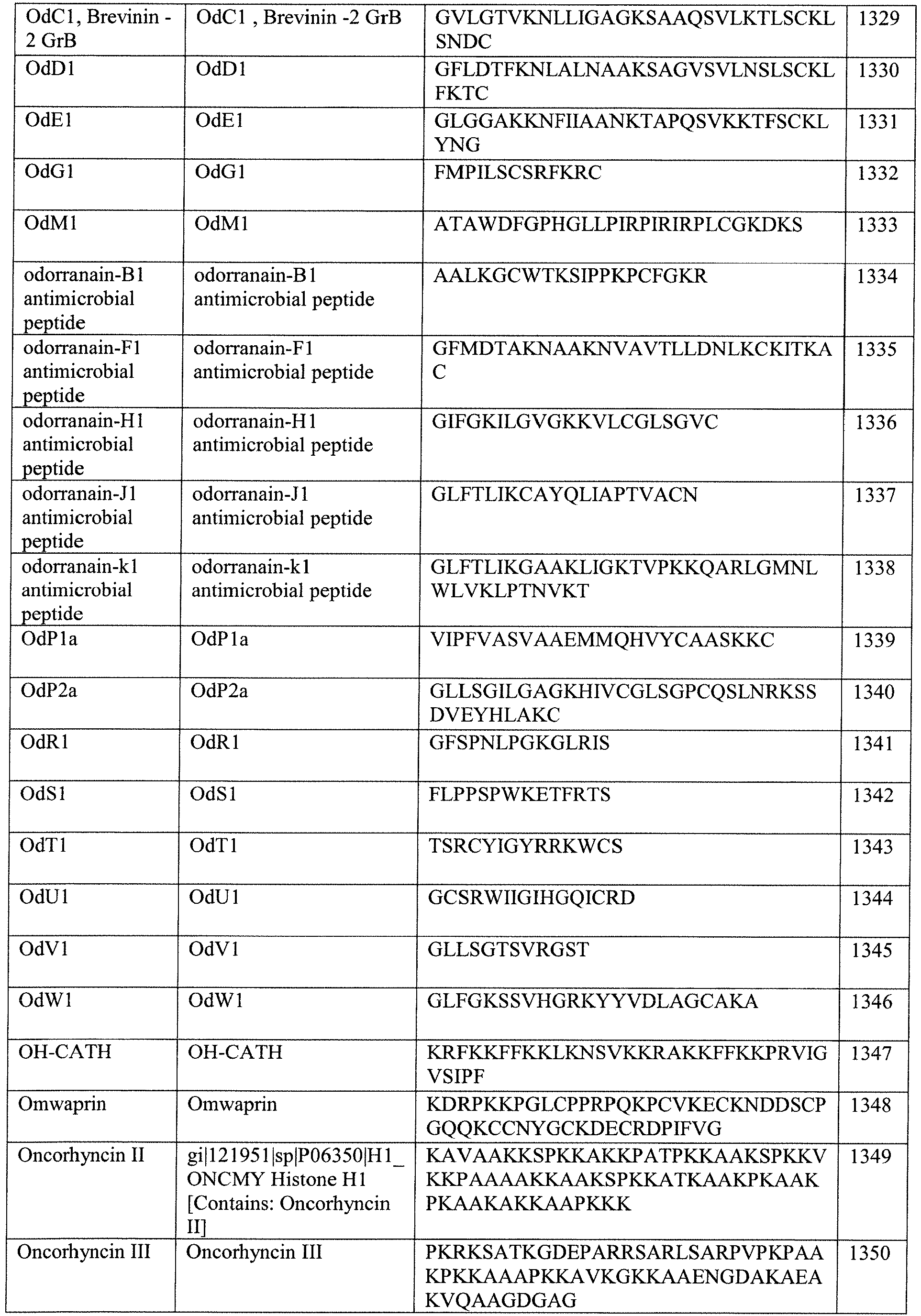

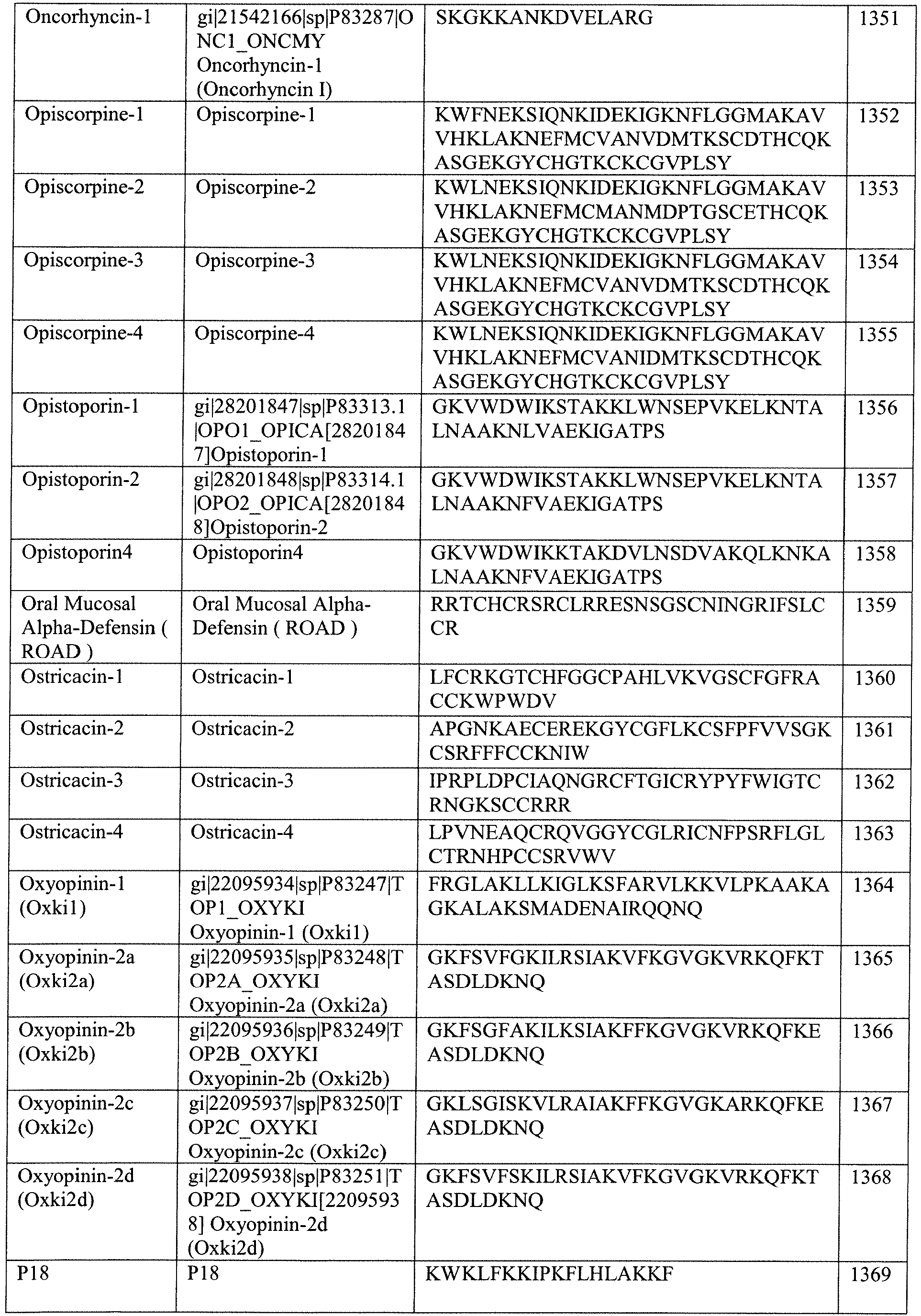

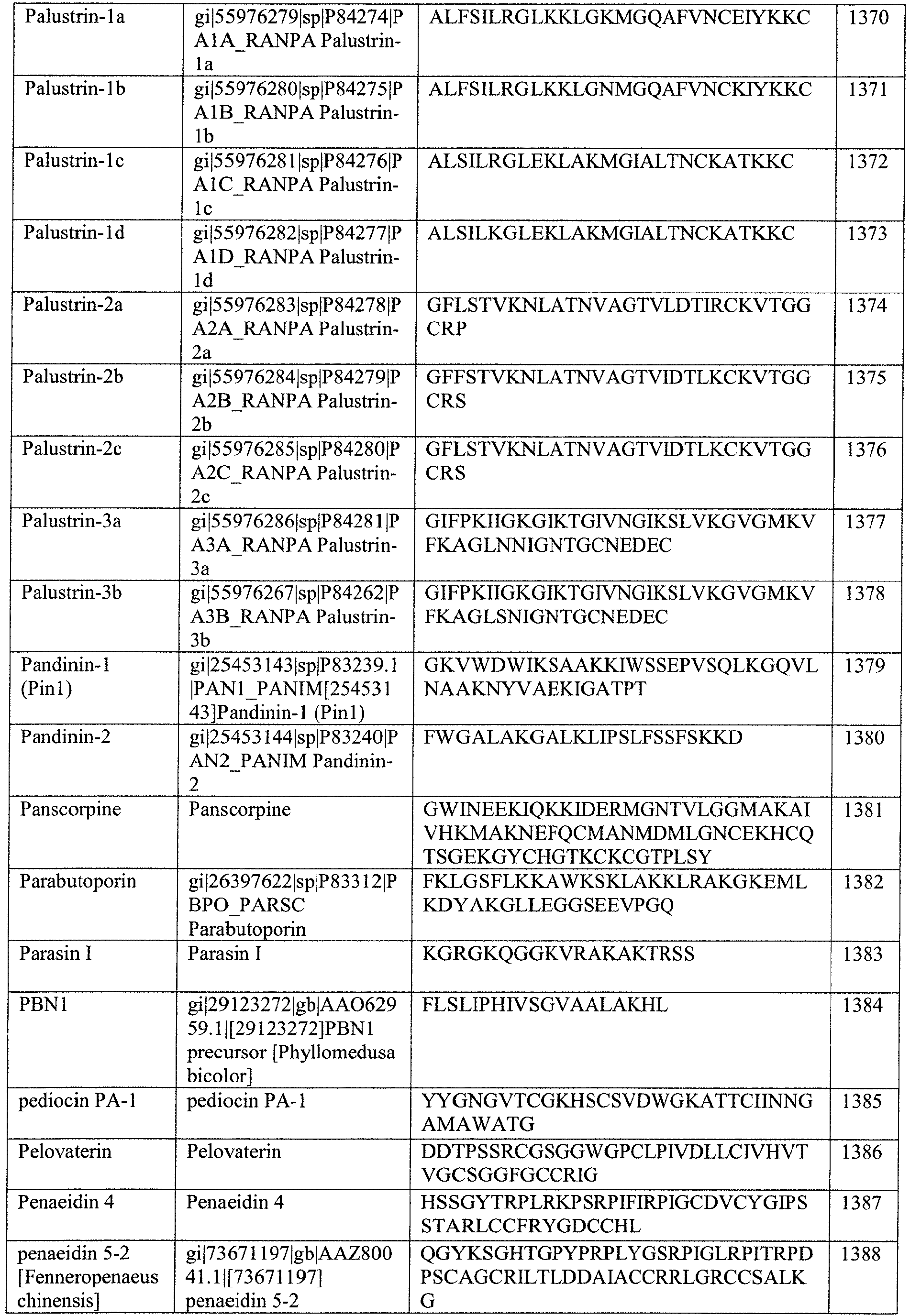

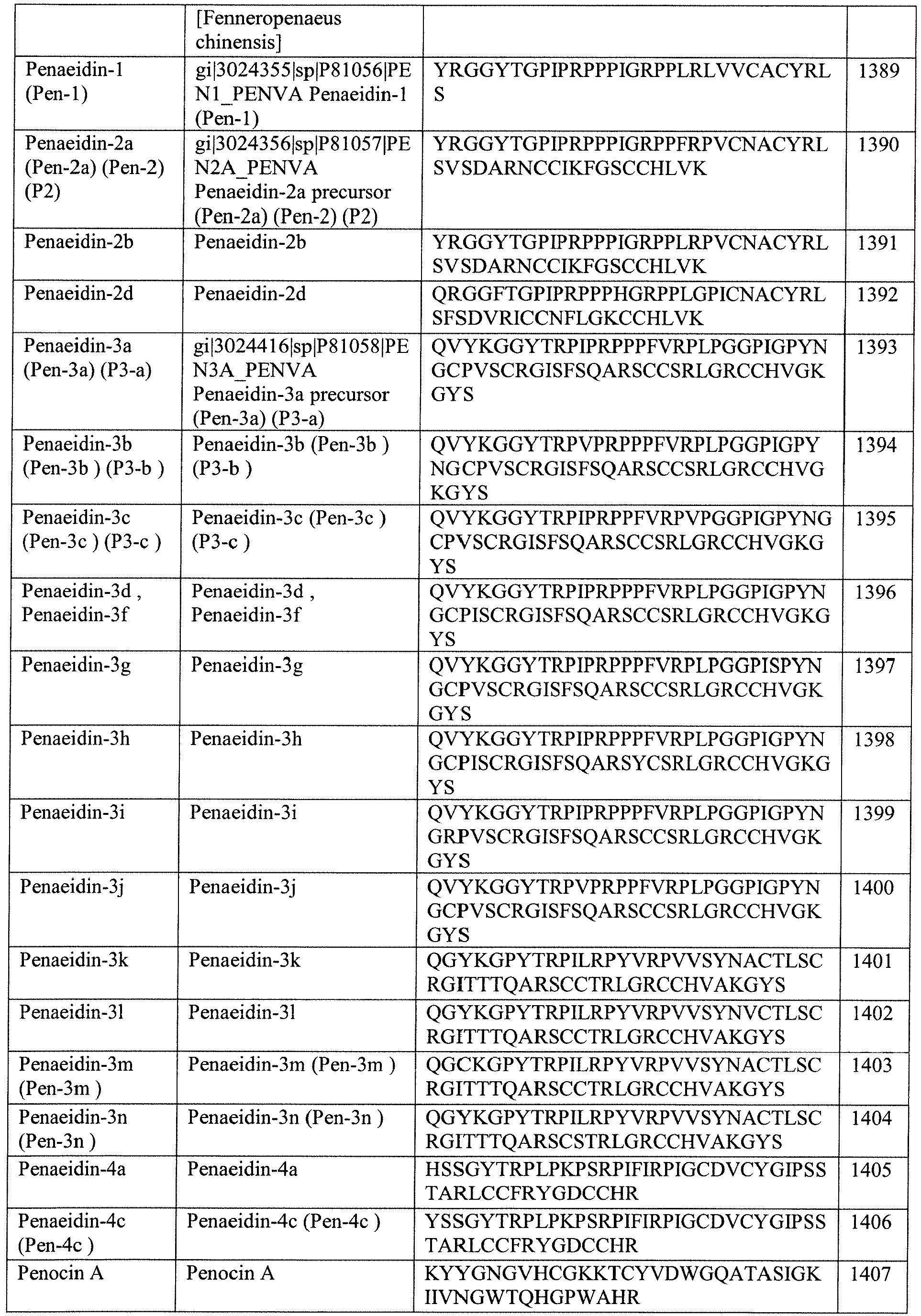

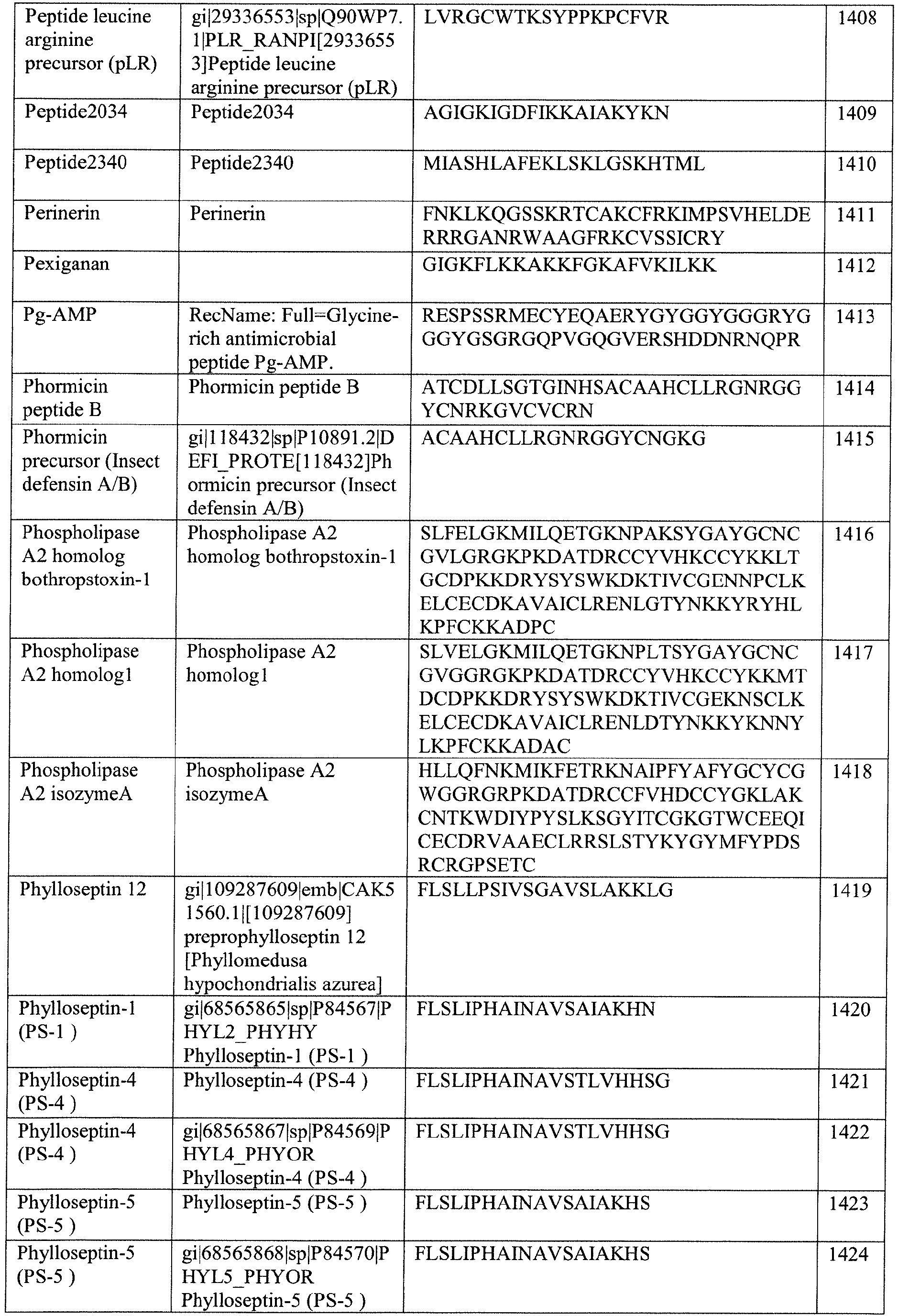

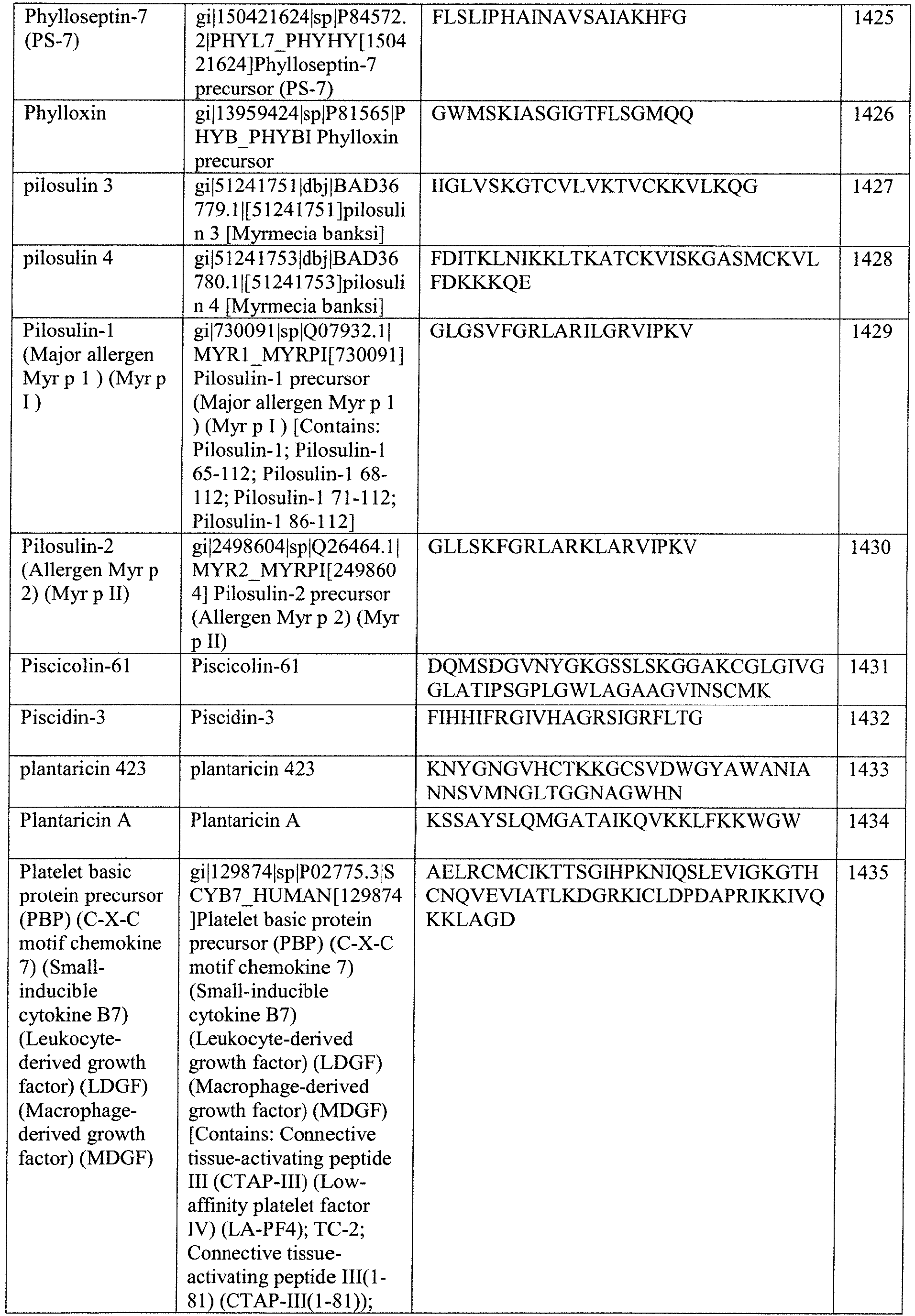

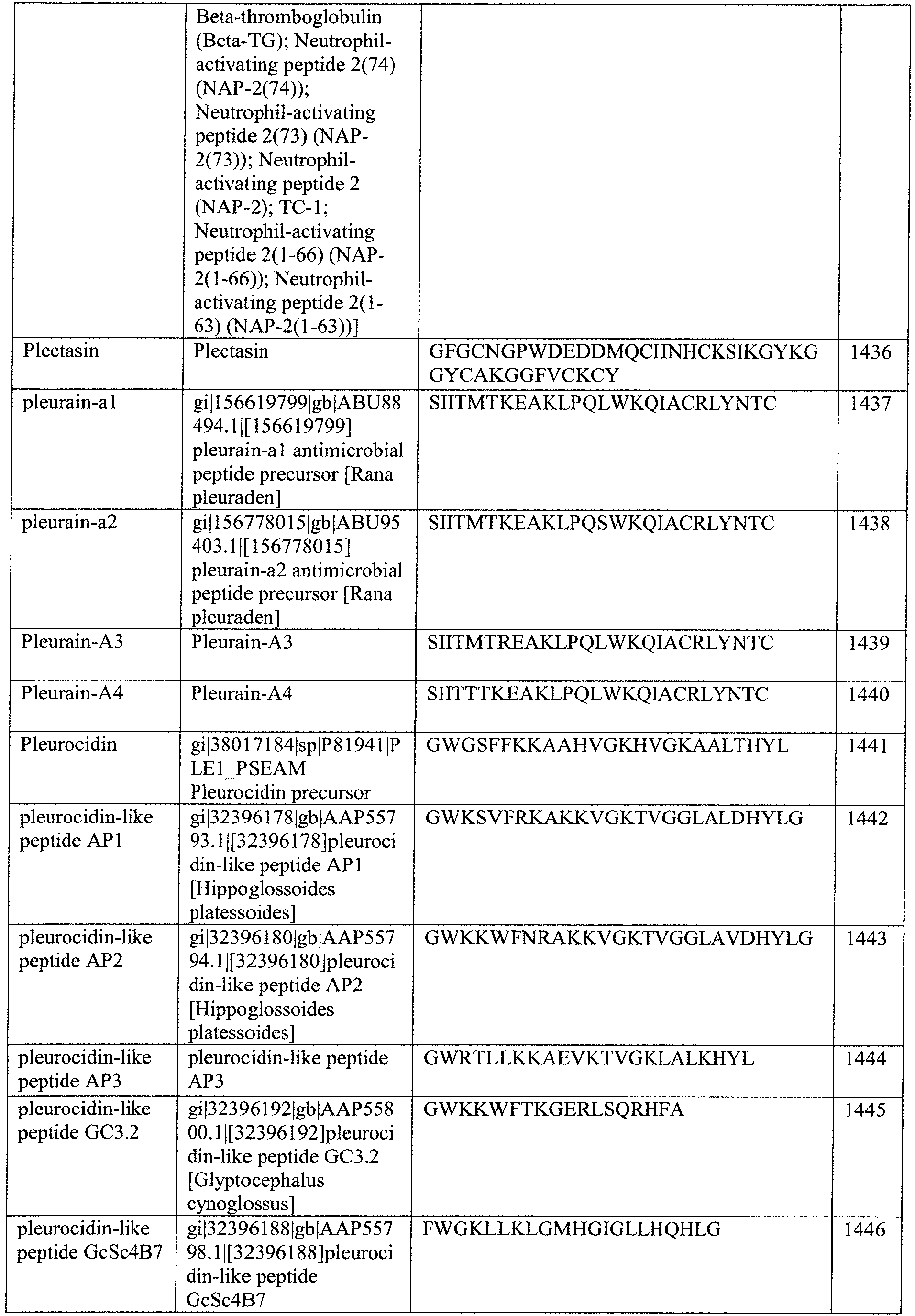

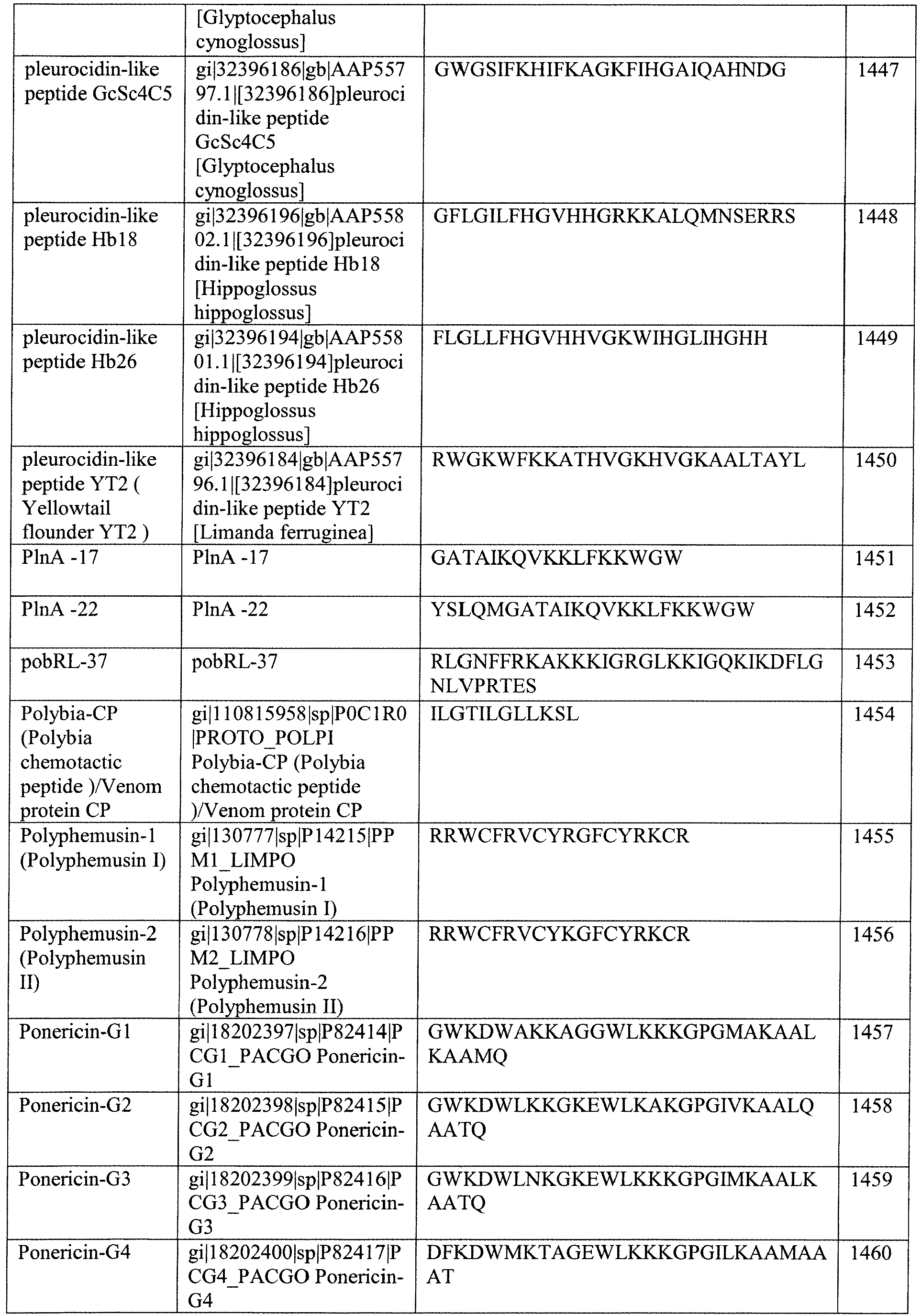

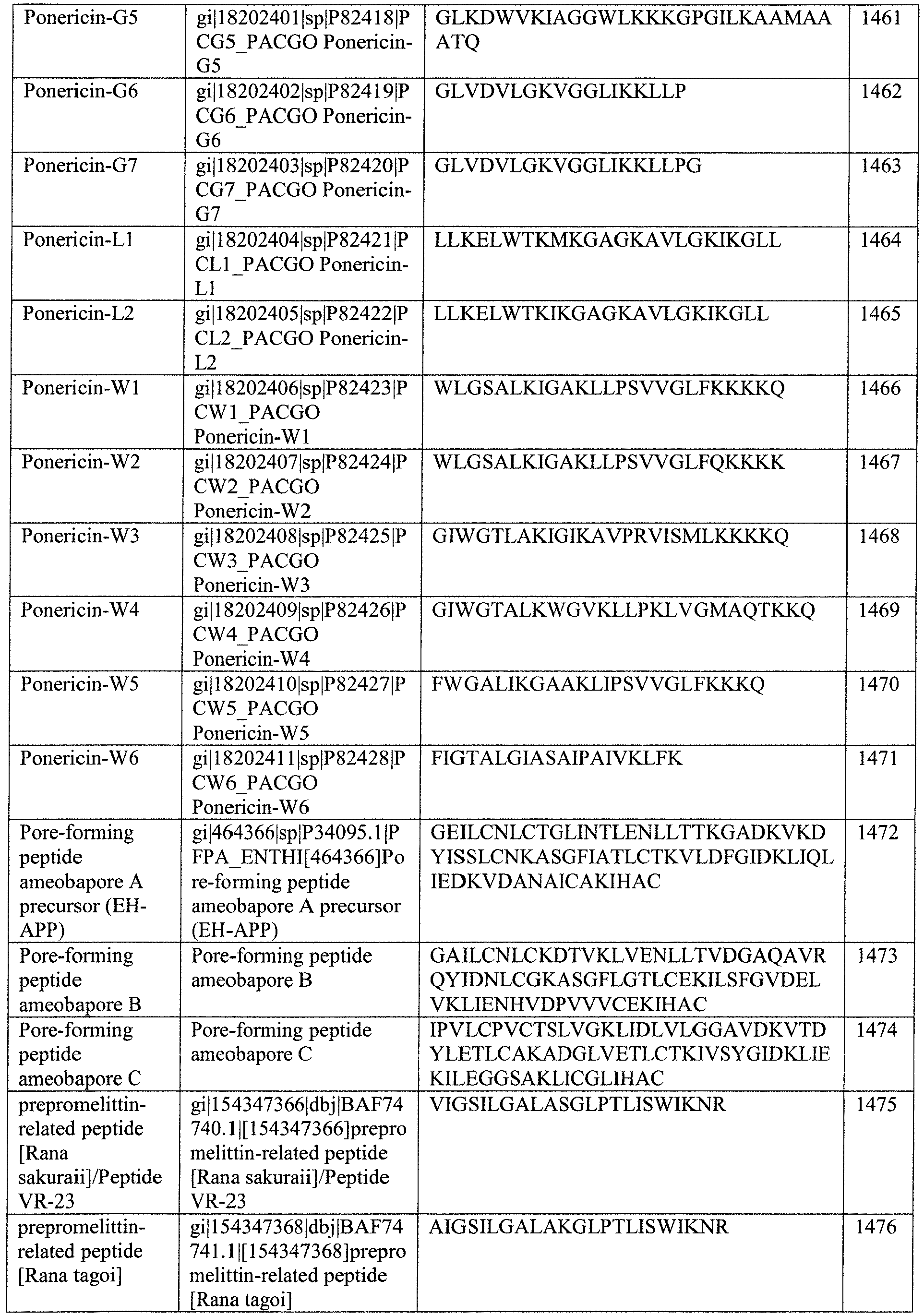

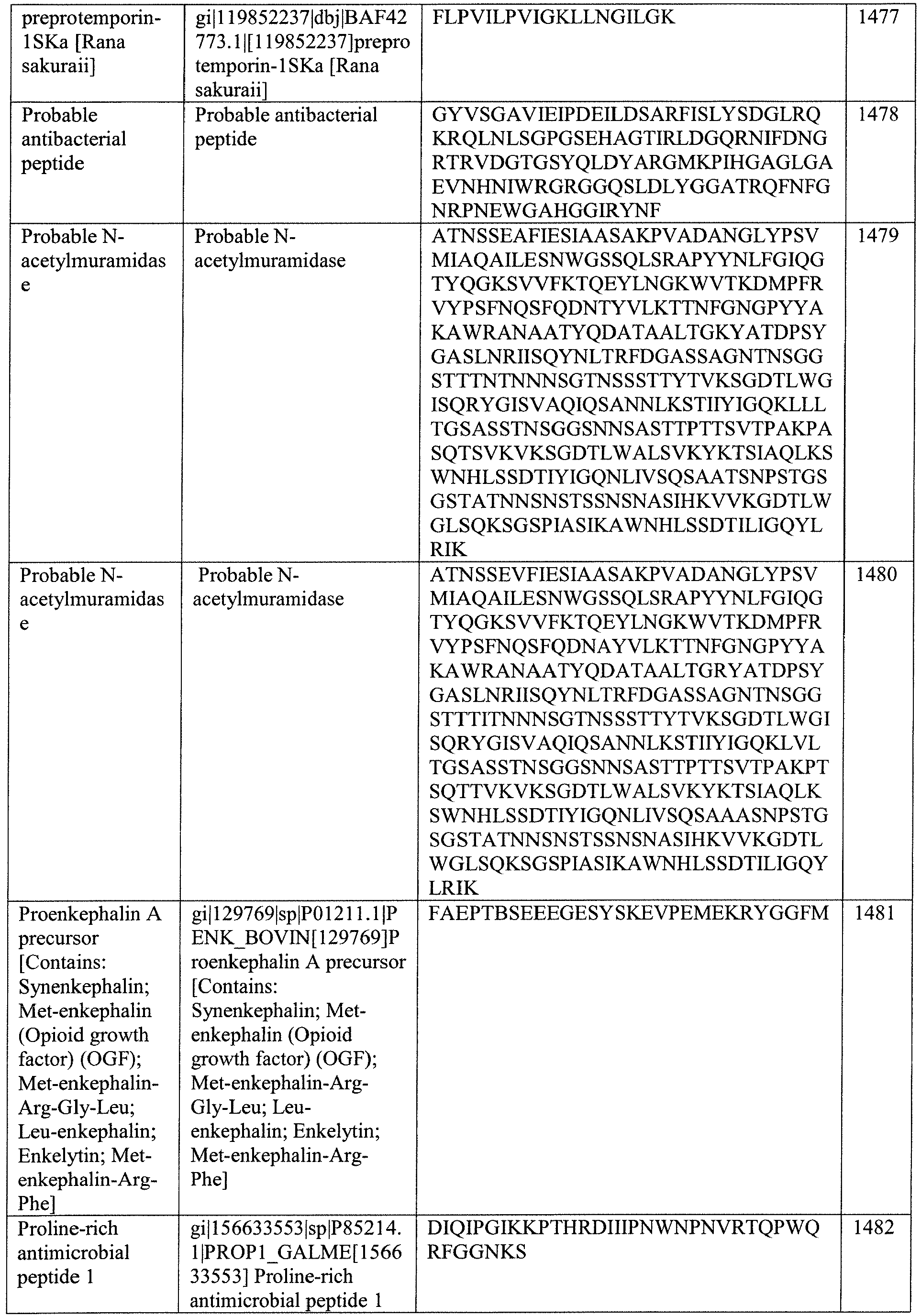

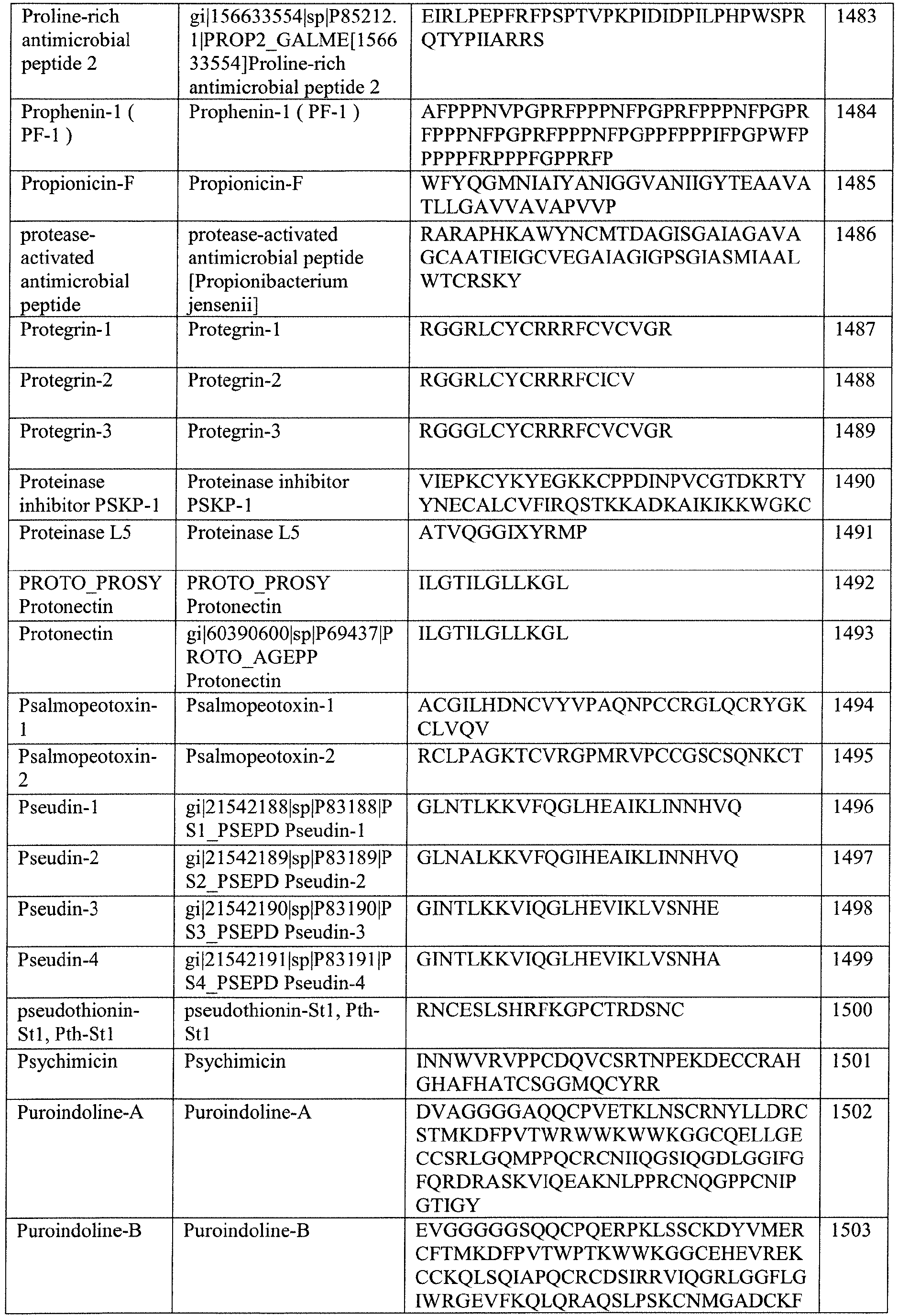

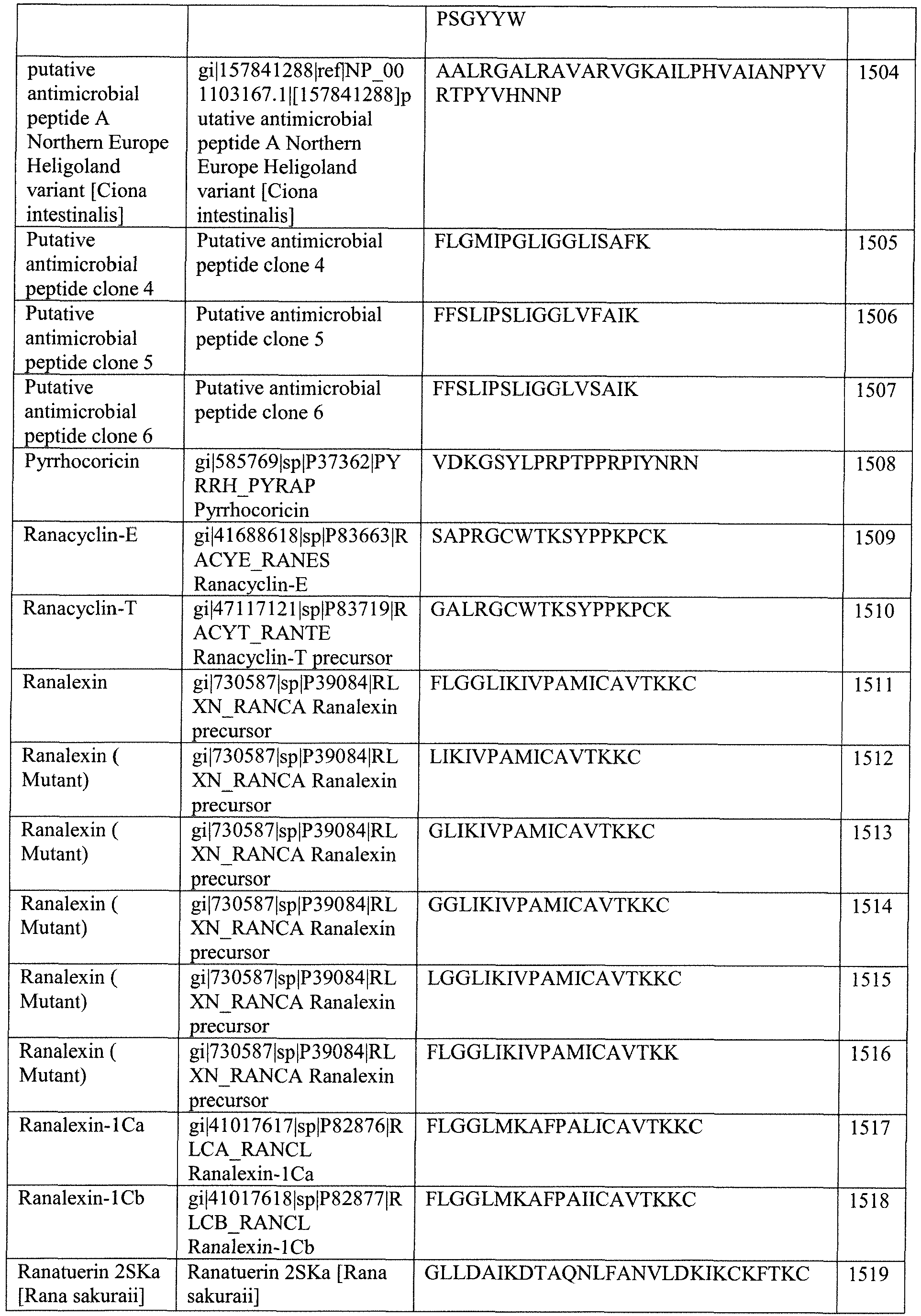

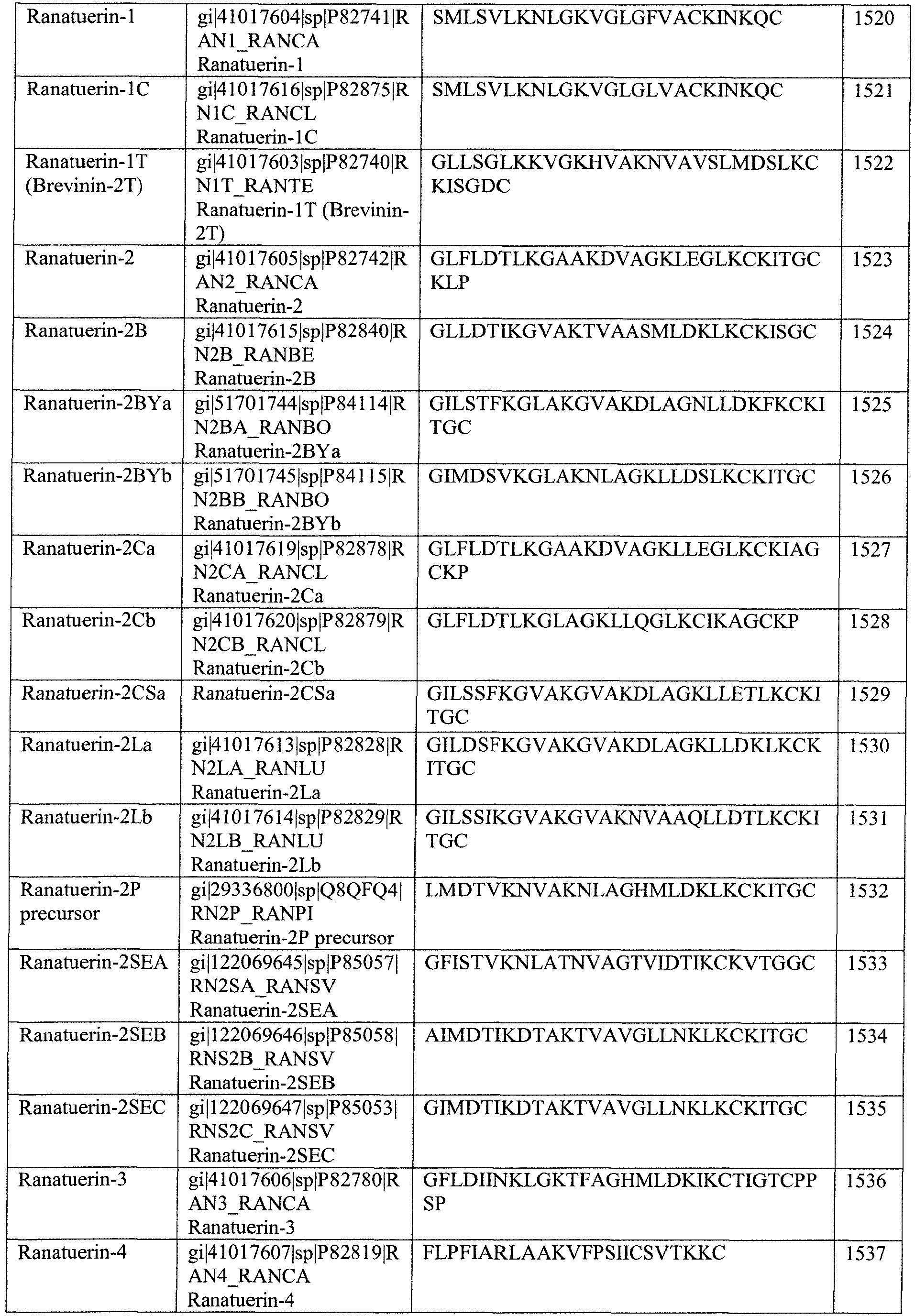

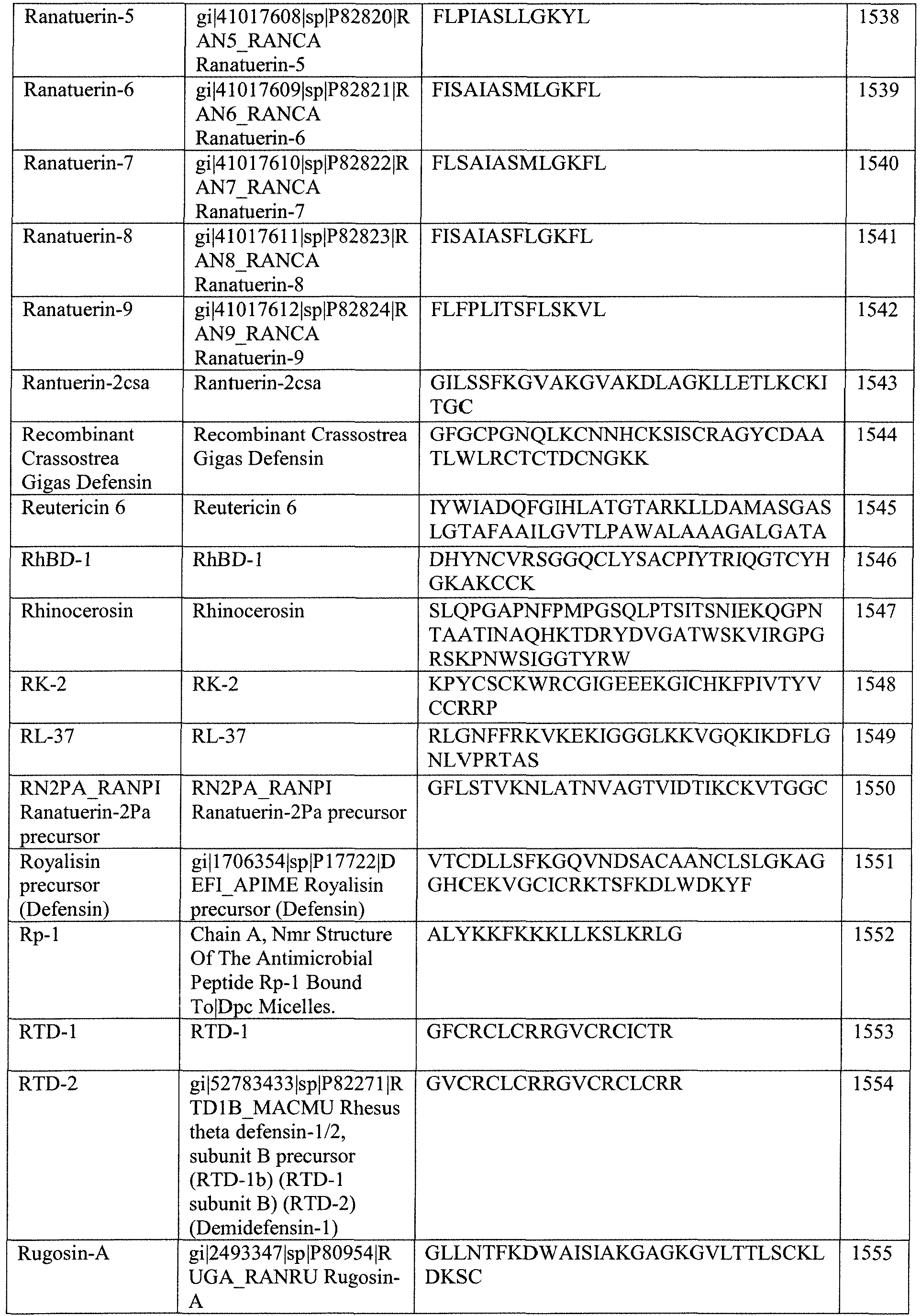

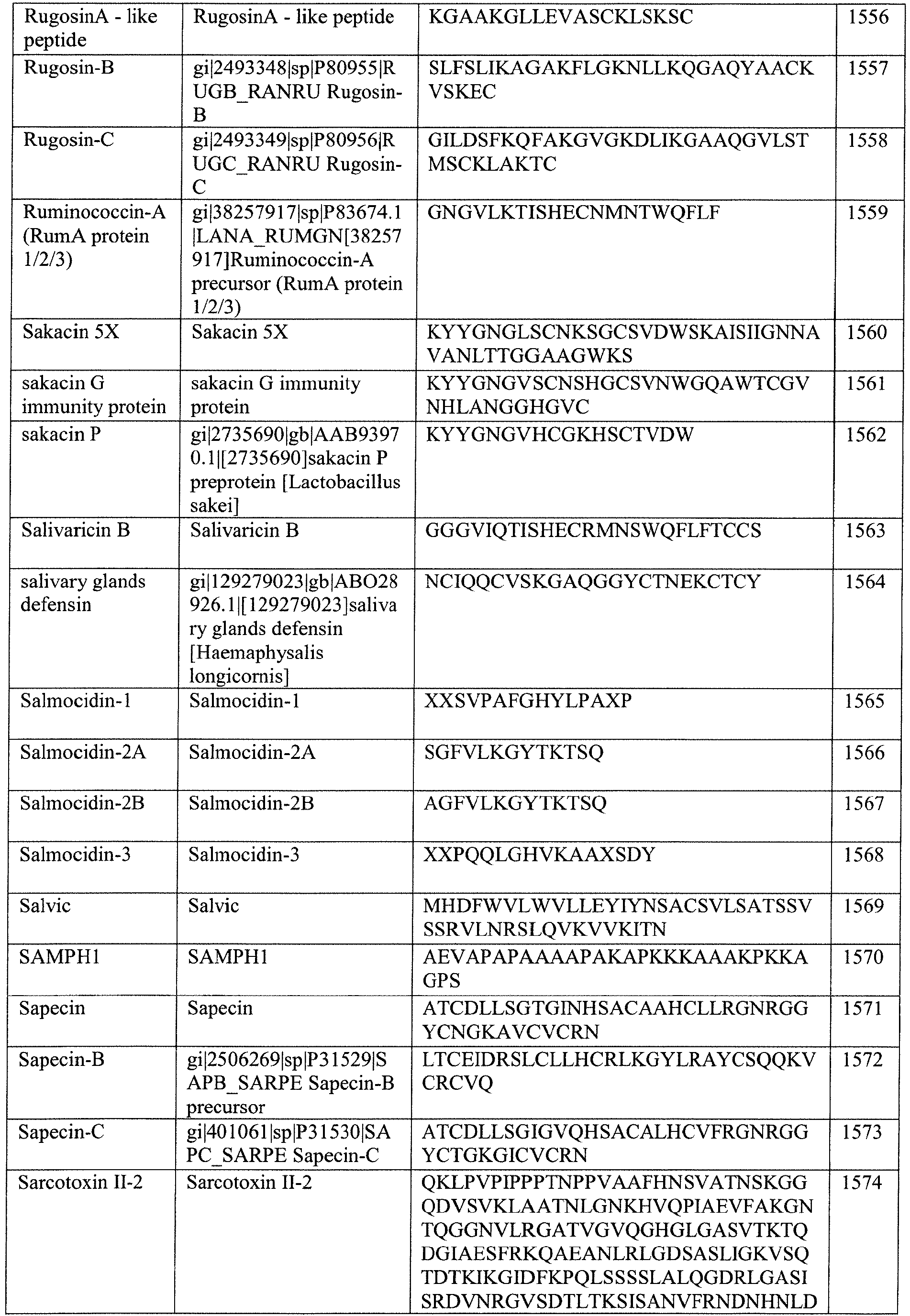

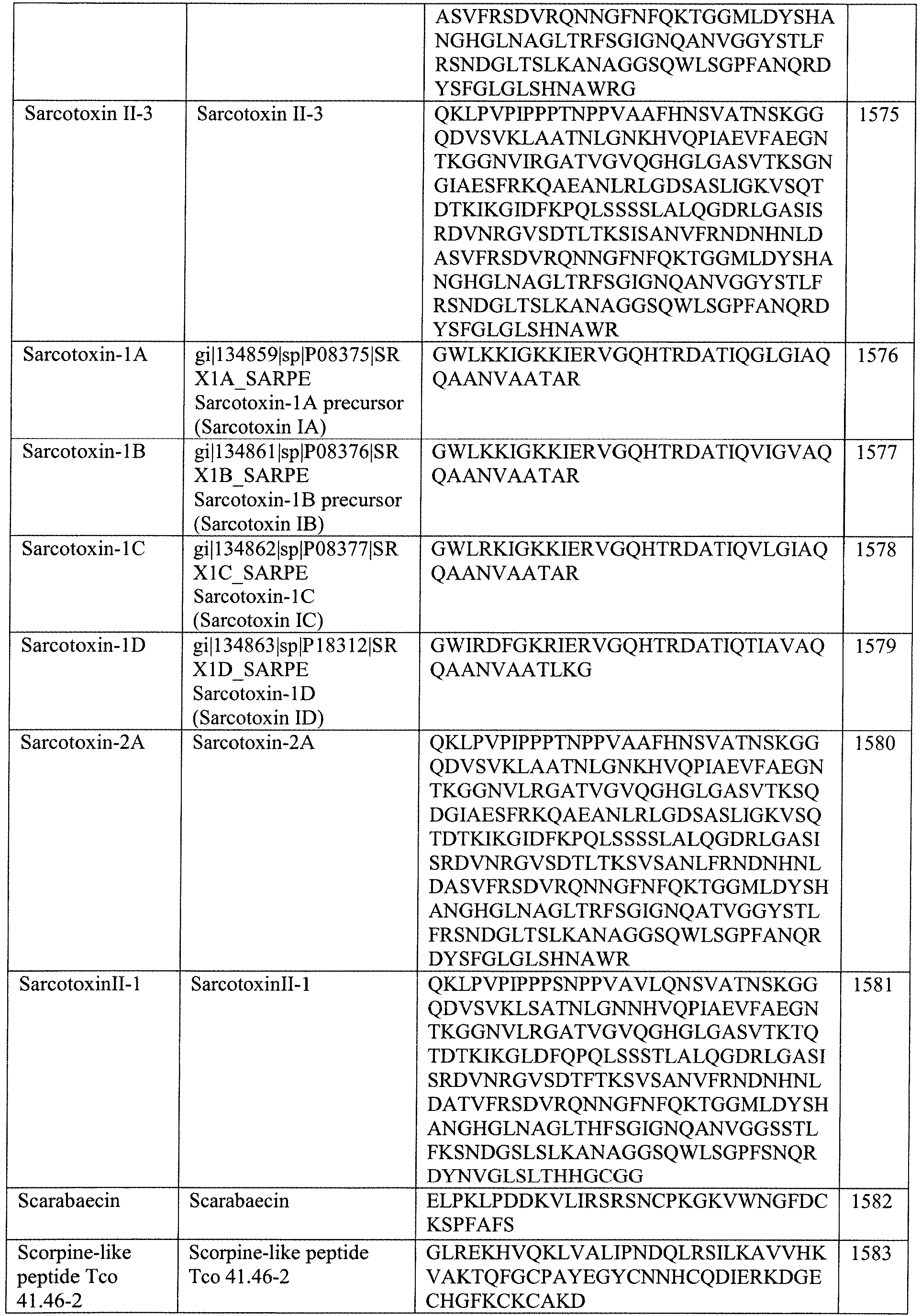

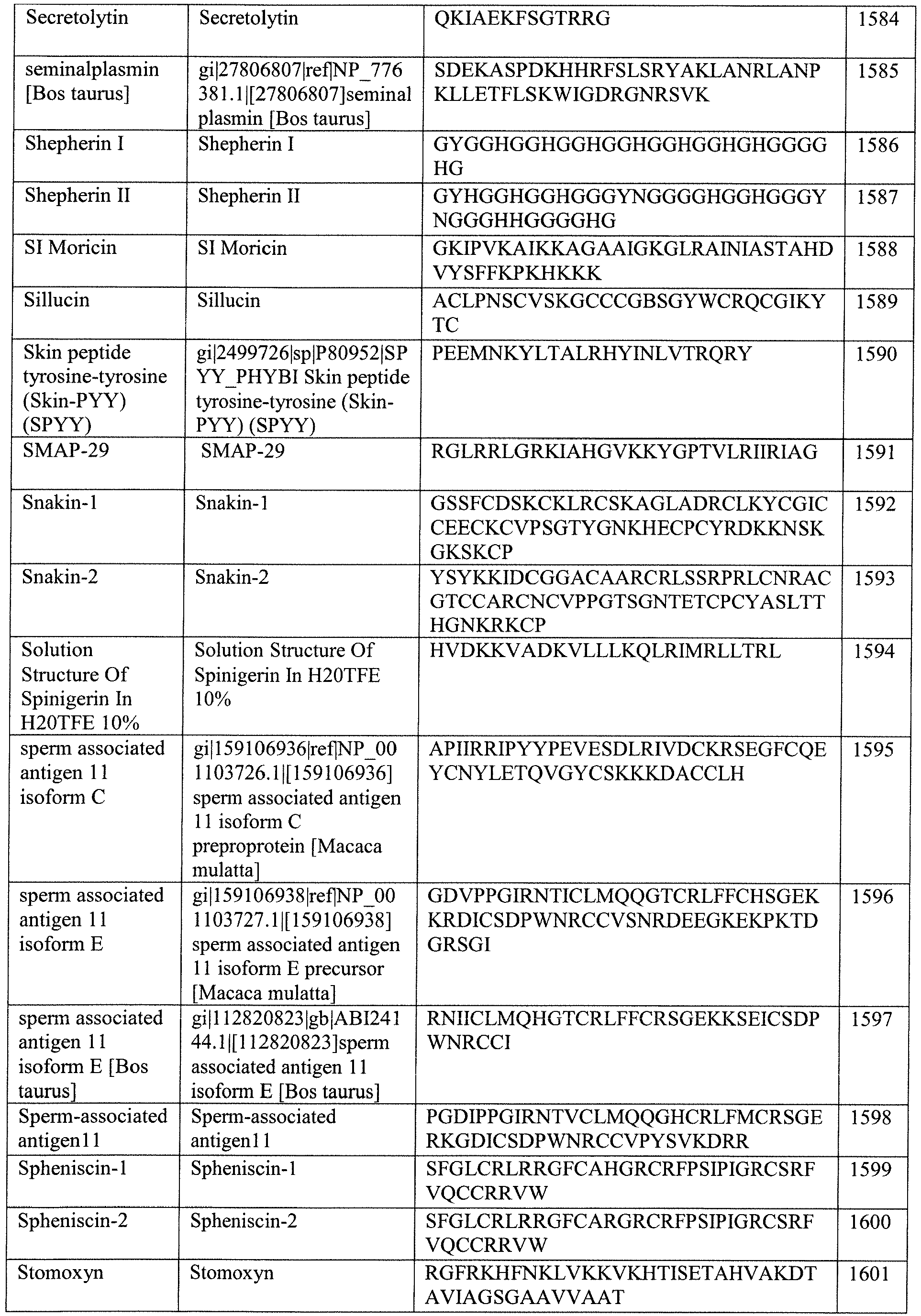

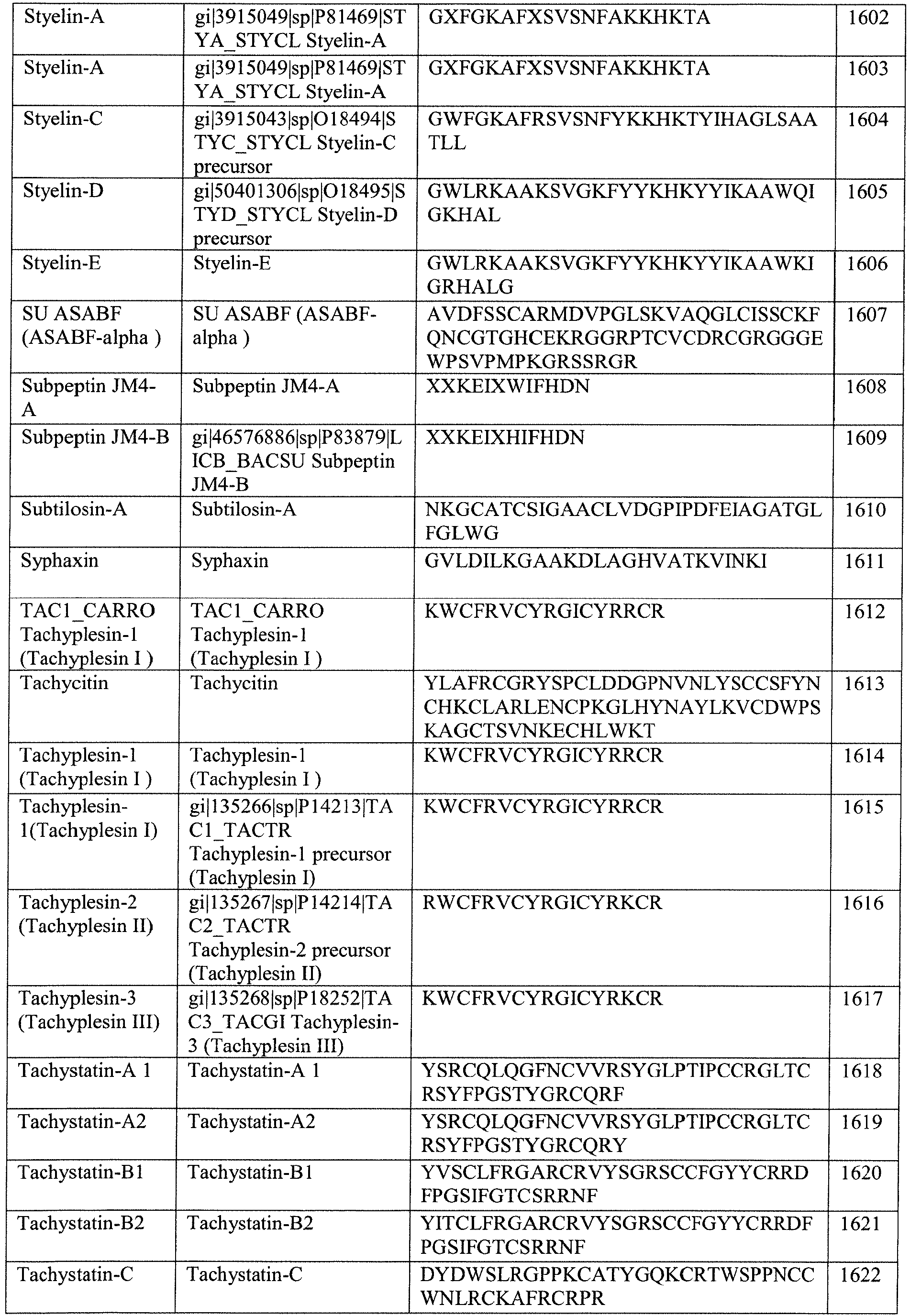

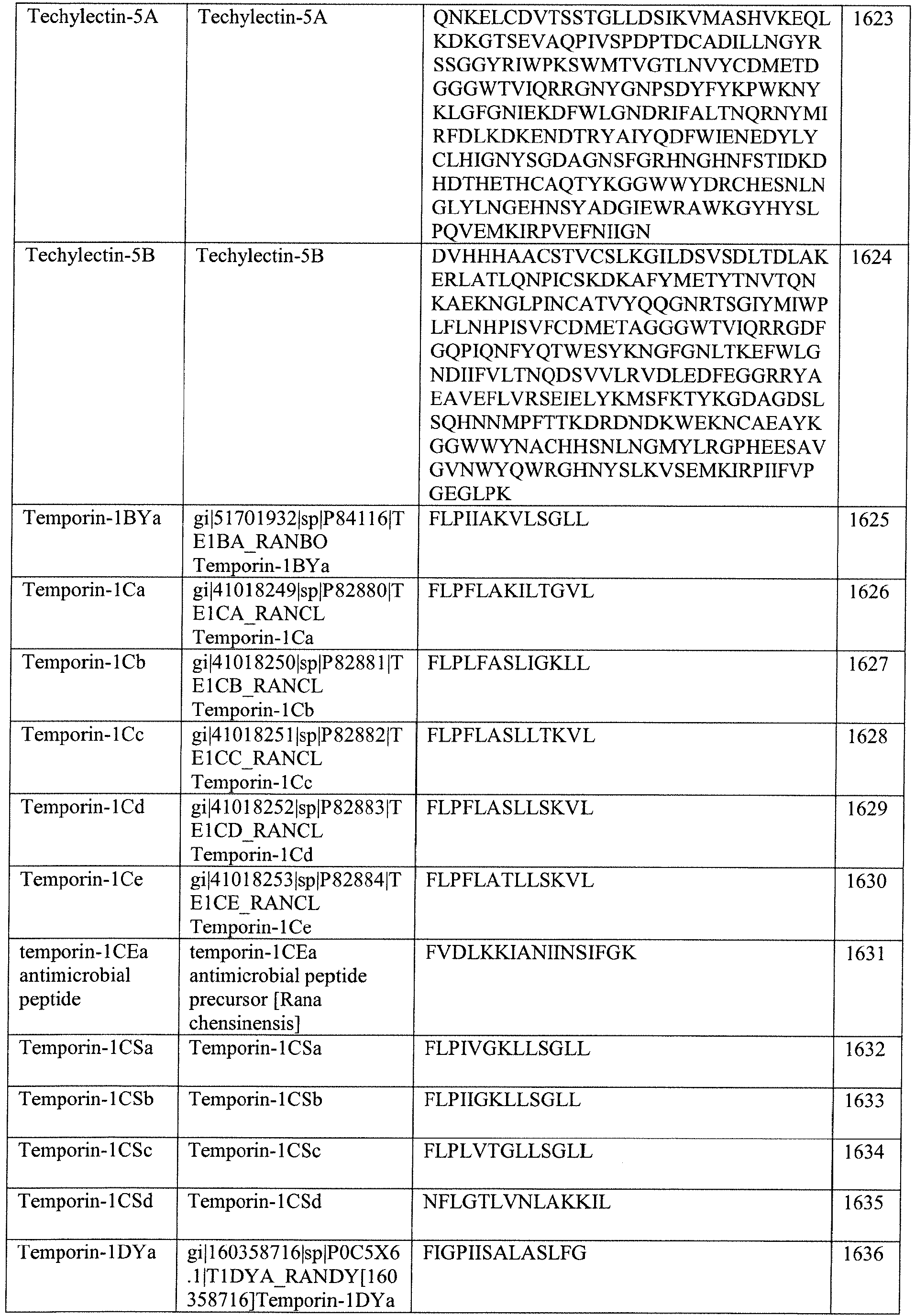

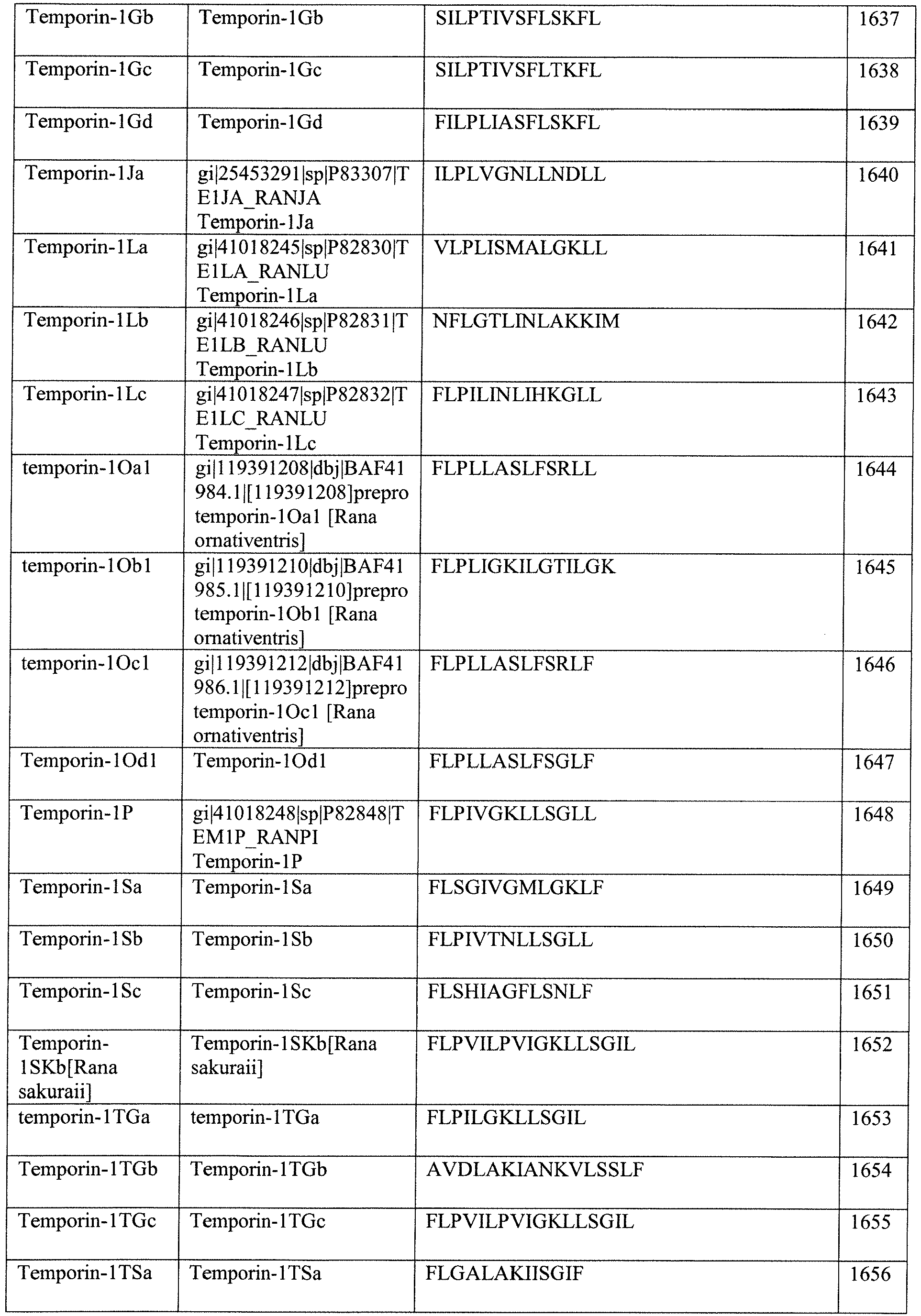

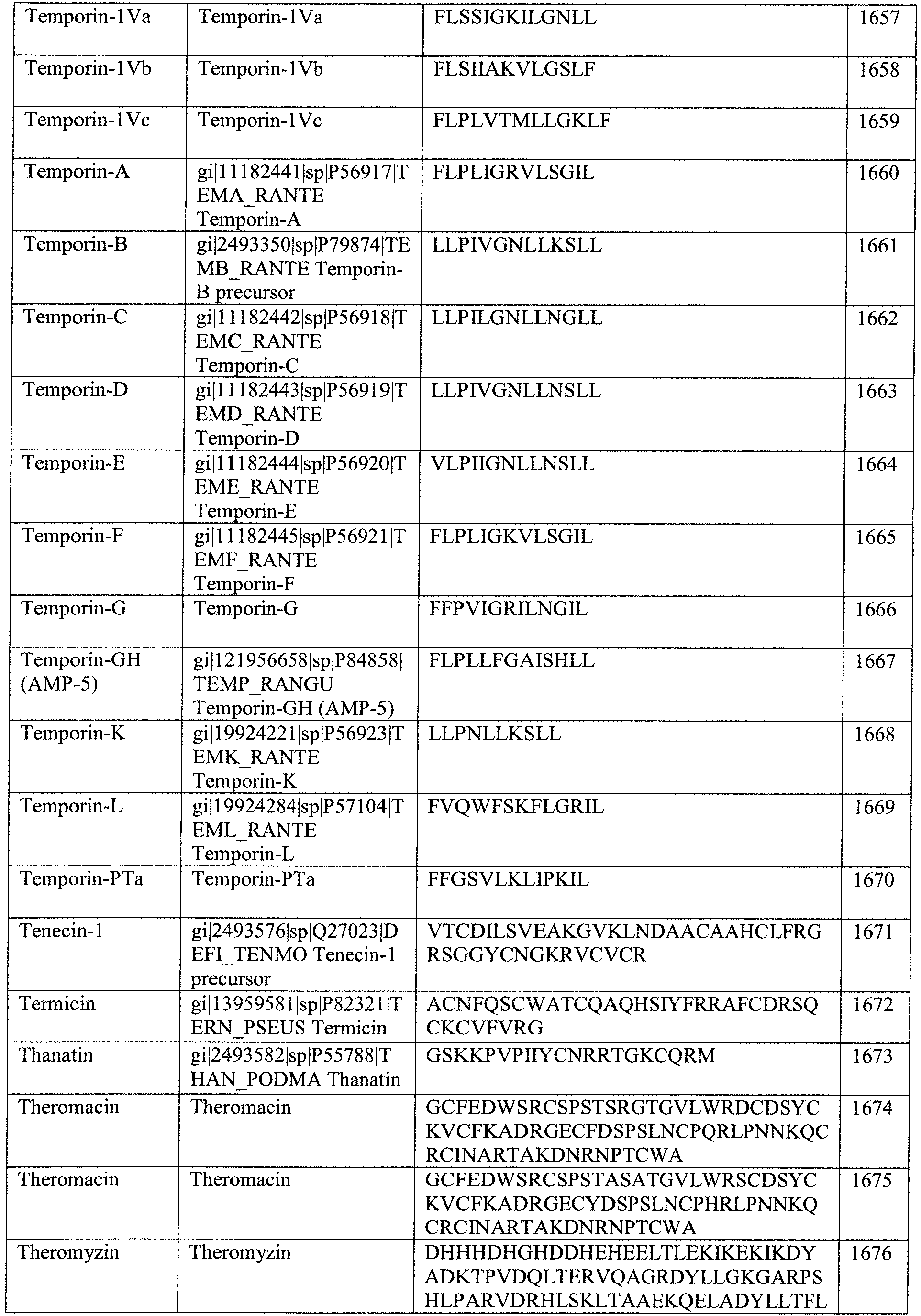

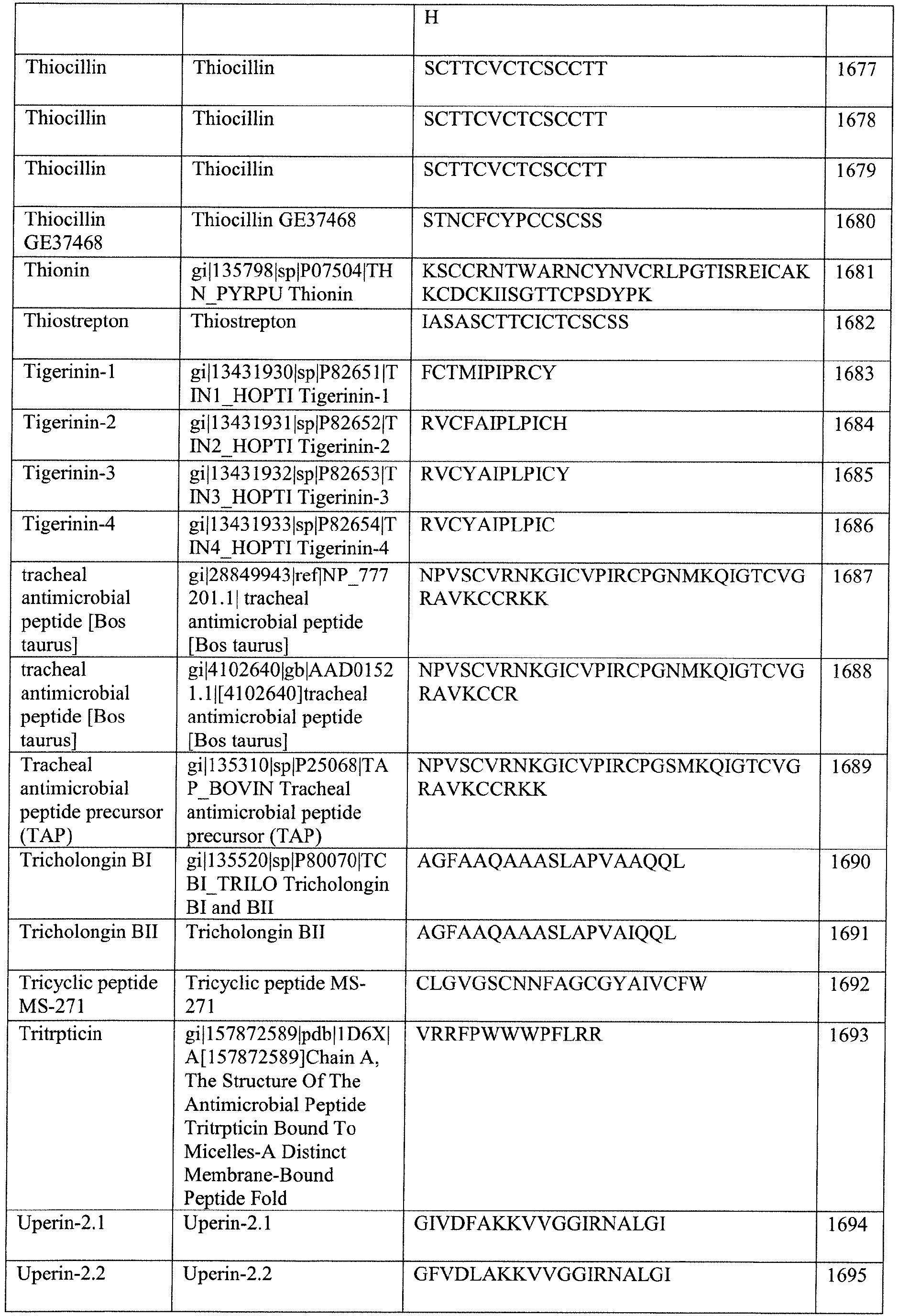

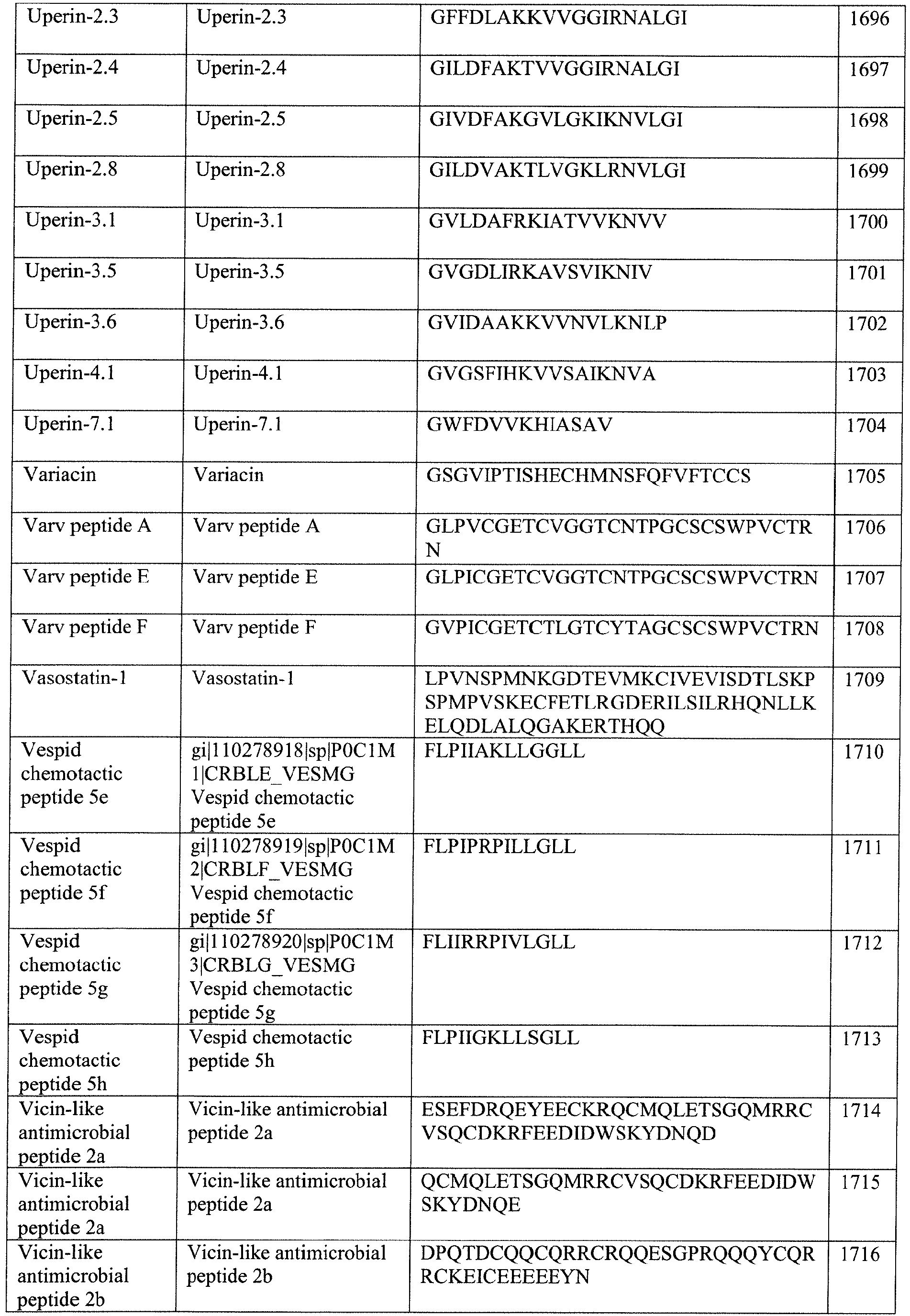

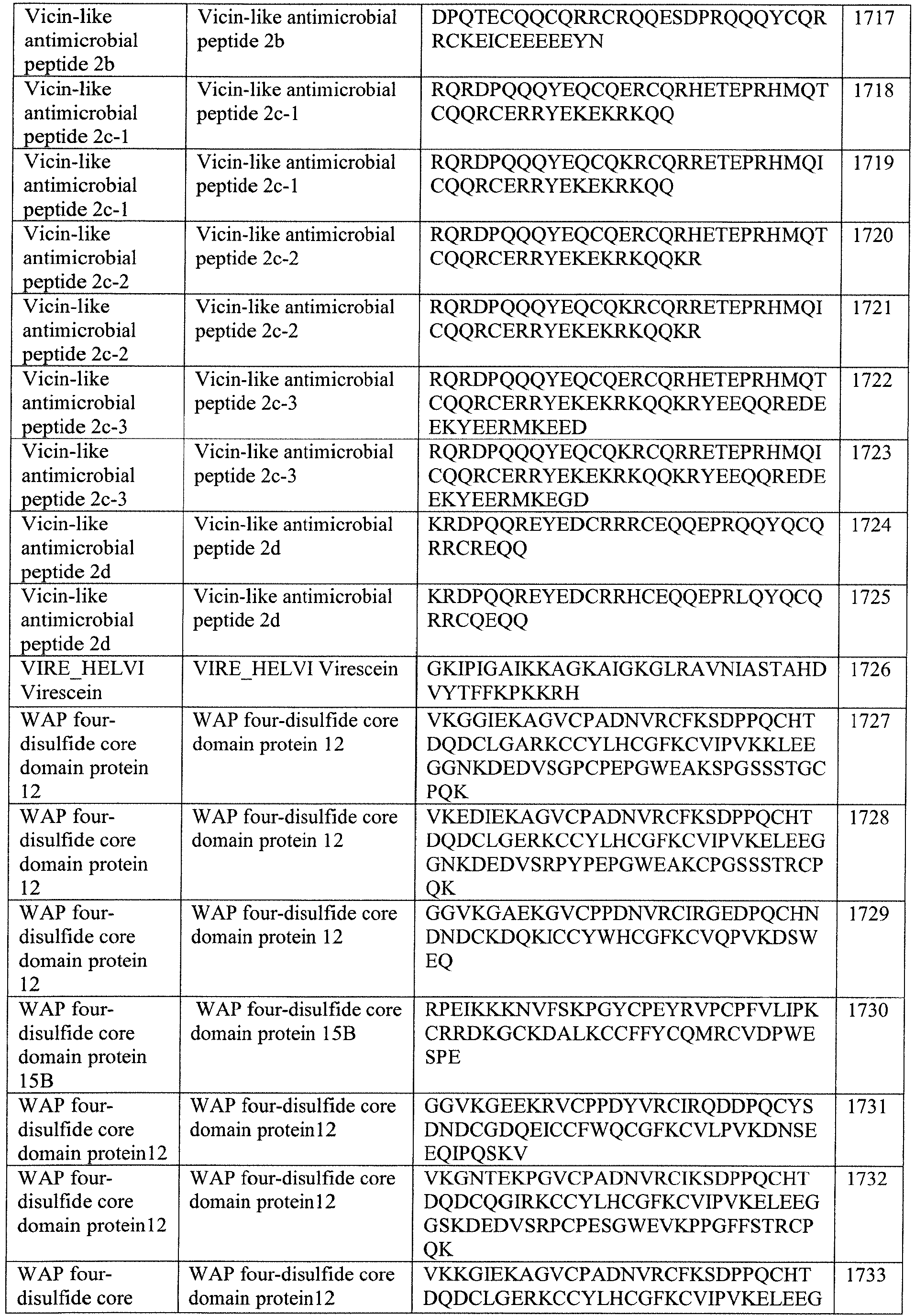

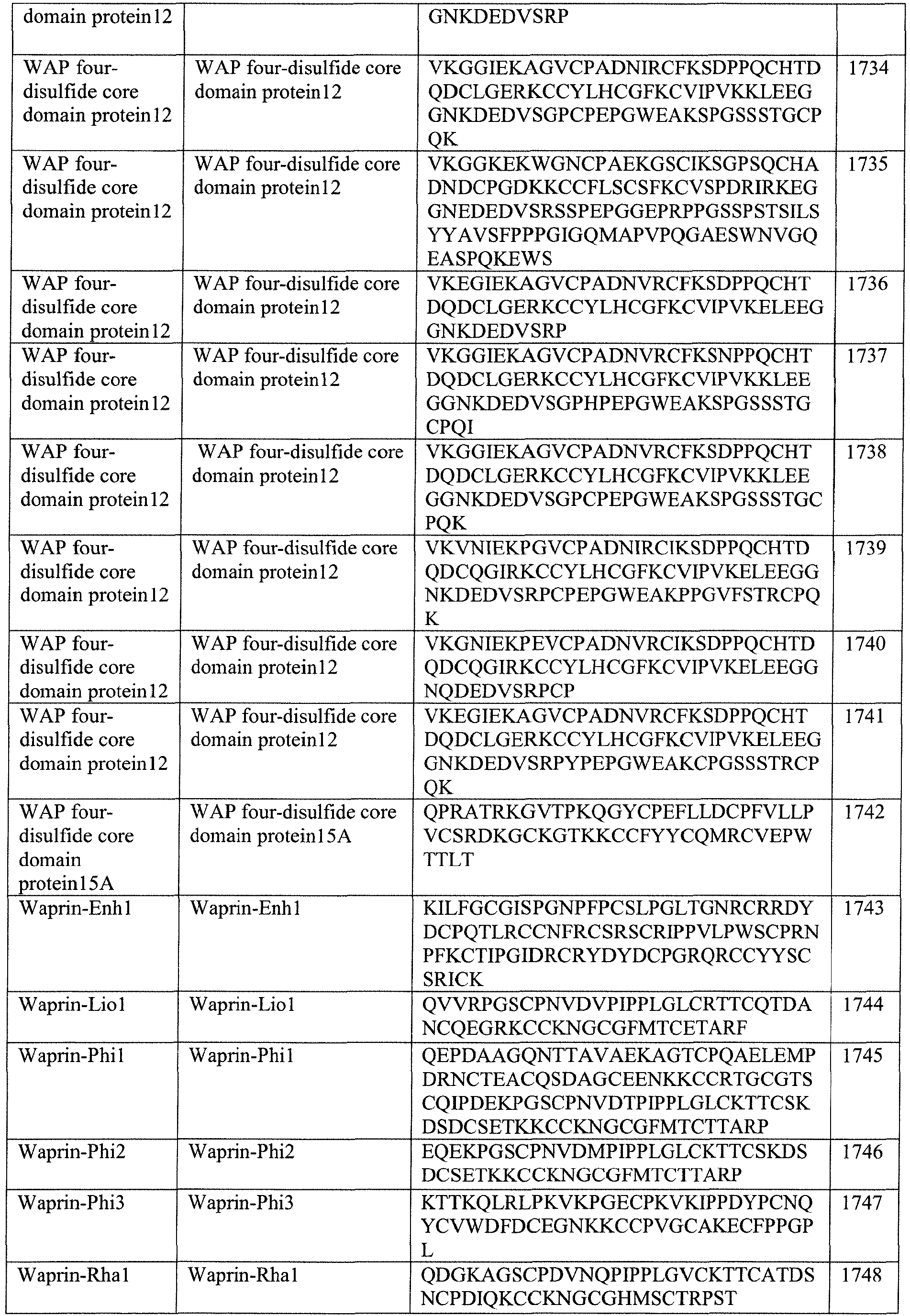

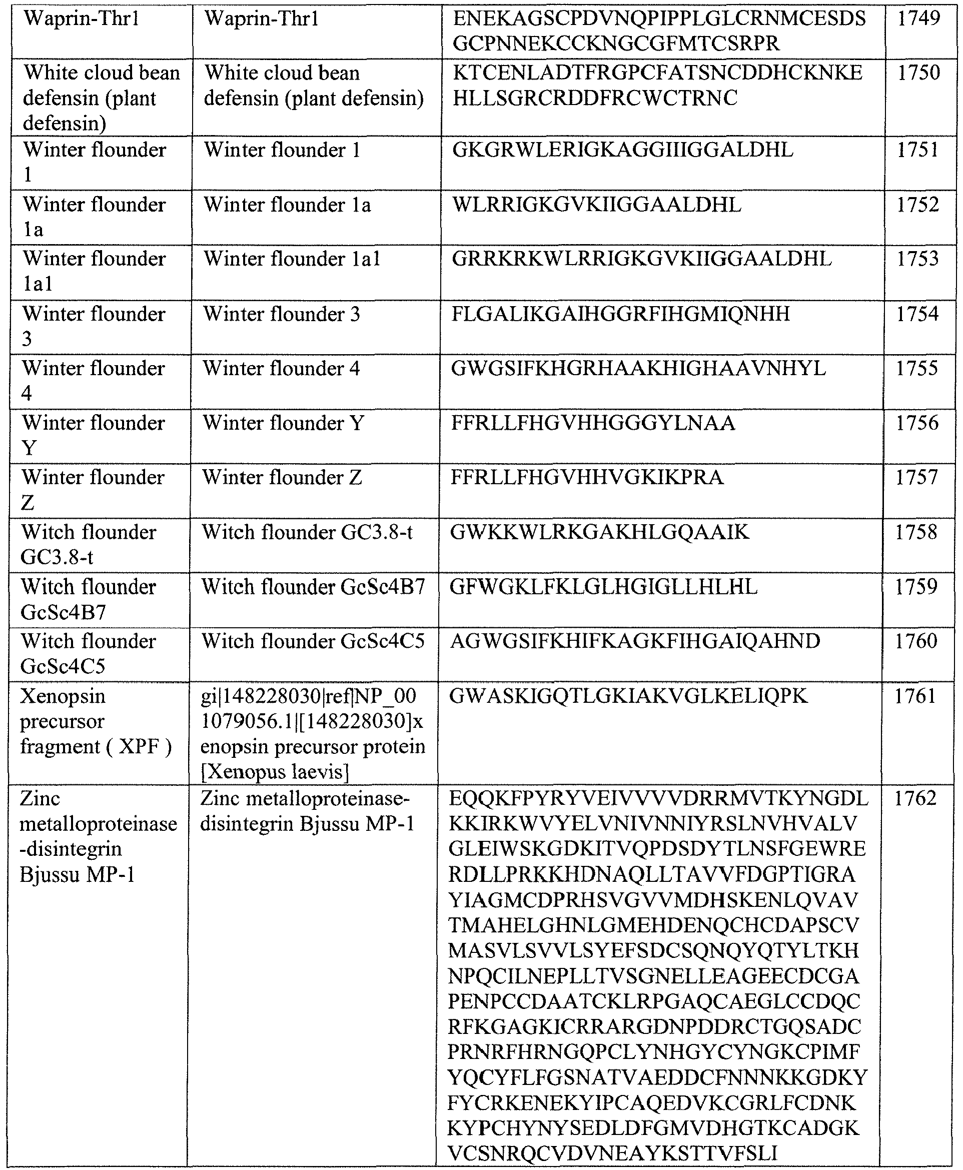

The present disclosure also comprises the following items:
1. A phannaceutical composition comprising:
   i) an effective amount of a synthetic messenger ribonucleic acid (mRNA) encoding an anti-viral polypeptide (AVP); and
   ii) a pharmaceutically acceptable carrier,
   wherein the synthetic mRNA comprises at least one nucleoside modification, and wherein the anti-viral polypeptide is about 6 to about 100 amino acids in length.
2. The phannaceutical composition of item 1, wherein the anti-viral polypeptide is about 6 to about 75 amino acids in length.
3. The phannaceutical composition of item 1, wherein the anti-viral polypeptide is about 6 to about 50 amino acids in length.
4. The pharmaceutical composition of item 1, wherein the anti-viral polypeptide is 15 to about 45 amino acids in length.
5. The phannaceutical composition of item 1, wherein the anti-viral polypeptide is substantially cationic and amphipathic.
6. The phannaceutical composition of item 1, wherein the anti-viral polypeptide is cytostatic or cytotoxic to a virus.
7. The phannaceutical composition of item 1, wherein the at least one nucleoside modification is selected from the group consisting of pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thiouridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.
8. The pharmaceutical composition of item 1, wherein the composition is suitable for administration selected from the group consisting of, systemic, local, intravenous, topical, oral, administration via a dressing, administration via a bandage, rectal, vaginal, intramuscular, transarterial, intraperitoneal, intranasally, subcutaneously, endoscopically, transdermally and intrathecally.
9. The pharmaceutical composition of item 8, wherein the administration is intravenous.
10. The pharmaceutical composition of item 8, wherein the administration is repeated at least once.
11. The pharmaceutical composition of item 1, wherein the anti-viral polypeptide is a secreted polypeptide.
12. The pharmaceutical composition of item 1, wherein the anti-viral polypeptide is useful in a treatment of an infection by a viral pathogen selected from the group consisting of human immunodeficiency viruses 1 and 2 (HIV-1 and HIV-2), human T-cell leukemia viruses 1 and 2 (HTLV-1 and HTLV-2), respiratory syncytial virus (RSV), human papilloma virus (HPV), adenovirus, hepatitis B virus (HBV), hepatitis C virus (HCV), Epstein-Barr virus (EBV), varicella zoster virus (VZV), cytomegalovirus (CMV), herpes simplex viruses 1 and 2 (HSV-1 and HSV-2), human herpes virus 8 (HHV-8), Yellow Fever virus, Dengue virus, Japanese Encephalitis and West Nile viruses.
13. The pharmaceutical composition of item 1, wherein the anti-viral polypeptide is selected from the group consisting of SEQ ID NOs: 1-1762.
14. The pharmaceutical composition of item 1, further comprising a lipid-based transfection reagent.
15. A method to treat a viral infection, comprising administering to a subject the pharmaceutical composition of item 1.
16. The method of item 15 wherein the viral infection is caused by a viral pathogen selected from the group consisting of human immunodeficiency viruses 1 and 2 (HIV-1 and HIV-2), human T-cell leukemia viruses 1 and 2 (HTLV-1 and HTLV-2), respiratory syncytial virus (RSV), human papilloma virus (HPV), adenovirus, hepatitis B virus (HBV), hepatitis C virus (HCV), Epstein-Barr virus (EBV), varicella zoster virus (VZV), cytomegalovirus (CMV), herpes simplex viruses 1 and 2 (HSV-1 and HSV-2), human herpes virus 8 (HHV-8), Yellow Fever virus, Dengue virus, Japanese Encephalitis and West Nile viruses.
17. The method of item 15, wherein the subject is human.
18. The method of item 15, wherein the subject is a livestock animal.
19. The method of item 15, wherein the pharmaceutical composition is administered by a route selected from the group consisting of systemic, local, intravenous, topical, oral, administration via a dressing, administration via a bandage, rectal, vaginal, intramuscular, transarterial, intraperitoneal, intranasally, subcutaneously, endoscopically, transdermally and intrathecally.
20. The method of item 19, wherein the route is intravenous.
21. The method of item 19, wherein the administration is repeated at least once.
22. The method of item 15, further comprising administering an effective amount of a small molecule anti-viral compound to the subject at the same time or at a different time from the administration of the pharmaceutical composition.
23. A kit, comprising the pharmaceutical composition of item 1, and packaging and instructions for use thereof.

## Claims

1. An mRNA comprising (i) a translatable region encoding an anti-viral polypeptide and (ii) at least one nucleoside modification.

2. The mRNA of claim 1 wherein the anti-viral polypeptide consists of from about 6 to about 100 amino acids.

3. The mRNA of claim 2 wherein the anti-viral polypeptide consists of from about 15 to about 45 amino acids.

4. The mRNA of any of the preceding claims wherein the anti-viral polypeptide is substantially cationic.

5. The mRNA of any of the preceding claims wherein the anti-viral polypeptide is substantially amphipathic.

6. The mRNA of any of the preceding claims wherein the anti-viral polypeptide is cytostatic to a virus.

7. The mRNA of any of the preceding claims wherein the anti-viral polypeptide is cytotoxic to a virus.

8. The mRNA of any of the preceding claims wherein the anti-viral polypeptide is anti-viral polypeptide is cytotoxic to a tumor or cancer cell.

9. The mRNA of any of the preceding claims wherein the anti-viral polypeptide is anti-viral polypeptide is cytostatic to a tumor or cancer cell.

10. The mRNA of any of the preceding claims wherein the anti-viral polypeptide is a secreted polypeptide.

11. A pharmaceutical composition comprising the mRNA as defined in any preceding claim and a pharmaceutically acceptable carrier.

12. The pharmaceutical composition of claim 11 wherein the mRNA does not comprise a substantial amount of a nucleotide or nucleotides selected from the group consisting of uridine, cytidine, and a combination of uridine and cytidine, and wherein the composition is suitable for repeated intravenous administration to a mammalian subject in need thereof.

13. The pharmaceutical composition of claims 11 or 12 wherein the mRNA is encapsulated in a nanoparticle.

14. The composition of claim 13 wherein the nanoparticle further comprises one or more targeting moieties.
